# EUROPEAN PATENT APPLICATION

(11) **EP 4 220 176 A2**
(43) Date of publication of application: **02.08.2023**
(21) Application number: 23164742.1
(22) Date of filing: 27.02.2018
(51) Int. Cl.: G01N 33/68, A61K 39/00

(54) **BIOMARKERS**

(30) Priority: 27.02.2017 GB 201703123
(62) Divisional of application: 18712262.7
(71) Applicant: St. George's Hospital Medical School, London, Greater London SW17 0RE (GB)
(72) Inventor: SOFAT, Nidhi, London, SW17 0RE (GB)
(74) Representative: J A Kemp LLP

(57) **Abstract**

The invention is in the field of osteoarthritis pain, particularly biomarkers which allow for diagnosis and monitoring of osteoarthritis pain. Methods of treating osteoarthritis pain are also provided, together with methods of screening for agents which provide potential therapies.

## Description

### Field of the Invention

The present invention relates to biomarkers associated with osteoarthritis pain, which allow for diagnosing that joint pain is a result of osteoarthritis. The present invention also relates to treating osteoarthritis pain by targeting the biomarkers and pathways that the biomarkers represent.

### Background of the Invention

Osteoarthritis (OA) is the most common form of arthritis worldwide. In the United States, OA affects 13.9% of adults aged 25 years and older and 33.6% (12.4 million) of those aged more than 65 in 2005, which is estimated at 26.9 million US adults in 2005 (Lawrence et al, Arthritis Rheum. 2008; 58(1): 26-35). Such figures are believed to be a conservative estimate, with new cases set to rise due to aging populations worldwide and the rising epidemic of obesity (Nicholls et al, Osteoarthritis Cartilage. 2014 22(12): 2041-50). The most common joints affected by OA involve the large weight-bearing joints including the hip and knee. The most common symptom that people with OA complain of is pain and yet the mechanisms of pain in OA are poorly understood.

The current standard of care for OA pain management according to UK NICE guidelines includes paracetamol and non-steroidal anti-inflammatory drugs (NSAIDs) (topical or oral) (https://www.nice.org.uk/guidance/cg177). In people where paracetamol and NSAIDs have not been effective, opioids can be used. Steroid injections can also be used for symptom flares, but are not effective in the long-term. However, all analgesics which currently are included in NICE guidance have significant co-morbidity and NICE itself states that "risks and benefits should be considered, particularly in older people". There remains a huge unmet need to improve pain management in OA.

Pain in OA is thought to arise from several structures within the arthritic joint, including the synovium via which prostaglandins, leukotrienes and inflammatory mediators are released (Sofat et al, Rheumatology, 2011, 50(12):2157-65). Synovitis is often observed by magnetic resonance imaging (MRI) in OA and strongly correlates with pain (Roemer et al, Osteoarthritis Cartilage 2010; 18: 1269-74). Cartilage degradation is one of the hallmarks of OA disease (Roy et al, Ann Rheum Dis. 1968; 27: 544-58), although pain is likely to arise from other structures since cartilage is an avascular, aneural structure composed largely of extracellular matrix (ECM) and few chondrocytes. Recent interest has grown in the importance of bone marrow lesions (BML) in relation to OA pain. Epidemiological studies have shown a strong correlation between BMLs observed by MRI and OA-related knee pain in several large cohorts (Felson et al, Ann. Internal Med.2001; 134: 541-549 and Sowers et al, Osteoarthritis Cartilage, 2003; 11: 387-93).

To date there has though been very little work investigating the functional significance of BMLs.

### Summary of the Invention

The invention provides a method of evaluating osteoarthritis pain in a patient, said method comprising measuring the amount of thrombospondin 4 and/or one or more other biomarkers identified in Table 1 in a sample from the patient.

The invention also provides use of thrombopsondin 4 and/or one or more other biomarkers identified in Table 1 in evaluating osteoarthritis pain in a patient.

The invention also provides use of thrombopsondin 4 and/or one or more other biomarkers identified in Table 1 in evaluating the size and/or severity of bone marrow lesions

The invention also provides a kit for evaluating osteoarthritis pain, comprising a reagent for measuring the amount of thrombospondin 4 and/or one or more other biomarkers of Table 1 and instructions for use of the kit.

The invention also provides:
- A method of treating or preventing osteoarthritis pain in a patient, said method comprising administering an inhibitor of the NOTCH 1/thrombospondin 4 signalling pathway.
- A method of treating or preventing osteoarthritis pain in a patient, said method comprising administering an inhibitor of microtubule activity to the patient

Further, the invention provides a method of screening for agents for treating of preventing osteoarthritis pain, said method comprising:
(i) identifying agents which interact with thrombospondin 4 or stathmin; and
(ii) assessing whether agents identified in part (i) inhibit thrombospondin 4 or stathmin 2.

Finally, the invention provides a method of treating or preventing osteoarthritis pain in a patient, said method comprising:
(i) performing the method above and identifying agents which inhibit thrombospondin 4 and/or stathmin 2; and
(ii) administering the agents identified in part (i) to a patient in need thereof

### Brief Description of the Figures

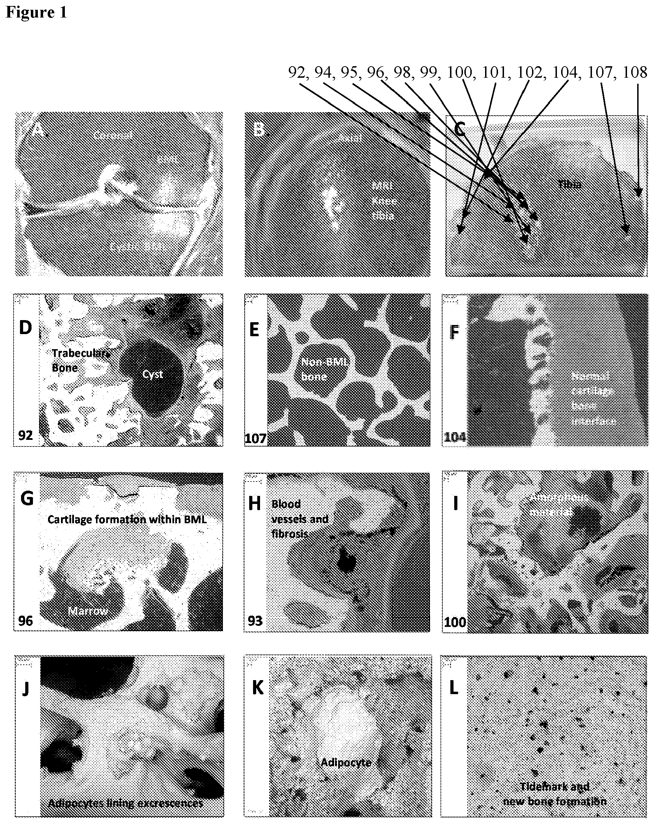

Figure 1 shows MRI correlation with BML analysis by scanning electron microscopy. Parts A and B show MRI knee scan showing medial bone marrow lesion with cyst within it. Part C shows BML bone tissue embedded in polymethacrylate and stained with iodine. Part D shows BML appearance by BML demonstrating cyst and enlarged trabecular bone (region 92 from bone sample in C). Part E shows normal bone marrow (region 107 shown from bone sample in C). Part F shows normal cartilage bone interface (region 104 from bone sample in C). Part G shows infiltration of marrow cavity in bone by new cartilage formation (region 96 from bone sample in C). Part H shows new blood vessel formation in BML Tissue (region from 93 bone sample in C). Part I shows infiltration of parts of bone marrow cavity by amorphous material (region 100 from bone sample in C). Parts J, K and L show macerated bone blocks of BML tissue revealing adipocyte-lined excrescences (J and K) and bone remodelling with tidemark (L).
Figure 2 shows MRI correlation with BML analysis by histology. Part A shows a MRI knee scan showing medial central tibial cysts and bone marrow lesion. Part B shows the naked eye appearance of cysts and bone marrow lesion. Part C shows H&E staining of cyst and trabecular bone within BML. Part D shows H&E stained blood vessel formation adjacent to enlarged trabecular bone cavity. Part E shows infiltration of regions of BML with amorphous material, blood vessels and fibrosis. Part F shows H&E staining of new cartilage formation within BML adjacent to marrow compartment.
Figure 3 (Table 1) shows the results of the transcriptomic analysis, with 218 genes showing significant differential expression. P Value^{a}: T-Test P value, P Value^{b}: Moderated T-Test P value.
Figure 4 shows microarray profiling of OA BML bone vs normal bone. Part A shows a heat map of regulated genes (218 in total) of BML bone vs normal non-OA bone. Hierarchical clustering and entities shown for comparisons with p<0.05. Part B shows PANTHER pathway analysis of genes detected from microarray. The predominant pathways detected are shown in the pie chart with classifications from recognised database pathways.

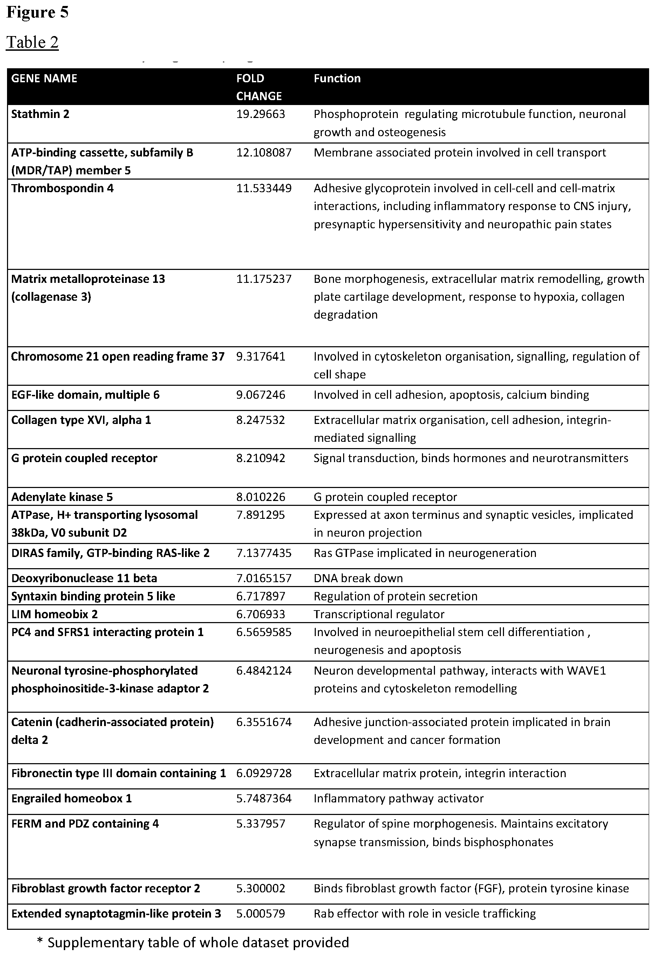

Figure 5 (Table 2) shows a summary of gene upregulation from OA bone marrow lesions.
Figure 6 part A shows quantitative PCR validation of OA bone marrow lesions compared with normal bone for the most highly upregulated genes from the microarray. Part B shows measurement of urinary CTX-II C-telopeptides by ELISA in advanced OA, early OA and healthy control subjects.
Figure 7 shows quantitative determination of STMN2 in human serum from 17 end-stage OA, 17 early OA and 7 healthy controls using ELISA.
Figure 8 shows quantitative measurement of thrombospondin 4 in serum from the end stage, early OA and control groups of patients using a human thrombospondin 4 sandwich enzyme immunoassay.

### Brief description of the sequence listing

SEQ ID NO: 1 presents the protein sequence of human stathmin 2 (isoform 1)
SEQ ID NO: 2 presents the protein sequence of human stathmin 2 (isoform 2)
SEQ ID NO: 3 shows the mRNA. sequence coding for human stathmin 2 (isoform 1)
SEQ ID NO: 4 shows the mRNA sequence coding for human stathmin 2 (isoform 2)
SEQ ID NO: 5 shows a cDNA sequence of human stathmin 2
SEQ ID NO: 6 shows the complete protein sequence human thrombospondin 4
SEQ ID NO: 7 shows the sequence of SEQ ID NO: 6 without the signal peptide
SEQ ID NOs: 8-11 show mRNA sequences coding for human thrombospondin 4
SEQ ID NOs: 12-15 show protein sequences of isoforms of ATP-binding cassette, sub-family B (MDR/TAP), member 5
SEQ ID NOs: 16-19 show mRNA sequences coding for ATP-binding cassette, sub-family B (MDR/TAP), member 5
SEQ ID NO: 20 shows the protein sequence of human matrix metalloproteinase 13.
SEQ ID NO: 21 shows the mRNA sequence of human matrix metalloproteinase 13.
SEQ ID NOs: 22 and 23 show protein sequences of human adenylate kinase 5.
SEQ ID NOs: 24 and 25 show mRNA sequences of human adenylate kinase 5.
SEQ ID NO: 26 shows the protein sequence of human ATPase, H+ transporting lysosomal 38 kDa V0 subunit D2.
SEQ ID NO: 27 shows the mRNA sequence of human ATPase, H+ transporting lysosomal 38 kDa V0 subunit D2.
SEQ ID NO: 28 shows the protein sequence of human DIRAS family, GTP binding RAS-like 2.
SEQ ID NO: 29 shows the mRNA sequence of human DIRAS family, GTP binding RAS-like 2.
SEQ ID NOs: 30 and 31 show protein sequences of human syntaxin binding protein 5-like.
SEQ ID NOs: 32-36 show mRNA sequences of human syntaxin binding protein 5-like.
SEQ ID NOs: 37-39 show protein sequences of human PC4 and SFRS1 interacting protein 1.
SEQ ID NOs: 40-44 show mRNA sequences of human PC4 and SFRS1 interacting protein 1.
SEQ ID NOs: 45-47 show protein sequences of human neuronal tyrosine-phosphorylated phosphoinositide-3-kinase adaptor 2.
SEQ ID NO: 48 shows the mRNA sequence of human neuronal tyrosine-phosphorylated phosphoinositide-3-kinase adaptor 2.
SEQ ID NOs: 49 and 50 show protein sequences of human catenin delta 2.
SEQ ID NOs: 51-54 show mRNA sequences of human catenin delta 2.
SEQ ID NO: 55 shows the protein sequence of human FERM and PDZ containing 4.
SEQ ID NO: 56 shows the mRNA sequence of human FERM and PDZ containing 4.
SEQ ID NOs: 57 and 58 show the protein sequences of human extended synaptotagmin-like protein 3.
SEQ ID NOs: 59-61 show the mRNA sequences of human extended synaptotagmin-like protein 3.
SEQ ID NOs: 62 and 63 show sense and antisense primers for matrix metalloproteinase 13.
SEQ ID NOs: 64 and 65 show sense and antisense primers for stathmin 2.
SEQ ID NOs: 66 and 67 show sense and antisense primers for thrombospondin 4.
SEQ ID NOs: 68 and 69 show sense and antisense primers for an OMD (NM_005014) positive tissue control.

The informal sequence listing that forms part of the description provides the authentic sequence listing for the application. The sequence listing filed for search purposes presents the mRNA sequences as DNA in the <212> field, so that the sequence listing is acceptable and meets the formal requirements. This is because the mRNA sequences, as identified in the listed databases entries, include "t" nucleotides in place of the "u" nucleotides.

### Detailed Description of the Invention

It is to be understood that different applications of the disclosed products and methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "an amino acid sequence" includes two or more such sequences, and the like.

All publications, patents and patent applications cited herein, whether supra or infra, are hereby incorporated by reference in their entirety.

### Method of evaluating osteoarthritis pain

The present invention provides a method of evaluating osteoarthritis pain in a patient. The method comprises measuring one or more biomarkers identified in Table 1 in a sample from the patient.

In the context of the present invention, "evaluating osteoarthritis pain" may mean identifying (diagnosing) that pain in a joint is a result of osteoarthritis. In other words, the method may involve diagnosing osteoarthritis and osteoarthritis pain. As discussed above, the most common joints affected by OA involve the large weight-bearing joints including the hip and knee. Osteoarthritis may also affect joints such as the lower back and neck, small joints of the fingers and the bases of the thumb and big toe. The methods of the present invention may therefore be used to identify that pain in e.g. the hip or knee is a result of osteoarthritis.

The methods of the present invention may be used in combination with other techniques used to diagnose osteoarthritis, such as physical examination of the joint, joint aspiration for the detection of crystals or joint deterioration, x-rays and MRI.

The patient is typically a human, but could also be a domestic/companion animal (such as a dog, cat, etc) or a livestock animal. The patient will typically have presented with symptoms indicative of osteoarthritis, including sore joints, limited motion or stiffness that goes away after movement, clicking or cracking sounds when a joint bends, mild swelling around a joint and pain that is worse after activity or towards the end of the day. For example, with osteoarthritis in the hips, pain may be felt in the groin area and sometimes on the inside of the knee or thigh. With knee osteoarthritis a grating or scraping sensation may occur when moving the knee. Osteoarthritic fingers may exhibit bony growths on the edge of joints which cause the fingers to become swollen, tender and red. In the feet, pain and tenderness may be felt in the large joint at the base of the big toe and there may be swelling in the ankles or toes.

Patients may also exhibit risk factors for osteoarthritis, including older age, obesity, or may have an occupation which places repetitive stress on a joint. Females are also at a higher risk of suffering from osteoarthritis.

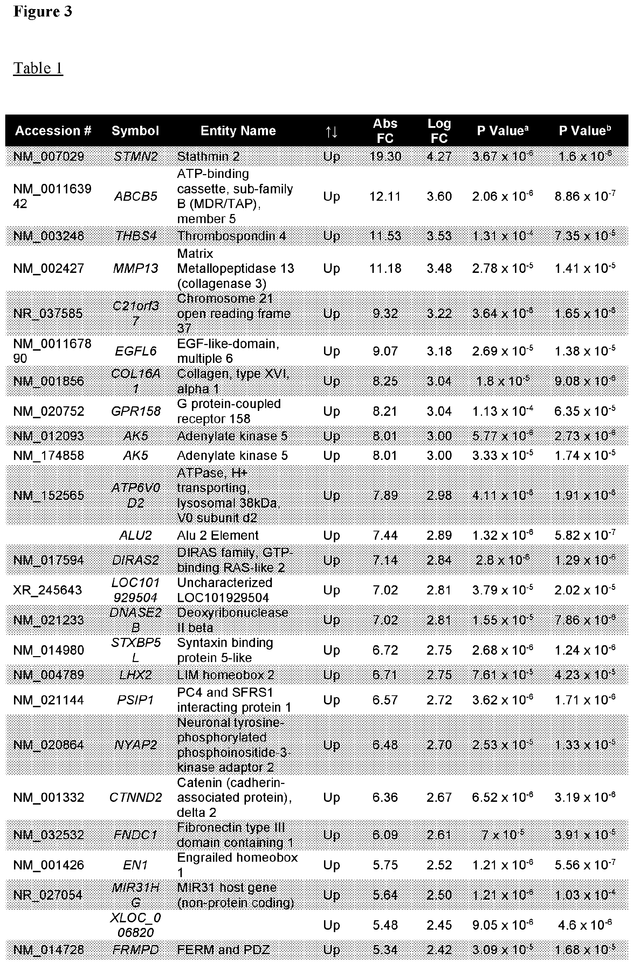

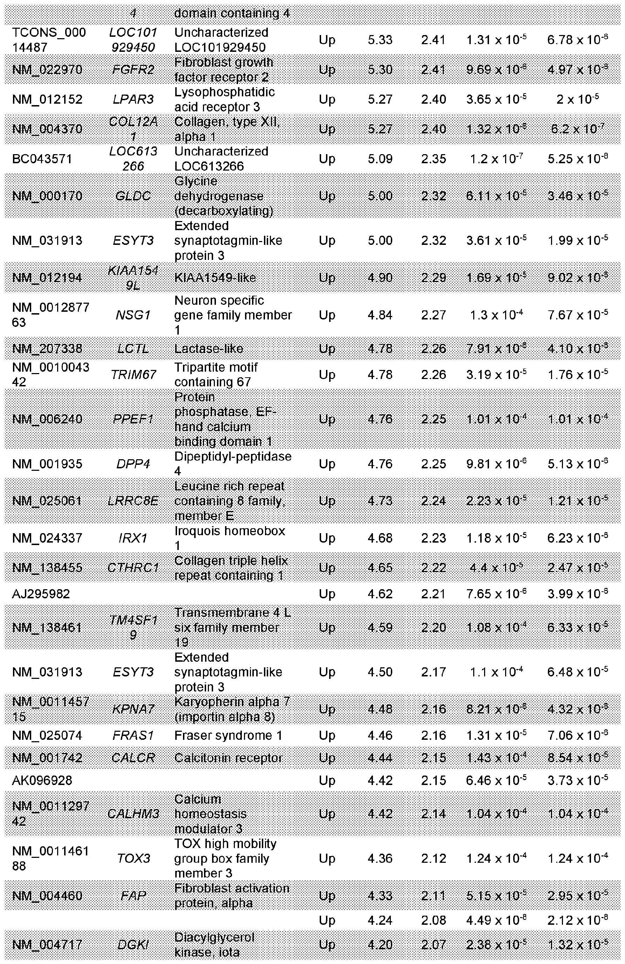

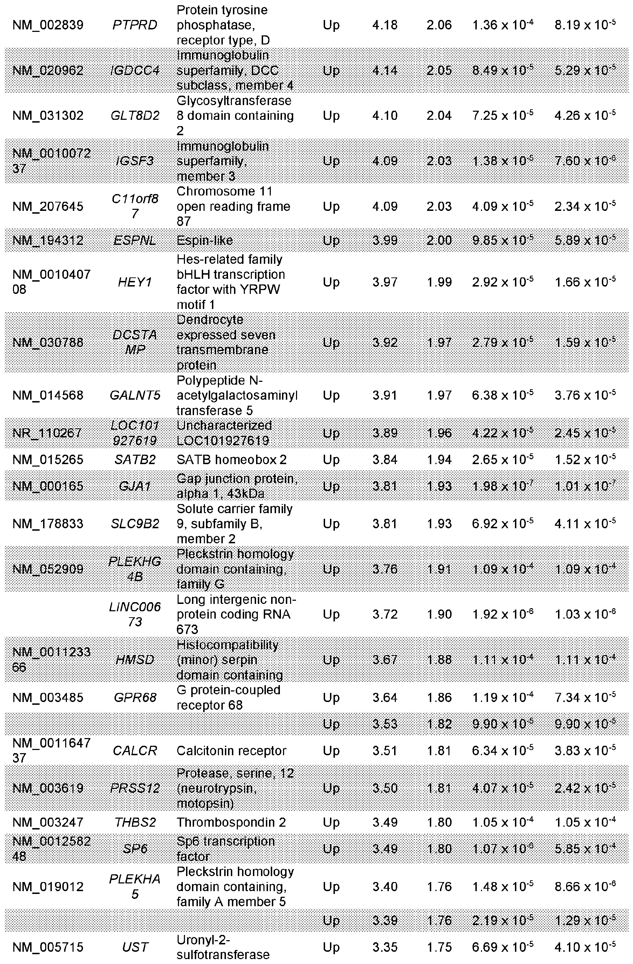

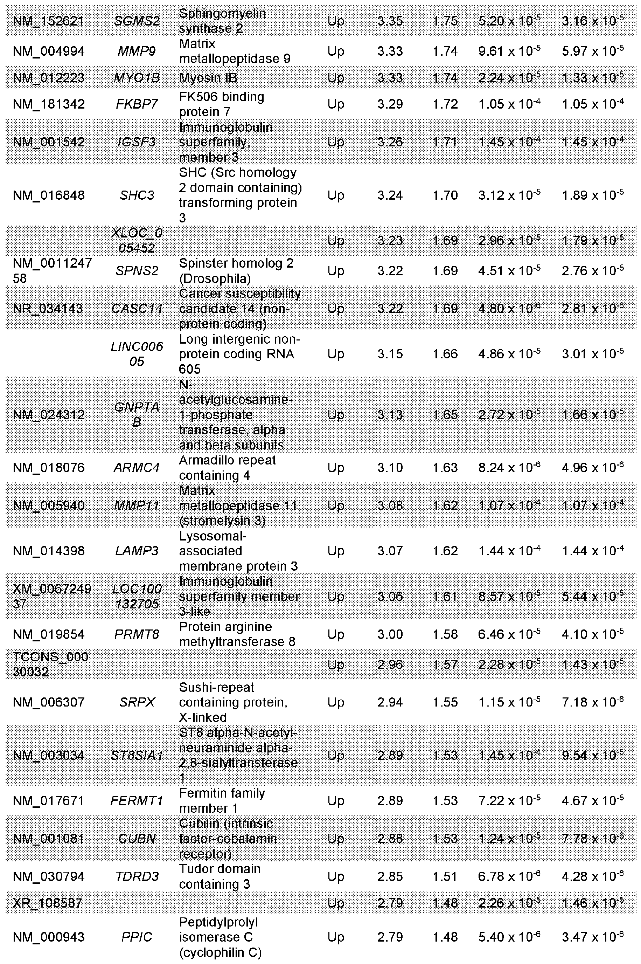

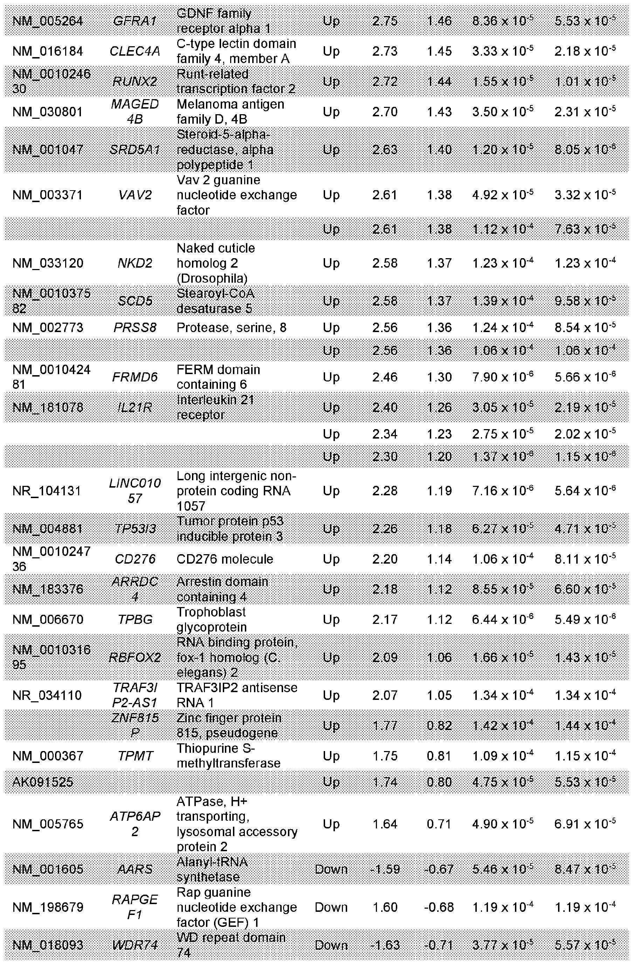

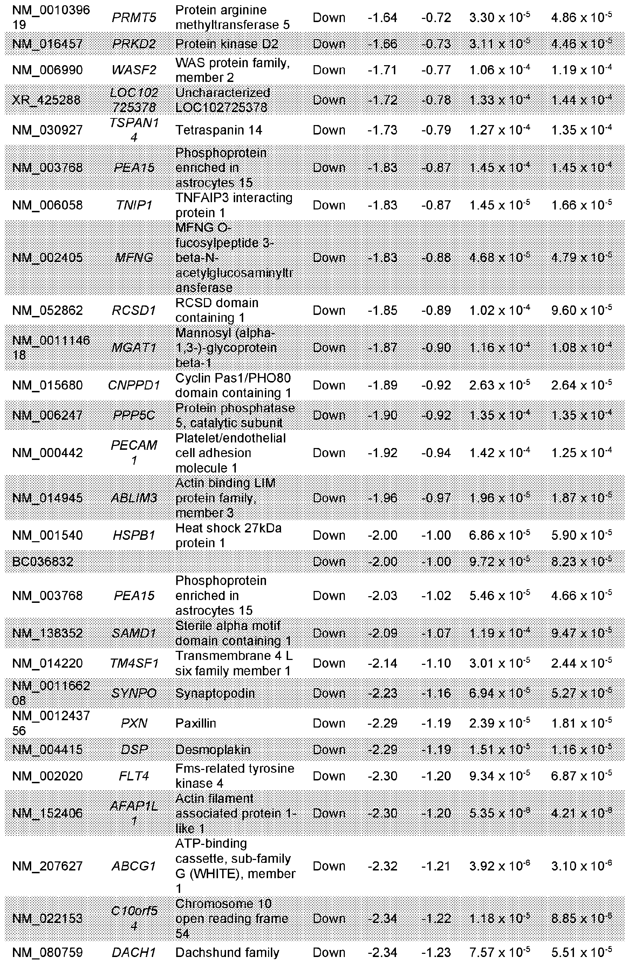

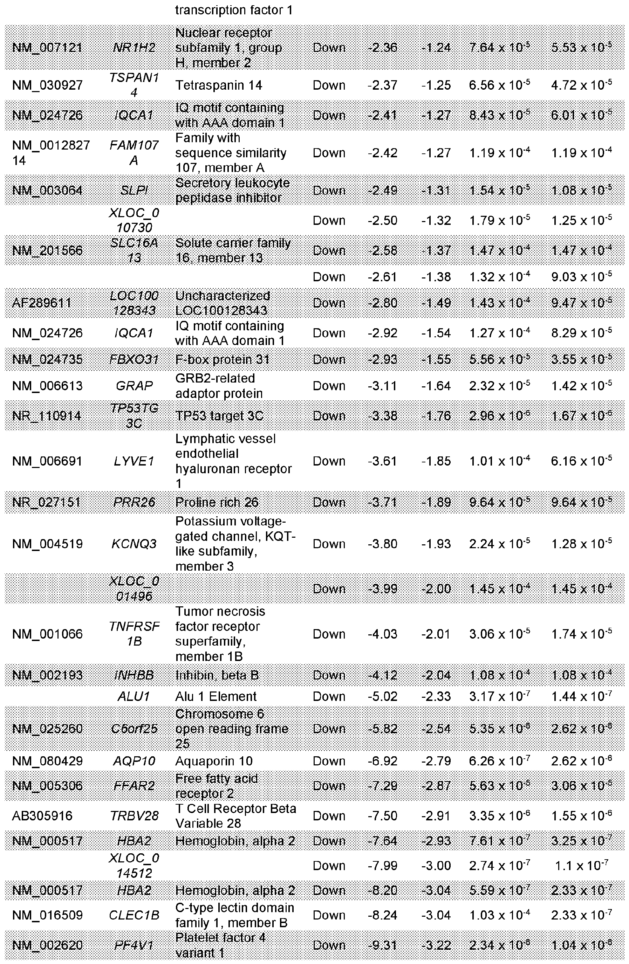

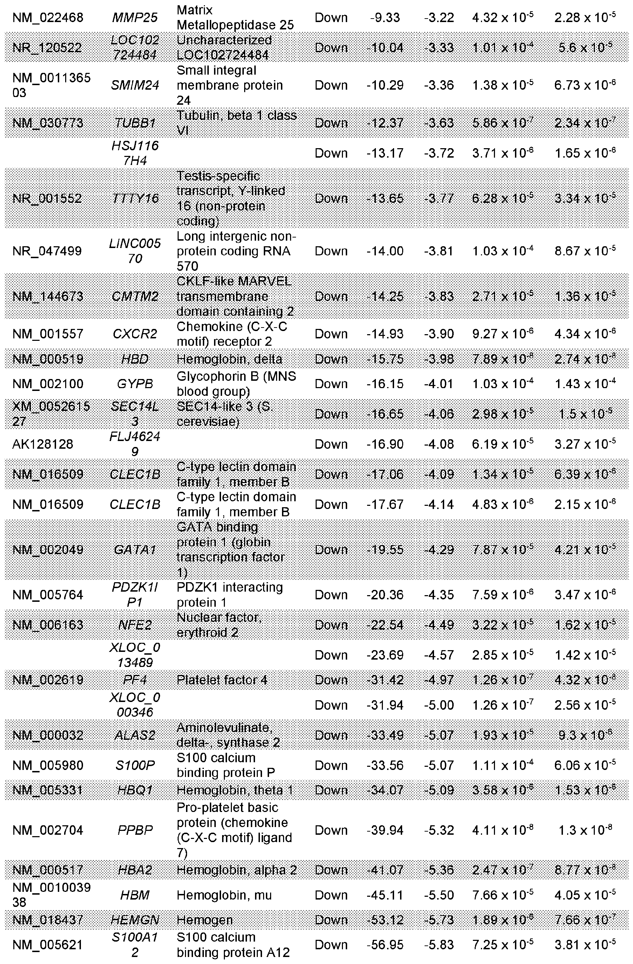

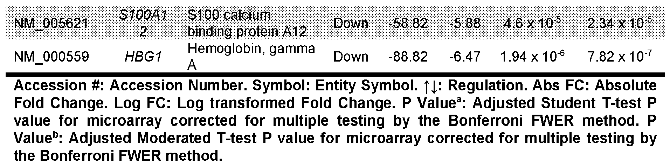

The methods of the present invention comprise measuring the amount of one or more biomarkers identified in Table 1 (Figure 3) in a sample from the patient. As discussed further below, Table 1 presents 218 genes which have been shown to be significantly differentially expressed in patients with osteoarthritis pain. In some instances, methods comprise measuring the amount of one or more biomarkers in Table 1, with the exception of matrix metalloproteinase 13 (collagenase 3).

The methods of the invention may involve measuring the amount or two or more biomarkers identified in Table 1. The methods of the invention may also involve measuring the amount of 5 or more, or 10 or more biomarkers identified in table 1.

Methods of the invention may also involve measuring the amount of at least one biomarker selected from the group consisting of
- ATP binding cassette subfamily B (MDR/TAP) member 5 (gene name ABCB5);
- matrix metalloproteinase 13 (gene name MMP13);
- adenylate kinase 5 (gene name AK5);
- ATPase H+ transporting lysosomal 38 kDa, V0 subunit D2 (V-type proton ATPase subunit d2) (gene name ATP6V0D2);
- DIRAS family, GTP-binding RAS-like 2 (gene name DIRAS2);
- syntaxin binding protein 5 like (gene name STXBP5L);
- PC4 and SFRS1 interacting protein 1 (gene name PSIP1);
- neuronal tyrosine-phosphorylated phosphoinositide-3-kinase adaptor 2 (gene name NYAP2);
- catenin delta (gene name C:TNND2);
- FERM and PDZ containing 4 (gene name FRMPD4);
- extended synaptotagmin-like protein 3 (gene name ESTY3).

In some cases, methods may involve measuring the amount of at least one biomarker selected from:
- ATP binding cassette subfamily B (MDR/TAP) member 5;
- ATPase H+ transporting lysosomal 38 kDa, V0 subunit D2;
- DIRAS family, GTP-binding RAS-like 2;
- syntaxin binding protein 5 like;
- PC4 and SFRS1 interacting protein 1;
- neuronal tyrosine-phosphorylated phosphoinositide-3-kinase adaptor 2;
- catenin delta;
- FERM and PDZ containing 4;
- extended synaptotagmin-like protein 3.

Methods may involve measuring the amount of one or more biomarkers selected from the group consisting of STMN2, ABCB2, THBS4, MMP13, C21orf37, EGFL6, COL16A1, GPR158, AK5, ATP6V0D2, ALU2, DIRAS2, LOC101929504, DNASE2B, STXBP5L, LHX2, PSIP1, NYAP2, CTNND2, FNDC1, EN1, MIR31HG, XLOC_006820, FRMPD4, LOC101929450, FGFR2, LPAR3, COL12A1, LOC613266, GLDC, ESYT3, KIAA1549L, NSG1, LCTL, TRIM67, PPEF1, DPP4, LRRC8E, IRX1, CTHRC1, TM4SF19, KPNA7, FRAS1, CACLR, CALHM3, TOX3, FAP, DGKI, PTPRD, IGDCC4, CiLT8D2, IGSF3, C11ORF87, ESPNL, HEY1, DCSTAMP, GALNT5, LOC101927619, SATB2, GJA1, SLC9B2, PLEKHG4B, LINC00673, HMSD, GPR68, CALCR, PRSS12, THBS2, SP6, PLEKHA5, UST, SGMS2, MMP9, MYO1B, FKBP7,IGSF3, SHC3, XLOC_005452, SPNS2, CASC14, LINC00605, GNPTAB, ARMC4, MMP11, LAMP3, LOC100132705, PRMT8, SRPX, ST8SIA1, FERMT1, CUBN, TDRD3, PPIC, GFRA1, CLEC4A, RUNX2, MAGED4B, SRD5A1, VAV2, NKD2, SCD5.

Methods may also involve measuring the amount of at least two biomarkers selected from the groups listed above (in any combinations). In some instances, methods of the invention may comprise measuring amounts of at least one biomarker listed in Table 2. Methods may also comprise measuring amounts of at least 2, at least 5 or at least 10 biomarkers listed in Table 2.

All combinations of biomarkers listed in Tables 1, 2 and the groups listed above are contemplated in the present invention.

Methods of the invention preferably involve measuring the amount of stathmin 2 or thrombospondin 4 (or both stathmin 2 and thrombospondin 4). Methods of the invention may for example comprise measuring the amount of stathmin 2 either on its own or in combination with other markers listed e.g. in Table 1 or 2, or in the groups presented above. Methods of the invention may also comprise measuring the amount of thrombospondin 4 either on its own or in combination with other markers listed e.g. in Table 1 or 2, or in the groups presented above. In some instances, methods of the invention comprise measuring amounts of both stathmin 2 and thrombospondin 4 (either on their own or in combination with other markers listed e.g. in Table 1 or 2, or the groups listed above).

Table 1 presents the gene name, gene symbol and accession number for each of the biomarkers. The proteins encoded by these genes are well known, and sequences coding for the proteins and sequences of the proteins themselves could readily be determined. Gene and protein sequences for the various biomarkers from different species are also available via databases such as http://www.uniprot.org and http://www.ncbi.nlm.nih.gov/genbank/.

For example, human stathmin 2 (gene name STMN2) exists in two isoforms, which are a result of alternative splicing. These two isoforms are presented in SEQ ID NOs: 1 and 2. An alternative name for stathmin 2 is superior cervical ganglion-10 protein.

SEQ ID NOs: 3 and 4 show mRNA sequences of human stathmin 2 and SEQ ID NO: 5 provides a cDNA sequence. Any sequencing coding for stathmin 2 (RNA or DNA) can be detected and measured in the methods of the present invention. Any of SEQ ID NOs: 1 to 5 can be detected and measured in the method of the present invention.

For example, in the methods of the invention amounts of protein may be determined. Methods for extracting, detecting and measuring amounts of a protein in a sample are well known in the art and any suitable method may be employed in the present invention. Methods may comprise contacting the sample with an antibody capable of binding to a protein of interest. For example immunohistochemistry or Western blotting may be employed, then used to quantify the amount of protein present. ELISA could also be used. ELISA is a heterogeneous, solid phase assay that requires the separation of reagents. ELISA is typically carried out using the sandwich technique or the competitive technique. The sandwich technique requires two antibodies. The first specifically binds the protein of interest and is bound to a solid support. The second antibody is bound to a marker, typically an enzyme conjugate. A substrate for the enzyme is used to quantify the protein-antibody complex and hence determine the amount of protein in a sample. The antigen competitive inhibition assay also typically requires an protein-specific antibody bound to a support. A protein -enzyme conjugate is added to the sample (containing the protein) to be assayed. Competitive inhibition between the protein-enzyme conjugate and unlabelled protein allows quantification of the amount of the protein of interest in a sample. The solid supports for ELISA reactions preferably contain wells.

The present invention may also employ methods of measuring proteins of interest that do not comprise antibodies. For example, High Performance Liquid Chromatography (HPLC) separation and fluorescence detection may be used as a method of determining the amount of a protein of interest. Alternatively, spectroscopic methods may also be employed.

The amino acid sequences of isoforms 1 and 2 of human stathmin 2 are shown in SEQ ID NOs: 1 and 2. Typically, for a human patient, amounts of protein comprising SEQ ID NOs: 1 and/or 2 are measured in the method of the present invention. However, amounts of variants of SEQ ID NOs: 1 and 2 may also be measured. These variants reflect the slight differences in stathmin 2 sequence that occur naturally between humans. In particular, variants of SEQ ID NOs: 1 and 2 have an amino acid sequence, which varies from that of SEQ ID NOs: 1 and 2, but still forms a functional stathmin 2 protein. As discussed below, stathmin 2 is a regulator of microtubule stability. Phosphorylation by MAPK8 results in microtubule stabilisation. Such variants typically comprise a sequence that is at least 90%, 95%, 97% or 99% homologous based on amino acid identity to SEQ ID NO: 1 or 2 over the entire sequence. Methods for determining homology are discussed below.

Such variants can readily be detected and measured, together with proteins comprising SEQ ID NOs: 1 and 2 themselves, using the techniques and methods for quantitating proteins described above. These methods can often tolerate slight changes in amino acid sequence, such as the changes that occur naturally between humans. For instance, methods may employ antibodies directed against an epitope in SEQ ID NOs: 1 and 2 which is conserved in most, if not all humans. Furthermore, methods could readily be adapted for any commonly occurring variants.

Variants may also, for example have up to 5, up to 10 or up to 20 deletions, substitutions or additions compared with SEQ ID NOs: 1 and 2.

Variants may also be fragments of SEQ ID NOs: 1 and 2. For example, fragments may comprise at least 50, at least 100 or at least 150 amino acids (typically contiguous amino acids) of SEQ ID NO: 1 and or SEQ ID NO: 2.

Standard methods in the art may be used to determine homology. For example the UWGCG Package provides the BESTFIT program which can be used to calculate homology, for example used on its default settings (Devereux et al (1984) Nucleic Acids Research 12, p387-395). The PILEUP and BLAST algorithms can be used to calculate homology or line up sequences (such as identifying equivalent residues or corresponding sequences (typically on their default settings)), for example as described in Altschul S. F. (1993) J Mol Evol 36:290-300; Altschul, S.F et al (1990) J Mol Biol 215:403-10. Software for performing BLAST analyses is publicly available through the National Center for Biotechnology Information (bttp://www.ncbi.nebi.nih.gov/).

As discussed above, amino acid substitutions may exist in the sequences of interest. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art and may be selected in accordance with the properties of the 20 main amino acids as defined in Table 3 below. Where amino acids have similar polarity, this can also be determined by reference to the hydropathy scale for amino acid side chains in Table 4 below.

**Table 3 - Chemical properties of amino acids**

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged(+) | Trp | aromatic, hydrophobic, neutral |
| Leu | aliphatic, hydrophobic, neutral | Tyr | aromatic, polar, hydrophobic |

**Table 4 - Hydropathy scale**

| Side Chain | Hydropathy |
|---|---|
| Ile | 4.5 |
| Val | 4.2 |
| Leu | 3.8 |
| Phe | 2.8 |
| Cys | 2.5 |
| Met | 1.9 |
| Ala | 1.8 |
| Gly | -0.4 |
| Thr | -0.7 |
| Ser | -0.8 |
| Trp | -0.9 |
| Tyr | -1.3 |
| Pro | -1.6 |
| His | -3.2 |
| Glu | -3.5 |
| Gln | -3.5 |
| Asp | -3.5 |
| Asn | -3.5 |
| Lys | -3.9 |
| Arg | -4.5 |

Kits are commercially available for detecting stathmin 2.

The amount of mRNA from a gene of interest may also be measured in the sample from the patient. Methods for extracting, detecting and measuring amounts of a particular mRNA are known in the art. Any suitable method may be used in the present invention. Methods include microarrays, Northern blotting, nuclease protection assays, *in situ* hybridization, reverse transcription-polymerase chain reaction (RT-PCR) and or RNA-seq (whole transcriptome sequencing).

Exemplary primers for stathmin 2 are shown in the Examples.

The cDNA sequence encoding stathmin 2 is shown in SEQ ID NO: 5, and the mRNA sequences of both isoforms are shown in SEQ ID NOs: 3 and 4. Typically, for a human patient, the amount of mRNA of SEQ ID NO: 3 and/or 4 is measured. However, amounts of variants of SEQ ID NO: 3 and/or 4 may also be measured. These variants reflect the slight differences in sequence that occur naturally between humans. In particular, variants of SEQ ID NOs: 3 and 4 have a sequence which varies from that of SEQ ID NOs: 3 and 4, but still codes for expression of a functional stathmin 2 protein. Stathmin 2 function is discussed above. Such variants typically comprise a sequence that is at least 90%, 95%, 97% or 99% homologous based on nucleotide identity to SEQ ID NOs: 3 and 4 over the entire sequence. Methods for determining homology are discussed above.

As these variants only reflect the slight differences in sequences that occur naturally between humans, the techniques described above can be used to detect and measure such variant mRNAs as well as mRNAs of SEQ ID NOs: 3 and 4. For example, the stringency of hybridisation of probes used in such techniques can be varied so that natural variants of SEQ ID NOs: 3 and 4 will still be detected together with mRNA of SEQ ID NOs: 3 and 4.

As mentioned above, amino acid, mRNA and DNA sequences of stathmin 2 from species other than humans are known and can be accessed via public databases. From these sequences, the skilled person could readily detect and measure stathmin 2 mRNA from such other species, in order to carry out the method of the invention. Methods for mRNA extraction, detection and quantification are discussed above.

SEQ ID NOS: 6 and 7 present the complete and mature forms (lacking the signal peptide) of human thrombospondin 4 (THBS4). SEQ ID NOs: 8-11 show mRNA sequences of human thrombospondin 4. Any sequence coding for thrombospondin 4 (RNA or DNA) can be detected and measured in the methods of the present invention. Any of SEQ ID NOs: 6-11 can be detected and measured in the method of the present invention.

Typically, for a human patient, amounts of protein comprising SEQ ID NOs:6 and/or 7 are measured in the method of the present invention. However, amounts of variants of SEQ ID NOs: 6 and 7 may also be measured. These variants reflect the slight differences in thrombospondin 4 sequence that occur naturally between humans.

Known variants include an L to Q substitution at position 55, an A to P substitution at position 387, an A to V substitution at position 420, a V to I substitution at position 646 and a V to I substitution at position 737 (wherein the numbering is based on the complete sequence of SEQ ID NO: 6). Methods of the invention may involve detecting any one of these variant sequences.

Variants of SEQ ID NOs: 6 and 7 have an amino acid sequence, which varies from that of SEQ ID NOs: 6 and 7, but may still form a functional protein. Such variants typically comprise a sequence that is at least 90%, 95%, 97% or 99% homologous based on amino acid identity to SEQ ID NO: 6 or 7 over the entire sequence. Methods for determining homology are discussed above.

Such variants can readily be detected and measured, together with proteins comprising SEQ ID NOs: 6 and 7 themselves, using the techniques and methods for quantitating proteins described above. These methods can often tolerate slight changes in amino acid sequence, such as the changes that occur naturally between humans. For instance, methods may employ antibodies directed against an epitope in SEQ ID NOs: 6 and 7 which is conserved in most, if not all humans. Furthermore, methods could readily be adapted for any commonly occurring variants.

Variants may also have up to 5, 10 or 20 substitutions, deletions or additions compared with SEQ ID NOs 6 and 7.

Variants may also be fragments of SEQ ID NOs: 6 and 7 Such fragments typically comprise at least 700, at least 800 or at least 900 amino acids of SEQ ID NO 6 or 7.

mRNA sequences are shown in SEQ ID NOs: 8-11. Typically, for a human patient, the amount of mRNA of SEQ ID NO: 8,9,10 or 11 is measured. However, amounts of variants of these sequences may also be measured. These variants reflect the slight differences in sequence that occur naturally between humans. In particular, variants of have a sequence which varies from that of SEQ ID NOs: 8-11 but still codes for expression of a functional protein. Such variants typically comprise a sequence that is at least 90%, 95%, 97% or 99% homologous based on nucleotide identity to SEQ ID NOs: 8-11 over the entire sequence. Methods for determining homology are discussed above.

Once again, exemplary primers for thrombospondin 4 are shown in the Examples.

As mentioned above, amino acid, mRNA and DNA sequences of thrombospondin 4 from species other than humans are known and can be accessed via public databases. From these sequences, the skilled person could readily detect and measure thrombospondin 4 mRNA from such other species, in order to carry out the method of the invention. Methods for mRNA extraction, detection and quantification are discussed above.

Table 1 presents the accession numbers for genes in question. The sequences of these genes (and proteins) are therefore incorporated by reference based on the accession number as at the filing date of the application.

The protein sequences of human ATP-binding cassette sub-family B member 5 (ABCB5) are presented in SEQ ID NOs: 12-15. These four isoforms are produced by alternative splicing. Methods of the invention may comprise detecting any of these sequences, or variants thereof. Variants are as described above and may have for example at least 90% identity to any of SEQ ID NOs: 12-15. Variants may also have for example less than 5, or less than 10 substitutions, deletions or insertions relative to SEQ ID NOs: 12-15. Naturally occurring variants exist, such as K560E, K669R, E675V, A880T, Q905H, A915T and E970K (based on SEQ ID NO: 12). mRNA sequences for human ATP-binding cassette sub-family B member 5 are presented in SEQ ID NOs: 16-19. Methods of the invention may comprise detecting any one of these sequences. Once again, the above comments about variants can be applied.

The protein sequence of human matrix metalloproteinase 13 (MMP13) is shown in SEQ ID NO: 20. This is the complete form, which undergoes processing into a mature form. Residues 1-19 are the signal peptide, and residues 20-103 are a propeptide. The proenzyme is activated by removal of this propeptide. Methods of the invention may involve detecting SEQ ID NO: 13, or the mature form of peptide (without the signal sequence/propeptide), or variants thereof. Naturally occurring variants exist, including H2L, F74S, F75S, M91T, W207G, H232N and D390G. The mRNA sequence of human MMP13 is presented in SEQ ID NO: 20. SEQ ID NO: 20, or a variant thereof, may be detected in the methods of the invention.

The sequences of two isoforms of human adenylate kinase 5 (AK5) are presented in SEQ ID NOs: 22 and 23. These two forms are produced by alternative splicing. Natural variants exist: R418W and A726T (based on SEQ ID NO: 22). mRNA sequences are presented in SEQ ID NOs: 24 and 25. Methods of the invention may involve detecting any of these sequences, or variants thereof.

The protein sequence of V-type proton ATPase subunit d2 is presented in SEQ ID NO: 26. Two natural variants occur: G272R and E295K. The mRNA sequence is presented in SEQ ID NO: 27. Methods of the invention may involve detecting any of these sequences, or variants thereof.

The complete sequence of DIRAS family, GTP binding RAS-like 2 (GTP-binding protein Di-Ras2) is presented in SEQ ID NO: 28. This complete protein is processed into a mature form, where the propeptide of residues 197-199 is removed. The mRNA sequence is presented in SEQ ID NO: 29. Methods of the invention may involve detecting any of these sequences, or variants thereof.

SEQ ID NOs: 30 and 31 show protein sequences of syntaxin binding protein 5-like. The two isoforms are produced by alternative splicing. mRNA sequences are show in SEQ ID NOs: 32-36. Methods of the invention may involve detecting any of these sequences, or variants thereof.

SEQ ID NOs: 37-39 show the sequences of the three isoforms of human PC4 and SFRS1-interacting protein (produced by alternative splicing). Variants which occur in disease are K360A, I365A, D366A or N, V370A, F406A and V408A. Methods of the invention may involve detecting any of these sequences, or variants thereof.

SEQ ID NOs: 45-47 show the sequences of the three isoforms of human neuronal tyrosine-phosphorylated phosphoinositide-3-kinase adaptor 2 produced by alternative splicing. An mRNA sequence is presented in SEQ ID NO: 48. Methods of the invention may involve detecting any of these sequences, or variants thereof.

SEQ ID NOs: 49 and 50 show the sequences of two isoforms of human catenin delta 2. mRNA sequences are presented in SEQ ID NOs: 51-54. Methods of the invention may involve detecting any of these sequences, or variants thereof.

SEQ ID NO: 55 shows the protein sequence of FERM and PDZ containing 4. A naturally occurring variant has an C553R substitution. An mRNA sequene is shown in SEQ ID NO: 56. Methods of the invention may involve detecting any of these sequences, or variants thereof.

SEQ ID NOs: 57 and 58 show protein sequences of human extended synaptotagmin-like protein 3 (produced by alterative splicing). P246Q, G416R, G590R and T662S are natural variants. mRNA sequences are shown in SEQ ID NOs: 59-61. Methods of the invention may involve detecting any of these sequences, or variants thereof.

In all cases, variants are as described above and typically have at least 90%, 95% or 99% identity to the disclosed sequences. Variants may have for example up to 5 or up to 10 substitutions, deletions or insertions.

In the method of the invention, the amount of biomarker or interest is measured *in vitro* in a sample from the patient. The sample may be any appropriate sample. The sample may comprise a body fluid or a tissue sample from the patient. The sample may be a urine sample from the patient, but could also be a sample taken from the joint of the patient. For example, the sample may be a synovial fluid or synovial membrane sample. Preferably, the sample is a blood or plasma sample, most preferably a serum sample. Methods for obtaining such samples are well known in the art. The sample may be processed prior to being assayed. For example, blood samples may be centrifuged. Amounts of the biomarker in question in the sample are typically measured immediately after the sample has been obtained from the patient.

Preferably, methods of the invention comprise measuring amounts of thrombospondin 4 and/or stathmin 2 protein (as discussed above) in a serum sample from the patient. As discussed above, other sample types include synovial fluid, synovial biopsy and urine.

As discussed above, methods of the invention may involve measuring the amount of one or more biomarkers as presented in Table 1 in a sample from a patient in order to identify that joint pain in a patient is the result of osteoarthritis (in other words, as a diagnosis for osteoarthritis/osteoarthritis pain). Methods of the invention may also be used to diagnose osteoarthritis severity i.e. early vs late (advanced) disease. Early and advanced osteoarthritis are discussed further below. Furthermore, methods may be used to stratify patients. For example, pain can be stratified into mild, moderate of severe pain. The methods of the invention may also be used to stratify patients into groups, such as patients (1) exhibiting pain and having structural damage, (2) patients exhibiting pain but exhibiting no structural damage and patients (3) exhibiting no pain but having structural damage. In some instances, the methods may for example identify for group (2) patients that their pain is a result of osteoarthritis.

In some cases, an increased amount of a biomarker may be indicative of osteoarthritis. In other cases, a decreased amount of a biomarker may be indicative of osteoarthritis. This can be determined from Table 1 by whether or not a biomarker was determined to up- or down-regulated in comparison with the healthy control.

For example, the amount of stathmin 2 may be measured in a sample from the patient in order to determine whether or not joint pain is the result of osteoarthritis. A increased amount of stathmin 2 in the sample is indicative that joint pain may be the result of osteoarthritis. A person skilled in the art is capable of determining whether or not the amount of stathmin 2 is increased in the sample from the patient. Comparisons may be made with a pre-determined amount in the patient itself, but are preferably made with known control amounts as discussed in more detail below.

In order to measure whether or not joint pain is a result of osteoarthritis, the method of the present invention preferably comprises a first step of measuring the amount of stathmin 2 in a sample from the patient. The amount of stathmin 2 in the sample from the patient is then preferably compared with a control amount of stathmin 2 (or a control level of stathmin 2 or a control concentration of stathmin 2). An increased amount in the sample from the patient compared with the control amount indicates that joint pain is a result of osteoarthritis, in some cases early stage osteoarthritis. An identical (i.e. no significant difference) or lower amount in the sample from the patient compared with the control amount may indicate that the patient does not have joint pain resulting from osteoarthritis. When the sample is a serum sample from a patient, identical or lower amounts in the sample from the patient compared with the control amount could also be indicative that patient does not have early stage osteoarthritis (but could have advanced osteoarthritis).

Early stage osteoarthritis, in the context of the present invention, can be defined as patients who have had symptoms and some pain, but where the symptoms and pain are not severe enough to require joint replacement surgery. Advanced (or end/late stage) osteoarthritis, in the context of the present invention, is defined as patients who have severe enough pain and symptoms to require joint replacement surgery.

Typically, control amounts are derived from "healthy" subjects who are known not to have osteoarthritis (i.e. who do not have any evidence of osteoarthritis). Standard methods for determining whether or not a person has osteoarthritis are described above. Controls amounts are determined for a particular sample type e.g. serum and can be based on protein/mRNA as appropriate.

The control amount may be a range of all the amounts of stathmin 2 present in samples from control subjects. The control amount may also refer to the average amount of all the amounts of stathmin 2 present in the samples from the control subjects. The sample types obtained from the control subjects may be any of those described above. The amounts of stathmin 2 in these samples may be determined by any of the methods described above. The control amount may be determined by measuring the amount of stathmin 2 in samples from any appropriate number of control subjects. Typically, amounts of stathmin 2 in samples from at least 5, at least 10, at least 20, at least 50 or at least 100 control patients may be measured. Amounts of stathmin 2 in samples from even higher numbers of subjects may also be measured.

The control amount of stathmin 2 may refer to either stathmin 2 mRNA or stathmin 2 protein, depending on which of these is measured in the sample from the patient. In order to allow an effective comparison, the control amount has the same units as the measured amount of stathmin 2 in the sample from the patient. Furthermore, control values are typically obtained under the same conditions as those under which the method of the invention is carried out. For example, control amounts are typically determined using the same method of stathmin 2 detection and measurement as used in the method of the present invention.

The control amount is obtained separately from the method of the invention. For instance, control amounts may be obtained beforehand and recorded e.g. on a computer. Most preferably, an average control amount is determined and used as a standard reference value.

As discussed above, the method of the present invention preferably involves determining that a patient's joint pain is a result of osteoarthritis if the amount of stathmin 2 in a sample from the patient is increased compared with the control amount.

When the control amount is a range of amounts of stathmin 2, a patient may be determined to have osteoarthritis based on the spread of the control data, the difference between the control data and the amount of stathmin 2 measured in the sample from the patient, and calculated confidence levels. A person skilled in the art is capable of doing this using standard methods.

A patient may be determined to have osteoarthritis, particularly early stage osteoarthritis, if the amount of stathmin 2 in the sample from the patient is increased compared with the highest amount of stathmin 2 in the control amount range.

Preferably, a patient is determined to have osteoarthritis if the amount of stathmin 2 in the sample from the patient is increased by at least 10% (i.e. at least 10% higher), at least 20%, at least 40%, at least 50%, at least 60%, at least 70%, at least 80%, at least 90% or at least 100% compared with the highest control amount of stathmin 2 in the range of control amounts. These values are particularly applicable to a serum sample (with protein amounts being measured), with a increase of at least 10%, preferably an increase of at least 20%, providing an indication that the patient has early stage osteoarthritis.

A patient may also have a stathmin 2 amount which is least 2 fold, at least 5 fold, at least 10 fold or at least 15 fold higher than the highest control amount.

More preferably, a patient may be determined to have osteoarthritis if the amount of stathmin 2 in the sample from the patient is increased compared to the average control amount of stathmin 2 (which is recorded as a standard control value). Once again, a patient may be determined to have osteoarthritis where the amount of stathmin 2 in the sample from the patient is increased by at least 10%, at least 20%, at least 50%, at least 60%, at least 70%, at least 80%, at least 90% or at least 100% compared with the average control amount. For example, in a serum sample (where protein amounts are measured) an increase of at least 10%, preferably an increase of at least 20% compared with the control provides an indication that the patient has early stage osteoarthritis.

A patient may also have a stathmin 2 amount which is least 5 fold, at least 10 fold or at least 15 fold higher than the average control amount.

For example, in a serum sample the control amount may range from 0.8-1.0 ng/ml using an ELISA assay as described in the examples (with an average control amount of approximately 0.9 ng/ml). A patient may be determined to have early stage osteoarthritis if the amount of stathmin 2 in a serum sample is at least 1.0 ng/ml, preferably at least 1.1 ng/ml using this assay.

For thrombospondin 4 the above comments about controls are also applicable. Once again, a patient is determined to be suffering from osteoarthritis when the amount of thrombospondin 4 in a sample from the patient is increased compared with the control amount.

Here, a patient may be determined to have osteoarthritis if thrombospondin 4 is present in the sample from the patient. In some cases, the amount of thrombospondin 4 in the sample from the patient is increased by at least 10% (i.e. at least 10% higher) or at least 20% compared with the highest control amount of thrombospondin 4 in the range of control amounts. More preferably, the amount of thrombospondin 4 is increased at least 50% or at least 100% compared with the highest control amount of thrombospondin 4 in the range of control amounts.

A patient may also have a thrombospondin 4 amount which is least 5 fold, at least 10 fold or at least 15 fold higher than the highest control amount.

More preferably, a patient may be determined to have osteoarthritis if the amount of thrombospondin 4 in the sample from the patient is increased compared to the average control amount of thrombospondin 4 (which is recorded as a standard control value). Once again, a patient may be determined to have osteoarthritis if thrombospondin 4 is present in the sample from the patient (where it is absent in the control), or where the amount of thrombospondin 4 in the sample from the patient is increased by at least 10%, at least 20%, at least 50%, at least 60%, at least 70%, at least 80%, at least 90% or at least 100% compared with the average control amount.

A patient may also have a thrombospondin 4 amount which is least 5 fold, at least 10 fold or at least 15 fold higher than the average control amount.

As shown in the Examples, in serum samples it was not possible to detect thrombospondin 4 using ELISA. Therefore, the presence of thrombospondin 4 in a serum sample from a patient is typically indicative that the patient has osteoarthritis related pain. Here, higher amounts of thrombospondin 4 are indicative of later stage osteoarthritis. A patient may for example be determined to have early stage osteoarthritis if an amount of thrombospondin 4 up to 0.2 ng/ml is measured. (although in some cases a patient could have early stage osteoarthritis and greater than 0.2 ng/ml), and determined to have advanced/end stage osteoarthritis if an amount above 0.2 ng/ml is measured (e.g. using the ELISA assay as described in the Examples).

As discussed above, methods of the invention may involve determining amounts of both stathmin 2 and thrombospondin 4.

Similar considerations apply for other genes/proteins identified in Table 1. For up-regulated genes, an increased amount relative to the control indicates that pain is likely to be a result of osteoarthritis. For down-regulated genes, a decreased amount relative to the control indicates that pain is likely to be a result of osteoarthritis. Up-regulated genes may for example show increases of at least 10%, at least 50% or at least 100% relative to the control. Up-regulated genes may for example also show an increase of at least 2 fold, at least 5 fold or at least 10 fold relative to the control. Down regulated genes may for example show a decrease of at least 10%, at least 50% or at least 75% relative to the control. Down-regulated genes may show at least a decrease of at least 2 fold, at least 5 fold or at least 10 fold. Once again, the skilled person could readily determine whether any increase/decrease observed relative to a control is significant.

Once a patient has been identified as suffering from pain as a result of osteoarthritis, appropriate intervention can then be considered.

Methods of the invention may also be used to determine that a patient is at risk of developing osteoarthritis pain, or who may be more likely to have progressive worsening of symptoms. For example, the patient may have shown other signs of osteoarthritis (e.g. those described above) and the methods of the invention may be used to evaluate whether intervention is necessary and what type of intervention would be appropriate.

As described above, amounts of one or more biomarkers identified in Table 1 may be measured in a sample from the patient. Sample types are discussed above, but are typically a serum sample. The methods typically involve determining levels of stathmin 2 and/or thrombospondin 4 and comparing with a control. Once again, stathmin 2, thrombospondin 4 and controls are described above. Increased amounts of stathmin 2 and/or thrombspondin 4 are indicative that a patient is at risk of suffering from osteoarthritis pain.

For example, as discussed above thrombospondin 4 was not observed in serum samples from healthy controls. Here, detection of thrombospondin 4 in a serum sample can be indicative that a patient is at risk of suffering from osteoarthritis and osteoarthritis pain. People with pain due to osteoarthritis who do not respond to usual painkillers e.g. paracetamol and have high throinobospondin 4 levels may also benefit from specific inhibitors of the thrombospondin 4 pathway e.g. gabapentinoids (see below). Likewise, an increased amount of stathmin 2 relative to a control amount may be indicative of osteoarthritis and later development of osteoarthritis pain. For example, in a serum sample, an increase in stathmin 2 relative to the control may be detected (see above).

Similar considerations apply for monitoring osteoarthritis disease progression. Here, the patient has typically been identified as already having osteoarthritis. However, measuring amounts of one or more biomarkers in Table 1 can be used to determine whether or not the disease is developing (typically worsening, but could also be improving), allowing consideration of interventions.

As discussed above, methods may involve measuring amounts of one or more biomarkers in Table 1. However, methods typically involve measuring levels of stathmin 2 and/or thrombospondin 4.

For example, methods of determining disease progression may involve repeated measurements of one or more biomarkers of Table 1 in a sample from the patient. In some cases, an increase in the amount of the biomarker in later samples will be indicative of a worsening in the disease (i.e. those markers in Table 1 known to be increased with osteoarthritis pain). In other cases, a decrease in the amount of biomarker will be indicative of a worsening in the disease (correlated with a biomarker showing a reduction in expression correlated with OA pain in Table 1). The opposite would apply for determining an improvement in the disease state. Samples are as described above.

For example, thrombospondin 4 amounts may be determined in consecutive serum samples from a patient. These consecutive serum samples may taken days, weeks, months or even years apart. A clinician could readily determine when would be appropriate to extract and test samples. Here, an increase in the amount of thrombospondin 4 between samples indicatives an increase in severity of the disease. A decrease in the amount of thrombospondin 4 may also be indicative of an improvement in the disease.

For this reason, methods of the invention could also be used to monitor efficacy of a treatment (where increases/decreases of markers as discussed above would indicate an improvement in disease state).

For stathmin 2, a decrease between samples could be indicative of progression from early to late osteoarthritis.

In some instances, the invention provides use of thrombospondin 4 and/or one or more other biomarkers as identified in Table 1 in evaluating osteoarthritis pain in a patient. The biomarkers may be any of those described above.

As shown in the Examples, the biomarkers of the present invention were identified by first investiging bone marrow lesions in osteoarthritis patients. Bone marrow lesions provide an accurate diagnosis of osteoarthritis. The biomarkers described herein are a reflection of the bone marrow lesions in an osteoarthritis patient (in particular the number, size and severity of the bone marrow lesions). Accordingly, the invention provides a more accurate diagnostic for osteoarthritis/osteoarthritis pain by evaluating the bone marrow lesions via the particular biomarkers. The invention also provides a more accurate way of following progression of osteoarthritis.

The invention therefore also provides use of thrombospondin 4 and/or one or more other biomarkers as identified in Table 1 in evaluating bone marrow lesions in a patient, particularly in evaluating the size and/or severity of bone marrow lesions. The biomarkers may be any of those described above. The patient may also be as described above and the features of the above described methods are also applicable to evaluating bone marrow lesions, for example, increased thrombospondin 4 is indicative of an increase in size/severity of bone marrow lesions. Likewise, increased stathmin 2 is indicative of increased size/severity of bone marrow lesions.

In some instances, the above methods may involve measuring the amount of one or more of the biomarkers and outputting the results, for example outputting the result that a patient has pain as a result of osteoarthritis if the patient has elevated or decreased levels of a biomarker discussed above (i.e. above/below one of the levels discussed above).

In some instances, once a patient has been diagnosed as having pain as a result of osteoarthritis the patient may then be treated for the pain and/or the osteoarthritis itself. In other words, an appropriate treatment is administered to the patient. Such treatments are known in the art and include lifestyle changes, medication and surgery. Exemplary medication for osteoarthritis pain includes non-steroidal anti-inflammatory drugs (e.g. ibuprofen, naproxen), acetaminophen and duloxetine. Patients may also undergo physiotherapy. In some cases, surgery may be required. Procedures include cortisone injections, lubricating injections (e.g. hyaluronic acid), re-aligning bones and joint replacement.

The methods of the invention may also be used for stratification of patients. For example detection of the presence/absence of a biomarker may be used to stratify patient groups for clinical trials (i.e. the identification of patients with shared biological characteristics). The methods of the invention may also be used to identify patients in need of joint replacement. For example, patients determined to have advanced osteoarthritis using the methods described above may benefit from joint replacement or specific pain-relieving medication, including non-steroidal anti-inflammatory agents, intra-articular steroid injections, nutraceuticals such as glucosamine or chondroitin sulphate, anti-nerve growth factor antibodies and centrally acting analgesics such as duloxetine or gabapentinoids.

### Kit for evaluating osteoarthritis

The present invention also relates to a kit for evaluating osteoarthritis and osteoarthritis pain (i.e. for use in any one of the methods described above). The kit comprises a reagent for measuring the amount of one or more of the biomarkers in Table 1 and instructions for use of the kit. The kit may also comprise a means for taking a sample from a patient.

The means for taking a sample from the patient may be any suitable means depending on the type of sample to be obtained. For example, for a blood, plasma, serum, synovial fluid, synovial fluid or urinesample a needle may be provided. The reagent(s) for measuring the amount of the biomarkers may be any suitable reagent. These reagent(s) maybe capable of detecting and/or measuring amounts of mRNA (e.g. a microarray) or protein. For example, reagents for detecting protein may include antibodies that specifically bind the protein of interest. The kit may, for example, comprise a monoclonal antibody, a polyclonal antibody, a single chain antibody, a chimeric antibody, a CDR-grafted antibody or a humanized antibody. The antibody may be an intact immunoglobulin molecule or a fragment thereof such as a Fab, F(ab')₂ or Fv fragment.

The kit may additionally comprise one or more other reagents or instruments which enables the method mentioned above to be carried out. Such reagents include suitable buffers, means to extract/isolate the biomarkers of interest from the sample or a support comprising wells on which quantitative reactions can be done. The kit may, optionally, comprise instructions to enable the kit to be used in the method of invention or details regarding patients on which the method may be carried out.

The kit preferably comprises reagents for measuring stathmin 2 and/or thrombospondin 4. For example, the kit may comprise antibodies for detecting stathmin 2 and/or thrombspondin 4. Such antibodies are commercially available.

### Method of treating or preventing osteoarthritis pain in a patient comprising administering an inhibitor of microtubule activity to the patient

The invention also relates to a method of treating or preventing osteoarthritis pain in a patient. The method comprises administering to the patient an inhibitor of microtubule activity and thereby treating or preventing osteoarthritis pain in the patient. The invention also relates to an inhibitor of microtubule activity for use in a method of treating or preventing osteoarthritis pain in a patient, said method comprising administering the inhibitor to the patient and thereby treating or preventing osteoarthritis pain. Furthermore, the invention relates to an inhibitor of microtubule activity for use in the manufacture of a medicament for treating or preventing osteoarthritis pain in a patient.

The patient has typically been diagnosed as suffering from osteoarthritis, particularly pain resulting from osteoarthritis, using any of the techniques and physical measurements mentioned above. The patient may in particular be determined as suffering from pain caused by osteoarthritis by the methods described above. Typically, the patient has been determined as having increased amounts of stathmin 2 in a sample from the patient. For example, methods may comprise a first step of measuring the amount of stathmin 2 in a sample from the patient. The amount of stathmin 2 in the sample may then be compared to a control amount of stathmin 2. Both of these steps may be performed as described above. If the amount in the sample from the patient is increased compared with the control amount the patient is administered an inhibitor of microtubule activity.

As discussed below, stathmin 2 is involved in physiological microtubule formation and neurite outgrowth. In particular, stathmin 2 is a regulator of microtubule stability. When phosphorylated by MAPK8 it stabilises microtubules and consequently controls neurite length in cortical neurons. It is therefore possible that bone marrow lesion tissue upregulated stathmin 2 is involved in developing new neuronal tissue expression and expansion of bone marrow lesion cavity in osteoarthritis, thereby causing pain as new blood vessels form within BML tissue and exposed to pain sensitizers including chemokines. Inhibitors of microtubule activity therefore have potential as therapeutics for treating or preventing osteoarthritis pain.

Microtubule inhibitors are a recognised class of therapeutic agent, as established by the FDA. Microtubule inhibitors may disrupt microtubules by binding to tubulin. Tubulin binding drugs are generally classified based on their mode of action and binding site. In some cases, microtubule inhibitors inhibit polymerisation of tubulin (such as colchine and vinca alkaloids, which act on different sites). Microtubule inhibitors may also act as depolymerisation inhibitors as they inhibit depolymerisation of polymerized tubulin and hence increase microtubule mass in the cell (taxanes).

*In vitro* and *in vivo* tubulin polymerisation assays are well known in the art, as are assays for determining microtubule growth. Kits for performing such assays are also commercially available. Accordingly, the skilled person would already be aware of, and could identify further, inhibitors of microtubule activity.

In some instances, the inhibitor of microtubule activity is a taxane. Taxanes are complex terpenes produced by plants of the genus Taxus (yews). Originally derived from the Pacific Yew Tree, taxanes are now made artificially. Taxanes are a well known class of compounds and feature a taxadiene core:

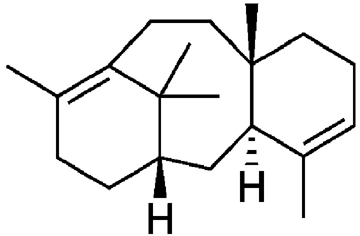

Paclitaxel and docetaxel are examples of taxanes which are already in clinical use to treat cancer.

### Paclitaxel:

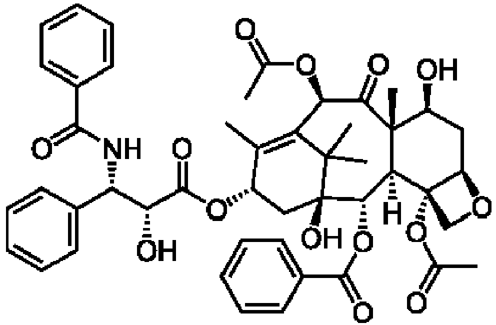

### Docetaxel:

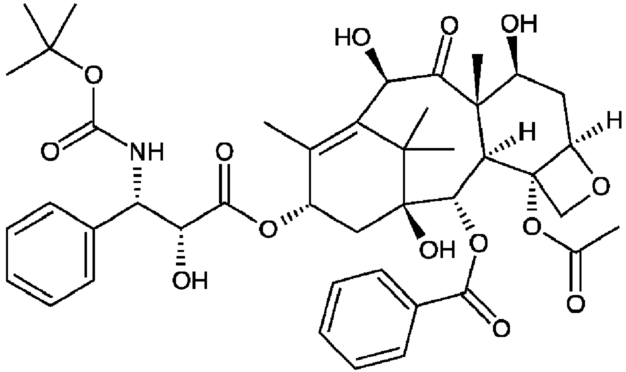

The principal mechanism of action of the taxane class of drugs is the disruption of microtubule function. Microtubules are essential to cell division, and taxanes stabilize GDP-bound tublin in the microtubule, thereby inhibiting the process of cell division as depolymerisation is prevented.

Other inhibitors of microtubule polymerisation are vinca alkaloids. As opposed to taxanes, vinca alkaloids act on tubulin and prevent formation into microtubules. Vinca alkaloids thus prevent microtubule polymerisation. Vinca alkaloids include vinblastine, vincristine, vindesine and vinorelbine. Other less common vinca alkaloids are vincaminol, vineridine and vinbumine.

Methods of the invention may also involve administration of colchicine. Colchicine is an alkaloid derived from the autumn crocus. As mentioned above, colchicine inhibits mitosis by inhibiting microtubule polymerisation. It is commonly used to treat gout.

Another microtubule inhibitor is podophyllotoxin. Podophyllotoxin is derived from the may apple plant and is used to treat viral skin infections. Synthetic analogues are used to treat cancer

Other tubulin depolymerisation inhibitors are epothilone and discodermolide. Other polymerisation inhibitors are crytophycin 52, halichondrins, dolastatins and hemiasterlins (which bind the vinca domain), and combretastatins, 2-Methoxyestradiol and E7010 (which bind the colchicine domain.

The inhibitor of microtubule formation may also be a stathmin 2 inhibitor. An inhibitor of stathmin 2 may be anything that reduces amounts of stathmin 2 present in the patient, or that prevents the functionality of stathmin 2. The stathmin 2 being inhibited typically comprises the amino acid sequence of SEQ ID NO: 1 and/or 2 or a naturally occurring variant thereof. Such variants of stathmin 2 are described above. In other animals, the amino acid sequence of stathmin 2 may be identified from databases such as http://www.uniprot.org. Again, the stathmin 2 may be a naturally occurring variant as described above.

Suitable inhibitors include antibodies and small molecule inhibitors. An example of an anti-stathmin 2 antibody is mentioned in the Examples below.

Inhibitors of stathmin 2 may also reduce amounts of stathmin 2 present in the patient, for example by knocking down expression of stathmin 2. Oligonucleotides, such as antisense, siRNA and shRNA technology for knocking down protein expression are well known in the art and standard methods can be employed to knock down expression of stathmin 2. Both antisense and siRNA technology interfere with mRNA. Antisense oligonucleotides interfere with mRNA by binding to (hybridising with) a section of the mRNA. The antisense oligonucleotide is therefore designed to be complementary to the mRNA (although the oligonucleotide does not have to be 100% complementary as discussed below). In other words, the antisense oligonucleotide may be a section of the cDNA. Again, the olignucleotide sequence may not be 100% identical to the cDNA sequence. This is also discussed below. siRNA involves double-stranded RNA (double-stranded versions of these oligonucleotides).

Methods of the present invention may therefore involve an oligonucleotide which specifically hybridises to a part of (a) SEQ ID NOs:3 and/or 4 (stathmin 2 mRNA) or (b) a variant sequence with at least 90% homology to SEQ ID NO: 3 or 4 as defined above. Oligonucleotides are short nucleotide polymers which typically have 50 or fewer nucleotides, such 40 or fewer, 30 or fewer, 22 or fewer, 21 or fewer, 20 or fewer, 10 or fewer or 5 or fewer nucleotides. The oligonucleotide is preferably 20 to 25 nucleotides in length, more preferably 21 or 22 nucleotides in length.

The nucleotides can be naturally occurring or artificial. A nucleotide typically contains a nucleobase, a sugar and at least one linking group, such as a phosphate, 2'O-methyl, 2' methoxy-ethyl, phosphoramidate, methylphosphonate or phosphorothioate group. The nucleobase is typically heterocyclic. Nucleobases include, but are not limited to, purines and pyrimidines and more specifically adenine (A), guanine (G), thymine (T), uracil (U) and cytosine (C). The sugar is typically a pentose sugar. Nucleotide sugars include, but are not limited to, ribose and deoxyribose. The nucleotide is typically a ribonucleotide or deoxyribonucleotide. The nucleotide typically contains a monophosphate, diphosphate or triphosphate. Phosphates may be attached on the 5' or 3' side of a nucleotide.

Nucleotides include, but are not limited to, adenosine monophosphate (AMP), adenosine diphosphate (ADP), adenosine triphosphate (ATP), guanosine monophosphate (GMP), guanosine diphosphate (GDP), guanosine triphosphate (GTP), thymidine monophosphate (TMP), thymidine diphosphate (TDP), thymidine triphosphate (TTP), uridine monophosphate (UMP), uridine diphosphate (UDP), uridine triphosphate (UTP), cytidine monophosphate (CMP), cytidine diphosphate (CDP), cytidine triphosphate (CTP), 5-methylcytidine monophosphate, 5-methylcytidine diphosphate, 5-methylcytidine triphosphate, 5-hydroxymethylcytidine monophosphate, 5-hydroxymethylcytidine diphosphate, 5-hydroxymethylcytidine triphosphate, cyclic adenosine monophosphate (cAMP), cyclic guanosine monophosphate (cGMP), deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), deoxythymidine monophosphate (dTMP), deoxythymidine diphosphate (dTDP), deoxythymidine triphosphate (dTTP), deoxyuridine monophosphate (dUMP), deoxyuridine diphosphate (dUDP), deoxyuridine triphosphate (dUTP), deoxycytidine monophosphate (dCMP), deoxycytidine diphosphate (dCDP) and deoxycytidine triphosphate (dCTP), 5-methyl-2'-deoxycytidine monophosphate, 5-methyl-2'-deoxycytidine diphosphate, 5-methyl-2'-deoxycytidine triphosphate, 5-hydroxymethyl-2'-deoxycytidine monophosphate, 5-hydroxymethyl-2'-deoxycytidine diphosphate and 5-hydroxymethyl-2'-deoxycytidine triphosphate. The nucleotides are preferably selected from AMP, TMP, GMP, UMP, dAMP, dTMP, dGMP or dCMP.

The nucleotides may contain additional modifications. In particular, suitable modified nucleotides include, but are not limited to, 2'amino pyrimidines (such as 2'-amino cytidine and 2'-amino uridine), 2'-hyrdroxyl purines (such as, 2'-fluoro pyrimidines (such as 2'-fluorocytidine and 2'fluoro uridine), hydroxyl pyrimidines (such as 5'-α-P-borano uridine), 2'-O-methyl nucleotides (such as 2'-O-methyl adenosine, 2'-O-methyl guanosine, 2'-O-methylcytidine and 2'-O-methyl uridine), 4'-thio pyrimidines (such as 4'-thio uridine and 4'-thio cytidine) and nucleotides have modifications of the nucleobase (such as 5-pentynyl-2'-deoxy uridine, 5-(3-aminopropyl)-uridine and 1,6-diaminohexyl-N-5-carbamoylmethyl uridine).

One or more nucleotides in the oligonucleotide can be oxidized or methylated. One or more nucleotides in the oligonucleotide may be damaged. For instance, the oligonucleotide may comprise a pyrimidine dimer. Such dimers are typically associated with damage by ultraviolet light.

The nucleotides in the oligonucleotide may be attached to each other in any manner. The nucleotides may be linked by phosphate, 2'O-methyl, 2' methoxy-ethyl, phosphoramidate, methylphosphonate or phosphorothioate linkages. The nucleotides are typically attached by their sugar and phosphate groups as in nucleic acids. The nucleotides may be connected via their nucleobases as in pyrimidine dimers.

The invention also provides an oligonucleotide which comprises 50 or fewer consecutive nucleotides from (a) SEQ ID NO: 5 (cDNA sequence of human stathmin 2) or (b) a variant sequence with at least 90% homology to SEQ ID NOs: 5 as defined above. The oligonucleotide may be any of the lengths discussed above. It is preferably 21 or 22 nucleotides in length. The oligonucleotide may comprise any of the nucleotides discussed above, including the modified nucleotides.

The oligonucleotide can be nucleic acids, such as deoxyribonucleic acid (DNA) or a ribonucleic acid (RNA). The polynucleotide may be any synthetic nucleic acid known in the art, such as peptide nucleic acid (PNA), glycerol nucleic acid (GNA), threose nucleic acid (TNA), locked nucleic acid (LNA), morpholino nucleic acid or other synthetic polymers with nucleotide side chains. The oligonucleotide may comprise any of the nucleotides discussed above, including the modified nucleotides. The oligonucleotide is preferably RNA.

The oligonucleotide is preferably single stranded. The oligonucleotide may be double stranded i.e. siRNA comprising an oligonucleotide with 50 or fewer consecutive nucleotides from SEQ ID NOs: 3 and/or 4 and 50 or fewer consecutive nucleotides from SEQ ID NO: 5. siRNA technology is well known in the art and standard methods can be employed to knock down stathmin 2 in the present invention.

An oligonucleotide preferably specifically hybridises to a part of a SEQ ID NO: 3 and/or 4 (stathmin 2 mRNA), hereafter called the target sequence. The length of the target sequence typically corresponds to the length of the oligonucleotide. For instance, a 21 or 22 nucleotide oligonucleotide typically specifically hybridises to a 21 or 22 nucleotide target sequence. The target sequence may therefore be any of the lengths discussed above with reference to the length of the oligonucleotide. The target sequence is typically consecutive nucleotides within the polynucleotide of the invention.

An oligonucleotide "specifically hybridises" to a target sequence when it hybridises with preferential or high affinity to the target sequence but does not substantially hybridise, does not hybridise or hybridises with only low affinity to other sequences.

An oligonucleotide "specifically hybridises" if it hybridises to the target sequence with a melting temperature (Tₘ) that is at least 2 °C, such as at least 3 °C, at least 4 °C, at least 5 °C, at least 6 °C, at least 7 °C, at least 8 °C, at least 9 °C or at least 10 °C, greater than its Tₘ for other sequences. More preferably, the oligonucleotide hybridises to the target sequence with a Tₘ that is at least 2 °C, such as at least 3 °C, at least 4 °C, at least 5 °C, at least 6 °C, at least 7 °C, at least 8 °C, at least 9 °C, at least 10 °C, at least 20 °C, at least 30 °C or at least 40 °C, greater than its Tₘ for other nucleic acids. Preferably, the portion hybridises to the target sequence with a Tₘ that is at least 2 °C, such as at least 3 °C, at least 4 °C, at least 5 °C, at least 6 °C, at least 7 °C, at least 8 °C, at least 9 °C, at least 10 °C, at least 20 °C, at least 30 °C or at least 40 °C, greater than its Tₘ for a sequence which differs from the target sequence by one or more nucleotides, such as by 1, 2, 3, 4 or 5 or more nucleotides. The portion typically hybridises to the target sequence with a Tₘ of at least 90 °C, such as at least 92 °C or at least 95 °C. Tₘ can be measured experimentally using known techniques, including the use of DNA microarrays, or can be calculated using publicly available Tₘ calculators, such as those available over the internet.

Conditions that permit the hybridisation are well-known in the art (for example, Sambrook et al., 2001, Molecular Cloning: a laboratory manual, 3rd edition, Cold Spring Harbour Laboratory Press; and Current Protocols in Molecular Biology, Chapter 2, Ausubel et al., Eds., Greene Publishing and Wiley-Interscience, New York (1995)). Hybridisation can be carried out under low stringency conditions, for example in the presence of a buffered solution of 30 to 35% formamide, 1 M NaCl and 1 % SDS (sodium dodecyl sulfate) at 37 °C followed by a 20 wash in from 1X (0.1650 M Na⁺) to 2X (0.33 M Na⁺) SSC (standard sodium citrate) at 50 °C. Hybridisation can be carried out under moderate stringency conditions, for example in the presence of a buffer solution of 40 to 45% formamide, 1 M NaCl, and 1 % SDS at 37 °C, followed by a wash in from 0.5X (0.0825 M Na⁺) to 1X (0.1650 M Na⁺) SSC at 55 °C. Hybridisation can be carried out under high stringency conditions, for example in the presence of a buffered solution of 50% formamide, 1 M NaCl, 1% SDS at 37 °C, followed by a wash in 0.1X (0.0165 M Na⁺) SSC at 60 °C.

The oligonucleotide may comprise a sequence which is substantially complementary to the target sequence. Typically, the oligonucleotides are 100% complementary. However, lower levels of complementarity may also be acceptable, such as 95%, 90%, 85% and even 80%. Complementarity below 100% is acceptable as long as the oligonucleotides specifically hybridise to the target sequence. An oligonucleotide may therefore have 1, 2, 3, 4, 5, 6, 7, 8, 9, 10 or more mismatches across a region of 5, 10, 15, 20, 21, 22, 30, 40 or 50 nucleotides.

Oligonucleotides may be synthesised using standard techniques known in the art. Alternatively, oligonucleotides may be purchased.

The ability of an inhibitor as a potential therapeutic for osteoarthritis pain can be tested using using bone marrow lesion explant cultures. Such cultures can be used to test the level of stathmin 2 inhibition.

In the methods of the invention, the inhibitor of microtubule formation is administered to the patient in order to treat or prevent osteoarthritis pain. Inhibitors of stathmin 2 may be administered to the patient in any appropriate way. In the invention, inhibitors may be administered in a variety of dosage forms. Thus, they can be administered orally, for example as tablets, troches, lozenges, aqueous or oily suspensions, dispersible powders or granules. They may also be administered parenterally, either subcutaneously, intravenously, intramuscularly, intrastemally, transdermally or by infusion techniques. They may also be administered as suppositories. A physician will be able to determine the required route of administration depending on the inhibitor for each particular patient.

The formulation of an inhibitor will depend upon factors such as the nature of the exact inhibitor, etc. An inhibitor may be formulated for simultaneous, separate or sequential use.

An inhibitor is typically formulated for administration in the present invention with a pharmaceutically acceptable carrier or diluent. The pharmaceutical carrier or diluent may be, for example, an isotonic solution. For example, solid oral forms may contain, together with the active substance, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, gum arabic, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non-toxic and pharmacologically inactive substances used in pharmaceutical formulations. Such pharmaceutical preparations may be manufactured in known manner, for example, by means of mixing, granulating, tabletting, sugar-coating, or film-coating processes.

Liquid dispersions for oral administration may be syrups, emulsions or suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspensions or solutions for intramuscular injections may contain, together with the active substance, a pharmaceutically acceptable carrier, e.g. sterile water, olive oil, ethyl oleate, glycols, e.g. propylene glycol, and if desired, a suitable amount of lidocaine hydrochloride.

Solutions for intravenous administration or infusion may contain as carrier, for example, sterile water or preferably they may be in the form of sterile, aqueous, isotonic saline solutions.

The oligonucleotides maybe naked nucleotide sequences or be in combination with cationic lipids, polymers or targeting systems. The oligonucleotides may be delivered by any available technique. For example, the oligonucleotide may be introduced by needle injection, preferably intradermally, subcutaneously or intramuscularly. Alternatively, the oligonucleotide may be delivered directly across the skin using a oligonucleotide delivery device such as particle-mediated gene delivery. The oligonucleotide may be administered topically to the skin, or to mucosal surfaces for example by intranasal, oral, or intrarectal administration.

Preferably, inhibitors are administered orally.

Uptake of oligonucleotide constructs may be enhanced by several known transfection techniques, for example those including the use of transfection agents. Examples of these agents includes cationic agents, for example, calcium phosphate and DEAE-Dextran and lipofectants, for example, lipofectam and transfectam. The dosage of the oligonucleotide to be administered can be altered.

A therapeutically effective amount of an inhibitor is administered to the patient. The dose may be determined according to various parameters, especially according to the substance used; the age, weight and condition of the patient to be treated; the route of administration; and the required regimen. Again, a physician will be able to determine the required route of administration and dosage for any particular patient. A typical daily dose is from about 0.1 to 50 mg per kg of body weight, according to the activity of the specific inhibitor, the age, weight and conditions of the subject to be treated and the frequency and route of administration. The dose may be provided as a single dose or may be provided as multiple doses, for example taken at regular intervals, for example 2, 3 or 4 doses administered hourly. Preferably, dosage levels of inhibitors are from 5 mg to 2 g.

Typically oligonucleotide inhibitors are administered in the range of 1 pg to 1 mg, preferably to 1 pg to 10 µg nucleic acid for particle mediated delivery and 10 µg to 1 mg for other routes.

### Method of treating osteoarthritis pain in a patient comprising administering an inhibitor of the NOTCH1/thrombospondin 4 signalling pathway

The invention also relates to a further method of treating or preventing osteoarthritis pain in a patient. The method comprises administering to the patient an inhibitor of the NOTCH1/thrombospondin 4 signalling pathway and thereby treating or preventing osteoarthritis pain in the patient. The invention also relates to an inhibitor of the NOTCH1/thrombospondin 4 signalling pathway for use in a method of treating or preventing osteoarthritis pain in a patient, said method comprising administering the inhibitor to the patient and thereby treating or preventing osteoarthritis pain. Furthermore, the invention relates to an inhibitor of the NOTCH1/thrombospondin 4 signalling pathway for use in the manufacture of a medicament for treating or preventing osteoarthritis pain in a patient.

The patient has been diagnosed as suffering from osteoarthritis, particularly pain resulting from osteoarthritis, using any of the techniques and physical measurements mentioned above. The patient may in particular be determined as suffering from pain caused by osteoarthritis by the methods described above. Typically, the patient has been determined as having increased amounts of thrombospondin 4 in a sample from the patient. For example, methods may comprise a first step of measuring the amount of thrombospondin 4 in a sample from the patient. The amount of thrombospondin 4 in the sample may then be compared to a control amount of thrombospondin 4. Both of these steps may be performed as described above. If the amount in the sample from the patient is increased compared with the control amount, the patient is administered an inhibitor of NOTCH1/thrombospondin 4.

Inhibitors of NOTCH1 signalling are known in the art and include γ-secretase inhibitors such as N-[N-(3,5-Difluorophenacetyl)-L-alanyl]-S-phenylglycine t-butyl ester (DAPT), BMS-906024, MK0752 and PF-03084014) and semgacestat. Structures of these inhibitors are shown in the table below:

| | |
|---|---|
| DAPT | 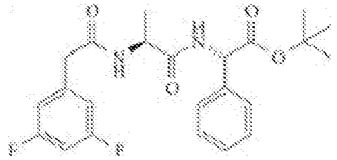 |
| BMS-906024 | 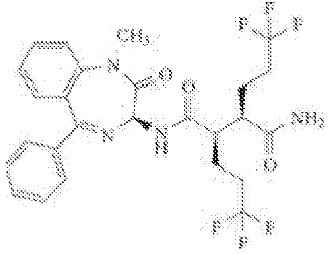 |
| MK0752 | 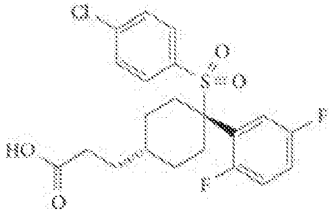 |
| PF-03084014 | 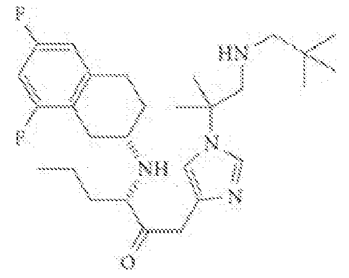 |
| Sernagacestat (LY-450139) | 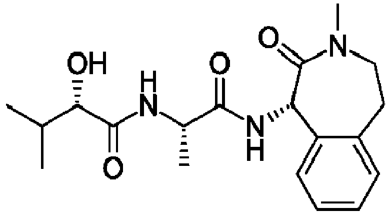 |

The inhibitor may also be a thrombospondin 4 inihbitor. An inhibitor of thrombospondin 4 may be anything that reduces amounts of thrombospondin 4 present in the patient, or that prevents the functionality of thrombospondin 4. The thrombospondin 4 being inhibited typically comprises the amino acid sequence of SEQ ID NO: 6 or 7 or a naturally occurring variant thereof. Such variants of thrombospondin 4 are described above. In other animals, the amino acid sequence of thrombospondin 4 may be identified from databases such as http://www.uniprot.org. Again, the thrombospondin 4 may be a naturally occurring variant as described above.

Suitable inhibitors include antibodies and small molecule inhibitors. An example of an anti- thrombospondin 4 antibody is mentioned in the Examples below.

As discussed above, inhibitors of thrombospondin 4 may also reduce amounts of thrombospondin 4 present in the patient, for example by knocking down expression of thrombospondin 4. As described above, oligonucleotides, including antisense, siRNA and shRNA technology may be used.

Methods of the present invention may therefore involve an oligonucleotide which specifically hybridises to a part of (a) SEQ ID NOs: 8-11 (thrombospondin 4 mRNA) or (b) a variant sequence with e.g. at least 90% homology to SEQ ID NOs:8-11 as defined above. Oligonucleotides are as described above.

The invention also provides an oligonucleotide which comprises 50 or fewer consecutive nucleotides from the cDNA sequence of human thrombospondin 4 or a variant sequence with at least 90% homology. The oligonucleotide may be any of the lengths discussed above. It is preferably 21 or 22 nucleotides in length. The oligonucleotide may comprise any of the nucleotides discussed above, including the modified nucleotides.

The oligonucleotide is preferably single stranded. The oligonucleotide may be double stranded i.e. siRNA comprising an oligonucleotide with 50 or fewer consecutive nucleotides. siRNA technology is well known in the art and standard methods can be employed to knock down thrombospondin 4 in the present invention.

An oligonucleotide preferably specifically hybridises to a part of a SEQ ID NO: 8-11 (thrombospondin 4 mRNA), hereafter called the target sequence. The length of the target sequence typically corresponds to the length of the oligonucleotide. For instance, a 21 or 22 nucleotide oligonucleotide typically specifically hybridises to a 21 or 22 nucleotide target sequence. The target sequence may therefore be any of the lengths discussed above with reference to the length of the oligonucleotide. The target sequence is typically consecutive nucleotides within the polynucleotide of the invention.

In the methods of the invention, the inhibitor of the thrombospondin 4 signalling pathway may also be a *gamma*-aminobutyric acid derivative, such as a gabapentinoid. The receptor for thrombospondin 4, α2δ-1, is targeted by such compounds.

The *gamma*-aminobutyric acid derivative may be a compound of formula (I) or a pharmaceutically acceptable salt thereof 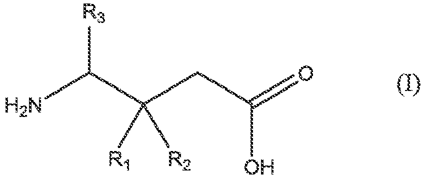 wherein
R₁ is a C₁₋₆ alkyl, phenyl, or C₃₋₆ cycloalkyl group;
R₂ is a hydrogen or methyl group; and
R₃ is a hydrogen, methyl, or carboxyl group.

An alkyl group may be a straight-chain or branched-chain alkyl group. C₁₋₆ alkyl includes methyl, ethyl, propyl, butyl, pentyl and hexyl. C₃₋₆ cycloalkyl includes cyclopropyl, cyclobutyl, cyclopentyl, and cyclohexyl.

Preferably R₁ is a C₁₋₆ alkyl group, more preferably a -(CH₂)₀₋₂-iC₄H₉ group and most preferably an -iC₄H₉ group (i.e., an isobutyl group). Preferably R₂ is hydrogen. Preferably R₃ is hydrogen. A particularly preferred compound of formula (I) is one in which R₁ is an -iC₄H₉ group and R₂ and R₃ are both hydrogen. A particularly preferred gamma-aminobutyric acid derivative is the compound of formula (I) in which R₁ is an -iC₄H₉ group and R₂ and R₃ are both hydrogen.

Most preferably the compound of formula (I) is pregabalin, i.e. (3S)-3-(aminomethyl)-5-methylhexanoic acid. The structure of pregabilin is shown below.

Other *gamma-*aminobutyric acid derivatives (gabapentinoids) used clinically are gabapentin and phenibut.

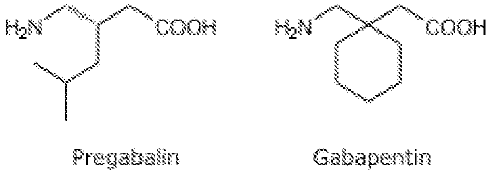

### Phenibut:

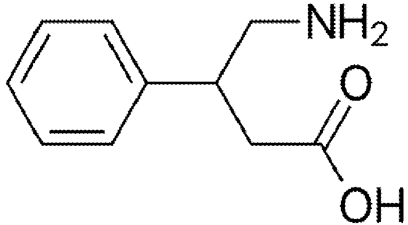

Inhibitors of thrombospondin 4 signalling may be particularly useful in two types of patient group:
1. Patients who have osteoarthritis pain who have pain sensitisation with features of pain sensitivity in regions further away from areas of joint damage. These patients are likely to have higher thrombospondin 4 levels.
2. Patients with inflammation in their joint fluid, redness and swelling, with no pain or swelling in other joints. These patients are likely to respond to local treatments such as anti-inflammatories and/or steroid injections.

Methods of administering inhibitors are as described above.

### Other methods of treating or preventing osteoarthritis pain

Methods of the invention may also involve targeting any of the other biomarkers identified in Table 1 (especially those listed above), or the pathways represented by these biomarkers. For example, where a biomarker has been shown to be increased in osteoarthritis pain, methods of the invention may inhibit this biomarker (or a pathway associated with the biomarker) in order to treat or prevent osteoarthritis pain. Inhibitition is as described above, and may involve specifically targeting the biomarker with for example an antibody or an oligonucleotide (such as siRNA or antisense RNA). In cases where a marker is down-regulated, methods of the invention may for example involve administering the protein (or a nucleic acid encoding the protein) in order to treat or prevent osteoarthritis pain.

### Method of screening for agents to reduce osteoarthritis pain

Finally, the invention provides a method of screening for agents which treat or prevent osteoarthritis pain. The methods may comprise identifying agents which interact with any of the biomarkers identified in Table 1. The methods may then involve identifying whether an agent is an inhibitor or activator i.e. for an upregulated gene/protein the method may comprise identifying an inhibitor.

Typically, the methods comprise identifying inhibitors which interact with stathmin 2 and/or thrombospondin 4 and assessing whether the agents inhibit stathmin 2 and/or thrombospondin 4.

The agents may be e.g. small molecule inhibitors, oligonucleotides (see above) or antibodies. Standard methods known in the art may be used to determine that an inhibitor interacts with the protein in question. Methods include fluorescence quenching, fluorescence polarization/anisotropy, partitioning techniques such as dialysis, gel filtration and filter binding, surface plasmon resonance and isothermal calorimetry. In particular, binding between an antibody and a target protein may be determined by surface plasmon resonance.

Methods of screening may also be applied to the other biomarkers identified in Table 1, or potentially to components in pathways associated with these biomarkers.

The following examples are presented below so as to provide those of ordinary skill in the art with a complete disclosure and description of how to make and use the methods and compositions of the invention. The examples are not intended to limit the scope of what the inventors regard as their invention.

### Examples

### Example 1: Initial analysis of BMLs

### Materials and Methods

All study procedures were carried out after Ethical Approval was granted by the UK Health Research Authority approval number 12/LO/1970. The study was also registered on clinical trials.gov identifier NCT02603939e with UK Clinical Research Network ID was 15707. Participants attending a large South London Orthopaedic centre were recruited at the time of their assessment for total knee replacement (TKR), which was the `late OA group'. For the `early OA' group, participants were recruited from rheumatology clinics at St George's University Hospitals NHS Foundation Trust. For tissue bone controls, participants were recruited from orthopaedic trauma lists for participants undergoing joint surgery following traumatic fractures or trochleoplasties, approval number 09/H0806/45. None of the tissue control participants had a clinical diagnosis of OA, inflammatory arthritis or other bone disorders. Blood and urine samples were also obtained with full consent for biomarker studies.

Inclusion criteria. Participants were eligible for screening if they were aged 35-90 years, had knee OA diagnosed by ACR criteria (Altman R et al. Arthritis Rheum 1986; 29: 1039-1049) confirmed by a rheumatologist and experiencing pain. Criteria also stated that participants experience sustained pain despite treatment with usual care as per current UK NICE guidelines for OA (https://www.nice.org.uk/guidance/cgl77). All participants underwent baseline knee radiography to confirm evidence of OA. Radiographic findings were used to confirm changes with a Kellgren-Lawrence grade of 2 or above in the affected knee joint (Kellgren et al, Ann Rheum Dis 1957; 16(4): 494-502). Participants were consented for knee MRI of the target joint and waste joint tissue was collected at the time of TKR. Participants without knee OA were recruited as control subjects aged 35-90 years.

Exclusion criteria. Exclusion was for other diagnoses e.g. rheumatoid arthritis, psoriatic arthritis, systemic lupus erythematosus (SLE), fibromyalgia, current or planned pregnancy, regular use of bisphosphonates within the last 6 months, history of clinically-diagnosed depression or anxiety disorder and recent surgery. Participants over 150 kg were also excluded since they were precluded from MRI scanning.

Clinical data collection. The primary pain score collected was the Western Ontario and McMaster Universities Osteoarthritis Index (WOMAC) with sub-scales for stiffness and function (Bellamy et al, Arthritis Care Res (Hoboken) 2015; 67(7): 972-80). Participants were asked to record their scores based on symptoms 'in the last 48 hours'. Data was also collected for body mass index (BMI), Numerical Rating Scale (NRS) pain rating 0-10 (Dworkin et al, Pain 2005; 113: 9-19), the Hospital Anxiety and Depression Scale (HADS) (Bjelland et al, Journal of Psychosomatic Research. 2002; 52(2): 69-77) and pain sensitization measures using quantitative sensory testing (QST) as we have previously described (Wajed et al, Rheumatology 2011; 50 (suppl 3): iii91(129) doi: 10.1093/rheumatology/ker160). QST data will be reported separately. All primary data were double entered and checked for consistency. Secondary data were checked after data entry.

Magnetic resonance imaging. A Philips 3T MRI scanner acquired images from participants within 6 weeks of TKR. Timing selected was to optimize the visualization of structural changes, including BML, synovitis and cartilage damage. Briefly, a T1 and T2 weighted protocol was used. All data was acquired using the manufacturers' approved imaging protocols with all scanner safety procedures and full CE-marking in place. Radiographic changes were recorded independently using the validated MRI Knee Osteoarthritis Score (MOAKS) (Hunter et al. Osteoarthritis Cartilage 2011; 19(8): 990-1002) by two consultant radiologists (VE and CH) independently and consensus scores reached. All contraindications to MRI scanning were adhered to, including recent surgery, presence of metal implants or claustrophobia. Multimodal MRI scans lasted 30 minutes and included scout images, T2-weighted imaging for lesion detection, 3D T1-weighted imaging for delineation of knee anatomical structures, with T1/T2 weighted imaging for vascular characterization.

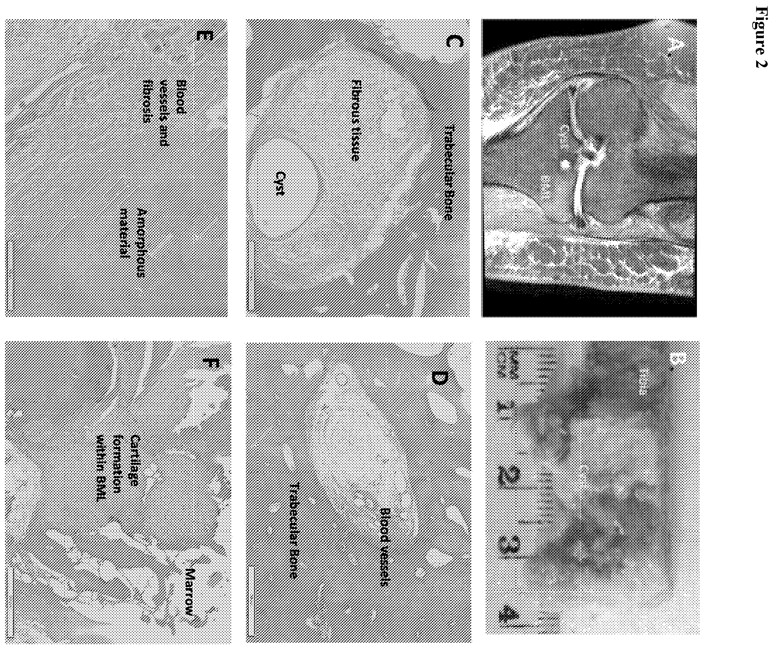

Region of interest localisation and RNA isolation. MR images were viewed as DICOM files using an image processing and analysis software (ImageJ, NIH, Wayne Rasband, 1997). Axial image dimensions were set as: pixel width 0.25mm and pixel height: 0.2mrn on ImageJ then scaled at 160mm x 160mm in line with the field of view. A 2cm x 2cm grid was drawn over the axial images and the MOAKS scoring was used to confirm the location of the lesions. The tibial plateau was placed over a grid of 2cm x 2cm with 1mm increments (Figure 2). This was used in line with the scaled axial images to measure and locate the BMLs. Once the lesions were located a non-BML control sample was taken approximately ≥5cm contralateral to the lesion and both regions were dissected using a hack saw. The samples were then divided equally into two: one part stored in - 80°C for expression studies and the other fixed in 10% natural buffered formalin (VWR, Leicestershire, UK) for histology. Total RNA was isolated from bone tissue obtained during surgery using the RNeasy Mini Kit (Qiagen, Crawley, UK) according to manufacturer's instructions (RNeasy mini Hand book isolation kit. Qiagen. Fourth Edition, June 2012. https://www.qiagen.com/es/resources/resaurcedetail?id=14e7cf6e-521a-4cf7-8cbc-bf9f6fa33e24&lang=en). Total RNA was measured via spectrophotometry using a Nanodrop 1000 (Thermo Scientific, Hertfordshire, UK). Integrity and purity of the RNA was assessed via microfluidic electrophoretic technology using an Agilent 2100 bioanalyzer (Agilent Technologies, Santa Clara, CA, US).

Scanning electron microscopy (SEM). A Zeiss SEM was used for all work. More than 3000 images were obtained from a subgroup of 15 participants undergoing TKR surgery. The techniques used employed correlation of MRI with bone tissue previously developed in our laboratories (Boyde A. Methods Mol Biol. 2012;816:365-400 and Boyde et al. J Anat. 2014 Oct;225(4):436-46). Tissue was embedded in PMMA, block surfaces trimmed, polished and examined uncoated using 20kV BSE SEM at 50Pa chamber pressure in a Zeiss EVOMA10 SEM (Boyde A. Eur Cell Mater. 2012 Jul 24;24:154-60). A 20 kV BSE-SEM gave electron optical section as extensive as the block surface approximately half a micron in thickness, showing mineral density/concentration almost equivalent to a microradiograph with higher resolution. Blocks were stained using iodine vapour or ammonium triiodide solution before SEM to provide good imaging of all soft tissue phases (Boyde et al, Microsc Res Tech. 2014 Dec;77(12) and Ley et al, Equine Vet J. 2016 Jan;48(1):57-64). MRI images were matched to SEM to aid visualisation of BML. The location of SEM images was mapped using block photos and montage imaging. Further processing of some blocks was required due to bone fragment impaction into marrow spaces during TKR. Blocks were re-cut and re-polished to provide multiple adjoining facets and a kind of 3D (2.5D) imaging. Other tissue pieces were 'macerated' using alkaline bacterial pronase solution (Tergazyme ^{™}): in some cases also 5% hydrogen peroxide solution, washed, dried and imaged using same SEM instrument and same conditions, but here image signals show topography rather than composition of the sample. Our images of bone mineralising fronts employed this technique. In addition, since we kept large pieces of tissue intact, we were able to correlate SEM image locations with 3D of the surgical sample(s) and MRI images.

Histological Analysis. All bone specimens were fixed in 10% neutral buffered formalin for 24 hours. After fixation specimens were decalcified to remove the calcium phosphate using formic acid containing 40% (v/v) formalin. Specimens were placed in the decalcification solution at 20 times the tissue volume for up to 14 days at room temperature (RT). Once decalcified the specimens were dehydrated and paraffin embedded before being sectioned using a microtome at 5µm (Leica RM2255, Milton Keynes, UK). The sections were stained in Haematoxylin and Eosin (H&E) (Leica, Milton Keynes, UK). Stained slides were scanned using a NanoZoomer 2.0-RS Digital Scanner (Hamamatsu, Hertfordshire, UK) to visualize tissue changes as high resolution digital data using the NanoZoomer Digital Pathology v2.0 viewing software (Hamamatsu, Hertfordshire, UK).

Whole Transcriptomic Analysis. Total RNA (400ng) isolated from bone samples from 10 healthy controls and 14 OA patients with extensive BMLs was used for one round of cRNA synthesis and amplification. Cyanine 3-labeled cRNAs were purified and hybridized to Agilent whole human genome 60k microarray chips (Agilent Technologies, Santa Clara, CA, USA). All procedures were carried out according to the manufacturer's instructions (Microarray-Based Gene Expression Analysis. Version 6.9.1. December 2015. https://www.agilent.com/cs/library/usermanuals/Public/G2505-90019 _ScannerC_User.pdf). The array signal intensities were further analysed by the Agilent Gene-Spring GX software (version 11.5). The dataset was normalized by quantile normalization. The significant differentially expressed entities between bone samples from healthy controls and OA patients were identified by a Bonferroni FWER corrected union of a student's t-test and moderated t-test. Probes with a threshold of ≥1.5 fold-change and a P-value <0.05 in gene expression were additionally analysed by the hierarchical clustering method, with a Pearson Correlation Coefficient algorithm and an average linkage method.

Pathways Analysis. Significant differentially expressed gene IDs were uploaded onto PANTHER pathway analysis (PANTHER Classification Systems 10.0; www.pantherdb.org) for functional classification. The online resource includes almost 5000 new protein families, 104 fully sequenced genomes spanning all kingdoms of life and inferring functional annotations from the GO Phylogenetic Annotation project for the biological interpretation of large-scale experimental datasets (Mi et al, Nucleic Acids Res 2016; 44(D1): D336-42). The biological interaction scores were defined by the IPA statistical algorithm: based on the z-score and P-value, which were calculated by the IPA regulation z-score algorithm and the Fischer's exact test. A positive or negative z-score of more than 1.5 or less than 1.5, and P-value less than 0.05 (-log10 ≥1.5) indicates a significant biological function and predicts that the biological process or condition is leaning towards an increase (z-score ≥1.5) or decrease (z-score ≤ -1.5). P values were corrected for multiple comparisons with the Benjaminie Hochberg test.

Quantitative Reverse-Transcription PCR Validation. We performed quantitative reverse-transcription PCR (qPCR) for expression of MMP13, OMD, STMN2 and THBS4 genes using ATP5B, CYC1, E1F4A2 and SDHA as endogenous reference genes. The genes of interest (GOI) were identified for verification on the basis of significant fold-change (≥1.5) comparing the healthy control and OA patient microarray results. To select a fixed group of reference genes the geNorm reference gene selection kit (PrimerDesign, Southampton, UK) was used. The kit incorporates 12 of the most notably cited candidate reference genes for which qPCR was used to measure expression in disease and non-disease tissue. The geNorm Biogazelle qbase PLUS software was used to analyse the data resulting in a list of the most optimal reference genes for accurate normalisation dependent on the stability of their expression. Two regions of interest (BML and non-BML) were analysed in 15 OA bone tissue samples using 13 healthy control bone samples for the control group. Total RNA from each region was converted into cDNA using Superscript II reverse transcriptase (Invitrogen, Paisley, UK). The qPCR was performed using GoTaq SYBR Green florescence system (Promega, Southampton, UK) according to the manufacturer's instructions. The qRT-PCR reactions were performed on the Bio-Rad CFX96 or CFX384 Real-Time PCR Detection System (Bio-Rad Laboratories, Hertfordshire, UK) and the fluorescent signal intensity was analysed by CFX Manager software (Bio-Rad Laboratories, Hertfordshire, UK). Primer sequences are presented below:

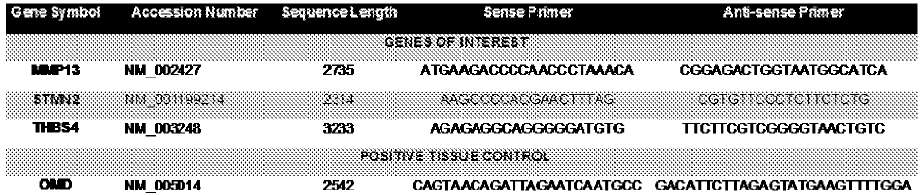

Two-tailed unpaired student t-tests were performed to evaluate for statistically significant differences in gene expression levels between regions.

Enzyme-Linked. ImmunoSorbent Assay. Detection of C-terminal telopeptides of type II collagen cleavage products was conducted using the Urine Cartilaps (CTX-II) EIA (Immunodiagnostic Systems) as per the manufacturers' instructions and as previously described (24). Urine samples collected at the first visit were sampled for type II collagen cleavage products. Matched urine samples were tested for creatinine for normalization. The absorbance of each well was read using a microplate reader at 450 nm.

### Results

The study demographics data demonstrate that participants were representative of a knee OA population. In particular, as shown in the table below, the knee OA participants who underwent TKR had a high BMI and had high pain scores measured by WOMAC.

**Table 5: Demographics showing characteristics of study population**

| | | **Advanced OA** | **Early OA** |
|---|---|---|---|
| **Number*** | | 72 | 12 |
| **Age** | | | |
| **Range** | | 51-88 | 49-79 |
| **Mean (SD)** | | 69.1 (7.7) | 62.2 (8.5) |
| **Gender** | | | |
| **Female N (%)** | | 55 (76.4%) | 9 (75%) |
| **Body Mass Index** | | | |
| **Mean (SD)** | | 32.5 (5.7) | 28.8 (3.9) |
| **WOMAC Pain** | | | |
| **Mean (SD)** | | 297.1 (106.7) | 154.4 (101.5) |
| **WOMAC Total** | | | |
| **Mean (SD)** | | 1436.2 (471.8) | 797.4 (549.6) |
| **NRS Pain** | | | |
| **Mean (SD)** | | 5.7 (2.3) | 2.6 (2.4) |
| **HADS** | | | |
| **Mean (SD)** | | 12.6 (7.2) | 9.6 (6.7) |
| **MOAKS N(%)** | | | |
| **BML** | MOAKS = 0 | 9 (14.1%) | 4 (57.1%) |
| | MOAKS = 1 | 52 (81.3%) | 3 (42.9%) |
| | MOAKS = 2 | 3 (4.7%) | 0 (0%) |
| | MOAKS = 3 | 0 (0%) | 0 (0%) |

| **Synovitis/Effusion** | | | |
|---|---|---|---|
| | MOAKS = 0 | 2 (3.1%) | 2 (28.6%) |
| | MOAKS = 1 | 28 (43.8%) | 2 (28.6%) |
| | MOAKS = 2 | 18 (28.1%) | 1 (14.3%) |
| | MOAKS = 3 | 16 (25%) | 2 (28.6%) |

| **Cartilage damage** | | | |
|---|---|---|---|
| | MOAKS = 0 | 0 (0%) | 4 (57.1%) |
| | MOAKS = 1 | 16 (25%) | 3 (42.9%) |
| | MOAKS = 2 | 41 (64.1%) | 0 (0%) |
| | MOAKS = 3 | 7 (10.9%) | 0 (0%) |
| **Clinical managment** | | Underwent knee replacement surgery | Medical management |

The mean (SD) total WOMAC scores in the groups were as follows: advanced OA 1436.2 (471.6), early OA 797.4 (549.6) and controls 10.5 (12.6), demonstrating that the advanced OA group had severe pain (p<0.0001). The MOAKS analysis found that the advanced OA group had severe knee OA, as demonstrated by 81.3% of the advanced OA group having BML of up to 33% bone involvement corresponding to a MOAKS score 1 in the 21 regions involved, in addition to significant levels of synovitis and cartilage damage (Table 5). The dataset provided an ideal starting point for targeted tissue analysis of BML by mapping MRI regions as a focus of further analysis by SEM and tissue microarray.

SEM analysis showed most normal bone marrow was adipocytic with adipocytes the major bone lining cells, often making and moulding trabecular excrescences (Figure 2). Bone volume fraction was starkly reduced in BML areas, with marrow replaced by dense fibrous connective tissue, hyaline cartilage and fibrocartilage. Areas of aggressive resorption were found at the periphery of BML patches and areas of calcified cartilage so deep within the bone that they could not be explained by impaction from the joint surfaces, but were arising by mineralization of cartilage formed deep within the bone organ (Figure 1). SEM demonstrated that what appeared to be cystic lesions on MRI were more heterogeneous than the structures appeared by MRI techniques. Areas of new blood vessels with fibrocartilage were identified, interspersed with regions of new cartilage formation. In other areas there was a more amorphous infiltrate of bone, including bone marrow infiltrated with connective tissue-like material. When bone tissue was macerated to remove debris, evidence of new bone formation with a tidemark was also observed. SEM was compared to light microscopy, finding using both techniques that constituent parts of OA BMLs comprise a number of distinct structures, including areas of chondrogenesis, fibrous tissue with fibrocartilage formation, angiogenesis, amorphous structures within marrow space and new bone formation (Figure 2).

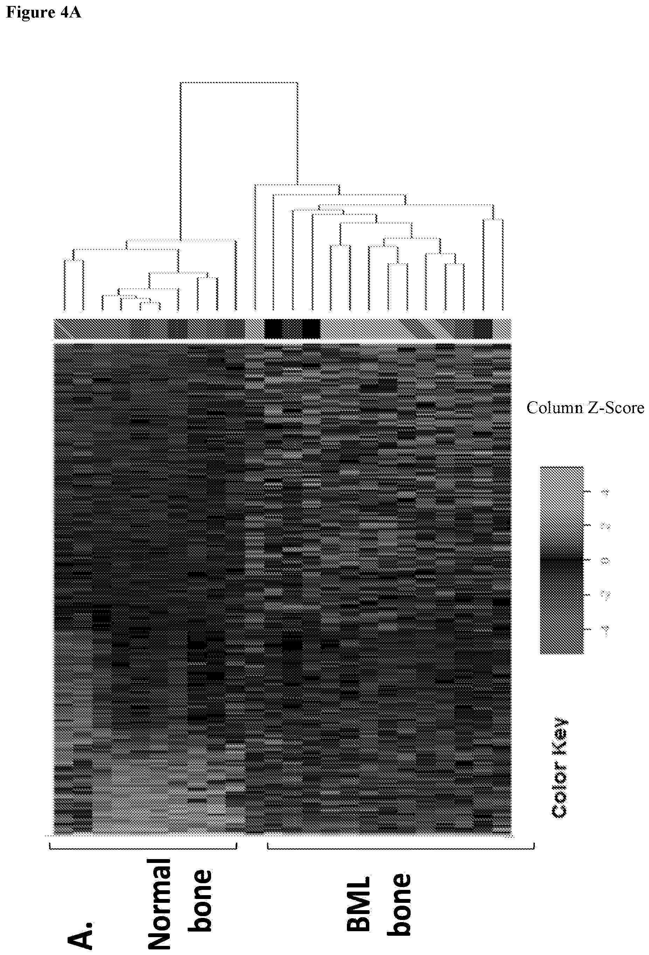

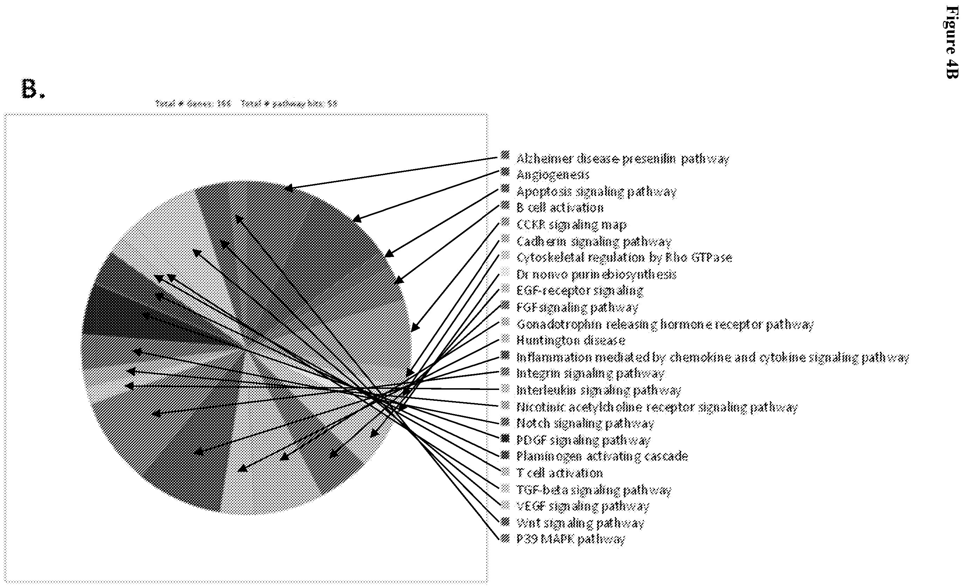

A subset ofn=24 samples from the OA BML and controls showed 218 genes were significantly differentially regulated compared with control samples from a total of 60,000 genes tested on the microarray plate (p<0.05) (Figure 4). The most highly upregulated genes were stathmin 2, ATP-binding cassette protein, thrombospondin 4, matrix metalloproteinase 13 and chromosome 21 open reading frame (see Figure 4). The most down regulated genes included haemoglobin, S100 calcium binding protein A12, hemogen, pro-platelet basic protein [(chemokine C-X-C) motif ligand 7] and delta amino levulinate synthase 2. The full list of genes showing significant differential expression is presented in Figure 3 (Table 1).

Among other significantly upregulated genes were the EGF-like domain which is involved in cell adhesion, apoptosis and calcium binding, collagen type XVI, with functions in ECM organisation, cell adhesion and integrin mediated signalling, G protein coupled receptor that is involved in signal transduction, binds hormones/neurotransmitters and ATPase H+ transporting lysosomal gene which is expressed at the axon terminus and synaptic vesicles and is implicated in neuron projection. Upregulation of the DIRAS family, GTP-binding RAS-like 2 was also found which is a Ras GTPase implicated in neurodegeneration. PC4 and SFRS1 interacting protein 1, molecules that are involved in neuroepithelial stem cell differentiation, neurogenesis and apoptosis, neuronal tyrosine phosphorylated phosphoinositide-3-kinase adaptor 2, a gene involved in neuronal development, interacting with WAVE1 proteins and is implicated in cytoskeletal modelling were detected. Catenin (cadherin-associated protein) upregulation, an adhesive junction associated protein implicated in brain development and cancer formation was also identified. From our PANTHER pathway analysis, 166 of the 218 regulated genes identified were recognised as being involved in 59 pathways, the most prevalent of which were angiogenesis, the Alzheimer disease-presenilin pathway, EGF/FGF/gonadotrophin signaling, inflammation mediated by chemokine and cytokine signaling with PDGF/Notch/VEGF and wnt signaling.

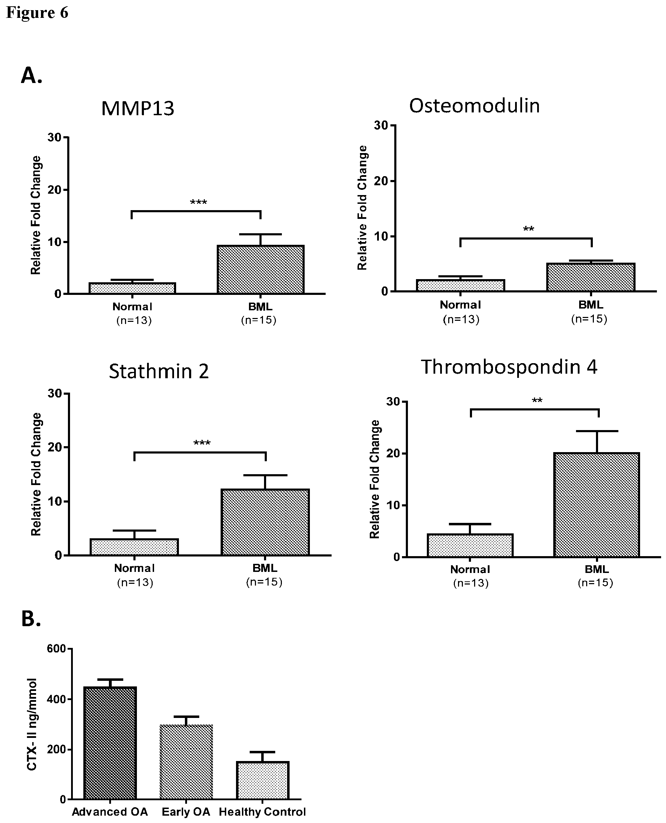

Further validation of the genes observed by microarray was conducted by Q-PCR experiments. For Q-PCR experiments RNA was isolated from 15 additional bone samples from human tissue including BML, non-BML regions in the same subjects (n=13) and analyzed samples by Q-PCR for the following genes: osteomodulin (selected as a bone turnover gene since it is expressed in osteoblasts and osteoclasts), stathmin 2, MMP-13 and thrombospondin 4. The Q-PCR results demonstrated that thrombospondin 4 and stathmin-2 were the most highly upregulated gene in OA BML vs normal bone, reflecting similar results to the microarray experiment (Figure 6). It was also confirmed that there are high levels of MMP-13 gene expression in BML bone. As confirmation of the bone lineage of the tissue, osteomodulin was found in OA bone and normal bone.

Next the functional significance of proteins in the serum and/or urine from genes identified in our microarray was tested. Since MMP-13 was strongly upregulated in OA BML tissue, we tested MMP-13 levels by ELISA in the serum of subjects from the study. No difference in MMP-13 protein levels in late OA, early OA or controls was found. Type II collagen is a substrate for active MMP-13 and C-terminal telopeptide levels of CTX-II generated by MMP cleavage were therefore tested for by ELISA in the urine samples from the study group. A ignificant increase in CTX-II levels i.e. cleavage products of type II collagen in the late OA group compared with early OA and controls was identified (Figure 6B).

### Discussion

BMLs are very well described radiographically by MRI in knee OA, but very little is known about their functional characteristics. This is the first to use a multimodal approach with MRI to locate BMLs, followed by detailed SEM analysis and gene microarray techniques to interrogate BMLs. Bone marrow signal changes were first described on MRI by Wilson et al. who used the term `bone marrow edema' (BME) to describe MRI findings in painful joints (Wilson et al, Radiology 1988; 167(3): 757-60). The first descriptions involved ill-defined signal intensity on T1-weighted images and increased signal intensity on T2-weighted images, but little information has hitherto been reported about BMLs at the pathological and functional level. Studies so far have focused on acquiring data from patients undergoing joint replacement surgery of the knee and hip. Zanetti et al. described that histology of BMLs contained normal fatty marrow with marrow necrosis, necrotic or remodelled trabeculae, oedema and bone marrow bleeding (Zanetti M et al, Radiology. 2000;215:835-840). The same group matched MRI changes to BML abnormalities in a study of participants undergoing total knee replacement (TKR), finding that the BMLs they analysed contained normal tissue in addition to areas of bone marrow fibrosis, edema and bleeding. In a study of hip and knee OA, Hunter et al. (2009) reported increased bone volume fraction but decreased tissue mineral density from light microscopy of BMLs (Hunter et al, Arthritis Res Ther. 2009; 11(1). Samples from the lesion area showed increased trabecular thickness, with granulation, edema, necrosis, fibrinoid deposition and hyperplasia of blood vessel walls. Taljanovic reported one of the largest studies of BML histology to date (Taljanovic et al, Skeletal Radiol. 2008; 37(5): 423-31). In their work, Taljanovic et al. observed regions of fibrosis and microfracture formation at different stages of healing in a study of hip OA by light microscopy. Leydet-Quilici et al. also described oedema, necrosis and fibrosis on BML biopsy (Leydet-Quilici et al, Osteoarthritis Cartilage 2010; 18(11): 1429-35).

In comparison to other published work, the novel SEM techniques described above have allowed improved visualization of BMLs, with direct observation for the first time of cystic structures within BMLs (which could explain the edema observed by other groups by light microscopy), surrounding areas of fibrosis, new cartilage formation within the bone compartment and blood vessel formation, which are findings we validated by light microscopy. SEM techniques allowed additional visualization of new bone formation enabled by 3D visualisation of bone: it was also observed that adipocytes were the main bone-lining cells, with bone excrescences moulded by adipocytes. Comparison of the SEM findings with light microscopy changes supported the observations of fibrosis, new vessel infiltration and inflammatory cells within BML. It is possible that previous studies which have focused mainly on histology of BMLs were not able to identify certain structures that we have observed by SEM, including cysts, cartilage and amorphous infiltration since some structural changes may not have withstood the rigorous decalcification process by which bone is treated for routine histology. The maceration technique used herein of treating bone also revealed the tidemark and new bone formation, which have not been previously described in BMLs.

Using MRI, Roemer et al. showed that, when compared to stable BMLs, subregions without BMLs are associated with a decreased risk of cartilage loss, while progression of BMLs and the development of BMLs is associated with an increased risk of cartilage loss in the same subregion (Roemer et al, Ann Rheum Dis 2009; 68(9): 1461-5). The study described herein found that the most identifiable areas of BML for tissue analysis were at the medial and lateral tibial compartments (see Figure 2). When considering cystic structures, it was found that the tissue analysis supported previous observations that subchondral cysts, which are well-defined rounded areas of fluid-like signal intensity on T2 fat-suppressed sequence MR images were observed in our study (see Figure 2). Carrino et al. (Carrino et al, Osteoarthritis Cartilage 2006; 14(10): 1081-5) reported that 87% of subchondral cysts were associated with BML abnormalities and the tissue analysis described herein supports this strong association. The previous hypotheses that BMLs could be precystic, but that not all BMLs become cystic is also supported by the findings, since by SEM cystic structures were observed within the areas of MRI defined cysts, with additional pathological changes adjacent to cystic areas including fibrocartilage, new vessel formation, chondrogenesis and amorphous tissue deposition in BML tissue. It was also found that towards the periphery of BMLs areas of increased bone turnover with new bone formation were observed.

From the microarray studies of OA bone marrow lesions, the highest regulated gene from our microarray was stathmin 2, which is a phosphoprotein involved in regulating microtubule function, neuronal growth and osteogenesis (Jin et al, FASEB J. 2004; 18(2): 287-99). Stathmin 2 is a regulator of microtubule stability. When it is phosphorylated by MAPK8, it stabilizes microtubules and consequently controls neurite length in cortical neurons. In the developing brain, it negatively regulates the rate of exit from multipolar stage and retards radial migration from the ventricular zone. It is possible that BML tissue-upregulated stathmin 2 is involved in developing new neuronal tissue expression (Liu et al, J Bone Miner Res. 2011; 26(9): 2052-67) and expansion of the BML cavity in OA, thereby causing pain as new blood vessels form within BML tissue and are exposed to pain sensitizers including chemokines; chemokine signalling molecules were identified including adenylate cyclase 5 which was highly upregulated in OA BML tissue but not in normal bone.

The microarray found elevated gene expression of thrombospondin 4, which is a molecule implicated in the inflammatory response to CNS injury, presynaptic hypersensitivity and neuropathic pain states (Kim et al, J Neursci 2012; 32(26): 8977-87). Thrombospondin 4 is an adhesive glycoprotein that mediates cell-to-cell and cell-to-matrix interactions and is involved in various processes including cellular proliferation, migration, adhesion and attachment (Arber S et al, J Cell Biol 1995; 131: 1083-1094). It is also involved in neurite outgrowth (Narouz-Ott L et al, J Biol Chem 2000; 275: 37110-37117), the inflammatory response to CNS injury (Kim et al, J Neursci 2012; 32(26): 8977-87), regulation of vascular inflammation, adaptive responses of the heart to pressure overload and in myocardial function and remodelling. Thrombospondin 4 binds to structural ECM proteins (Adams JC. Annu Rev Cell Dev Biol 2001; 17: 25-51) and has been proposed to contribute to spinal presynaptic spinal hypersensitivity and neuropathic pain states after peripheral nerve injury (Kim et al, J Neursci 2012; 32(26): 8977-87). It may also play a role in regulating protective astrogenesis from the subventricular zone (SVZ) niche after injury in a NUTCH1-dependent manner (Benner et al Nature 2013; 497(7449): 369-73). The spinal NOTCH signalling pathway has recently been implicated in the development of neuropathic pain (Sun et al. Mol Brain 2012; 5: 23). Such observations from other groups and the study described herein suggest that thrombospondin 4 could be a key molecule mediating pain sensitization in OA.

Other upregulated genes that are involved in neuronal morphogenesis from the microarray study included ATPase, H+ transporting lysosomal 38 kDa gene, PC4 and SFRS1 interacting protein 1, neuronal tyrosine-phosphorylated phosphoinositide-3-kinase adaptor 2, FERM and PDZ containing 4, which are implicated in a variety of processes including inflammatory response to CNS injury, presynaptic hypersensitivity and neuropathic pain states (Foulkes et al PLoS Genetics 2008; 4(7) and Swaminathan et al. J Biol Chem. 2016; 291(39): 20303-14). Another group of genes that were strongly upregulated from study included genes involved in extracellular matrix formation and its turnover, including matrix metalloproteinase 13 (MMP-13) and collagens, including type XVI collagen, fibronectins and growth factors which are known to be bound within the ECM (Sofat, International Journal of Experimental Pathology, 2009; 90:463-479). The data suggest that BMLs represent regions of high metabolic activity with increased cell turnover, bone, neuronal, inflammatory and genes. The PANTHER pathway analysis revealed gene pathways involved in chemokine, integrin and cytokine signaling. Evidence of neurodevelopment and pain pathway signaling represented by the Alzheimer's, Notch, catenin and wnt pathways was also observed. There was also evidence of angiogenesis with VEGF and angiogenesis pathway expression. Recently, Kusumbe et al. (Kusumbe et al, Nature 2014; 507(7492): 323-8) described how growth of blood vessels in bone and osteogenesis are coupled, proposing that type H endothelial cells mediate local growth of the vasculature and provide niche signals for perivascular osteoprogenitors (Benner et al Nature 2013; 497(7449): 369-73). The same group reported that endothelial notch activity promotes angiogenesis and osteogenesis in bone (Ramasamy et al, Nature 2014; 507(7492): 376-80). It was also demonstrated that osteomulin expression in BML tissue: Ninomiya et al. showed that osteoclast activity induces osteomodulin expression in bone (Ninomiya et al, Biochemical and Biophysical Research Communications. 2007; 362(2): 460-466), demonstrating that our BML tissue was metabolically active and expressed bone turnover markers. Upregulation of Notch, wnt and catenin signalling pathways was observed in the microarray, in addition to VEGF and angiogenesis. It is therefore proposed that the new vessel formation and bone formation observed by SEM that was validated by gene expression studies are likely to be coupled in BML formation. Since blood vessels are formed within neurovascular bundles, it is likely that the increased nerve pathway gene expression we observed, including stathmin 2, thrombospondin 4, PC4, SFRS1 interacting protein, neuronal tyrosine-phosphorylated phosphoinositide-3-kinase adaptor 2 are all important genes in BML that are expressed during neural pathway development and new nerve formation. The PANTHER pathway analysis showed high representation of signalling pathways involving pro-inflammatory chemokines and cytokine signalling; other groups have also implicated chemokines in OA pain e.g. CCR2 was recently reported to mediate pain in a murine model of OA (Miller et al, Proc Natl Acad Sci USA 2012; 109(50): 20602-7). The data also suggest that chemokine pathway molecules could be pain sensitizers in BMLs.

One of our most highly expressed genes was MMP-13, traditionally thought to be an enzyme expressed in cartilage which is involved in regulating ECM turnover and cartilage destruction in OA (Little et al, Arthritis Rheum 2009; 60(12): 3723-33). The data showed that type II collagen degradation products were increased in urine from the late OA population, suggesting that increased production of MMP-13 by BMLs has significance at the protein level reflected in increased cleavage products of type II collagen, likely to be generated by MMP-13 activity. It is conceivable that new cartilage formation we found by SEM in BMLs, coupled with gene and protein expression of MMP-13, as we observed by microarray and ELISA, could represent reversion to the embryonic phenotype in OA BMLs, with the bone compartment expressing increased MMP-13.

In conclusion, it has been shown for the first time the structure-function relationship of BMLs which are strongly implicated in the pathogenesis of OA. The work has demonstrated that BMLs are regions of high metabolic activity, with expression of genes involved in neuronal development and pain, extracellular matrix turnover and cartilage formation, bone formation and angiogenesis.

### Example 2 - Further measurements of stathmin 2 and thrombospondin 4 as biomarkers in OA

From the microarray studies of OA bone marrow lesions, the highest regulated gene from was stathmin 2, which is a phosphoprotein involved in regulating microtubule function, neuronal growth and osteogenesis. Stathmin 2 is a regulator of microtubule stability. When it is phosphorylated by MAPK8, it stabilizes microtubules and consequently controls neurite length in cortical neurons. In the developing brain, it negatively regulates the rate of exit from multipolar stage and retards radial migration from the ventricular zone. It is possible that BML tissue-upregulated stathmin 2 is involved in developing new neuronal tissue expression (Liu et al, J Bone Miner Res. 2011; 26(9): 2052-67) and expansion of the BML cavity in OA, thereby causing pain as new blood vessels form within BML tissue and are exposed to pain sensitizers including chemokines; we identified chemokine signalling molecules including adenylate cyclase 5 which was highly upregulated in OA BML tissue but not in normal bone. As stathmin 2 is a strong candidate as a key molecule in BML-mediated bone pain, serum from participants in the study was tested using an ELISA assay outlined below.

Quantitative determination of STMN2 in human serum from 17 end-stage OA, 17 early OA and 7 healthy controls was performed using the US Biological STMN2 BioAssay ELISA. Kit for human samples (United States Biological, Salem, MA, USA.) adhering to manufacturer instructions. The ELISA kit employs the competitive enzyme-linked immunoassay technique with a colorimetric detection method measured at 450nm on a Molecular Devices Spectra Max 340 microplate reader (Molecular Devices, Berkshire, UK). The intensity of the absorbance is inversely proportional to the concentration of STMN2 present within the serum sample or standards. A standard curve was plotted relating the absorbance (OD) to the concentration of the standards (ng/ml). The STMN2 concentration in each sample was interpolated from the standard curve and results plotted on GraphPad Prism 7.01 (GraphPad Prism, San Diego, CA, USA).

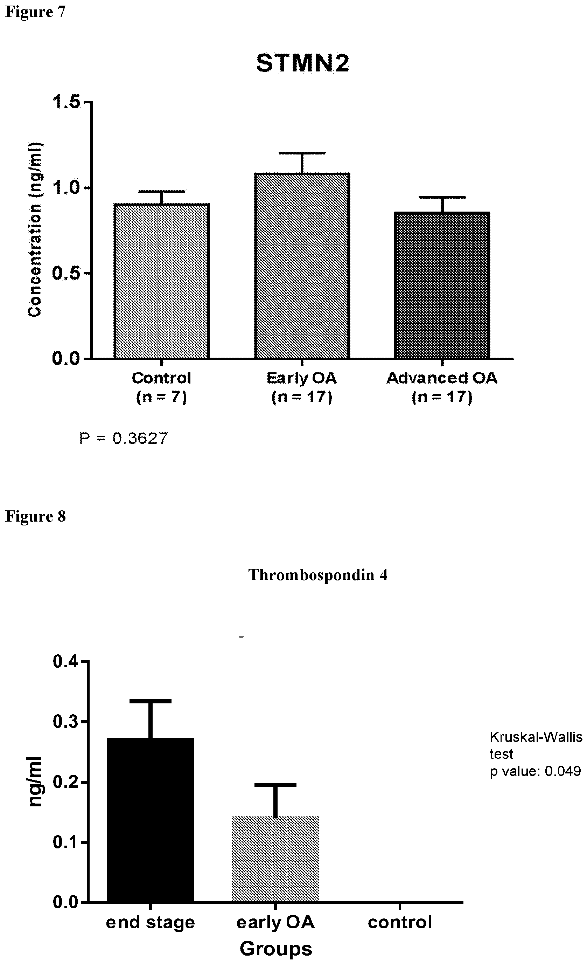

Results are presented in Figure 7. The data show that stathmin 2 was elevated in participants with early OA, but not late OA, suggesting that stathmin 2 is higher in the serum when OA lesions are developing.

The microarray also found elevated gene expression of thrombospondin 4, which is a molecule implicated in the inflammatory response to CNS injury, presynaptic hypersensitivity and neuropathic pain states (Kim et al, J Neursci 2012; 32(26): 8977-87). Thrombospondin 4 is an adhesive glycoprotein that mediates cell-to-cell and cell-to-matrix interactions and is involved in various processes including cellular proliferation, migration, adhesion and attachment (Arber et al, J Cell Biol 1995; 131: 1083-1094). It is also involved in neurite outgrowth (Narouz-Ott et al, J Biol Chem 2000; 275: 37110-37117), the inflammatory response to CNS injury (Kim et al, J Neursci 2012; 32(26): 8977-87), regulation of vascular inflammation, adaptive responses of the heart to pressure overload and in myocardial function and remodelling. Thrombospondin 4 binds to structural ECM proteins (Adams, Annu Rev Cell Dev Biol 2001; 17: 25-51) and has been proposed to contribute to spinal presynaptic spinal hypersensitivity and neuropathic pain states after peripheral nerve injury (Kim et al, J Neursci 2012; 32(26): 8977-87). It may also play a role in regulating protective astrogenesis from the subventricular zone (SVZ) niche after injury in a NOTCH1-dependent manner (Benner et al, Nature 2013; 497(7449): 369-73). The spinal NOTCH signalling pathway has recently been implicated in the development of neuropathic pain (Sun et al, Mol Brain 2012; 5: 23).

A human thrombospondin 4 sandwich enzyme immunoassay was used for in vitro quantitative measurement of thrombospondin 4 in serum from the subject samples (DL Sci & Tech Development Co Ltd). The ELISA kit employs an immunoassay technique with a colorimetric detection method measured at 450nm on a Molecular Devices Spectra Max 340 microplate reader (Molecular Devices, Berkshire, UK). A standard curve was plotted relating the absorbance (OD) to the concentration of the standards (ng/ml). The THBS4 concentration in each sample was interpolated from the standard curve and results plotted on GraphPad Prism 7.01 (GraphPad Prism, San Diego, CA, USA).

Results are presented in Figure 8. Higher levels of thrombospondin 4 were observed in end stage OA patients compared with the early OA patients. Thrombospondin 4 was not detected for the controls.

### Example 3 - Additional measurement of thrombospondin 4 levels

Methods: Samples were evaluated from participants in the Pain Perception in Osteoarthritis study of participants with knee OA. Serum and urine samples for biomarker studies were assessed. Three groups were evaluated: advanced OA, undergoing joint replacement surgery, mild OA, receiving usual care for knee OA including non-steroidal anti-inflammatory agents and/or physical therapies and normal healthy volunteers. Thrombospondin 4 (TSP4) ELISA: TSP4 levels were measured in serum collected at the first visit using the human thrombospondin 4 ELISA kit (DLDEVELOP). Absorbance of each well was read at 450 nm. CTX-II ELISA: for the detection of MMP-13 activity, we measured the levels of C-terminal telopeptides of type II collagen cleavage products using the Urine Cartilaps (CTX-II) EIA (Immunodiagnostic Systems). Urine samples collected at the first study visit were sampled for type II collagen cleavage products. Matched urine samples were tested for creatinine for normalisation. Absorbance of each well was read at 450 nm. For all ELISA assays, significance across all the groups was compared using Kruskal-Wallis comparisons, with significance considered at p < 0.05. Analysis of variance - ANOVA was also performed for group comparisons to assess the influence of structural damage measured by the MRI knee osteoarthritis score (MOAKS) with adjustment for age, BMI and gender as confounders in relation to the biomarkers tested.

Results: Demographics. A total of 110 participants were evaluated with advanced OA (n=72), mild OA (n=32) and healthy volunteers (n=6). (See table below)

For TSP4 serum protein measurements, it was found that TSP4 levels were undetectable in normal healthy controls. TSP4 levels were raised significantly in the advanced OA group [Mean (SD) (0.228 (0.27)] compared with mild OA groups [Mean (SD) 0.095 (0.148)] and healthy controls (p=0.0046). A significant correlation was also deteced between cartilage degradation and TSP4 levels (p=0.014) when adjusting for age, BMI and gender as confounders. Urinary CTX-II levels were significantly increased in the advanced OA group [Mean (SD) 447 (269)], compared with the mild OA [Mean (SD) 320 (174)] and healthy control [Mean (SD) 149.8 (81.0)] groups (p=0.0034). T here was a trend for increasing breakdown products of type II collagen with worsening OA disease severity. There were significant correlations between CTX-II degradation products and cartilage damage (p=0.047) and Hoffa synovitis (p=0.001) with adjustment of age, BMI and gender as confounders.

Conclusions: The highest elevations of TSP4 and urinary CTX-II were observed in the advanced OA group. The de novo cartilage formation that observed within BMLs from the microarray study, coupled with the increased transcriptomic expression of MMP-13 observed and the detection of MMP-13 cleavage products in this biomarker study, suggest recapitulation of the embryonic bone development phenotype within OA with detection of MMP-13 cleavage products in the urine potentially from MMP-13 protease activity arising from BMLs and cartilage. It was also found that TSP4 was elevated in subjects with advanced OA at the most significant level. TSP4 has been implicated in the inflammatory response to central nervous system injury, presynaptic hypersensitivity and neuropathic pain states. The findings raise the possibility of serum TSP4 and urine type II collagen degradation products in combination as pain, BML- and cartilage-specific OA biomarkers.

**Demographics table:**

| Parameter | Advanced OA | Mild OA | Healthy control |
|---|---|---|---|
| Age mean (SD) | 69.1 (7.7) | 62.3 (8.3) | 45 (5.5) |
| BMI mean (SD) | 32.5 (5.7) | 29.4 (4.4) | 23.3 (3.6) |
| Gender M:F | 17:55 | 7:25 | 0:6 |

### Example 4 - Relation of radiographic severity of knee osteoarthritis to clinical pain scores

The aim was to gain a deeper understanding of OA pathophysiology by assessing how structural changes including bone marrow lesions (BMLs), cartilage loss, synovitis or effusion relate to clinical pain measures in people with knee OA.

Methods: A prospective, observational study was conducted in people with advanced and mild OA who were attending rheumatology and orthopaedic clinics at a London teaching hospital. Participants were assessed for their level of pain using the Western Ontario and MacMaster Universities Osteoarthritis Index (WOMAC) pain, stiffness and function scores, the Visual Analogue Scale (VAS) for pain, sensitisation scores using painDETECT and the Hospital Anxiety and Depression Scale (HADS). In addition, each subject underwent magnetic resonance imaging (MRI) of their target painful knee using standard T1-weighted and fat-suppressed T2-weighted images with a 3T scanner. Two Consultant Radiologists independently scored the level of cartilage degradation, BMLs, synovitis and effusion severity using the semi-quantitative MRI knee osteoarthritis score (MOAKS). Radiographic severity was compared for structural changes in the two groups: mild OA (receiving standard medical management for knee OA) and advanced OA (undergoing joint replacement surgery). A correlation analyses of radiographic changes was also conducted with individual pain scores using the analysis of covariance (ANCOVA).

Results: n=88 participants were evaluated who had completed pain and function questionnaires and undergone MRI evaluation of their target painful knee. The group comprised of participants with mild OA (n=22) and advanced OA (n=66). The mean age in the mild OA group was 61.8 (8.2) and the advanced OA group was 68.8 (7.8). The mean BMI in the mild OA group was 28.6 (4.1) and the advanced group was 32.3 (5.7). A summary of the MOAKS grading, WOMAC pain and VAS pain are summarized (see Table). A significant correlation was obsereved between MOAKS identified cartilage loss and WOMAC pain (p=0.009), WOMAC stiffness (p=0.004), WOMAC function (p=0.004) and VAS for pain (p=0.047) when adjusting for BMI and age as confounders. A correlation was also observed between MOAKS-identified BMLs and VAS pain (p=0.029) - however, this correlation was not significant after adjusting for BMI and age as confounders (p=0.057). A significant correlation was also observed between VAS pain and WOMAC pain and HADS scores (p<0.05). No significant correlations were observed between synovitis/effusion and pain scores (data not shown).

Conclusions: The study found that the strongest correlation was between the outcome scores for WOMAC pain, stiffness, function and VAS pain with cartilage degradation. No significant correlation was observed between painDETECT and MOAKS-assessed structural damage, suggesting that painDETECT is a marker of pain sensitization rather than nociceptive pain reflected by structural damage. The data also demonstrate that participants with knee OA who report the highest pain levels are also more likely to experience significant anxiety and/or depression. The data show that in a cohort of knee OA subjects with a range of structural damage from mild OA to advanced OA, the strongest structural correlates with clinical pain are cartilage degradation.

| Summary of MOAKS damage and clinical pain scores | | | |
|---|---|---|---|
| MOAKS parameter | Frequency N(%) | WOMAC Pain Mean (SD) | VAS Pain Mean (SD) |
| Bone Marrow Lesion Severity Score | | | |
| MOAKS=0 | MOAKS=0 24 (27.3) | 46.1 (25.9) | 4.1 (2.8) |
| MOAKS=1 | 61 (69.3) | 58.3 (22.2) | 5.7 (2.2) |
| MOAKS=2 | 3 (3.4) | 40.1 (21.6) | 4.7 (3.3) |
| MOAKS=3 | - | - | - |

| Cartilage Damage Severity Score | | | |
|---|---|---|---|
| MOAKS=0 | 8 (9.0) | 28.6 (24.7) | 2.6 (2.6) |
| MOAKS=1 | 27 (30.7) | 46.6 (19.7) | 4.5 (2.1) |
| MOAKS=2 | 46 (52.3) | 63.2 (22.2) | 6.1 (22) |
| MOAKS=3 | ::7 (8.0) | 55.8 (18.6) | 5.6 (3.3) |

### Sequence listing

**SEQ ID NO: 1 - Human stathmin 2 isoform 1 (Taken from Uniprot ID Q93045 as at the filing date)** 
MAKTAMAYKE KMKELSMLSL ICSCFYPEPR NINIYTYDDM EVKQINKRAS
GQAFELILKP PSPISEAPRT LASPKKKDLS LEEIQKKLEA AEERRKSQEA
QVLKQLAEKR EHEREVLQKA LEENNNFSKM AEEKLILKME QIKENREANL
AAIIERLQEK ERHAAEVRRN KELQVELSG
**SEQ ID NO: 2 - Human stathmin 2 isoform 2 (Taken from Uniprot ID Q93045 as at the filing date)** 
MAKTAMAYKE KMKELSMLSL ICSCFYPEPR NINIYTYDDM EVKQINKRAS
GQAFELILKP PSPISEAPRT LASPKKKDLS LEEIQKKLEA AEERRKSQEA
QVLKQLAEKR EHEREVLQKA LEENNNFSKM AEEKLILKME QIKENREANL
AAIIERLQEK LVKFISSELK ESIESQFLEL QREGEKQ
**SEQ ID NO: 3 - Human stathmin 2 mRNA (isoform 1) (Taken from accession number NM_007029 as at the filing date)** 
agctcctagg aagcttcagg gcttaaagct ccactctact tggactgtac
tatcaggccc ccaaaatggg gggagccgac agggaaggac tgatttccat
ttcaaactgc attctggtac tttgtactcc agcaccattg gccgatcaat
atttaatgct tggagattct gactctgcgg gagtcatgtc aggggacctt
gggagccaat ctgcttgagc ttctgagtga taattattca tgggctcctg
cctcttgctc tttctctagc acggtcccac tctgcagact cagtgcctta
ttcagtcttc tctctcgctc tctccgctgc tgtagccgga ccctttgcct
tcgccactgc tcagcgtctg cacatcccta caatggctaa aacagcaatg
gcctacaagg aaaaaatgaa ggagctgtcc atgctgtcac tgatctgctc
ttgcttttac ccggaacctc gcaacatcaa catctatact tacgatgata
tggaagtgaa gcaaatcaac aaacgtgcct ctggccaggc ttttgagctg
atcttgaagc caccatctcc tatctcagaa gccccacgaa ctttagcttc
tccaaagaag aaagacctgt ccctggagga gatccagaag aaactggagg
 
ctgcagagga aagaagaaag tctcaggagg cccaggtgct gaaacaattg
gcagagaaga gggaacacga gcgagaagtc cttcagaagg ctttggagga
gaacaacaac ttcagcaaga tggcggagga aaagctgatc ctgaaaatgg
aacaaattaa ggaaaaccgt gaggctaatc tagctgctat tattgaacgt
ctgcaggaaa aggagaggca tgctgcggag gtgcgcagga acaaggaact
ccaggttgaa ctgtctggct gaagcaaggg agggtctggc acgccccacc
aatagtaaat ccccctgcct atattataat ggatcatgcg atatcaggat
ggggaatgta tgacatggtt taaaaagaac tcattataaa aaaaaaaaaa
caaaaaaaat caaaaattaa aaaaaatcaa tgcggtctct ttgcagaatg
ttttgcttga tgtttaaaaa ataccttgga tcttattttg taaatactta
catttttgtt aaaaaataca agtattgcat tatgcaagtt atttcataat
cttacatgtc ctgtaacagg cttttgatgt tgtgtctttc cactcaaatg
aatttgctag gtctgttctt tttgaagctc cccatgtcta actccattcc
aaaagaaaaa tgaggtcagt agacagtcta tggtgctaga aacccaccat
tgcctaatga cctagaaggc tttgttgtct ctgagcttga ctaagaccat
acctagatca caggtattat gactccacat gaaccttcac atttgttcgc
tcataatcta cttactgcct aaaaactaca aaaccaggct aagaaatacc
accagtcata gcatttactt ctgcttctcc tggattatgt gctacaaatg
tgctttggct ttagaaaggg atggatgaga agacagacct gagaccaatc
tgggtagaag caaaaagttg aaccttttaa agtgctgaac acaaatccaa
attcgaatgg ttcaagcagc cgtgaaatcg ctcttcataa agtgggctta
attctctagt ttaagttctt ttgatggaat gaattaatta atgtgtcagg
tggcttattt gtggatgcca tgattgatga tgttcatttt aagctcttac
ctatagtaca agtacatgat gctactgaat atttttccac ttggaaactg
tgagctggtt gttgcattaa aacacacata caaacaaaat caaaaacact
gcggactttc actcaagctg gtctttcttc cccagtgtaa ggcaatcctg
cctactaaca acaccaacaa caaaacactc catctgtgaa gctgacgcag
ttaagggggc taggcagggc atttgtgcca actaagaatc accagatacc
caccataagt acctatcgca gttttgaagt cgtttctccc caactcccaa
ctcctgaagg ttgctgcctg catatttact cttcattagt gctattttcc
tgtatgtcat tgtgagcaag ctgtgattaa taaagaattg gagttctgtg
aactaataaa ggtttggtct gttaaaaaaa aa
**SEQ ID NO: 4 - Human stathmin 2 mRNA (isoform 1) (Taken from accession number NM_001199214 as at the filing date)** 
agctcctagg aagcttcagg gcttaaagct ccactctact tggactgtac
tatcaggccc ccaaaatggg gggagccgac agggaaggac tgatttccat
ttcaaactgc attctggtac tttgtactcc agcaccattg gccgatcaat
atttaatgct tggagattct gactctgcgg gagtcatgtc aggggacctt
gggagccaat ctgcttgagc ttctgagtga taattattca tgggctcctg
cctcttgctc tttctctagc acggtcccac tctgcagact cagtgcctta
ttcagtcttc tctctcgctc tctccgctgc tgtagccgga ccctttgcct
tcgccactgc tcagcgtctg cacatcccta caatggctaa aacagcaatg
gcctacaagg aaaaaatgaa ggagctgtcc atgctgtcac tgatctgctc
ttgcttttac ccggaacctc gcaacatcaa catctatact tacgatgata
tggaagtgaa gcaaatcaac aaacgtgcct ctggccaggc ttttgagctg
atcttgaagc caccatctcc tatctcagaa gccccacgaa ctttagcttc
tccaaagaag aaagacctgt ccctggagga gatccagaag aaactggagg
ctgcagagga aagaagaaag tctcaggagg cccaggtgct gaaacaattg
gcagagaaga gggaacacga gcgagaagtc cttcagaagg ctttggagga
gaacaacaac ttcagcaaga tggcggagga aaagctgatc ctgaaaatgg
aacaaattaa ggaaaaccgt gaggctaatc tagctgctat tattgaacgt
ctgcaggaaa agctggtcaa gtttatttct tctgaactaa aagaatctat
agagtctcaa tttctggagc ttcagaggga aggagagaag caatgagagg
catgctgcgg aggtgcgcag gaacaaggaa ctccaggttg aactgtctgg
ctgaagcaag ggagggtctg gcacgcccca ccaatagtaa atccccctgc
ctatattata atggatcatg cgatatcagg atggggaatg tatgacatgg
tttaaaaaga actcattata aaaaaaaaaa aacaaaaaaa atcaaaaatt
aaaaaaaatc aatgcggtct ctttgcagaa tgttttgctt gatgtttaaa
aaataccttg gatcttattt tgtaaatact tacatttttg ttaaaaaata
caagtattgc attatgcaag ttatttcata atcttacatg tcctgtaaca
ggcttttgat gttgtgtctt tccactcaaa tgaatttgct aggtctgttc
tttttgaagc tccccatgtc taactccatt ccaaaagaaa aatgaggtca
gtagacagtc tatggtgcta gaaacccacc attgcctaat gacctagaag
gctttgttgt ctctgagctt gactaagacc atacctagat cacaggtatt
 
atgactccac atgaaccttc acatttgttc gctcataatc tacttactgc
ctaaaaacta caaaaccagg ctaagaaata ccaccagtca tagcatttac
ttctgcttct cctggattat gtgctacaaa tgtgctttgg ctttagaaag
ggatggatga gaagacagac ctgagaccaa tctgggtaga agcaaaaagt
tgaacctttt aaagtgctga acacaaatcc aaattcgaat ggttcaagca
gccgtgaaat cgctcttcat aaagtgggct taattctcta gtttaagttc
ttttgatgga atgaattaat taatgtgtca ggtggcttat ttgtggatgc
catgattgat gatgttcatt ttaagctctt acctatagta caagtacatg
atgctactga atatttttcc acttggaaac tgtgagctgg ttgttgcatt
aaaacacaca tacaaacaaa atcaaaaaca ctgcggactt tcactcaagc
tggtctttct tccccagtgt aaggcaatcc tgcctactaa caacaccaac
aacaaaacac tccatctgtg aagctgacgc agttaagggg gctaggcagg
gcatttgtgc caactaagaa tcaccagata cccaccataa gtacctatcg
cagttttgaa gtcgtttctc cccaactccc aactcctgaa ggttgctgcc
tgcatattta ctcttcatta gtgctatttt cctgtatgtc attgtgagca
agctgtgatt aataaagaat tggagttctg tgaactaata aaggtttggt
ctgttaaaaa aaaa
**SEQ ID NO: 5 - Stathmin 2 cDNA (Taken from accession number CR456833 as at the filing date)** 
ATGGCTAAAACAGCAATGGCCTACAAGGAAAAAATGAAGGAGCTGTCCATGCTGTCACTG
ATCTGCTCTTGCTTTTACCCGGAACCTCGCAACATCAACATCTATACTTACGATGATATG
GAAGTGAAGCAAATCAACAAACGTGCCTCTGGCCAGGCTTTTGAGCTGATCTTGAAGCCA
CCATCTCCTATCTCAGAAGCCCCACGAACTTTAGCTTCTCCAAAGAAGAAAGACCTGTCC
CTGGAGGAGATCCAGAAGAAACTGGAGGCTGCAGAGGAAAGAAGAAAGTCTCAGGAGGCC
CAGGTGCTGAAACAATTGGCAGAGAAGAGGGAACACGAGCGAGAAGTCCTTCAGAAGGCT
TTGGAGGAGAACAACAACTTCAGCAAGATGGCGGAGGAAAAGCTGATCCTGAAAATGGAA
CAAATTAAGGAAAACCGTGAGGCTAATCTAGCTGCTATTATTGAACGTCTGCAGGAAAAG
GAGAGGCATGCTGCGGAGGTGCGCAGGAACAAGGAACTCCAGGTTGAACTGTCTGGTTAA
**SEQ ID NO 6 - Human thrombospondin 4 complete sequence (Taken from UniProt ID P35443 as at the filing date)** 
MLAPRGAAVL LLHLVLQRWL AAGAQATPQV FDLLPSSSQR LNPGALLPVL
TDPALNDLYV ISTFKLQTKS SATIFGLYSS TDNSKYFEFT VMGRLNKAIL
RYLKNDGKVH LVVFNNLQLA DGRRHRILLR LSNLQRGAGS LELYLDCIQV
DSVHNLPRAF AGPSQKPETI ELRTFQRKPQ DFLEELKLVV RGSLFQVASL
QDCFLQQSEP LAATGTGDFN RQFLGQMTQL NQLLGEVKDL LRQQVKETSF
LRNTIAECQA CGPLKFQSPT PSTVVPPAPP APPTRPPRRC DSNPCFRGVQ
CTDSRDGFQC GPCPEGYTGN GITCIDVDEC KYHPCYPGVH CINLSPGFRC
DACPVGFTGP MVQGVGISFA KSNKQVCTDI DECRNGACVP NSICVNTLGS
YRCGPCKPGY TGDQIRGCKA ERNCRNPELN PCSVNAQCIE ERQGDVTCVC
GVGWAGDGYI CGKDVDIDSY PDEELPCSAR NCKKDNCKYV PNSGQEDADR
DGIGDACDED ADGDGILNEQ DNCVLIHNVD QRNSDKDIFG DACDNCLSVL
NNDQKDTDGD GRGDACDDDM DGDGIKNILD NCPKFPNRDQ RDKDGDGVGD
ACDSCPDVSN PNQSDVDNDL VGDSCDTNQD SDGDGHQDST DNCPTVINSA
QLDTDKDGIG DECDDDDDND GIPDLVPPGP DNCRLVPNPA QEDSNSDGVG
DICESDFDQD QVIDRIDVCP ENAEVTLTDF RAYQTVVLDP EGDAQIDPNW
VVLNQGMEIV QTMNSDPGLA VGYTAFNGVD FEGTFHVNTQ TDDDYAGFIF
GYQDSSSFYV VMWKQTEQTY WQATPFRAVA EPGIQLKAVK SKTGPGEHLR
NSLWHTGDTS DQVRLLWKDS RNVGWKDKVS YRWFLQHRPQ VGYIRVRFYE
GSELVADSGV TIDTTMRGGR LGVFCFSQEN IIWSNLKYRC NDTIPEDFQE
FQTQNFDRFD N
**SEQ ID NO 7 - Human thrombospondin 4 mature form (without signal peptide) (Taken from UniProt ID P35443 as at the filing date)** 
TPQV FDLLPSSSQR LNPGALLPVL
TDPALNDLYV ISTFKLQTKS SATIFGLYSS TDNSKYFEFT VMGRLNKAIL
RYLKNDGKVH LVVFNNLQLA DGRRHRILLR LSNLQRGAGS LELYLDCIQV
DSVHNLPRAF AGPSQKPETI ELRTFQRKPQ DFLEELKLVV RGSLFQVASL
QDCFLQQSEP LAATGTGDFN RQFLGQMTQL NQLLGEVKDL LRQQVKETSF
LRNTIAECQA CGPLKFQSPT PSTVVPPAPP APPTRPPRRC DSNPCFRGVQ
CTDSRDGFQC GPCPEGYTGN GITCIDVDEC KYHPCYPGVH CINLSPGFRC
DACPVGFTGP MVQGVGISFA KSNKQVCTDI DECRNGACVP NSICVNTLGS
YRCGPCKPGY TGDQIRGCKA ERNCRNPELN PCSVNAQCIE ERQGDVTCVC
 
GVGWAGDGYI CGKDVDIDSY PDEELPCSAR NCKKDNCKYV PNSGQEDADR
DGIGDACDED ADGDGILNEQ DNCVLIHNVD QRNSDKDIFG DACDNCLSVL
NNDQKDTDGD GRGDACDDDM DGDGIKNILD NCPKFPNRDQ RDKDGDGVGD
ACDSCPDVSN PNQSDVDNDL VGDSCDTNQD SDGDGHQDST DNCPTVINSA
QLDTDKDGIG DECDDDDDND GIPDLVPPGP DNCRLVPNPA QEDSNSDGVG
DICESDFDQD QVIDRIDVCP ENAEVTLTDF RAYQTVVLDP EGDAQIDPNW
VVLNQGMEIV QTMNSDPGLA VGYTAFNGVD FEGTFHVNTQ TDDDYAGFIF
GYQDSSSFYV VMWKQTEQTY WQATPFRAVA EPGIQLKAVK SKTGPGEHLR
NSLWHTGDTS DQVRLLWKDS RNVGWKDKVS YRWFLQHRPQ VGYIRVRFYE
GSELVADSGV TIDTTMRGGR LGVFCFSQEN IIWSNLKYRC NDTIPEDFQE
FQTQNFDRFD N
**SEQ ID NO: 8 - Human thrombospondin 4 mRNA (complete sequence, transcript variant 1) (Taken from accession number NM_003248 as at the filing date)** 
tcccccgccc ggccgcacca taaagcgccc ggccgctgcc gcggagccca
gcagccagct ccccagcacc gcacggcggg gacgcgagcg cgcccccgac
ggcagcccgg acgccgagca cgggtcacct gcggcgccgg cccgggcgcc
gaccgaggtt caacgcacgg cccggggacc cccaggcggg gccaacgccg
ccgtcgcccc cggcctcgcg gggagcagga agagccaaca tgctggcccc
gcgcggagcc gccgtcctcc tgctgcacct ggtcctgcag cggtggctag
cggcaggcgc ccaggccacc ccccaggtct ttgaccttct cccatcttcc
agtcagaggc taaacccagg cgctctgctg ccagtcctga cagaccccgc
cctgaatgat ctctatgtga tttccacctt caagctgcag actaaaagtt
cagccaccat cttcggtctt tactcttcaa ctgacaacag taaatatttt
gaatttactg tgatgggacg cttaaacaaa gccatcctcc gttacctgaa
gaacgatggg aaggtgcatt tggtggtttt caacaacctg cagctggcag
acggaaggcg gcacaggatc ctcctgaggc tgagcaattt gcagcgaggg
gccggctccc tagagctcta cctggactgc atccaggtgg attccgttca
caatctcccc agggcctttg ctggcccctc ccagaaacct gagaccattg
aattgaggac tttccagagg aagccacagg acttcttgga agagctgaag
ctggtggtga gaggctcact gttccaggtg gccagcctgc aagactgctt
cctgcagcag agtgagccac tggctgccac aggcacaggg gactttaacc
 
ggcagttctt gggtcaaatg acacaattaa accaactcct gggagaggtg
aaggaccttc tgagacagca ggttaaggaa acatcatttt tgcgaaacac
catagctgaa tgccaggctt gcggtcctct caagtttcag tctccgaccc
caagcacggt ggtgcccccg gctccccctg caccgccaac acgcccacct
cgtcggtgtg actccaaccc atgtttccga ggtgtccaat gtaccgacag
tagagatggc ttccagtgtg ggccctgccc cgagggctac acaggaaacg
ggatcacctg tattgatgtt gatgagtgca aataccatcc ctgctacccg
ggcgtgcact gcataaattt gtctcctggc ttcagatgtg acgcctgccc
agtgggcttc acagggccca tggtgcaggg tgttgggatc agttttgcca
agtcaaacaa gcaggtctgc actgacattg atgagtgtcg aaatggagcg
tgcgttccca actcgatctg cgttaatact ttgggatctt accgctgtgg
gccttgtaag ccggggtata ctggtgatca gataagggga tgcaaagcgg
aaagaaactg cagaaaccca gagctgaacc cttgcagtgt gaatgcccag
tgcattgaag agaggcaggg ggatgtgaca tgtgtgtgtg gagtcggttg
ggctggagat ggctatatct gtggaaagga tgtggacatc gacagttacc
ccgacgaaga actgccatgc tctgccagga actgtaaaaa ggacaactgc
aaatatgtgc caaattctgg ccaagaagat gcagacagag atggcattgg
cgacgcttgt gacgaggatg ctgacggaga tgggatcctg aatgagcagg
ataactgtgt cctgattcat aatgtggacc aaaggaacag cgataaagat
atctttgggg atgcctgtga taactgcctg agtgtcttaa ataacgacca
gaaagacacc gatggggatg gaagaggaga tgcctgtgat gatgacatgg
atggagatgg aataaaaaac attctggaca actgcccaaa atttcccaat
cgtgaccaac gggacaagga tggtgatggt gtgggggatg cctgtgacag
ttgtcctgat gtcagcaacc ctaaccagtc tgatgtggat aatgatctgg
ttggggactc ctgtgacacc aatcaggaca gtgatggaga tgggcaccag
gacagcacag acaactgccc caccgtcatt aacagtgccc agctggacac
cgataaggat ggaattggtg acgagtgtga tgatgatgat gacaatgatg
gtatcccaga cctggtgccc cctggaccag acaactgccg gctggtcccc
aacccagccc aggaggatag caacagcgac ggagtgggag acatctgtga
gtctgacttt gaccaggacc aggtcatcga tcggatcgac gtctgcccag
agaacgcaga ggtcaccctg accgacttca gggcttacca gaccgtggtc
ctggatcctg aaggggatgc ccagatcgat cccaactggg tggtcctgaa
ccagggcatg gagattgtac agaccatgaa cagtgatcct ggcctggcag
 
tggggtacac agcttttaat ggagttgact tcgaagggac cttccatgtg
aatacccaga cagatgatga ctatgcaggc tttatctttg gctaccaaga
tagctccagc ttctacgtgg tcatgtggaa gcagacggag cagacatatt
ggcaagccac cccattccga gcagttgcag aacctggcat tcagctcaag
gctgtgaagt ctaagacagg tccaggggag catctccgga actccctgtg
gcacacgggg gacaccagtg accaggtcag gctgctgtgg aaggactcca
ggaatgtggg ctggaaggac aaggtgtcct accgctggtt cctacagcac
aggccccagg tgggctacat cagggtacga ttttatgaag gctctgagtt
ggtggctgac tctggcgtca ccatagacac cacaatgcgt ggaggccgac
ttggcgtttt ctgcttctct caagaaaaca tcatctggtc caacctcaag
tatcgctgca atgacaccat ccctgaggac ttccaagagt ttcaaaccca
gaatttcgac cgcttcgata attaaaccaa ggaagcaatc tgtaactgct
tttcggaaca ctaaaaccat atatatttta acttcaattt tctttagctt
ttaccaaccc aaatatatca aaacgtttta tgtgaatgtg gcaataaagg
agaagagatc atttttaaaa aaaaaaaaaa a
**SEQ ID NO: 9 - Human thrombospondin 4 mRNA (transcript variant 2) (Taken from accession number NM_001306212 as at the filing date)** 
gtgaaacgcg ggggcgtggc taagggaggg aagggtaatt tgggggctct
tgagagatga gagaaacaac gactggaggg attaagaaga actcttagga
gcagcttggc ctaaaggctg tgtgggacgt gcctgctttg ccttcgcctt
ccaccatgat ctctttgacc ttctcccatc ttccagtcag aggctaaacc
caggcgctct gctgccagtc ctgacagacc ccgccctgaa tgatctctat
gtgatttcca ccttcaagct gcagactaaa agttcagcca ccatcttcgg
tctttactct tcaactgaca acagtaaata ttttgaattt actgtgatgg
gacgcttaaa caaagccatc ctccgttacc tgaagaacga tgggaaggtg
catttggtgg ttttcaacaa cctgcagctg gcagacggaa ggcggcacag
gatcctcctg aggctgagca atttgcagcg aggggccggc tccctagagc
tctacctgga ctgcatccag gtggattccg ttcacaatct ccccagggcc
tttgctggcc cctcccagaa acctgagacc attgaattga ggactttcca
gaggaagcca caggacttct tggaagagct gaagctggtg gtgagaggct
cactgttcca ggtggccagc ctgcaagact gcttcctgca gcagagtgag
ccactggctg ccacaggcac aggggacttt aaccggcagt tcttgggtca
aatgacacaa ttaaaccaac tcctgggaga ggtgaaggac cttctgagac
agcaggttaa ggaaacatca tttttgcgaa acaccatagc tgaatgccag
gcttgcggtc ctctcaagtt tcagtctccg accccaagca cggtggtgcc
cccggctccc cctgcaccgc caacacgccc acctcgtcgg tgtgactcca
 
acccatgttt ccgaggtgtc caatgtaccg acagtagaga tggcttccag
tgtgggccct gccccgaggg ctacacagga aacgggatca cctgtattga
tgttgatgag tgcaaatacc atccctgcta cccgggcgtg cactgcataa
atttgtctcc tggcttcaga tgtgacgcct gcccagtggg cttcacaggg
cccatggtgc agggtgttgg gatcagtttt gccaagtcaa acaagcaggt
ctgcactgac attgatgagt gtcgaaatgg agcgtgcgtt cccaactcga
tctgcgttaa tactttggga tcttaccgct gtgggccttg taagccgggg
tatactggtg atcagataag gggatgcaaa gcggaaagaa actgcagaaa
cccagagctg aacccttgca gtgtgaatgc ccagtgcatt gaagagaggc
agggggatgt gacatgtgtg tgtggagtcg gttgggctgg agatggctat
atctgtggaa aggatgtgga catcgacagt taccccgacg aagaactgcc
atgctctgcc aggaactgta aaaaggacaa ctgcaaatat gtgccaaatt
ctggccaaga agatgcagac agagatggca ttggcgacgc ttgtgacgag
gatgctgacg gagatgggat cctgaatgag caggataact gtgtcctgat
tcataatgtg gaccaaagga acagcgataa agatatcttt ggggatgcct
gtgataactg cctgagtgtc ttaaataacg accagaaaga caccgatggg
gatggaagag gagatgcctg tgatgatgac atggatggag atggaataaa
aaacattctg gacaactgcc caaaatttcc caatcgtgac caacgggaca
aggatggtga tggtgtgggg gatgcctgtg acagttgtcc tgatgtcagc
aaccctaacc agtctgatgt ggataatgat ctggttgggg actcctgtga
caccaatcag gacagtgatg gagatgggca ccaggacagc acagacaact
gccccaccgt cattaacagt gcccagctgg acaccgataa ggatggaatt
ggtgacgagt gtgatgatga tgatgacaat gatggtatcc cagacctggt
gccccctgga ccagacaact gccggctggt ccccaaccca gcccaggagg
atagcaacag cgacggagtg ggagacatct gtgagtctga ctttgaccag
gaccaggtca tcgatcggat cgacgtctgc ccagagaacg cagaggtcac
cctgaccgac ttcagggctt accagaccgt ggtcctggat cctgaagggg
atgcccagat cgatcccaac tgggtggtcc tgaaccaggg catggagatt
gtacagacca tgaacagtga tcctggcctg gcagtggggt acacagcttt
taatggagtt gacttcgaag ggaccttcca tgtgaatacc cagacagatg
atgactatgc aggctttatc tttggctacc aagatagctc cagcttctac
gtggtcatgt ggaagcagac ggagcagaca tattggcaag ccaccccatt
ccgagcagtt gcagaacctg gcattcagct caaggctgtg aagtctaaga
caggtccagg ggagcatctc cggaactccc tgtggcacac gggggacacc
agtgaccagg tcaggctgct gtggaaggac tccaggaatg tgggctggaa
ggacaaggtg tcctaccgct ggttcctaca gcacaggccc caggtgggct
acatcagggt acgattttat gaaggctctg agttggtggc tgactctggc
gtcaccatag acaccacaat gcgtggaggc cgacttggcg ttttctgctt
ctctcaagaa aacatcatct ggtccaacct caagtatcgc tgcaatgaca
ccatccctga ggacttccaa gagtttcaaa cccagaattt cgaccgcttc
gataattaaa ccaaggaagc aatctgtaac tgcttttcgg aacactaaaa
ccatatatat tttaacttca attttcttta gcttttacca acccaaatat
atcaaaacgt tttatgtgaa tgtggcaata aaggagaaga gatcattttt
aaaaaaaaaa aaaaa
**SEQ ID NO: 10 - Human thrombospondin 4 mRNA (transcript variant 3) (Taken from accession number NM_001306213 as at the filing date)** 
tgaaacgcg ggggcgtggc taagggaggg aagggtaatt tgggggctct
tgagagatga gagaaacaac gactggaggg attaagaaga actcttagga
gcagcttggc ctaaaggctg tgtgggacgt gcctgctttg ccttcgcctt
ccaccatgat cgtaagtttt ctgagacccc ctcagccatg tttcctgtag
agcctgtgga gtttctttga ccttctccca tcttccagtc agaggctaaa
cccaggcgct ctgctgccag tcctgacaga ccccgccctg aatgatctct
atgtgatttc caccttcaag ctgcagacta aaagttcagc caccatcttc
ggtctttact cttcaactga caacagtaaa tattttgaat ttactgtgat
gggacgctta aacaaagcca tcctccgtta cctgaagaac gatgggaagg
tgcatttggt ggttttcaac aacctgcagc tggcagacgg aaggcggcac
aggatcctcc tgaggctgag caatttgcag cgaggggccg gctccctaga
gctctacctg gactgcatcc aggtggattc cgttcacaat ctccccaggg
cctttgctgg cccctcccag aaacctgaga ccattgaatt gaggactttc
cagaggaagc cacaggactt cttggaagag ctgaagctgg tggtgagagg
ctcactgttc caggtggcca gcctgcaaga ctgcttcctg cagcagagtg
agccactggc tgccacaggc acaggggact ttaaccggca gttcttgggt
caaatgacac aattaaacca actcctggga gaggtgaagg accttctgag
acagcaggtt aaggaaacat catttttgcg aaacaccata gctgaatgcc
aggcttgcgg tcctctcaag tttcagtctc cgaccccaag cacggtggtg
cccccggctc cccctgcacc gccaacacgc ccacctcgtc ggtgtgactc
caacccatgt ttccgaggtg tccaatgtac cgacagtaga gatggcttcc
agtgtgggcc ctgccccgag ggctacacag gaaacgggat cacctgtatt
gatgttgatg agtgcaaata ccatccctgc tacccgggcg tgcactgcat
aaatttgtct cctggcttca gatgtgacgc ctgcccagtg ggcttcacag
ggcccatggt gcagggtgtt gggatcagtt ttgccaagtc aaacaagcag
gtctgcactg acattgatga gtgtcgaaat ggagcgtgcg ttcccaactc
gatctgcgtt aatactttgg gatcttaccg ctgtgggcct tgtaagccgg
ggtatactgg tgatcagata aggggatgca aagcggaaag aaactgcaga
aacccagagc tgaacccttg cagtgtgaat gcccagtgca ttgaagagag
gcagggggat gtgacatgtg tgtgtggagt cggttgggct ggagatggct
atatctgtgg aaaggatgtg gacatcgaca gttaccccga cgaagaactg
ccatgctctg ccaggaactg taaaaaggac aactgcaaat atgtgccaaa
ttctggccaa gaagatgcag acagagatgg cattggcgac gcttgtgacg
aggatgctga cggagatggg atcctgaatg agcaggataa ctgtgtcctg
attcataatg tggaccaaag gaacagcgat aaagatatct ttggggatgc
ctgtgataac tgcctgagtg tcttaaataa cgaccagaaa gacaccgatg
gggatggaag aggagatgcc tgtgatgatg acatggatgg agatggaata
aaaaacattc tggacaactg cccaaaattt cccaatcgtg accaacggga
caaggatggt gatggtgtgg gggatgcctg tgacagttgt cctgatgtca
gcaaccctaa ccagtctgat gtggataatg atctggttgg ggactcctgt
gacaccaatc aggacagtga tggagatggg caccaggaca gcacagacaa
ctgccccacc gtcattaaca gtgcccagct ggacaccgat aaggatggaa
ttggtgacga gtgtgatgat gatgatgaca atgatggtat cccagacctg
gtgccccctg gaccagacaa ctgccggctg gtccccaacc cagcccagga
ggatagcaac agcgacggag tgggagacat ctgtgagtct gactttgacc
aggaccaggt catcgatcgg atcgacgtct gcccagagaa cgcagaggtc
accctgaccg acttcagggc ttaccagacc gtggtcctgg atcctgaagg
ggatgcccag atcgatccca actgggtggt cctgaaccag ggcatggaga
ttgtacagac catgaacagt gatcctggcc tggcagtggg gtacacagct
 
tttaatggag ttgacttcga agggaccttc catgtgaata cccagacaga
tgatgactat gcaggcttta tctttggcta ccaagatagc tccagcttct
acgtggtcat gtggaagcag acggagcaga catattggca agccacccca
ttccgagcag ttgcagaacc tggcattcag ctcaaggctg tgaagtctaa
gacaggtcca ggggagcatc tccggaactc cctgtggcac acgggggaca
ccagtgacca ggtcaggctg ctgtggaagg actccaggaa tgtgggctgg
aaggacaagg tgtcctaccg ctggttccta cagcacaggc cccaggtggg
ctacatcagg gtacgatttt atgaaggctc tgagttggtg gctgactctg
gcgtcaccat agacaccaca atgcgtggag gccgacttgg cgttttctgc
ttctctcaag aaaacatcat ctggtccaac ctcaagtatc gctgcaatga
caccatccct gaggacttcc aagagtttca aacccagaat ttcgaccgct
tcgataatta aaccaaggaa gcaatctgta actgcttttc ggaacactaa
aaccatatat attttaactt caattttctt tagcttttac caacccaaat
atatcaaaac gttttatgtg aatgtggcaa taaaggagaa gagatcattt
ttaaaaaaaa aaaaaaa
**SEQ ID NO: 11 - Human thrombospondin 4 mRNA (transcript variant 4) (Taken from accession number NM_001306213 as at the filing date)** 
ggagcgatgt atgcccagag tctgctcttg acatccctga gaaagacgca
gaattactct acttgccctg ccacctgcat tttctgctct gactttgctt
gaacgacaaa aagaaagttt atttcaagga ttggcttgat gtcttaattt
ctgagtcttt gaccttctcc catcttccag tcagaggcta aacccaggcg
ctctgctgcc agtcctgaca gaccccgccc tgaatgatct ctatgtgatt
tccaccttca agctgcagac taaaagttca gccaccatct tcggtcttta
ctcttcaact gacaacagta aatattttga atttactgtg atgggacgct
taaacaaagc catcctccgt tacctgaaga acgatgggaa ggtgcatttg
gtggttttca acaacctgca gctggcagac ggaaggcggc acaggatcct
cctgaggctg agcaatttgc agcgaggggc cggctcccta gagctctacc
tggactgcat ccaggtggat tccgttcaca atctccccag ggcctttgct
ggcccctccc agaaacctga gaccattgaa ttgaggactt tccagaggaa
gccacaggac ttcttggaag agctgaagct ggtggtgaga ggctcactgt
tccaggtggc cagcctgcaa gactgcttcc tgcagcagag tgagccactg
gctgccacag gcacagggga ctttaaccgg cagttcttgg gtcaaatgac
acaattaaac caactcctgg gagaggtgaa ggaccttctg agacagcagg
ttaaggaaac atcatttttg cgaaacacca tagctgaatg ccaggcttgc
ggtcctctca agtttcagtc tccgacccca agcacggtgg tgcccccggc
tccccctgca ccgccaacac gcccacctcg tcggtgtgac tccaacccat
gtttccgagg tgtccaatgt accgacagta gagatggctt ccagtgtggg
ccctgccccg agggctacac aggaaacggg atcacctgta ttgatgttga
tgagtgcaaa taccatccct gctacccggg cgtgcactgc ataaatttgt
ctcctggctt cagatgtgac gcctgcccag tgggcttcac agggcccatg
gtgcagggtg ttgggatcag ttttgccaag tcaaacaagc aggtctgcac
tgacattgat gagtgtcgaa atggagcgtg cgttcccaac tcgatctgcg
ttaatacttt gggatcttac cgctgtgggc cttgtaagcc ggggtatact
ggtgatcaga taaggggatg caaagcggaa agaaactgca gaaacccaga
 
gctgaaccct tgcagtgtga atgcccagtg cattgaagag aggcaggggg
atgtgacatg tgtgtgtgga gtcggttggg ctggagatgg ctatatctgt
ggaaaggatg tggacatcga cagttacccc gacgaagaac tgccatgctc
tgccaggaac tgtaaaaagg acaactgcaa atatgtgcca aattctggcc
aagaagatgc agacagagat ggcattggcg acgcttgtga cgaggatgct
gacggagatg ggatcctgaa tgagcaggat aactgtgtcc tgattcataa
tgtggaccaa aggaacagcg ataaagatat ctttggggat gcctgtgata
actgcctgag tgtcttaaat aacgaccaga aagacaccga tggggatgga
agaggagatg cctgtgatga tgacatggat ggagatggaa taaaaaacat
tctggacaac tgcccaaaat ttcccaatcg tgaccaacgg gacaaggatg
gtgatggtgt gggggatgcc tgtgacagtt gtcctgatgt cagcaaccct
aaccagtctg atgtggataa tgatctggtt ggggactcct gtgacaccaa
tcaggacagt gatggagatg ggcaccagga cagcacagac aactgcccca
ccgtcattaa cagtgcccag ctggacaccg ataaggatgg aattggtgac
gagtgtgatg atgatgatga caatgatggt atcccagacc tggtgccccc
tggaccagac aactgccggc tggtccccaa cccagcccag gaggatagca
acagcgacgg agtgggagac atctgtgagt ctgactttga ccaggaccag
gtcatcgatc ggatcgacgt ctgcccagag aacgcagagg tcaccctgac
cgacttcagg gcttaccaga ccgtggtcct ggatcctgaa ggggatgccc
agatcgatcc caactgggtg gtcctgaacc agggcatgga gattgtacag
accatgaaca gtgatcctgg cctggcagtg gggtacacag cttttaatgg
agttgacttc gaagggacct tccatgtgaa tacccagaca gatgatgact
atgcaggctt tatctttggc taccaagata gctccagctt ctacgtggtc
atgtggaagc agacggagca gacatattgg caagccaccc cattccgagc
agttgcagaa cctggcattc agctcaaggc tgtgaagtct aagacaggtc
caggggagca tctccggaac tccctgtggc acacggggga caccagtgac
caggtcaggc tgctgtggaa ggactccagg aatgtgggct ggaaggacaa
ggtgtcctac cgctggttcc tacagcacag gccccaggtg ggctacatca
gggtacgatt ttatgaaggc tctgagttgg tggctgactc tggcgtcacc
atagacacca caatgcgtgg aggccgactt ggcgttttct gcttctctca
agaaaacatc atctggtcca acctcaagta tcgctgcaat gacaccatcc
ctgaggactt ccaagagttt caaacccaga atttcgaccg cttcgataat
taaaccaagg aagcaatctg taactgcttt tcggaacact aaaaccatat
atattttaac ttcaattttc tttagctttt accaacccaa atatatcaaa
acgttttatg tgaatgtggc aataaaggag aagagatcat ttttaaaaaa
aaaaaaaaa
**SEQ ID NO: 12 - Human ATP-binding cassette sub-family B member 5 (ABCB5) (Isoform 4) (Taken from UniProt ID Q2M3G0 as at the filing date)** 
MENSERAEEM QENYQRNGTA EEQPKLRKEA VGSIEIFRFA DGLDITLMIL
GILASLVNGA CLPLMPLVLG EMSDNLISGC LVQTNTTNYQ NCTQSQEKLN
EDMTLLTLYY VGIGVAALIF GYIQISLWII TAARQTKRIR KQFFHSVLAQ
DIGWFDSCDI GELNTRMTDD IDKISDGIGD KIALLFQNMS TFSIGLAVGL
VKGWKLTLVT LSTSPLIMAS AAACSRMVIS LTSKELSAYS KAGAVAEEVL
SSIRTVIAFR AQEKELQRYT QNLKDAKDFG IKRTIASKVS LGAVYFFMNG
 
TYGLAFWYGT SLILNGEPGY TIGTVLAVFF SVIHSSYCIG AAVPHFETFA
IARGAAFHIF QVIDKKPSID NFSTAGYKPE SIEGTVEFKN VSFNYPSRPS
IKILKGLNLR IKSGETVALV GLNGSGKSTV VQLLQRLYDP DDGFIMVDEN
DIRALNVRHY RDHIGVVSQE PVLFGTTISN NIKYGRDDVT DEEMERAARE
ANAYDFIMEF PNKFNTLVGE KGAQMSGGQK QRIAIARALV RNPKILILDE
ATSALDSESK SAVQAALEKA SKGRTTIVVA HRLSTIRSAD LIVTLKDGML
AEKGAHAELM AKRGLYYSLV MSQDIKKADE QMESMTYSTE RKTNSLPLHS
VKSIKSDFID KAEESTQSKE ISLPEVSLLK ILKLNKPEWP FVVLGTLASV
LNGTVHPVFS IIFAKIITMF GNNDKTTLKH DAEIYSMIFV ILGVICFVSY
FMQGLFYGRA GEILTMRLRH LAFKAMLYQD IAWFDEKENS TGGLTTILAI
DIAQIQGATG SRIGVLTQNA TNMGLSVIIS FIYGWEMTFL ILSIAPVLAV
TGMIETAAMT GFANKDKQEL KHAGKIATEA LENIRTIVSL TREKAFEQMY
EEMLQTQHRN TSKKAQIIGS CYAFSHAFIY FAYAAGFRFG AYLIQAGRMT
PEGMFIVFTA IAYGAMAIGE TLVLAPEYSK AKSGAAHLFA LLEKKPNIDS
RSQEGKKPDT CEGNLEFREV SFFYPCRPDV FILRGLSLSI ERGKTVAFVG
SSGCGKSTSV QLLQRLYDPV QGQVLFDGVD AKELNVQWLR SQIAIVPQEP
VLFNCSIAEN IAYGDNSRVV PLDEIKEAAN AANIHSFIEG LPEKYNTQVG
LKGAQLSGGQ KQRLAIARAL LQKPKILLLD EATSALDNDS EKVVQHALDK
ARTGRTCLVV THRLSAIQNA DLIVVLHNGK IKEQGTHQEL LRNRDIYFKL
VNAQSVQ
**SEQ ID NO: 13 - Human ATP-binding cassette sub-family B member 5 (ABCB5) (Isoform 2) (Taken from UniProt ID Q2R3G0 as at the filing date)** 
MVDENDIRAL NVRHYRDHIG VVSQEPVLFG TTISNNIKYG RDDVTDEEME
RAAREANAYD FIMEFPNKFN TLVGEKGAQM SGGQKQRIAI ARALVRNPKI
LILDEATSAL DSESKSAVQA ALEKDTPRYS F
**SEQ ID NO: 14 - Human ATP-binding cassette sub-family B member 5 (ABCB5) (Isoform 3) (Taken from UniProt ID Q2R3G0 as at the filing date)** 
MVDENDIRAL NVRHYRDHIG VVSQEPVLFG TTISNNIKYG RDDVTDEEME
RAAREANAYD FIMEFPNKFN TLVGEKGAQM SGGQKQRIAI ARALVRNPKI
LILDEATSAL DSESKSAVQA ALEKKK
**SEQ ID NO: 15 - Human ATP-binding cassette sub-family B member 5 (ABCB5) (Isoform 1) (Taken from UniProt ID Q2M3G0 as at the filing date)** 
MVDENDIRAL NVRHYRDHIG VVSQEPVLFG TTISNNIKYG RDDVTDEEME
RAAREANAYD FIMEFPNKFN TLVGEKGAQM SGGQKQRIAI ARALVRNPKI
LILDEATSAL DSESKSAVQA ALEKASKGRT TIVVAHRLST IRSADLIVTL
KDGMLAEKGA HAELMAKRGL YYSLVMSQDI KKADEQMESM TYSTERKTNS
LPLHSVKSIK SDFIDKAEES TQSKEISLPE VSLLKILKLN KPEWPFVVLG
TLASVLNGTV HPVFSIIFAK IITMFGNNDK TTLKHDAEIY SMIFVILGVI
CFVSYFMQGL FYGRAGEILT MRLRHLAFKA MLYQDIAWFD EKENSTGGLT
TILAIDIAQI QGATGSRIGV LTQNATNMGL SVIISFIYGW EMTFLILSIA
 
PVLAVTGMIE TAAMTGFANK DKQELKHAGK IATEALENIR TIVSLTREKA
FEQMYEEMLQ TQHRNTSKKA QIIGSCYAFS HAFIYFAYAA GFRFGAYLIQ
AGRMTPEGMF IVFTAIAYGA MAIGETLVLA PEYSKAKSGA AHLFALLEKK
PNIDSRSQEG KKPDTCEGNL EFREVSFFYP CRPDVFILRG LSLSIERGKT
VAFVGSSGCG KSTSVQLLQR LYDPVQGQVL FDGVDAKELN VQWLRSQIAI
VPQEPVLFNC SIAENIAYGD NSRVVPLDEI KEAANAANIH SFIEGLPEKY
NTQVGLKGAQ LSGGQKQRLA IARALLQKPK ILLLDEATSA LDNDSEKVVQ
HALDKARTGR TCLVVTHRLS AIQNADLIVV LHNGKIKEQG THQELLRNRD
IYFKLVNAQS VQ
**SEQ ID NO: 16 - Human ATP-binding cassette sub-family B member 5 (ABCB5) (transcript variant 3 mRNA) (Taken from NM_001163942 as at the filing date)** 
ATTGCTTCTCGGCCTTTTGGCTAAGATCAAGTGTAATCTGTGTTCTTTTTTATTTGGTCAT
ATCTTCCATTCTTTCTTACCTAATTCCTCTAATATCTCTCTGTGAGCCTAAACCAATAATT
ATATATTACATTCTATTGTCTTTCTTATATAACTGCAGAAAGATAAATATCACTTTGTTTG
TTCCTGTAGGTTTTCTTTAGTGTAATCCATAGCAGTTATTGCATTGGAGCAGCAGTCCCTC
ACTTTGAAACCTTCGCAATAGCCCGAGGAGCTGCCTTTCATATTTTCCAGGTTATTGATAA
GAAACCCAGTATAGATAACTTTTCCACAGCTGGATATAAACCTGAATCCATAGAAGGAACT
GTGGAATTTAAAAATGTTTCTTTCAATTATCCATCAAGACCATCTATCAAGATTCTGAAAG
GTCTGAATCTCAGAATTAAGTCTGGAGAGACAGTCGCCTTGGTCGGTCTCAATGGCAGTGG
GAAGAGTACGGTAGTCCAGCTTCTGCAGAGGTTATATGATCCGGATGATGGCTTTATCATG
GTGGATGAGAATGACATCAGAGCTTTAAATGTGCGGCATTATCGAGACCATATTGGAGTGG
TTAGTCAAGAGCCTGTTTTGTTCGGGACCACCATCAGTAACAATATCAAGTATGGACGAGA
TGATGTGACTGATGAAGAGATGGAGAGAGCAGCAAGGGAAGCAAATGCGTATGATTTTATC
ATGGAGTTTCCTAATAAATTTAATACATTGGTAGGGGAAAAAGGAGCTCAAATGAGTGGAG
GGCAGAAACAGAGGATCGCAATTGCTCGTGCCTTAGTTCGAAACCCCAAGATTCTGATTTT
AGATGAGGCTACGTCTGCCCTGGATTCAGAAAGCAAGTCAGCTGTTCAAGCTGCACTGGAG
AAGGATACCCCCAGGTATTCATTTTGACCTAATTTCACCTCAAGTGGAGAATCGCTGACCT
TGAACCAGCGCCCTTCGACAGCTCTGGCCCCTCAAACCTCACCCTGACCTCCTGCTGCCTA
TGAGCTACTGCACATACCTCAAGGCCATATGCAGTTGTGGCCCTGCACCAAATTACACTGA
ATCTAGGAGGGGAGTTGGCAGTGGCGGTATGAAAAACCATTGAACAGTTTTCTCGATGGCC
TGACTCCCTTATAAACCAGAGCCTTCAGACCCCTTACAAGGCTTAATGGCACATTTTACTT
TGCATTTGCTTGGAAGTGAGTTAAGCGTTTTTTTTTCTCTAAGAAAATCGCAGGCTTCTTT
TTTTAAAATGCTGACTTTATGGAACCAGAAACGGATACTGGCAGGCTAAATGTCCACTGCT
TCTTGAAAAATCTCTTAGTTACTATTATTTAGTAGCAAATCAGATTGTGCAAGTTTGAACG
CTTGTTTCCCTCCCATAATAACAATGCCACATGTTGCTTGTGTGATAAATAATCTTTGTTT
ACTGTGGACACTTCGGCAATTTGGTTCATCTTGGTACAGAAAATACAAAGACAGGACTAAT
ATTTTATTCATTTTTTATACTTAGTAACTAGGACAGGGATCTGTATTTAGACTATATTCAA
GCCTCGCTCTGTCACCCATGCTTGAGTGCAACCTCTGCCCACTGTGTTCAAGCGATTCTCC
TGCCTCAGCCTCCTGAGTAGCTGGGAGTACAGGCGTGCATCACCACACCCAGCTAATTTTT
GTATTTTTAGTAGAGACAAGGTTTTGCCATGTTGACCAGGCTGGTCTCAAACTCCTGACCT
CAGGTGATCCGCCCACCTCGGCCTCCCAAAGTGCTGGGATTACAGGTGTGCGTCTCCGCGC
CCGGCCAGTTACATAAGATTTTAAAAAATGTATTGTAATAGGCTCTCTGATTGTTTAGAAG
GTTTCATGTTGTGCCCGCAGTATTTTCATCCACTGGTAATCACTTTAGGTAATCAAAACTA
GTTCAGACTTTTAAAATAAATAGTTGCTTGGTGACATGATAAACTAATAAATGTTATTCTT
ATTGGTTAATATGAAACATCTATCAAAAGTGAAAAAAGAAGAATGCAGAATGTTTGTAAAC
TGATTCCAAAATTACACAAAATAGCAACTGTGAAAAAAATGAAGGCAATATATGAAAATAA
 
TAACAGCTATGTGGCATAATTATGGGGGAATTTAACTTATTTCAAAAATTCATTTACTGTC
CCTGTTTTTCAGTTGTTTAAAGGAGAAAGAGATTAAATCAATGTAGAAATA
**SEQ ID NO: 17 - Human ATP-binding cassette sub-family B member 5 (ABCB5) (transcript variant 1 mRNA) (Taken from NM_001163941 as at the filing date)** 
TGTAGAGAGAGAAAGAGAAAACACGCTCATGTAATTGTGGTGACTGGCACTTCCAAAATCCACAAGCCAG
ACTAGAAGGCTGGAAATTCTGGCAAAAAATTGATACCTTGTATCTGAAAGCTTTATCCTATCCTCTTCAG
AAGACTTCAGTCTTTTCTCTTAAGGACTTCAACTGACTGGATGGGGCCCACTCAAAACAGCATCTAAGGA
ATTAAAAAGAAGTAAATTGTAAATAAGATGGAAAATTCAGAAAGAGCTGAAGAAATGCAAGAAAATTATC
AGAGAAATGGAACTGCAGAAGAACAGCCAAAACTGAGAAAGGAAGCAGTTGGATCTATTGAGATATTCCG
CTTTGCTGATGGACTGGACATCACACTCATGATCCTGGGTATACTGGCATCACTGGTCAATGGAGCCTGC
CTTCCTTTAATGCCACTGGTTTTAGGAGAAATGAGTGATAACCTTATTAGTGGATGTCTAGTCCAAACTA
ACACAACAAATTATCAGAACTGTACTCAGTCTCAAGAGAAGCTGAATGAAGATATGACTCTGTTGACCCT
GTATTATGTTGGAATAGGTGTTGCTGCCTTGATTTTTGGTTACATACAGATTTCCTTGTGGATTATAACT
GCAGCACGACAGACCAAGAGGATTCGAAAACAGTTTTTTCATTCAGTTTTGGCACAGGACATCGGCTGGT
TTGATAGCTGTGACATCGGTGAACTTAACACTCGCATGACAGATGACATTGACAAAATCAGTGATGGTAT
TGGAGATAAGATTGCTCTGTTGTTTCAAAACATGTCTACTTTTTCGATTGGCCTGGCAGTTGGTTTGGTG
AAGGGCTGGAAACTCACCCTAGTGACTCTATCCACGTCTCCTCTTATAATGGCTTCAGCGGCAGCATGTT
CTAGGATGGTCATCTCATTGACCAGTAAGGAATTAAGTGCCTATTCCAAAGCTGGGGCTGTGGCAGAAGA
AGTCTTGTCATCAATCCGAACAGTCATAGCCTTTAGGGCCCAGGAGAAAGAACTTCAAAGGTATACACAG
AATCTCAAAGATGCAAAGGATTTTGGCATAAAAAGGACTATAGCTTCAAAAGTGTCTCTTGGTGCTGTGT
ACTTCTTTATGAATGGAACCTATGGACTTGCTTTTTGGTATGGAACCTCCTTGATTCTTAATGGAGAACC
TGGATATACCATCGGGACTGTTCTTGCTGTTTTCTTTAGTGTAATCCATAGCAGTTATTGCATTGGAGCA
GCAGTCCCTCACTTTGAAACCTTCGCAATAGCCCGAGGAGCTGCCTTTCATATTTTCCAGGTTATTGATA
AGAAACCCAGTATAGATAACTTTTCCACAGCTGGATATAAACCTGAATCCATAGAAGGAACTGTGGAATT
TAAAAATGTTTCTTTCAATTATCCATCAAGACCATCTATCAAGATTCTGAAAGGTCTGAATCTCAGAATT
AAGTCTGGAGAGACAGTCGCCTTGGTCGGTCTCAATGGCAGTGGGAAGAGTACGGTAGTCCAGCTTCTGC
AGAGGTTATATGATCCGGATGATGGCTTTATCATGGTGGATGAGAATGACATCAGAGCTTTAAATGTGCG
GCATTATCGAGACCATATTGGAGTGGTTAGTCAAGAGCCTGTTTTGTTCGGGACCACCATCAGTAACAAT
ATCAAGTATGGACGAGATGATGTGACTGATGAAGAGATGGAGAGAGCAGCAAGGGAAGCAAATGCGTATG
ATTTTATCATGGAGTTTCCTAATAAATTTAATACATTGGTAGGGGAAAAAGGAGCTCAAATGAGTGGAGG
GCAGAAACAGAGGATCGCAATTGCTCGTGCCTTAGTTCGAAACCCCAAGATTCTGATTTTAGATGAGGCT
ACGTCTGCCCTGGATTCAGAAAGCAAGTCAGCTGTTCAAGCTGCACTGGAGAAGGCGAGCAAAGGTCGGA
CTACAATCGTGGTAGCACACCGACTTTCTACTATTCGAAGTGCAGATTTGATTGTGACCCTAAAGGATGG
AATGCTGGCGGAGAAAGGAGCACATGCTGAACTAATGGCAAAACGAGGTCTATATTATTCACTTGTGATG
TCACAGGATATTAAAAAAGCTGATGAACAGATGGAGTCAATGACATATTCTACTGAAAGAAAGACCAACT
CACTTCCTCTGCACTCTGTGAAGAGCATCAAGTCAGACTTCATTGACAAGGCTGAGGAATCCACCCAATC
TAAAGAGATAAGTCTTCCTGAAGTCTCTCTATTAAAAATTTTAAAGTTAAACAAGCCTGAATGGCCTTTT
GTGGTTCTGGGGACATTGGCTTCTGTTCTAAATGGAACTGTTCATCCAGTATTTTCCATCATCTTTGCAA
AAATTATAACCATGTTTGGAAATAATGATAAAACCACATTAAAGCATGATGCAGAAATTTATTCCATGAT
ATTCGTCATTTTGGGTGTTATTTGCTTTGTCAGTTATTTCATGCAGGGATTATTTTACGGCAGAGCAGGG
GAAATTTTAACGATGAGATTAAGACACTTGGCCTTCAAAGCCATGTTATATCAGGATATTGCCTGGTTTG
ATGAAAAGGAAAACAGCACAGGAGGCTTGACAACAATATTAGCCATAGATATAGCACAAATTCAAGGAGC
AACAGGTTCCAGGATTGGCGTCTTAACACAAAATGCAACTAACATGGGACTTTCAGTTATCATTTCCTTT
ATATATGGATGGGAGATGACATTCCTGATTCTGAGTATTGCTCCAGTACTTGCCGTGACAGGAATGATTG
AAACCGCAGCAATGACTGGATTTGCCAACAAAGATAAGCAAGAACTTAAGCATGCTGGAAAGATAGCAAC
TGAAGCTTTGGAGAATATACGTACTATAGTGTCATTAACAAGGGAAAAAGCCTTCGAGCAAATGTATGAA
GAGATGCTTCAGACTCAACACAGAAATACCTCGAAGAAAGCACAGATTATTGGAAGCTGTTATGCATTCA
GCCATGCCTTTATATATTTTGCCTATGCGGCAGGGTTTCGATTTGGAGCCTATTTAATTCAAGCTGGACG
AATGACCCCAGAGGGCATGTTCATAGTTTTTACTGCAATTGCATATGGAGCTATGGCCATCGGAGAAACG
CTCGTTTTGGCTCCTGAATATTCCAAAGCCAAATCGGGGGCTGCGCATCTGTTTGCCTTGTTGGAAAAGA
AACCAAATATAGACAGCCGCAGTCAAGAAGGGAAAAAGCCAGACACATGTGAAGGGAATTTAGAGTTTCG
AGAAGTCTCTTTCTTCTATCCATGTCGCCCAGATGTTTTCATCCTCCGTGGCTTATCCCTCAGTATTGAG
CGAGGAAAGACAGTAGCATTTGTGGGGAGCAGCGGCTGTGGGAAAAGCACTTCTGTTCAACTTCTGCAGA
 
GACTTTATGACCCCGTGCAAGGACAAGTGCTGTTTGATGGTGTGGATGCAAAAGAATTGAATGTACAGTG
GCTCCGTTCCCAAATAGCAATCGTTCCTCAAGAGCCTGTGCTCTTCAACTGCAGCATTGCTGAGAACATC
GCCTATGGTGACAACAGCCGTGTGGTGCCATTAGATGAGATCAAAGAAGCCGCAAATGCAGCAAATATCC
ATTCTTTTATTGAAGGTCTCCCTGAGAAATACAACACACAAGTTGGACTGAAAGGAGCACAGCTTTCTGG
CGGCCAGAAACAAAGACTAGCTATTGCAAGGGCTCTTCTCCAAAAACCCAAAATTTTATTGTTGGATGAG
GCCACTTCAGCCCTCGATAATGACAGTGAGAAGGTGGTTCAGCATGCCCTTGATAAAGCCAGGACGGGAA
GGACATGCCTAGTGGTCACTCACAGGCTCTCTGCAATTCAGAACGCAGATTTGATAGTGGTTCTGCACAA
TGGAAAGATAAAGGAACAAGGAACTCATCAAGAGCTCCTGAGAAATCGAGACATATATTTTAAGTTAGTG
AATGCACAGTCAGTGCAGTGATGCTGTTGAGGTAGCACATATTTTGATGTTCGTGTAATGCAAAGAAGGA
GTACTTAATAATTACTTGGCAAGCTTTGATCTCTTTTATTGCATATATCAATACCTAGAATCATGCTACT
CAAGTACATACATGTTCTATTCACACACCATCTGACCTTCAGATTTTTAAAAGGAAGCAAAAATTTGCTT
ATTTCATGTAAGTGAAATAATGCTTATATCCTTCACTTTATAAAACTATTCTAGCACATTTGCTTGTAAA
GCAGTTTTCTACAAGGTGAATTTATTTCCCATCAACTTCTGCTATAAAATCGGAAATATGTTTCCAGGGG
GAATATTATCCAATTAACCATGTTGAAGGTTTTAGCAAAGGCAGTGTAAGATAGAGTGGGGCCTGTAGCA
TTGCAGGGAGAGTGTCTTTCACTTGGAATTTTGTTTTGCAGCACATATTACAGTAGTTTTGCTAGTCCCT
TTTCTCCAGACCGTAGGGATTTCTCTCAATAAGTATTCACTATTTCTCTAAATTTTATTCTATTTTTTTG
TTGAGCAGGGAATAGAAAGGATTACGATGTAAAATTTCTGGGAGGATTAGGTAGCTATCTCCTACTTCAC
CAGTAAGTGAAGTGCCTCACATGAGCCATCCCAAAGATTCATTATTCCAAACCTTGGGTTTGGCAGTATA
AGTCACAGGCCTACCTGTTTATGAAAACTTACTTACTTAAAATAAGAGCTACTTTTGGGCCGGGTGCGGT
GGCTCACGCCTGTAATCCCAGAACTTTGGGAGGCCGAGGAGGGCGGATCACTTGAGGTCAGGAGTTCGAG
ACCAGCCTGGCCAACATGGTGAAACCCCGTCTCTACTAAAAACACAAAAATTAGCCAATCTTGGTGGCGG
GCACCTGGAATCCCAGCTACTTGGGAGGCTGAGGCAGGAGAATCATTTGAACCTAGGAGGCAGAGGTTGC
AGTGAGCCGAGATCTCACCACTGCACTCCAGCCTGCGCAACAGAGCGAGACTCCATCTCAAAAAATAATA
AATAAGAGCTAATTTTATTGTGGGTGAAAATTTTTAAACGTCTTTCTCTATAATAAAATAATTTCCTTAA
ATTTTATATATACTTTATCATATATAATGTGTGAATGATTTTAAAGTTCTGTGTAAATAACAATATTGGT
AAAATGAGTTACATTTTCAACTTACTTAAATATGTAATGTCACCTGGTGATTTTATCTTTATTCTTCAGT
GTATTTTCTTCCATTTACACATTTAGCTAGCCTCCCTAAAGTGTACTCTACCAATAATTGAAATCTTGTT
AAACAAAATTAAAACCATTTATATATTATGCTGCTTTCTTTAAAATGCAAAATAAAAATAAGATTGGGGA
CTTGAGAATCA
**SEQ ID NO: 18 - Human ATP-binding cassette sub-family B member 5 (ABCB5) (transcript variant 2 mRNA) (Taken from NM_178559 as at the filing date)** 
ATTGCTTCTCGGCCTTTTGGCTAAGATCAAGTGTAATCTGTGTTCTTTTTTATTTGGTCATATCTTCCAT
TCTTTCTTACCTAATTCCTCTAATATCTCTCTGTGAGCCTAAACCAATAATTATATATTACATTCTATTG
TCTTTCTTATATAACTGCAGAAAGATAAATATCACTTTGTTTGTTCCTGTAGGTTTTCTTTAGTGTAATC
CATAGCAGTTATTGCATTGGAGCAGCAGTCCCTCACTTTGAAACCTTCGCAATAGCCCGAGGAGCTGCCT
TTCATATTTTCCAGGTTATTGATAAGAAACCCAGTATAGATAACTTTTCCACAGCTGGATATAAACCTGA
ATCCATAGAAGGAACTGTGGAATTTAAAAATGTTTCTTTCAATTATCCATCAAGACCATCTATCAAGATT
CTGAAAGGTCTGAATCTCAGAATTAAGTCTGGAGAGACAGTCGCCTTGGTCGGTCTCAATGGCAGTGGGA
AGAGTACGGTAGTCCAGCTTCTGCAGAGGTTATATGATCCGGATGATGGCTTTATCATGGTGGATGAGAA
TGACATCAGAGCTTTAAATGTGCGGCATTATCGAGACCATATTGGAGTGGTTAGTCAAGAGCCTGTTTTG
TTCGGGACCACCATCAGTAACAATATCAAGTATGGACGAGATGATGTGACTGATGAAGAGATGGAGAGAG
CAGCAAGGGAAGCAAATGCGTATGATTTTATCATGGAGTTTCCTAATAAATTTAATACATTGGTAGGGGA
AAAAGGAGCTCAAATGAGTGGAGGGCAGAAACAGAGGATCGCAATTGCTCGTGCCTTAGTTCGAAACCCC
AAGATTCTGATTTTAGATGAGGCTACGTCTGCCCTGGATTCAGAAAGCAAGTCAGCTGTTCAAGCTGCAC
TGGAGAAGGCGAGCAAAGGTCGGACTACAATCGTGGTAGCACACCGACTTTCTACTATTCGAAGTGCAGA
TTTGATTGTGACCCTAAAGGATGGAATGCTGGCGGAGAAAGGAGCACATGCTGAACTAATGGCAAAACGA
GGTCTATATTATTCACTTGTGATGTCACAGGATATTAAAAAAGCTGATGAACAGATGGAGTCAATGACAT
ATTCTACTGAAAGAAAGACCAACTCACTTCCTCTGCACTCTGTGAAGAGCATCAAGTCAGACTTCATTGA
CAAGGCTGAGGAATCCACCCAATCTAAAGAGATAAGTCTTCCTGAAGTCTCTCTATTAAAAATTTTAAAG
TTAAACAAGCCTGAATGGCCTTTTGTGGTTCTGGGGACATTGGCTTCTGTTCTAAATGGAACTGTTCATC
CAGTATTTTCCATCATCTTTGCAAAAATTATAACCATGTTTGGAAATAATGATAAAACCACATTAAAGCA
TGATGCAGAAATTTATTCCATGATATTCGTCATTTTGGGTGTTATTTGCTTTGTCAGTTATTTCATGCAG
GGATTATTTTACGGCAGAGCAGGGGAAATTTTAACGATGAGATTAAGACACTTGGCCTTCAAAGCCATGT
 
TATATCAGGATATTGCCTGGTTTGATGAAAAGGAAAACAGCACAGGAGGCTTGACAACAATATTAGCCAT
AGATATAGCACAAATTCAAGGAGCAACAGGTTCCAGGATTGGCGTCTTAACACAAAATGCAACTAACATG
GGACTTTCAGTTATCATTTCCTTTATATATGGATGGGAGATGACATTCCTGATTCTGAGTATTGCTCCAG
TACTTGCCGTGACAGGAATGATTGAAACCGCAGCAATGACTGGATTTGCCAACAAAGATAAGCAAGAACT
TAAGCATGCTGGAAAGATAGCAACTGAAGCTTTGGAGAATATACGTACTATAGTGTCATTAACAAGGGAA
AAAGCCTTCGAGCAAATGTATGAAGAGATGCTTCAGACTCAACACAGAAATACCTCGAAGAAAGCACAGA
TTATTGGAAGCTGTTATGCATTCAGCCATGCCTTTATATATTTTGCCTATGCGGCAGGGTTTCGATTTGG
AGCCTATTTAATTCAAGCTGGACGAATGACCCCAGAGGGCATGTTCATAGTTTTTACTGCAATTGCATAT
GGAGCTATGGCCATCGGAGAAACGCTCGTTTTGGCTCCTGAATATTCCAAAGCCAAATCGGGGGCTGCGC
ATCTGTTTGCCTTGTTGGAAAAGAAACCAAATATAGACAGCCGCAGTCAAGAAGGGAAAAAGCCAGACAC
ATGTGAAGGGAATTTAGAGTTTCGAGAAGTCTCTTTCTTCTATCCATGTCGCCCAGATGTTTTCATCCTC
CGTGGCTTATCCCTCAGTATTGAGCGAGGAAAGACAGTAGCATTTGTGGGGAGCAGCGGCTGTGGGAAAA
GCACTTCTGTTCAACTTCTGCAGAGACTTTATGACCCCGTGCAAGGACAAGTGCTGTTTGATGGTGTGGA
TGCAAAAGAATTGAATGTACAGTGGCTCCGTTCCCAAATAGCAATCGTTCCTCAAGAGCCTGTGCTCTTC
AACTGCAGCATTGCTGAGAACATCGCCTATGGTGACAACAGCCGTGTGGTGCCATTAGATGAGATCAAAG
AAGCCGCAAATGCAGCAAATATCCATTCTTTTATTGAAGGTCTCCCTGAGAAATACAACACACAAGTTGG
ACTGAAAGGAGCACAGCTTTCTGGCGGCCAGAAACAAAGACTAGCTATTGCAAGGGCTCTTCTCCAAAAA
CCCAAAATTTTATTGTTGGATGAGGCCACTTCAGCCCTCGATAATGACAGTGAGAAGGTGGTTCAGCATG
CCCTTGATAAAGCCAGGACGGGAAGGACATGCCTAGTGGTCACTCACAGGCTCTCTGCAATTCAGAACGC
AGATTTGATAGTGGTTCTGCACAATGGAAAGATAAAGGAACAAGGAACTCATCAAGAGCTCCTGAGAAAT
CGAGACATATATTTTAAGTTAGTGAATGCACAGTCAGTGCAGTGATGCTGTTGAGGTAGCACATATTTTG
ATGTTCGTGTAATGCAAAGAAGGAGTACTTAATAATTACTTGGCAAGCTTTGATCTCTTTTATTGCATAT
ATCAATACCTAGAATCATGCTACTCAAGTACATACATGTTCTATTCACACACCATCTGACCTTCAGATTT
TTAAAAGGAAGCAAAAATTTGCTTATTTCATGTAAGTGAAATAATGCTTATATCCTTCACTTTATAAAAC
TATTCTAGCACATTTGCTTGTAAAGCAGTTTTCTACAAGGTGAATTTATTTCCCATCAACTTCTGCTATA
AAATCGGAAATATGTTTCCAGGGGGAATATTATCCAATTAACCATGTTGAAGGTTTTAGCAAAGGCAGTG
TAAGATAGAGTGGGGCCTGTAGCATTGCAGGGAGAGTGTCTTTCACTTGGAATTTTGTTTTGCAGCACAT
ATTACAGTAGTTTTGCTAGTCCCTTTTCTCCAGACCGTAGGGATTTCTCTCAATAAGTATTCACTATTTC
TCTAAATTTTATTCTATTTTTTTGTTGAGCAGGGAATAGAAAGGATTACGATGTAAAATTTCTGGGAGGA
TTAGGTAGCTATCTCCTACTTCACCAGTAAGTGAAGTGCCTCACATGAGCCATCCCAAAGATTCATTATT
CCAAACCTTGGGTTTGGCAGTATAAGTCACAGGCCTACCTGTTTATGAAAACTTACTTACTTAAAATAAG
AGCTACTTTTGGGCCGGGTGCGGTGGCTCACGCCTGTAATCCCAGAACTTTGGGAGGCCGAGGAGGGCGG
ATCACTTGAGGTCAGGAGTTCGAGACCAGCCTGGCCAACATGGTGAAACCCCGTCTCTACTAAAAACACA
AAAATTAGCCAATCTTGGTGGCGGGCACCTGGAATCCCAGCTACTTGGGAGGCTGAGGCAGGAGAATCAT
TTGAACCTAGGAGGCAGAGGTTGCAGTGAGCCGAGATCTCACCACTGCACTCCAGCCTGCGCAACAGAGC
GAGACTCCATCTCAAAAAATAATAAATAAGAGCTAATTTTATTGTGGGTGAAAATTTTTAAACGTCTTTC
TCTATAATAAAATAATTTCCTTAAATTTTATATATACTTTATCATATATAATGTGTGAATGATTTTAAAG
TTCTGTGTAAATAACAATATTGGTAAAATGAGTTACATTTTCAACTTACTTAAATATGTAATGTCACCTG
GTGATTTTATCTTTATTCTTCAGTGTATTTTCTTCCATTTACACATTTAGCTAGCCTCCCTAAAGTGTAC
TCTACCAATAATTGAAATCTTGTTAAACAAAATTAAAACCATTTATATATTATGCTGCTTTCTTTAAAAT
GCAAAATAAAAATAAGATTGGGGACTTGAGAATCA
**SEQ ID NO: 19 - Human ATP-binding cassette sub-family B member 5 (ABCB5) (transcript variant 4 mRNA) (Taken from NM_001163941 as at the filing date)** 
ATTGCTTCTCGGCCTTTTGGCTAAGATCAAGTGTAATCTGTGTTCTTTTTTATTTGGTCATATCTTCCAT
TCTTTCTTACCTAATTCCTCTAATATCTCTCTGTGAGCCTAAACCAATAATTATATATTACATTCTATTG
TCTTTCTTATATAACTGCAGAAAGATAAATATCACTTTGTTTGTTCCTGTAGGTTTTCTTTAGTGTAATC
CATAGCAGTTATTGCATTGGAGCAGCAGTCCCTCACTTTGAAACCTTCGCAATAGCCCGAGGAGCTGCCT
TTCATATTTTCCAGGTTATTGATAAGAAACCCAGTATAGATAACTTTTCCACAGCTGGATATAAACCTGA
ATCCATAGAAGGAACTGTGGAATTTAAAAATGTTTCTTTCAATTATCCATCAAGACCATCTATCAAGATT
CTGAAAGGTCTGAATCTCAGAATTAAGTCTGGAGAGACAGTCGCCTTGGTCGGTCTCAATGGCAGTGGGA
AGAGTACGGTAGTCCAGCTTCTGCAGAGGTTATATGATCCGGATGATGGCTTTATCATGGTGGATGAGAA
TGACATCAGAGCTTTAAATGTGCGGCATTATCGAGACCATATTGGAGTGGTTAGTCAAGAGCCTGTTTTG
TTCGGGACCACCATCAGTAACAATATCAAGTATGGACGAGATGATGTGACTGATGAAGAGATGGAGAGAG
CAGCAAGGGAAGCAAATGCGTATGATTTTATCATGGAGTTTCCTAATAAATTTAATACATTGGTAGGGGA
AAAAGGAGCTCAAATGAGTGGAGGGCAGAAACAGAGGATCGCAATTGCTCGTGCCTTAGTTCGAAACCCC
 
AAGATTCTGATTTTAGATGAGGCTACGTCTGCCCTGGATTCAGAAAGCAAGTCAGCTGTTCAAGCTGCAC
TGGAGAAGAAAAAATAATACAACAAAGACGTATTGATAATCTACCACACTATCTACGTTGAGGTATCTGG
AACCACAGTGGTATGAATTGCACTATCTCCCTAATCTTTACACTAATTCTGGCTAAAAGACAGCATCAGC
TCAAATTTTCCTGATCAGTTTTTTAGCAGGCTTTGGCTGCCAAAATCTCTTCTGTGTTTTAAGTCTGAAG
CAATTACAGTTGAATATTTTGTTGTTGTTCACTATGAGGAGTATATCGGTTTTAGGTCACATGAATGATC
TAGTAGAGATGTATTTTTCTAGTACACTTGATAGTACCTTGAATATGTACAGTACATGTAGGCCCATTTT
ATGGACCTCTGAGGCACAGTTTTTTCCCTTTTAAGTTATTAGATATTTCTGTGATTTATTTGATGCACTC
AGATACAAATTCATGCTATAATAAGTTAAATTGTAACATAATATCTACCTTAATCAAATATTTATAGTTT
AGGCCAGGAGCCTGGCAAAGTAAAGATGACACAGATGAAATGCTTGATAAGTAAATAAATATGAGAAGAG
ATATATATTAAAACACCTGTGAAGTGAATCCCCTGTGAAGAAAAGAAGGTCTCTAACAATATAAACCTGT
ATCAGGAAACCCTGTGATTCTGATTCTGTGCACAGAAGGATACATGGAATTCTCAGACCTTTTCTAGTTG
CAACCTTTTAATTGTTTGACTTTATATTCTGTCCTCTCAAATTCTCCTTAGACTGTGACGTGTTAATGCT
CTCTTTTATATTTTCTAATTAAATTCCATACACATTAATTTGAG
**SEQ ID NO: 20 - Protein sequence of human matrix metalloproteinase 13 (MMP13) complete form (Taken from UniProt ID P45452 as at the filing date)**
**SEQ ID NO: 21 -Human matrix metalloproteinase 13 (MMP13) mRHA (NM_002427 as at the filing date)** 
ACAACAGTCCCCAGGCATCACCATTCAAGATGCATCCAGGGGTCCTGGCTGCCTTCCTCTTCTTGAGCTG
GACTCATTGTCGGGCCCTGCCCCTTCCCAGTGGTGGTGATGAAGATGATTTGTCTGAGGAAGACCTCCAG
TTTGCAGAGCGCTACCTGAGATCATACTACCATCCTACAAATCTCGCGGGAATCCTGAAGGAGAATGCAG
CAAGCTCCATGACTGAGAGGCTCCGAGAAATGCAGTCTTTCTTCGGCTTAGAGGTGACTGGCAAACTTGA
CGATAACACCTTAGATGTCATGAAAAAGCCAAGATGCGGGGTTCCTGATGTGGGTGAATACAATGTTTTC
CCTCGAACTCTTAAATGGTCCAAAATGAATTTAACCTACAGAATTGTGAATTACACCCCTGATATGACTC
ATTCTGAAGTCGAAAAGGCATTCAAAAAAGCCTTCAAAGTTTGGTCCGATGTAACTCCTCTGAATTTTAC
CAGACTTCACGATGGCATTGCTGACATCATGATCTCTTTTGGAATTAAGGAGCATGGCGACTTCTACCCA
TTTGATGGGCCCTCTGGCCTGCTGGCTCATGCTTTTCCTCCTGGGCCAAATTATGGAGGAGATGCCCATT
TTGATGATGATGAAACCTGGACAAGTAGTTCCAAAGGCTACAACTTGTTTCTTGTTGCTGCGCATGAGTT
CGGCCACTCCTTAGGTCTTGACCACTCCAAGGACCCTGGAGCACTCATGTTTCCTATCTACACCTACACC
GGCAAAAGCCACTTTATGCTTCCTGATGACGATGTACAAGGGATCCAGTCTCTCTATGGTCCAGGAGATG
AAGACCCCAACCCTAAACATCCAAAAACGCCAGACAAATGTGACCCTTCCTTATCCCTTGATGCCATTAC
CAGTCTCCGAGGAGAAACAATGATCTTTAAAGACAGATTCTTCTGGCGCCTGCATCCTCAGCAGGTTGAT
GCGGAGCTGTTTTTAACGAAATCATTTTGGCCAGAACTTCCCAACCGTATTGATGCTGCATATGAGCACC
CTTCTCATGACCTCATCTTCATCTTCAGAGGTAGAAAATTTTGGGCTCTTAATGGTTATGACATTCTGGA
AGGTTATCCCAAAAAAATATCTGAACTGGGTCTTCCAAAAGAAGTTAAGAAGATAAGTGCAGCTGTTCAC
TTTGAGGATACAGGCAAGACTCTCCTGTTCTCAGGAAACCAGGTCTGGAGATATGATGATACTAACCATA
TTATGGATAAAGACTATCCGAGACTAATAGAAGAAGACTTCCCAGGAATTGGTGATAAAGTAGATGCTGT
CTATGAGAAAAATGGTTATATCTATTTTTTCAACGGACCCATACAGTTTGAATACAGCATCTGGAGTAAC
CGTATTGTTCGCGTCATGCCAGCAAATTCCATTTTGTGGTGTTAAGTGTCTTTTTAAAAATTGTTATTTA
AATCCTGAAGAGCATTTGGGGTAATACTTCCAGAAGTGCGGGGTAGGGGAAGAAGAGCTATCAGGAGAAA
GCTTGGTTCTGTGAACAAGCTTCAGTAAGTTATCTTTGAATATGTAGTATCTATATGACTATGCGTGGCT
GGAACCACATTGAAGAATGTTAGAGTAATGAAATGGAGGATCTCTAAAGAGCATCTGATTCTTGTTGCTG
TACAAAAGCAATGGTTGATGATACTTCCCACACCACAAATGGGACACATGGTCTGTCAATGAGAGCATAA
TTTAAAAATATATTTATAAGGAAATTTTACAAGGGCATAAAGTAAATACATGCATATAATGAATAAATCA
TTCTTACTAAAAAGTATAAAATAGTATGAAAATGGAAATTTGGGAGAGCCATACATAAAAGAAATAAACC
 
AAAGGAAAATGTCTGTAATAATAGACTGTAACTTCCAAATAAATAATTTTCATTTTGCACTGAGGATATT
CAGATGTATGTGCCCTTCTTCACACAGACACTAACGAAATATCAAAGTCATTAAAGACAGGAGACAAAAG
AGCAGTGGTAAGAATAGTAGATGTGGCCTTTGAATTCTGTTTAATTTTCACTTTTGGCAATGACTCAAAG
TCTGCTCTCATATAAGACAAATATTCCTTTGCATATTATAAAGGATAAAGAAGGATGATGTCTTTTTATT
AAAATATTTCAGGTTCTTCAGAAGTCACACATTACAAAGTTAAAATTGTTATCAAAATAGTCTAAGGCCA
TGGCATCCCTTTTTCATAAATTATTTGATTATTTAAGACTAAAAGTTGCATTTTAACCCTATTTTACCTA
GCTAATTATTTAATTGTCCAGTTTGTCTTGGATATATAGGCTATTTTCTAAAGACTTGTATAGCATGAAA
TAAAATATATCTTATAAAGTGGAAGTATGTATATTAAAAAAGAGACATCCAAATTTTTTTTTAAAGCAGT
CTACTAGATTGTGATCCCTTGAGATATGGAAGGATGCCTTTTTTTCTCTGCATTTAAAAAAATCCCCCAG
CACTTCCCACAGTGCCTATTGATACTTGGGGAGGGTGCTTGGCACTTATTGAATATATGATCGGCCATCA
AGGGAAGAACTATTGTGCTCAGAGACACTGTTGATAAAAACTCAGGCAAAGAAAATGAAATGCATATTTG
CAAAGTGTATTAGGAAGTGTTTATGTTGTTTATAATAAAAATATATTTTCAACAGACAAAAAAAAAAAA
AAAAA
**SEQ ID NO: 22 - Protein sequence of human adenylate kinase 5 (A isoform 1 (taken from UniProt ID 095622 as at the filing date)**
**SEQ ID NO: 23 - Protein sequence of human adenylate kinase 5 isoform 2 (taken from UniProt ID 095622 as at the filing date)** 
MKSQKEGCCS RGDLSIQTGP GGEWAPRRLV SNVLIFSCTN IVGVCTHYPA
EVSQRQAFQE TRECIQARLH SQRENQQQER LLLSVLPRHV AMEMKADINA
KQEDMMFHKI YIQKHDNVSI LFADIEGFTS LASQCTAQEL VMTLNELFAR
FDKLAAENHC LRIKILGDCY YCVSGLPEAR ADHAHCCVEM GMDMIEAISL
VREVTGVNVN MRVGIHSGRV HCGVLGLRKW QFDVWSNDVT LANHMEAGGK
AGRIHITKAT LNYLNGDYEV EPGCGGERNA YLKEHSIETF LILRCTQKRK
EEKAMIAKMN RQRTNSIGHN PPHWGAERPF YNHLGGNQVS KEMKRMGFED
PKDKNAQESA NPEDEVDEFL GRAIDARSID RLRSEHVRKF LLTFREPDLE
KKYSKQVDDR FGAYVACASL VFLFICFVQI TIVPHSIFML SFYLTCSLLL
TLVVFVSVIY SCVKLFPSPL QTLSRKIVRS KMNSTLVGVF TITLVFLAAF
VNMFTCNSRD LLGCLAQEHN ISASQVNACH VAESAVNYSL GDEQGFCGSP
 
WPNCNFPEYF TYSVLLSLLA CSVFLQISCI GKLVLMLAIE LIYVLIVEVP
GVTLFDNADL LVTANAIDFF NNGTSQCPEH ATKVALKVVT PIIISVFVLA
LYLHAQQVES TARLDFLWKL QATEEKEEME ELQAYNRRLL HNILPKDVAA
HFLARERRND ELYYQSCECV AVMFASIANF SEFYVELEAN NEGVECLRLL
NEIIADFDEI ISEDRFRQLE KIKTIGSTYM AASGLNDSTY DKVGKTHIKA
LADFAMKLMD QMKYINEHSF NNFQMKIGLN IGPVVAGVIG ARKPQYDIWG
NTVNVASRMD STGVPDRIQV TTDMYQVLAA NTYQLECRGV VKVKGKGEMM
TYFLNGGPPL S
**SEQ ID NO: 24 - mRNA of human adenylate kinase 5 (taken from NK_174858 as at the filing date)** 
GCCACGCTGTCCTCCCTCCTCCGCTCCCTGCGCCCCACCCCTGGCGGCCGCGCTGCCTGGCAGCCCGGGA
AGCCGCGGCACAGCTGCTCGGCGCCTGCAGCTCCGGCTCGGGGGCTGGAACCGAAGCGGGGGCGGCGGGA
GCGCGGAGACCACAGCCCCCGGGGAGAGGCGGAGGGGGTCCCTGGCCTGGGCGGAGAGGCTGAGCTGAGT
GCGCGTGAGAAAGAGGGCTGCACCGCTGCTCGGCGCGGACTCTGCCAGCCCCAGCTTCAGCCCCGGCTCA
GGTCGCCGCAGCCCGGGAGCCTCCCCGCTTGCGCCCCAAGGCACGCGCGGCACAGCCATGAACACCAACG
ATGCCAAGGAGTATCTGGCCCGGAGGGAAATCCCTCAGCTTTTTGAGAGCCTTTTGAATGGACTGATGTG
TTCTAAGCCCGAAGATCCAGTAGAATACTTGGAAAGTTGTTTACAAAAAGTAAAGGAACTGGGTGGCTGT
GACAAGGTGAAATGGGATACATTTGTAAGCCAGGAAAAGAAGACCTTACCTCCACTAAATGGAGGACAGT
CACGGAGATCCTTTCTAAGAAATGTAATGCCTGAAAACTCAAACTTTCCATATCGGCGGTATGACCGGCT
CCCTCCAATCCATCAATTCTCCATAGAAAGTGACACGGATCTCTCTGAGACTGCAGAGTTGATTGAGGAG
TATGAGGTTTTTGATCCTACCAGACCTCGACCAAAAATCATTCTTGTTATAGGTGGTCCAGGAAGTGGAA
AGGGTACTCAGAGTTTGAAAATTGCAGAACGATATGGATTCCAATACATTTCTGTGGGAGAATTATTAAG
AAAGAAGATCCACAGTACCAGCAGCAATAGGAAATGGAGTCTTATTGCCAAGATAATTACAACTGGAGAA
TTGGCCCCACAGGAAACAACAATTACAGAGATAAAACAAAAATTGATGCAAATACCTGATGAAGAGGGCA
TTGTTATTGATGGATTTCCAAGAGATGTTGCCCAGGCTCTATCTTTTGAGGACCAAATCTGTACCCCCGA
TTTGGTGGTATTCCTGGCTTGTGCTAATCAGAGACTCAAAGAAAGATTACTGAAGCGTGCAGAACAGCAG
GGCCGACCAGACGACAATGTAAAAGCTACCCAAAGGAGACTAATGAACTTCAAGCAGAATGCTGCTCCAT
TGGTTAAATACTTCCAGGAAAAGGGGCTCATCATGACATTTGATGCCGACCGCGATGAGGATGAGGTGTT
CTATGACATCAGCATGGCAGTTGACAACAAGTTATTTCCAAACAAAGAGGCTGCAGCAGGTTCAAGTGAC
CTTGATCCTTCGATGATATTGGACACTGGAGAGATCATTGATACAGGATCTGATTATGAAGATCAGGGTG
ATGACCAGTTAAATGTATTTGGAGAGGACACTATGGGAGGTTTCATGGAAGATTTGAGAAAGTGTAAAAT
TATTTTCATAATTGGTGGTCCTGGCTCTGGCAAAGGCACACAGTGTGAAAAGCTGGTGGAAAAATATGGA
TTTACACATCTCTCAACTGGCGAGCTCCTGCGTGAGGAACTGGCATCAGAATCTGAAAGAAGCAAATTGA
TCAGAGACATTATGGAACGTGGAGACCTGGTGCCCTCAGGCATCGTTTTGGAGCTCCTGAAGGAGGCCAT
GGTGGCCAGCCTCGGGGACACCAGGGGCTTCCTGATTGACGGCTATCCTCGGGAGGTGAAGCAAGGGGAA
GAGTTCGGACGCAGGATTGGAGACCCACAGTTGGTGATCTGTATGGACTGCTCGGCAGACACCATGACCA
ACCGCCTTCTCCAAAGGAGCCGGAGCAGCCTGCCTGTGGACGACACCACCAAGACCATCGCCAAGCGCCT
AGAAGCCTACTACCGAGCGTCCATCCCCGTGATCGCCTACTACGAGACAAAAACACAGCTACACAAGATA
AATGCAGAGGGAACACCAGAGGACGTTTTTCTTCAACTCTGCACAGCTATTGACTCTATTTTCTGAAGGC
AAAAATGCATGTTTGTTAGAATGGAAACAGAAAAACATTAAAAAGTTCATTCCTTAACACAATGTTTCAA
GTTAAACCTTTTGTGTCACCGCCCCCACCAACCACCACCTCCTAAATCCTGACAGCACTGTTTGCTTCCC
AGCTAGACCTGTGTGAGAGGTGTCTGGAAATCATGCATGGTGTATTTGGGACTATATCAACCTATTCTCC
ACACTTCAGACAACTGTCTGCACTCACGGCACACACACTTTGTATCATGCAGGCCACACTCAGAGCTAGT
CAGTACATGAACAGTGGTGCGGTGCCAGTCTGTGTCCGTTGTGATCACAGGCCTTGCTAGACCCTGATCA
TCTGGTTCTCCTCTCATTAAGCATCCCTAACCCCCAGTCACACCTTCCTCTTACATACTGTTCCCCAATG
GAGGCCCCTGGCATAGGGGACAGCCCTGGGCATCTTCCTTTGGTGTCTGGCTGTTTTGTCAACTCTCATC
CACTGGTGGCTCAGAGCCATAAGGTGGGTTGATTACACAATGCCTTGTACATGATATAGAGGCATCAAGC
AAGTAAAATTTGACAGAAATTTTAAAATATGAAGATGTATAGCTTTCCCAAGATGATGGTAAAACCCAGG
TTAGTCATCAGTAACCTTCTCTATTATTATTATTTTTTAGAAACTTGGAATACTGTCGTCATGGCTAAGA
GAACAAATCTGATAAATTGTGTAACCTAGTCTCTTCTCTACATGGTGATGCATTTCAGCAATTATAAATT
AATATAAATGACCAAAAGTAACTTAAAAGCATGAGATATTTGCTATTTCATTCATTGGGCACATATCAAA
TTATAATTTTGATTTTAAATGGTCACCCATGTATTTATTTGTTGCCAAGCAAGTAAAAAAATACCCTAAC
AAACCTGATGTGGGTGGGAGGGGCATGTCAGTAAGTGGTGTGTTCAATGTGTTTGTTTCATATGGCCCCT
TTCCAGGAGTTTGCAAACCTTGTCATACCCATATGCAAAACTGTGTTTCCTTGCATTAAACCAGTGAAGT
TTGGGTTCTCTTTTGTGCTATCAATCAGTTGTAAAATCAGAGCTGCTTATATATTCTACTGGAATAACTG
CATCTTCCACTCAGTCACTACAAAAAAGCATAGTTTCAGTTTGCATGAATTTTTTTTTTTTTCTTCAATG
GCTGTGCAGATAAGGATCCATTTCTGGGATAGAATTGTATTTTTTAAGTCATTTTTTTTTCTTGAAATGG
 ATATGTACAAATAAAATAAATGGAAGACAGGATAAA
**SEQ ID NO: 25 - mRNA of human adenylate kinase 5 (taken from NM_012093 as at the filing date)** 
AGTCTTCCAGCAGGGTTCTGAAAGCAGCCTGCTCCCATCGCGCCCCTCGGATGTGGACACCCAGGGTGGG
CTGCAGACCGCGGCCGGGCCGCACTCTCTGGGGGCGAGGGCCAGGTCAGGTGGCTGCACTGTCGCCCAGC
AGCGAGTAAAGACTCGAAACTGAGGGAGCCAGAGCCCTAGTCACTCTGCTGGTCGAGGGTCGGAACCGCT
GGGGGAAAGAGGAAATGTGGACGAAGGCAGCCCTTCCTGGAGACAGCTTGGAAGAGAGGGATGAAGATGC
AAAAAGCAAAACCCAACCTATCAAGTGCCACCTCCCCGGTGACAGGCCCCCAGCCTTGCTCTGCGCTTTC
GCTGAATGAACTCCTGAGCACGTTTTTCCCGTTGCCTAGGAAGGGTTGGGCCGCAGGTTTCGCCAGTGCT
CGACAACCCACCTGGAGATAGCGGGTCGGGGAGGGAGCTGCGAGGGGGGCGGACTCCTGTTGCTCAGTCT
TCAGACCGCAGCACAGCATCATCTGCACCGGGACCCGGGAATGGGGGGACACTTGGAGACTGAGAGAGAG
GAGGCTCGAGGAATGAACCTTGGTCTGTTCTAAGCCACAACCCCTGTCTCAGGTCGTTTGGCTTCCTTTC
CTTCTCTTTGTATTTGAATTTAAAAACATTCCTGAATCTCAAGTGCTTTTGCTTGAAGTAGGGGGGAGGA
GGAGAAAAGAATAGAAGCAAGCCAGGCTTCCTAAGCGAGCTCTGAAAGGAGAAATCCATTCACTGCAAAC
CTCGTTCCCCATTTCTGAAAACGGATAGACTGATATCGTAAACTAAAGGTCATGAGGGTTTTTCCCCCCG
GTTTGGGGGATTTGTTCTGTTAAAAGGAATTTTAAAGCACCACTTGAGAAAATAATTTTGCCTCAGTTTC
CCTAAACTATCTTTAAAATGGGTTGCCATAATTACAGGTCTTCTTTTCTCTCACCTCTATAAGCCACCGT
TTTCAGGTCATCTGAAGTCAAGATTTCCAGCCTTTTGAATGGACTGATGTGTTCTAAGCCCGAAGATCCA
GTAGAATACTTGGAAAGTTGTTTACAAAAAGTAAAGGAACTGGGTGGCTGTGACAAGGTGAAATGGGATA
CATTTGTAAGCCAGGAAAAGAAGACCTTACCTCCACTAAATGGAGGACAGTCACGGAGATCCTTTCTAAG
AAATGTAATGCCTGAAAACTCAAACTTTCCATATCGGCGGTATGACCGGCTCCCTCCAATCCATCAATTC
TCCATAGAAAGTGACACGGATCTCTCTGAGACTGCAGAGTTGATTGAGGAGTATGAGGTTTTTGATCCTA
CCAGACCTCGACCAAAAATCATTCTTGTTATAGGTGGTCCAGGAAGTGGAAAGGGTACTCAGAGTTTGAA
AATTGCAGAACGATATGGATTCCAATACATTTCTGTGGGAGAATTATTAAGAAAGAAGATCCACAGTACC
AGCAGCAATAGGAAATGGAGTCTTATTGCCAAGATAATTACAACTGGAGAATTGGCCCCACAGGAAACAA
CAATTACAGAGATAAAACAAAAATTGATGCAAATACCTGATGAAGAGGGCATTGTTATTGATGGATTTCC
AAGAGATGTTGCCCAGGCTCTATCTTTTGAGGACCAAATCTGTACCCCCGATTTGGTGGTATTCCTGGCT
TGTGCTAATCAGAGACTCAAAGAAAGATTACTGAAGCGTGCAGAACAGCAGGGCCGACCAGACGACAATG
TAAAAGCTACCCAAAGGAGACTAATGAACTTCAAGCAGAATGCTGCTCCATTGGTTAAATACTTCCAGGA
AAAGGGGCTCATCATGACATTTGATGCCGACCGCGATGAGGATGAGGTGTTCTATGACATCAGCATGGCA
GTTGACAACAAGTTATTTCCAAACAAAGAGGCTGCAGCAGGTTCAAGTGACCTTGATCCTTCGATGATAT
TGGACACTGGAGAGATCATTGATACAGGATCTGATTATGAAGATCAGGGTGATGACCAGTTAAATGTATT
TGGAGAGGACACTATGGGAGGTTTCATGGAAGATTTGAGAAAGTGTAAAATTATTTTCATAATTGGTGGT
CCTGGCTCTGGCAAAGGCACACAGTGTGAAAAGCTGGTGGAAAAATATGGATTTACACATCTCTCAACTG
GCGAGCTCCTGCGTGAGGAACTGGCATCAGAATCTGAAAGAAGCAAATTGATCAGAGACATTATGGAACG
TGGAGACCTGGTGCCCTCAGGCATCGTTTTGGAGCTCCTGAAGGAGGCCATGGTGGCCAGCCTCGGGGAC
ACCAGGGGCTTCCTGATTGACGGCTATCCTCGGGAGGTGAAGCAAGGGGAAGAGTTCGGACGCAGGATTG
GAGACCCACAGTTGGTGATCTGTATGGACTGCTCGGCAGACACCATGACCAACCGCCTTCTCCAAAGGAG
CCGGAGCAGCCTGCCTGTGGACGACACCACCAAGACCATCGCCAAGCGCCTAGAAGCCTACTACCGAGCG
TCCATCCCCGTGATCGCCTACTACGAGACAAAAACACAGCTACACAAGATAAATGCAGAGGGAACACCAG
AGGACGTTTTTCTTCAACTCTGCACAGCTATTGACTCTATTTTCTGAAGGCAAAAATGCATGTTTGTTAG
AATGGAAACAGAAAAACATTAAAAAGTTCATTCCTTAACACAATGTTTCAAGTTAAACCTTTTGTGTCAC
CGCCCCCACCAACCACCACCTCCTAAATCCTGACAGCACTGTTTGCTTCCCAGCTAGACCTGTGTGAGAG
GTGTCTGGAAATCATGCATGGTGTATTTGGGACTATATCAACCTATTCTCCACACTTCAGACAACTGTCT
GCACTCACGGCACACACACTTTGTATCATGCAGGCCACACTCAGAGCTAGTCAGTACATGAACAGTGGTG
CGGTGCCAGTCTGTGTCCGTTGTGATCACAGGCCTTGCTAGACCCTGATCATCTGGTTCTCCTCTCATTA
AGCATCCCTAACCCCCAGTCACACCTTCCTCTTACATACTGTTCCCCAATGGAGGCCCCTGGCATAGGGG
ACAGCCCTGGGCATCTTCCTTTGGTGTCTGGCTGTTTTGTCAACTCTCATCCACTGGTGGCTCAGAGCCA
TAAGGTGGGTTGATTACACAATGCCTTGTACATGATATAGAGGCATCAAGCAAGTAAAATTTGACAGAAA
TTTTAAAATATGAAGATGTATAGCTTTCCCAAGATGATGGTAAAACCCAGGTTAGTCATCAGTAACCTTC
TCTATTATTATTATTTTTTAGAAACTTGGAATACTGTCGTCATGGCTAAGAGAACAAATCTGATAAATTG
TGTAACCTAGTCTCTTCTCTACATGGTGATGCATTTCAGCAATTATAAATTAATATAAATGACCAAAAGT
AACTTAAAAGCATGAGATATTTGCTATTTCATTCATTGGGCACATATCAAATTATAATTTTGATTTTAAA
TGGTCACCCATGTATTTATTTGTTGCCAAGCAAGTAAAAAAATACCCTAACAAACCTGATGTGGGTGGGA
GGGGCATGTCAGTAAGTGGTGTGTTCAATGTGTTTGTTTCATATGGGCCCTTTCCAGGAGTTTGCAAACC
TTGTCATACCCATATGCAAAACTGTGTTTCCTTGCATTAAACCAGTGAAGTTTGGGTTCTCTTTTGTGCT
 
ATCAATCAGTTGTAAAATCAGAGCTGCTTATATATTCTACTGGAATAACTGCATCTTCCACTCAGTCACT
ACAAAAAAGCATAGTTTCAGTTTGCATGAATTTTTTTTTTTTTCTTCAATGGCTGTGCAGATAAGGATCC
ATTTCTGGGATAGAATTGTATTTTTTAAGTCATTTTTTTTTCTTGAAATGGATATGTACAAATAAAATAA
ATGGAAGACAGGATAAA
**SEQ ID NO: 26 - protein sequence of V-type proton ATPase subunit d 2 (taken from UniProt Q8N8Y2 as at the filing date)** 
MLEGAELYFN VDHGYLEGLV RGCKASLLTQ QDYINLVQCE TLEDLKIHLQ
TTDYGNFLAN HTNPLTVSKI DTEMRKRLCG EFEYFRNHSL EPLSTFLTYM
TCSYMIDNVI LLMNGALQKK SVKEILGKCH PLGRFTEMEA VNIAETPSDL
FNAILIETPL APFFQDCMSE NALDELNIEL LRNKLYKSYL EAFYKFCKNH
GDVTAEVMCP ILEFEADRRA FIITLNSFGT ELSKEDRETL YPTFGKLYPE
GLRLLAQAED FDQMKNVADH YGVYKPLFEA VGGSGGKTLE DVFYEREVQM
NVLAFNRQFH YGVFYAYVKL KEQEIRNIVW IAECISQRHR TKINSYIPIL
**SEQ ID NO: 27 - mRNA sequence of V-type proton ATPase subunit d 2 (taken from NM_152565 as at the filing date)** 
GCAAATCTTCAGGGGCCGTCCAGGACTACAGAGCTGTTTCACCCTACCTTGGCTTCAATCTCTTCCCCCA
TGCTCGAAGGTGCGGAGCTGTACTTCAACGTGGACCATGGCTACCTGGAGGGCCTGGTTCGAGGATGCAA
GGCCAGCCTCCTGACCCAGCAAGACTATATCAACCTGGTCCAGTGTGAGACCCTAGAAGACCTGAAAATT
CATCTCCAGACTACTGATTATGGTAACTTTTTGGCTAATCACACAAATCCTCTTACTGTTTCCAAAATTG
ACACTGAGATGAGGAAAAGACTATGTGGAGAATTTGAGTATTTCCGGAATCATTCCCTGGAGCCCCTCAG
CACATTTCTCACCTATATGACGTGCAGTTATATGATAGACAATGTGATTCTGCTGATGAATGGTGCATTG
CAGAAAAAATCTGTGAAAGAAATTCTGGGGAAGTGCCACCCCTTGGGCCGTTTCACAGAAATGGAAGCTG
TCAACATTGCAGAGACACCTTCAGATCTCTTTAATGCCATTCTGATCGAAACGCCATTAGCTCCATTCTT
CCAAGACTGCATGTCTGAAAATGCTCTAGATGAACTGAATATTGAATTGCTACGCAATAAACTATACAAG
TCTTACCTTGAGGCATTCTATAAATTCTGTAAGAATCATGGTGATGTCACAGCAGAAGTTATGTGTCCCA
TTCTTGAGTTTGAGGCCGACAGACGTGCTTTTATCATCACTCTTAACTCCTTTGGCACTGAATTGAGCAA
AGAAGACCGAGAGACCCTCTATCCAACCTTCGGCAAACTCTATCCTGAGGGGTTGCGGCTGTTGGCTCAA
GCAGAAGACTTTGACCAGATGAAGAACGTAGCGGATCATTACGGAGTATACAAACCTTTATTTGAAGCTG
TAGGTGGCAGTGGGGGAAAGACATTGGAGGACGTGTTTTACGAGCGTGAGGTACAAATGAATGTGCTGGC
ATTCAACAGACAGTTCCACTACGGTGTGTTTTATGCATATGTAAAGCTGAAGGAACAGGAAATTAGAAAT
ATTGTGTGGATAGCAGAATGTATTTCACAGAGGCATCGAACTAAAATCAACAGTTACATTCCAATTTTAT
AACCCAAGTAAGGTTCTCAAATGTAGAAAATTATAAATGTTAAAAGGAAGTTATTGAAGAAAATAAAAGA
AATTATGTTATATTATCTAGACTACACAAAAGTAAGCCACACTATATCTTCATGAGTTGCAAATCCATGG
AAACACAGTAAACCAGCCCTGAAACAAAGCATTTCCTTGTTTTCAGTGGTATTAGATCTTGTTTCCACAT
GTCTGTCTCATTCTTCACTGGGCCTTACAGGTTAGTTTTAATTAACTCTATGGTATTTTTCTTATTCTTG
TTTGATCATGTTAAAAATTGGACCTAATAAAAGTATTTTATTCTTGCTTTTCCATGCTTCTCTACAGGTC
CAAATACTGAATGTCTCCTTTACTTTTTCTCTTTTAAATTTTTTTCTAGACAGGGTCTCACTCTGTCACC
TAGGCTACAGTGCAGTGGTGTGATCACAGCTCACTGCAGCCTCGACTTCCCAGGCTCAAGTGATCCTCCC
AGCTCTCAGCCTCCAAAGTAGCTGGCACTACAAGTGTACACCCCCACACAAGGCTAAGTTTTGTATTTTT
TGTAGAGACAGGGTTTCAACATATTATCCAGGCTGGTGTCGAATTCCTGGGCTCCAGGGATCCACAGTCC
CCCTTGGCCTCCCAAAGTGTTGGGATTACATGCATGAGCCACTGTGCTGGGCTTCATTTACATTTTAACT
GTCTGTTCCTTGCCTAGATTCACAGAAATCCAAAGCTGTATGTAGTCAACATGGTTCACAAGTGTTGGAA
AATGTGTTTTTTGTTTTGTTTTGTTTTGTTTCGTTTTGTTTTGAGACAGAGTTTCCCTCTGTCGCCCAGG
CTAGAGTGCAATGGCGTGATCTCGGCTCACTGCAACCTCCACCTCCCAGATTCAAGCAACTCTCTGCCTC
AGCCTCCCGAGTAGCTGGGATTACAAGCACCCACCACTACACTCAGCTAATTTTTTGTATTTTTAGTAGA
GCCGGGGTTTCACCATCTTGGCCAGGCTGATCTTGAACTCCTGAGCTCATGATCCACCCGCCTCAGCCTC
CCAAAGTGCTGGGATTACAGGCCCCTTGTTCAGCCACTGCACCTGGCCCCTTATTTTGTTTTTGTTTTCT
AATATACTTTGATGTAATCAGCTTGAGAAAGCAACACAATTTCAAATCCTATCTTCTAGATGCAAGCAGT
GTTAAATTTGTTAATAAATTTGCTTTTCACACCTTTCTTTAAATAAAAGGTATATCTCTC
**SEQ ID NO: 28 - complete protein sequence of DIRAS family, GTP binding RAS-like 2 (taken from UniProt Q96HU8 as at the filing date)** MPEQSNDYRV AVFGAGGVGK SSLVLRFVKG TFRESYIPTV EDTYRQVISC 
DKSICTLQIT DTTGSHQFPA MQRLSISKGH AFILVYSITS RQSLEELKPI
YEQICEIKGD VESIPIMLVG NKCDESPSRE VQSSEAEALA RTWKCAFMET
SAKLNHNVKE LFQELLNLEK RRTVSLQIDG KKSKQQKRKE KLKGKCVIM
**SEQ ID NO: 29 - mRNA sequence of DIRAS family, GTP binding RAS-like 2 (taken from NM_017594 as at the filing date)** 
ATTTAAGCAGGAGCAGCAGCCTCTGCAGCAGTTGGCACATCTGAGAAGGCAGAGATTGAAGAGAGTAGGA
GGAGCGTGCAGCGGCGGTGACAGTGCCAGGCCGGAGCGGCTCAGGCCAGCGCAGGTGCAGGGGCTGCGAG
CGGGTGGAGCGCGAGGGCGCTGGGCGCAGGGAGCACGGCCGGTGGGCGGTGCAGGGCGCACGGCGGGCGA
GCAGCTGACAGCATCGTCACGGCGGCGGGAGGGAGTGCGCTGCGCCTGCCTCCGGGAGGAGCCGCATCCA
CACACCCTGCGCTGCCCTGTCCTGCGCGAGTGGAGCTCTGAAGAAGCTCTGAGCGGAGTTGTGTTCTTCC
CCAGGTGCGTCCTGGCTGAGAGTTGGAGCTCTCCAGCAACATGCCTGAGCAGAGTAACGATTACCGGGTG
GCCGTGTTTGGGGCTGGCGGTGTTGGCAAGAGCTCCCTGGTGTTGAGGTTTGTGAAAGGCACATTCCGGG
AGAGCTACATCCCGACGGTGGAAGACACCTACCGGCAAGTGATCAGCTGTGACAAGAGCATATGCACATT
GCAGATCACCGACACGACGGGGAGCCACCAGTTCCCGGCCATGCAGCGGCTGTCCATCTCCAAAGGGCAC
GCCTTCATCCTGGTGTACTCCATTACCAGCCGACAGTCCTTGGAGGAGCTCAAGCCCATCTACGAACAAA
TCTGCGAGATCAAAGGGGACGTGGAGAGCATCCCCATCATGCTGGTGGGGAACAAGTGTGATGAGAGCCC
CAGCCGCGAGGTGCAGAGCAGCGAGGCGGAGGCCTTGGCCCGCACATGGAAGTGTGCCTTCATGGAGACC
TCAGCCAAGCTCAACCATAACGTGAAGGAGCTTTTCCAGGAGCTGCTCAACCTGGAGAAGCGCAGGACCG
TGAGTCTCCAGATCGACGGGAAAAAGAGCAAGCAGCAGAAAAGGAAAGAGAAGCTCAAAGGCAAGTGCGT
GATCATGTGAAGGCCCTTCCTGCGGGAGGAGCAGCTGTGTGTCCCCGGCACCTCACTCCCCCAAAATGAC
ACCCACCGTCGTCAGGGTAGCATGTATAATGCCCACGTGTTAAACATTGCATTTAATCGAGATGCGTCCT
ATTGTCCTTAAGAGGGCGTTTCACACCACCAACAGTAAGCCACCCACTCTGGAGTCACAGAATCTGCCAG
GCGGTTCAAGTGAAAACCAACACACTCAGCATCCCTGGGAACTGAGAGGTGCCAGCAATTGCTGAAGGTG
GCGATGAACACCCGAAGGTGGGAGGGAGGACTGGTACCCACAAAGCAACATGTACCGAGAGGACTAAATG
TCATCTACGTGCATGTGAGAGCGTGTTAACCTAGAGTTACCTGCACCAACCCCAGACAGAAGCCAATCAC
ATCTTTGGGGGAGGGGAGGGGCAGGAAGAGGTGAGAAGATCAGATGGTCCAAAGTGGACCACACTTGGTC
CATTTTACACTTTTTTAAAGGGGATTAAAAAACACAGCCTCTCCCCCAAAGGGTGTCCGTTCTTAATTCC
CACCTGGCCTGTTAGGAGCCTTGCTACCCTGAGGGGATGTGTTCACCTTACCTAGACCTAGTTAGGAAGT
ATCATTTTAAGCTATTAGAGTATTTATCTTCATGTGCAGGGATAAGTGCACTAACAGTGTGCTGCTCTGT
CGGAAGTTCTTCAGTTTTTAAGTGAGGATATCGTGACAGTATTAAAACATCGCAATAATGTTCCTGTGTG
TTATACATCGAGGGTTTTAGAAATGTGATTTTCTTCTTTTGACCTGTGAGGAGTATAACTTCTTTCAGCC
CTCAGATTTTAAATACAAGCAAATAAACTCACTATTTTTAGACGTTTTTTTCCTCCAAGGTGGTTTTCTT
CTCTTAAATAACTCGATCTGTACCCAGCTGGGTAGCAGCCAGCAAAGGCCATCAGACAACCAGAAGCACA
TCCATTTTTGTAGTGTCACAAACATGTATATGCCACACTTTGCACCTTAATGAAATACTTTGAAACAGAA
GTTATTCACTGTGTTTTTGATGATCTATCTGTATTGGAAATATGTTCCTGGAAAATGCATTTAAATAATA
GTAAATTCTCTTGCATGTTCCATTATACGTGTCTTCTAAGAGCTGTTCAATACAGTATTCACTCTAGAAA
CAATTATCTTTTTCTCTTAATGATTTTGTGTGCATCTTTAATCTTTCAAGCCAAATTACAGCTATTTCAG
GTTTCCTGTGTTAGCTTGGGGATAGGATGGTGGCTGGAGACAGGCAGGCTTCTCTGCCCTGGGAAGAGCC
CACTCAGCTTAATTGCTCTGCCATCGTAGAGCCTGGTTGGACTTGGCTTCCTGAAAACTCCCACTGATAG
TGCCTGTTAGATCTCCTGTTTGTTTCAGTTGGCAGAACATTTACTGGCCCCAACTGTGGCATCATCCTCT
CAGCAGTCTTCCTGTCACCCGCCTGGCAGGCAGAAGGAGCTGCAGTCCCACGTGGGCCTGCCTGGGGGGG
TGGGGGCTGCATGGCTGTTGGGTGGCAGTGTCAGCACAGGGAGGGCTTAAGTTGGGGATGTTTGACCAGG
CCACCTCCTGCAACTGCTGTTTCTCCTGTCCCTCCTATGCAGGGCTTGCAGCAGCAGCAGTGTGGCCATC
TCCATCCCCCAAAGCACACTTGCTCTCTCAATATGTCCTAGTTTTCTTCAGCCTTTTCTGGTTCAGTTCC
CTTGTCCTGATCTCATCCTCTCTGGTCTCCCAATAACTCACCCTTGGGATGTGTTTAGAGCGTGGGAGGT
GCCTTTGAGAACTGCTTGACTCCATGATCTCCTAGAACAAAACCGCCCTGACTTTACAGGGGGAACACTC
ATGCTGAGCTGAGAAAGCAGAGAAGTGGCGTGGGAGCCAGCTGGGGGTGAAGAGCATTTGGGCCAGTCCC
GTGGCCCCCTTCAGATTCCTCAAGCAGGATTGTTCTGTTCTAAAAAGCTGTTGCACAGCATTCGCAATGA
GATCTTTAGTTGGCGGATTTTCTGGAACATTTGTTTTTCAACTTGTCCCGACATTTTTTTTCTGTTTCTA
TTCTGAGAGAGAGATGATCAAGTTTTAATTTGGGTATAGGTTAAATGGAAGAAGAAACAGAACTTCATGG
CCAAAGTAGACCTATAGATTTTGATTGGGTTCTTTGTTAACAGTAGAATGCGATCTTTGCCACTGACTGT
AGTATTAATAAGGTTTTAATGTGAGATATTCCTGCAAACCATCCCATTTCTACTGATTGTAAGTCAGAAT
TTCTTTTATCCCTTTCAAATCAGTTTCTACATGTTTAAGTGTTCAGGGCTTCATCAGCATGAGAAGTTTG
TAATTACTGAAAGTCTGATTTCATTCAGGACACATTTTTTCCTTCATATTTTTTCTGTGAATTTATAGGC
TAGGAAGGCTATTGAAGCCTCAATTATGGGTCTTCATTTTGAGATCGTTTTCTATGAGCTGAACTGAGGA
TATCAATGGTTATCTCAAAATCGTCTTTTAGGAGATCCCCAATTGACTCAGAGTTTGAGGAGTTAGTATC
 
ACAGAATTAGATTTTTTTAAAGCATTTGTACGTTTCCATTCCCAAATATGTAGCTGTGGTTCTTGAAAAC
ACATCCTACATTGCATATGGGCATAGCAGTTTTTGACCCAGGCAGAATAAGTTAATATTTAATTAAATAT
TGCTTTGAAGATGGCGCTCTGGGCATGAGCATGGGGCTCCATGACTTCCCTTCTATCCCCATGAGCCCCT
CCTCCATCCAGCGACAAGCCATGGGCATGCATACAATGCAGCAAGACCAACACAAGAGCAATATTGAATT
GTTCATTCTATCTAAAATTACATGTATATAAAATATATAATTTATCTTCCTGCATTTTTGAAGTATAAAG
TCATAAATTGTACATATCTGTAAGCTAGTATATTTGTTTCACTGTTTGTAATATTTAAGAAATGCTCATT
CTTTGTAGAACAAAAATGTATTAAATATTTTAAAAATTGCTCTGTGATACTTAATTTTTTTCCCCAAAAT
TTGTAATGTGTTGCTTCTACATAAGTTCTCTGGAAATATCTACAACTAATAGGACACATGTAAATCCTTG
AAGACACATCCTGGAATTCATACCCCACAAGGACAGTGTGTATACAAAGTATTTGCAGAGCATGACTTTT
ATATGTGTGGGATATCAATGTGTATATTTATATTTAAAGTGTATTTATTGTTACAAGTCTATTCTCTATT
ATATTTTATTTACTCTGCGGTTATAAAAATCACCCTTGCATACAAGTTTCTAGTTGCCAGTGATGTTCTG
GAAATAATGGGAGATATTACAATAAAGCTACAGTTATGACACCCTG
**SEQ ID NO: 30 - protein sequence of syntaxin binding protein 5-like isoform 1 (taken from UniProt Q9Y2K9 as at the filing date)** 
MKKFNFRKVL DGLTASSPGS GSSSGSNSGG GAGSGSVHPA GTAGVLREEI
QETLTSEYFQ ICKTVRHGFP HQPTALAFDP VQKILAIGTR TGAIRILGRP
GVDCYCQHES GAAVLQLQFL INEGALVSAS SDDTLHLWNL RQKRPAILHS
LKFNRERITY CHLPFQSKWL YVGTERGNTH IVNIESFILS GYVIMWNKAI
ELSTKTHPGP VVHLSDSPRD EGKLLIGYEN GTVVFWDLKS KRAELRVYYD
EAIHSIDWHH EGKQFMCSHS DGSLTLWNLK SPSRPFQTTI PHGKSQREGR
KSESCKPILK VEYKTCKNSE PFIIFSGGLS YDKACRRPSL TIMHGKAITV
LEMDHPIVEF LTLCETPYPN EFQEPYAVVV LLEKDLIVVD LTQSNFPIFE
NPYPMDIHES PVTCTAYFAD CPPDLILVLY SIGVKHKKQG YSNKEWPISG
GAWNLGAQTY PEIIITGHAD GSIKFWDASA ITLQMLYKLK TSKVFEKQKV
GEGKQTCEIV EEDPFAIQMI YWCPESRIFC VSGVSAYVII YKFSRHEITT
EIVSLEVRLQ YDVEDIITPE PETSPPFPDL SAQLPSSRSL SGSTNTVASE
GVTKDSIPCL NVKTRPVRMP PGYQAELVIQ LVWVDGEPPQ QITSLAVSSA
YGIVAFGNCN GLAVVDFIQK TVLLSMGTID LYRSSDLYQR QPRSPRKNKQ
FIADNFCMRG LSNFYPDLTK RIRTSYQSLT ELNDSPVPLE LERCKSPTSD
HVNGHCTSPT SQSCSSGKRL SSADVSKVNR WGPGRPPFRK AQSAACMEIS
LPVTTEENRE NSYNRSRSSS ISSIDKDSKE AITALYFMDS FARKNDSTIS
PCLFVGTSLG MVLIISLNLP LADEQRFTEP VMVLPSGTFL SLKGAVLTFS
CMDRMGGLMQ PPYEVWRDPN NIDENEKSWR RKVVMNSSSA SQEIGDHQYT
IICSEKQAKV FSLPSQTCLY VHNITETSFI LQANVVVMCS SACLACFCAN
GHIMIMSLPS LRPMLDVNYL PLTDMRIART FCFTNEGQAL YLVSPTEIQR
LTYSQEMCDN LQDMLGDLFT PIETPEAQNR GFLKGLFGGS GQTFDREELF
GEASAGKASR SLAQHIPGPG SIEGMKGAAG GVMGELTRAR IALDERGQRL
GELEEKTAGM MTSAEAFSKH AHELMLKYKD KKWYQF
**SEQ ID NO: 31 - protein sequence of syntaxin binding protein 5-like isoform 2 (taken from UniProt Q9Y2K9 as at the filing date)** 
MKKFNFRKVL DGLTASSPGS GSSSGSNSGG GAGSGSVHPA GTAGVLREEI
QETLTSEYFQ ICKFGMVFLI SPQH
**SEQ ID NO: 32 - mRNA sequence of syntaxin binding protein 5-like transcript 1(taken from NM_014980 as at the filing date)** 
CTGACTGCTCGGTTAGGACCTCGGAGAGCGCCAGGCGCCGCGACCAGAGGGCCCAGAGAAGCGGCCGGAG
CCCGCCTACCTCGGCCCCCTCAGCTTCCCGGGCTGGCAGGCGGCTAGAGGCGTCTGAGGAAGTGTTTAAA
ATGAAGAAGTTTAATTTCCGAAAAGTTTTGGATGGCTTAACTGCCTCCTCCCCTGGCAGTGGTAGCAGCA
GTGGCAGTAACAGTGGTGGTGGGGCTGGAAGTGGTTCCGTACATCCGGCGGGGACTGCAGGGGTTCTCAG
AGAGGAAATTCAGGAAACTTTGACTTCGGAGTATTTCCAGATTTGCAAGACAGTTCGGCATGGTTTTCCT
CATCAGCCCACAGCATTAGCCTTTGATCCAGTTCAGAAAATCTTGGCTATTGGGACGAGAACAGGTGCTA
TACGAATACTCGGGAGACCTGGTGTTGATTGCTATTGCCAACATGAAAGTGGTGCAGCTGTCCTACAGCT
CCAATTTTTGATCAATGAGGGTGCCTTGGTCAGTGCAAGTTCAGATGATACACTTCATTTGTGGAACCTT
 
AGACAAAAAAGGCCAGCCATACTCCATTCTCTTAAATTTAACCGGGAACGAATTACTTACTGTCATCTAC
CTTTCCAGAGTAAATGGCTTTATGTTGGAACAGAAAGAGGAAATACACATATTGTAAATATTGAATCTTT
CATTCTTTCTGGATATGTTATCATGTGGAACAAGGCAATTGAACTATCCACTAAGACTCATCCAGGTCCA
GTTGTACATTTAAGCGATAGCCCAAGAGATGAAGGCAAACTGCTAATAGGTTATGAAAATGGTACTGTAG
TATTCTGGGACTTGAAATCTAAAAGAGCAGAACTGAGAGTTTATTATGATGAGGCTATTCATTCAATTGA
TTGGCATCATGAGGGCAAACAGTTCATGTGCAGCCATTCAGATGGTAGTTTGACTTTATGGAACCTGAAA
AGCCCAAGTCGCCCTTTCCAGACCACAATTCCACATGGAAAAAGTCAAAGAGAAGGAAGAAAATCTGAAT
CTTGTAAACCAATTCTTAAAGTAGAATACAAGACCTGCAAAAACAGCGAACCATTCATAATATTCTCTGG
TGGGCTGTCCTATGACAAAGCTTGTAGAAGACCAAGTTTAACCATCATGCATGGAAAAGCAATTACAGTA
CTTGAAATGGATCATCCTATTGTTGAATTTCTAACTTTATGTGAAACGCCCTATCCAAATGAATTTCAAG
AACCCTATGCTGTCGTGGTACTTCTGGAGAAAGATCTCATTGTAGTTGATCTGACACAAAGCAATTTTCC
AATCTTTGAAAATCCATATCCCATGGACATTCATGAATCACCAGTTACATGCACAGCATACTTTGCAGAT
TGTCCTCCGGATTTGATTCTAGTACTGTATTCTATAGGAGTCAAGCATAAAAAACAAGGATACAGTAATA
AGGAGTGGCCAATCAGTGGAGGAGCTTGGAACCTTGGAGCACAAACATATCCAGAAATTATTATTACTGG
TCATGCAGATGGATCAATAAAATTTTGGGATGCTTCTGCAATAACTCTGCAGATGCTGTACAAGCTAAAA
ACTTCAAAAGTGTTTGAAAAACAGAAGGTAGGAGAAGGAAAACAAACATGTGAAATTGTAGAGGAAGACC
CATTTGCCATTCAGATGATTTACTGGTGTCCAGAGAGCAGAATATTCTGTGTATCAGGAGTCTCTGCATA
TGTCATAATTTATAAATTCAGCAGACATGAAATTACAACAGAAATAGTGTCATTAGAGGTACGACTTCAG
TATGATGTTGAAGATATTATTACCCCTGAACCAGAAACAAGTCCTCCGTTTCCAGATCTCTCAGCCCAGC
TTCCTTCTTCAAGGAGTCTTTCTGGGAGCACTAACACTGTTGCTAGTGAAGGAGTAACAAAGGACAGTAT
TCCATGCCTCAATGTGAAGACACGGCCAGTGCGAATGCCTCCAGGATATCAAGCAGAACTTGTTATTCAA
TTGGTGTGGGTAGATGGTGAACCTCCACAACAGATTACTAGTCTTGCTGTAAGCTCAGCATATGGAATAG
TTGCATTTGGGAACTGCAATGGGTTGGCTGTGGTGGATTTTATACAGAAGACAGTACTGTTAAGCATGGG
GACCATTGACCTATATAGATCAAGTGACTTATACCAGCGACAACCACGGTCTCCTCGAAAAAACAAACAG
TTCATTGCAGACAACTTTTGCATGCGTGGCCTGTCTAACTTTTATCCTGATTTAACGAAACGGATCCGTA
CTTCCTATCAGAGTTTAACTGAACTGAATGACAGTCCAGTTCCCCTAGAACTTGAGCGCTGCAAGTCTCC
TACCTCAGACCATGTAAATGGACACTGCACAAGTCCAACTTCTCAGAGTTGCAGTTCTGGAAAACGTCTT
TCTAGTGCCGATGTTTCAAAAGTAAATCGCTGGGGTCCTGGAAGACCACCATTTCGAAAGGCCCAGTCAG
CAGCCTGCATGGAGATTTCTTTACCAGTTACAACAGAAGAAAACCGAGAAAATTCCTATAATCGTTCTAG
AAGCTCTAGTATCTCCAGTATTGACAAAGATTCTAAAGAAGCAATTACAGCACTATACTTCATGGACTCC
TTTGCACGGAAAAATGACTCTACCATCTCTCCTTGTCTGTTCGTTGGAACCAGTCTGGGAATGGTGTTAA
TCATCTCCTTAAACCTACCATTAGCAGATGAACAAAGGTTTACAGAGCCAGTCATGGTATTGCCAAGTGG
TACATTCCTCTCATTGAAAGGAGCTGTGCTAACATTCTCCTGTATGGACCGAATGGGTGGATTAATGCAA
CCGCCATATGAAGTTTGGAGGGATCCAAACAACATAGATGAAAATGAAAAATCTTGGAGAAGGAAAGTGG
TAATGAACTCATCTTCTGCATCCCAAGAAATAGGAGATCATCAGTATACAATAATCTGCTCAGAAAAACA
AGCCAAAGTCTTCTCACTGCCTTCTCAGACTTGCCTTTATGTTCATAACATCACGGAGACATCTTTTATA
CTGCAAGCAAATGTGGTGGTCATGTGTAGCAGTGCCTGCTTGGCATGCTTTTGTGCTAACGGACATATCA
TGATAATGAGCCTACCTAGTCTTCGCCCAATGTTGGATGTTAATTATTTGCCACTGACAGACATGAGGAT
AGCACGAACATTTTGTTTTACCAATGAAGGACAGGCATTATACCTCGTCTCTCCTACTGAAATTCAGCGG
TTAACATACAGCCAAGAAATGTGTGATAATCTACAGGACATGCTAGGAGATTTGTTTACTCCCATAGAGA
CACCAGAAGCTCAAAACAGAGGCTTTCTCAAGGGACTGTTTGGTGGAAGCGGACAAACATTTGACAGAGA
AGAGCTCTTTGGGGAAGCTTCGGCAGGAAAAGCATCCCGCAGCCTTGCGCAACACATTCCTGGACCAGGT
AGTATAGAAGGGATGAAAGGCGCTGCTGGAGGGGTGATGGGAGAGCTGACCCGTGCACGGATTGCACTTG
ATGAAAGAGGACAGAGGCTAGGAGAGCTGGAAGAGAAAACTGCAGGCATGATGACCAGTGCAGAAGCATT
TTCCAAACATGCACATGAGTTAATGCTGAAATACAAGGATAAGAAATGGTACCAATTCTGACTTCTAAAG
AAGCTGTGACTGCTTTGAGAAACCATATTCAGGGAAACCAAATTTATTCCCAGCATCACTACTCAACTGC
TAGAAGCCAGTTTCTTCTACAAAATGTTCCATTTACAGTCAATCTGAAATTTTCATAAAAGAGATGTATC
AGAGACACTGTGCAACCCAAAGGCTGGAGCCCTGGTTAAAATCCCAAAATACGGCTGAATTTGCCTTTTC
CCATGTGGAATGGAGCTATCATCTTGGCATGTCATTCGATAAGGATTTATATTTAGTAACTACGGGGAGT
CCTCTTGATTTGAAAATTCCTGTACAAACAAAAGCATTAATGCACTTAATGAAAAAAAATCTTTGCATGA
TAAATTAACATCTTAAGGGGAAAAGAAAAAAAAGCATGTTGTGATAGAAGAAAAAAAGATTCATGGTAAA
CTATTTCTATCAATTGGGCCACACTCAACAATTTAAAAAATCTGCATTTGAAGATGTCAGTGCATTTCAA
ATATATTTGCCATACCCAACTGAAACGGAAAACAAAACAAAACAAGAACCCAGAGTTTTCTCCTCATCTG
TAACTTTCATTGTACTAGAACCTTATTGAATTCTTATGGCAAGAGTCTGATTAATGTTCTCTCTCCATGT
TTTACATCATTCACAGTTGTGTCAAAATCAAATATTGCCAGTATAGTAAATTATTCCCAACACAATCAGT
TTCATTTCTATCACCTTCAGAATGGGTTGCTGTTTTTAGCACAAACCATGCAGGGTTGGGTCACCTCAAA
GCATTTTCTTCAGCCTGGCTTGTACTCTATAGGGGAGTTGGTAGTAGGTGTGGGGAATCTATAGGAACAA
TAGAGTTAGCTATGAGAACTGACTTCACAAAAAGGGAAATTTCACACACCCCAATTTCAGCTTCTAAAGT
GATATATTTTAAAAGCTTCATTTGTATCCTTGTCTGCCAGTTACACAGTATATTACTGTTCAAACCCAGT
 
AGGGTCTGCAATCGAAAGGTGCTCTTGCAGCCTGACATTCACAAACCAAACTGGTCATTCTCTTTAGTGT
GATTCAGTAAAATCTCAGTTAATGTTTTGGGAAAGGGATTCTGATTTATGACATTTCTGATTTACAAGGG
CTTTCCTTAGAAAATACCTTTCGGTTTGATACTTACCACTGGAAATTGGTTCGATACTTACCACTGGAAA
TCTTTATAAAATATAGTGATCTGTTTGCTATCTTGGTCTCAGTCATCATTATAGACAATAGAAATACATA
AAGAGTTTAATGATACAAAATCAATGCTTTTGTCCCTAGGTCAAATAATATAAGCCACTAGATTATGTCA
ATAGATGCCAAACACTTGTCCCTTATTAAAGACTCTTTTAAAGCTGGAATAAAGAATATCCTTAACATGA
TACAAGGTATCTATAACTCACCAGAAAAGCACCAGAGTCAAAACCAAATAGAGACACCTACTACCATTTA
ACATGATTCTGGAAGTCAAAAGTTATAGGAATGTATATATACACATTCCAGGACCCATCCTGTAATATGT
TGTTTCGTCAAGAAAAGTTTTAATTATGGTTTTGGCTTATTGAATTTTTGTTTATCAAGGTCTCATTGGA
TTTACATTTGTGTTTCTTAAGACTTCACAATTTTCCTGGATTTCAGGTTATTTTATTAGCCAAAAAATCG
TATTTTTATTTCCTTTTTAGTCTAATTTTTTGCTAGAAGTTGTACTTTCTAGCAAATGTTTCTAGTGGTA
CAATGTTCTCAGTAATTTTGAGGAGAACAAAGATGAGTGAAAGAGTTAGATAATGTACACTAGGATAGCA
TTTCTCAGAGTCCTTGAATCCTCTTGTTAAGAAAGCCTCTCTGAATCTCCCAGACTCCTGTGAACAGCCT
TACGGTAACAGAATCTGGCTTGGAAACACAGACATTACATGACTGTATAATTTATAGAATTATAAAATTA
TATGGTAAAAATATTAAAGAAACTTAAGAGTAGGATCTAGAAAATACTAGTGGCCAAATTGCAATGTATT
CTTTGTACCATCTGTTTCAAAGACTTAGTTTTCTGATTTTCAGAACAAATACTTTATCGTTTCTGTATTT
TAAAAAAAACTAAAAGAGTAAAATATTAGTTTATTTTAGAAATAATGAAATTCAGTCCAAAGTAGCAGTA
CATTTGATGAGTATTTCTGAAGCCTTCAATAAGATTTAACTTGTTTTAACTTGATCTACATCGAGATTTC
TCAAAAGCAGCACTATTAGCTGAAAGTCTTTGTTGTGGGGAACTGTCCTGTGTATTGTAGGATGTTAGCA
ACATCCCTGGCCTCTGTCTACTAGATGCCAGTAAGAACCTCCTAGTAATGACAATCAAAATGTCTCCAGT
CATTGCCAAAATTTCTCCCAGTTCACAGCCACTATTCTACATCATACAAAGAGGTAGATTGTGTGAATAT
GTAATATATGTTGTTGTATAAAACTGTACAATAAGTATACAATGTCTTAGCAAGATAAATTATTATACCC
AAAATTACATGTGTGGTTCATATGCAATAAGATACAATTTTGAAGAAAGCTTATAGGAATCAGTGAAAAA
TAACAACTACAGAATATTTTAAGGCACACAGAGAAAATATCAAAAAGGACATTATAGCATTTCACTGTAG
AAGTGCTTAAAACCTTGGCCTCAGTGAATAGATCAAAAGTTTATAATTAGCACACCAATTGGAAGTAAAC
AGGTGCTTCTCAAATGCTTGAAAAGAAAAGGCTCTTTGAAGTAGTTAAAACGTGTCTTTCAAAATTGCTT
TTATAAGCCCTTCCTTAACGTAAGGGTAACTTCATCTCACAAACTCCTGGTTAGTGGAATCCAAATTAAT
GAGGTTTTACTGTATCAAAATTATTTTTATAATTCAAAGAGATGATAGCAATAAGAAGAATCTAATGTGG
ATGTGATTTTTCTCTTTGAACAATTCTGTTCTGTTGCACTGACCTTAGCACGCACGCACACACACACACA
CCATCGCCACCACTACCATCATCATCTTATCCTCATCTTTGGAAAATAAGTACTGTTGATCCATGTAATC
CTTTGCATAGTCTCATCTTTACTATCTTTCTTCTATTTCGGAACATAAGAAAAATACTCCTAATAACTTG
GACAATGATACAGAAAGTGACTCTGAGCCTTAGTTACCATCAAAATTACATTCTATCTGCTAAAGCGACA
TCAGAAGATAAGGCTTGCAAACCACAGTCCCAACAAGTTGACCTTGTCTCCCTATGATTTCCTTGGAAGA
TAAGGCCTTTTTAGTGTTACAGTAGAAAATGGGCCTTTTCATTTATTCATCTGTGAGAAGCTAGAGGTAT
TTGTTTCCCTGATAATAACGTGAGCAATAGTCCCTACCTTGAAATCATCCATTAGTCTTGGAGTTTGGAG
GGAGGAATGATTGATTCCTCATTTGTTTTAATCACCTACAAAAGCAGTACTACTGACTTTGCTTCTTACA
CTTCACTGAGCCCCAGACTTTAGGGCCCCAATCATTGTCAAGATCATTGTCTAACTGGAACTCTGAAAGG
ACAGTGACAGAACTGAATGAGATGTTTTCCAGTGATTCAGTCACAAAGACAATTTTCTCAGATCTTTTGA
AAGTGATTCTCAAATAAATAAATCAAAATCCAACTCCTTTCCCTCAAAGTTTCTAGGGATCTGAAGGAAA
AGGTTTACTGTTGGAGTCCCTTTTTATGTGACCCATGTACCAGTTTTAAAGGCATCATCTATACTTTATC
TACATGAAGGGATCACCATGACTTTGAGAGAAAAAAGAATCAGATTAGGCCTAACATTGGTTGAAGAAGA
TTAAAATAGATTCTTATATTCAAGCAGAAGGGAAAAATGAAGCCAATCTAGAAAAGTAAGCTAAAAATCC
TCTCCTTGGCTTAGGATCATTGGGTCAAACTGGGAGTATATCTGTGTGATTACAACCTGCTGCTATGAGA
AAAAAGAATTGTAACTTGACCATGTTGGCTCACACAGGGCAATGCAAAAGAAGTCAAAACATTCATATCA
GGTCTTGGTATCTGTGTCAGGAGAAACGTGGTGGCTTTCACAGCTTCTTCACCTCTGTCCTCTGTCATGC
AACTCCATTTTTGGCTAGGTACTATTTCACATGCACACTGTCCTGGGTGCCAGAAATACTTGTGGTGTAC
TTGGTTTGAGCAGTACCTCTAGTATGCACCATTCCAGGGAACATGGTTGTCAACATAACAAGTTTAGTGT
GTGTATTTGCAGACAACATAGCCCTGAAAGCATGAAATGTAGAATAGGTAAGGTCTGTCTTAGAAAAGGA
GGAGGAAGTACCAATTTGGTGCAAGAAGATCGTGGTGAGGAAGGACATGGGAGCCACAGTGAAGTGAAAG
AGGCTTTAAGGAATCAGATGCACAGATGGATGCTTTGATTTACACATTTATCTAGAGGAGTGATTCTCAA
CCTTGACTGTACATAAGAATCACCTCAGGAAATTTTTAAATTCCCAATTATATTAAAATCATTTGGAGTG
GGAACCAGACACCAATATTTGCGAAAGCCTCCAAGGCGGTTCTAATGTCCAGCCAAAGTTGATTATCACT
GCTTTATGGCCAACTAAGGAAACCTTGTTTGTCTGCCTTGGCTGGTGCTCCAGCCCCAGTTCTGAGTGGT
CAGGGAAGGCAAGATGTAAGATATAGTAATGGTATTTATTCTGTATGTCTCACAGAACAAAAGCATTTGA
GAATTTCTGACCAGGCAGTGCTCCATCAACTCTTAATAATCATCTTTGTGATATTCTCCTATGTGACTTG
TCCATCAATTATTCCAATTCAAAGGTCTAGGTTTTTTTTCAATTGTCTTACACATGTGACATCACAAGAT
ACCAGAAGTAGCACAGTAAATTCAAGAGCATATAAAGCTAAGTGGAAAACTAATTCTTCATATTTAGACA
GTGGAAATTCTCAAAACAATCATTTGCTAAATTAGGTCCAAATTTATTATTCATTCTTCTTCTGGAGGAG
ACATGGGTACCAGGTACCTGTTCTGCTGTAGGTATATTTCACTGGACTTGGTCTTAGCAAAAAGGAAGAA
 
CAAATCTTCCTTTTTGATTGTTATCTCAATCAAAAGCTCCCAGGGCCACTTGGATTTATTTTCTTAGTCT
TTGGACATGTGTTTAAAGGAAAAGAATACCTGTAACTTCCTATATGACATCCAGTGCCCTGAAAGGGTGC
TTACACCTTCAAAAAAGGAGAGTCCAGCCTATCAAAGGCAGTAACACATAATGAGAAGAAACTCAGAGTT
ATTGCCTTTCATACTGCTTTCCCTCTGCTGTTGGCCCACCCTTCTTGACTGCAAAGCCAGAAAATAAACT
AGATGGTGGTTTTGGACAATTGTACACAGGAACAGAATCTCTAATAACCTATAATTTTTAGTACTTAATT
ATGTGATGTTTTATTCTAGGAAATAAGAAACAGATGACATCATTGATGTATCTTTCTTTCATCTTCAGAA
TTTTTGCATCTTTTCAGAAAATGACAAATTACTATTTTTCTGTTGCACTCAGCAATTGTGACTATACTTA
ACAAAATTCTTGTAGTCTGTAGAGATATCAATAAAATTGTATATGTTTGTACATAAAAAAAAAAAAAAA
AA
**SEQ ID NO: 33 - mRNA sequence of syntaxin binding protein 5-like transcript 2(taken from NM_001308330 as at the filing date)** 
CTGACTGCTCGGTTAGGACCTCGGAGAGCGCCAGGCGCCGCGACCAGAGGGCCCAGAGAAGCGGCCGGAG
CCCGCCTACCTCGGCCCCCTCAGCTTCCCGGGCTGGCAGGCGGCTAGAGGCGTCTGAGGAAGTGTTTAAA
ATGAAGAAGTTTAATTTCCGAAAAGTTTTGGATGGCTTAACTGCCTCCTCCCCTGGCAGTGGTAGCAGCA
GTGGCAGTAACAGTGGTGGTGGGGCTGGAAGTGGTTCCGTACATCCGGCGGGGACTGCAGGGGTTCTCAG
AGAGGAAATTCAGGAAACTTTGACTTCGGAGTATTTCCAGATTTGCAAGACAGTTCGGCATGGTTTTCCT
CATCAGCCCACAGCATTAGCCTTTGATCCAGTTCAGAAAATCTTGGCTATTGGGACGAGAACAGGTGCTA
TACGAATACTCGGGAGACCTGGTGTTGATTGCTATTGCCAACATGAAAGTGGTGCAGCTGTCCTACAGCT
CCAATTTTTGATCAATGAGGGTGCCTTGGTCAGTGCAAGTTCAGATGATACACTTCATTTGTGGAACCTT
AGACAAAAAAGGCCAGCCATACTCCATTCTCTTAAATTTAACCGGGAACGAATTACTTACTGTCATCTAC
CTTTCCAGAGTAAATGGCTTTATGTTGGAACAGAAAGAGGAAATACACATATTGTAAATATTGAATCTTT
CATTCTTTCTGGATATGTTATCATGTGGAACAAGGCAATTGAACTATCCACTAAGACTCATCCAGGTCCA
GTTGTACATTTAAGCGATAGCCCAAGAGATGAAGGCAAACTGCTAATAGGTTATGAAAATGGTACTGTAG
TATTCTGGGACTTGAAATCTAAAAGAGCAGAACTGAGAGTTTATTATGATGAGGCTATTCATTCAATTGA
TTGGCATCATGAGGGCAAACAGTTCATGTGCAGCCATTCAGATGGTAGTTTGACTTTATGGAACCTGAAA
AGCCCAAGTCGCCCTTTCCAGACCACAATTCCACATGGAAAAAGTCAAAGAGAAGGAAGAAAATCTGAAT
CTTGTAAACCAATTCTTAAAGTAGAATACAAGACCTGCAAAAACAGCGAACCATTCATAATATTCTCTGG
TGGGCTGTCCTATGACAAAGCTTGTAGAAGACCAAGTTTAACCATCATGCATGGAAAAGCAATTACAGTA
CTTGAAATGGATCATCCTATTGTTGAATTTCTAACTTTATGTGAAACGCCCTATCCAAATGAATTTCAAG
AACCCTATGCTGTCGTGGTACTTCTGGAGAAAGATCTCATTGTAGTTGATCTGACACAAAGCAATTTTCC
AATCTTTGAAAATCCATATCCCATGGACATTCATGAATCACCAGTTACATGCACAGCATACTTTGCAGAT
TGTCCTCCGGATTTGATTCTAGTACTGTATTCTATAGGAGTCAAGCATAAAAAACAAGGATACAGTAATA
AGGAGTGGCCAATCAGTGGAGGAGCTTGGAACCTTGGAGCACAAACATATCCAGAAATTATTATTACTGG
TCATGCAGATGGATCAATAAAATTTTGGGATGCTTCTGCAATAACTCTGCAGATGCTGTACAAGCTAAAA
ACTTCAAAAGTGTTTGAAAAACAGAAGGTAGGAGAAGGAAAACAAACATGTGAAATTGTAGAGGAAGACC
CATTTGCCATTCAGATGATTTACTGGTGTCCAGAGAGCAGAATATTCTGTGTATCAGGAGTCTCTGCATA
TGTCATAATTTATAAATTCAGCAGACATGAAATTACAACAGAAATAGTGTCATTAGAGGTACGACTTCAG
TATGATGTTGAAGATATTATTACCCCTGAACCAGAAACAAGTCCTCCGTTTCCAGATCTCTCAGCCCAGC
TTCCTTCTTCAAGGAGTCTTTCTGGGAGCACTAACACTGTTGCTAGTGAAGGAGTAACAAAGGACAGTAT
TCCATGCCTCAATGTGAAGACACGGCCAGTGCGAATGCCTCCAGGATATCAAGCAGAACTTGTTATTCAA
TTGGTGTGGGTAGATGGTGAACCTCCACAACAGATTACTAGTCTTGCTGTAAGCTCAGCATATGGAATAG
TTGCATTTGGGAACTGCAATGGGTTGGCTGTGGTGGATTTTATACAGAAGACAGTACTGTTAAGCATGGG
GACCATTGACCTATATAGATCAAGTGACTTATACCAGCGACAACCACGGTCTCCTCGAAAAAACAAACAG
TTCATTGCAGGTTTAACTGAACTGAATGACAGTCCAGTTCCCCTAGAACTTGAGCGCTGCAAGTCTCCTA
CCTCAGACCATGTAAATGGACACTGCACAAGTCCAACTTCTCAGAGTTGCAGTTCTGGAAAACGTCTTTC
TAGTGCCGATGTTTCAAAAGTAAATCGCTGGGGTCCTGGAAGACCACCATTTCGAAAGGCCCAGTCAGCA
GCCTGCATGGAGATTTCTTTACCAGTTACAACAGAAGAAAACCGAGAAAATTCCTATAATCGTTCTAGAA
GCTCTAGTATCTCCAGTATTGACAAAGATTCTAAAGAAGCAATTACAGCACTATACTTCATGGACTCCTT
TGCACGGAAAAATGACTCTACCATCTCTCCTTGTCTGTTCGTTGGAACCAGTCTGGGAATGGTGTTAATC
ATCTCCTTAAACCTACCATTAGCAGATGAACAAAGGTTTACAGAGCCAGTCATGGTATTGCCAAGTGGTA
CATTCCTCTCATTGAAAGGAGCTGTGCTAACATTCTCCTGTATGGACCGAATGGGTGGATTAATGCAACC
GCCATATGAAGTTTGGAGGGATCCAAACAACATAGATGAAAATGAAAAATCTTGGAGAAGGAAAGTGGTA
ATGAACTCATCTTCTGCATCCCAAGAAATAGGAGATCATCAGTATACAATAATCTGCTCAGAAAAACAAG
CCAAAGTCTTCTCACTGCCTTCTCAGACTTGCCTTTATGTTCATAACATCACGGAGACATCTTTTATACT
GCAAGCAAATGTGGTGGTCATGTGTAGCAGTGCCTGCTTGGCATGCTTTTGTGCTAACGGACATATCATG
ATAATGAGCCTACCTAGTCTTCGCCCAATGTTGGATGTTAATTATTTGCCACTGACAGACATGAGGATAG
CACGAACATTTTGTTTTACCAATGAAGGACAGGCATTATACCTCGTCTCTCCTACTGAAATTCAGCGGTT
 
AACATACAGCCAAGAAATGTGTGATAATCTACAGGACATGCTAGGAGATTTGTTTACTCCCATAGAGACA
CCAGAAGCTCAAAACAGAGGCTTTCTCAAGGGACTGTTTGGTGGAAGCGGACAAACATTTGACAGAGAAG
AGCTCTTTGGGGAAGCTTCGGCAGGAAAAGCATCCCGCAGCCTTGCGCAACACATTCCTGGACCAGGTAG
TATAGAAGGGATGAAAGGCGCTGCTGGAGGGGTGATGGGAGAGCTGACCCGTGCACGGATTGCACTTGAT
GAAAGAGGACAGAGGCTAGGAGAGCTGGAAGAGAAAACTGCAGGCATGATGACCAGTGCAGAAGCATTTT
CCAAACATGCACATGAGTTAATGCTGAAATACAAGGATAAGAAATGGTACCAATTCTGACTTCTAAAGAA
GCTGTGACTGCTTTGAGAAACCATATTCAGGGAAACCAAATTTATTCCCAGCATCACTACTCAACTGCTA
GAAGCCAGTTTCTTCTACAAAATGTTCCATTTACAGTCAATCTGAAATTTTCATAAAAGAGATGTATCAG
AGACACTGTGCAACCCAAAGGCTGGAGCCCTGGTTAAAATCCCAAAATACGGCTGAATTTGCCTTTTCCC
ATGTGGAATGGAGCTATCATCTTGGCATGTCATTCGATAAGGATTTATATTTAGTAACTACGGGGAGTCC
TCTTGATTTGAAAATTCCTGTACAAACAAAAGCATTAATGCACTTAATGAAAAAAAATCTTTGCATGATA
AATTAACATCTTAAGGGGAAAAGAAAAAAAAGCATGTTGTGATAGAAGAAAAAAAGATTCATGGTAAACT
ATTTCTATCAATTGGGCCACACTCAACAATTTAAAAAATCTGCATTTGAAGATGTCAGTGCATTTCAAAT
ATATTTGCCATACCCAACTGAAACGGAAAACAAAACAAAACAAGAACCCAGAGTTTTCTCCTCATCTGTA
ACTTTCATTGTACTAGAACCTTATTGAATTCTTATGGCAAGAGTCTGATTAATGTTCTCTCTCCATGTTT
TACATCATTCACAGTTGTGTCAAAATCAAATATTGCCAGTATAGTAAATTATTCCCAACACAATCAGTTT
CATTTCTATCACCTTCAGAATGGGTTGCTGTTTTTAGCACAAACCATGCAGGGTTGGGTCACCTCAAAGC
ATTTTCTTCAGCCTGGCTTGTACTCTATAGGGGAGTTGGTAGTAGGTGTGGGGAATCTATAGGAACAATA
GAGTTAGCTATGAGAACTGACTTCACAAAAAGGGAAATTTCACACACCCCAATTTCAGCTTCTAAAGTGA
TATATTTTAAAAGCTTCATTTGTATCCTTGTCTGCCAGTTACACAGTATATTACTGTTCAAACCCAGTAG
GGTCTGCAATCGAAAGGTGCTCTTGCAGCCTGACATTCACAAACCAAACTGGTCATTCTCTTTAGTGTGA
TTCAGTAAAATCTCAGTTAATGTTTTGGGAAAGGGATTCTGATTTATGACATTTCTGATTTACAAGGGCT
TTCCTTAGAAAATACCTTTCGGTTTGATACTTACCACTGGAAATTGGTTCGATACTTACCACTGGAAATC
TTTATAAAATATAGTGATCTGTTTGCTATCTTGGTCTCAGTCATCATTATAGACAATAGAAATACATAAA
GAGTTTAATGATACAAAATCAATGCTTTTGTCCCTAGGTCAAATAATATAAGCCACTAGATTATGTCAAT
AGATGCCAAACACTTGTCCCTTATTAAAGACTCTTTTAAAGCTGGAATAAAGAATATCCTTAACATGATA
CAAGGTATCTATAACTCACCAGAAAAGCACCAGAGTCAAAACCAAATAGAGACACCTACTACCATTTAAC
ATGATTCTGGAAGTCAAAAGTTATAGGAATGTATATATACACATTCCAGGACCCATCCTGTAATATGTTG
TTTCGTCAAGAAAAGTTTTAATTATGGTTTTGGCTTATTGAATTTTTGTTTATCAAGGTCTCATTGGATT
TACATTTGTGTTTCTTAAGACTTCACAATTTTCCTGGATTTCAGGTTATTTTATTAGCCAAAAAATCGTA
TTTTTATTTCCTTTTTAGTCTAATTTTTTGCTAGAAGTTGTACTTTCTAGCAAATGTTTCTAGTGGTACA
ATGTTCTCAGTAATTTTGAGGAGAACAAAGATGAGTGAAAGAGTTAGATAATGTACACTAGGATAGCATT
TCTCAGAGTCCTTGAATCCTCTTGTTAAGAAAGCCTCTCTGAATCTCCCAGACTCCTGTGAACAGCCTTA
CGGTAACAGAATCTGGCTTGGAAACACAGACATTACATGACTGTATAATTTATAGAATTATAAAATTATA
TGGTAAAAATATTAAAGAAACTTAAGAGTAGGATCTAGAAAATACTAGTGGCCAAATTGCAATGTATTCT
TTGTACCATCTGTTTCAAAGACTTAGTTTTCTGATTTTCAGAACAAATACTTTATCGTTTCTGTATTTTA
AAAAAAACTAAAAGAGTAAAATATTAGTTTATTTTAGAAATAATGAAATTCAGTCCAAAGTAGCAGTACA
TTTGATGAGTATTTCTGAAGCCTTCAATAAGATTTAACTTGTTTTAACTTGATCTACATCGAGATTTCTC
AAAAGCAGCACTATTAGCTGAAAGTCTTTGTTGTGGGGAACTGTCCTGTGTATTGTAGGATGTTAGCAAC
ATCCCTGGCCTCTGTCTACTAGATGCCAGTAAGAACCTCCTAGTAATGACAATCAAAATGTCTCCAGTCA
TTGCCAAAATTTCTCCCAGTTCACAGCCACTATTCTACATCATACAAAGAGGTAGATTGTGTGAATATGT
AATATATGTTGTTGTATAAAACTGTACAATAAGTATACAATGTCTTAGCAAGATAAATTATTATACCCAA
AATTACATGTGTGGTTCATATGCAATAAGATACAATTTTGAAGAAAGCTTATAGGAATCAGTGAAAAATA
ACAACTACAGAATATTTTAAGGCACACAGAGAAAATATCAAAAAGGACATTATAGCATTTCACTGTAGAA
GTGCTTAAAACCTTGGCCTCAGTGAATAGATCAAAAGTTTATAATTAGCACACCAATTGGAAGTAAACAG
GTGCTTCTCAAATGCTTGAAAAGAAAAGGCTCTTTGAAGTAGTTAAAACGTGTCTTTCAAAATTGCTTTT
ATAAGCCCTTCCTTAACGTAAGGGTAACTTCATCTCACAAACTCCTGGTTAGTGGAATCCAAATTAATGA
GGTTTTACTGTATCAAAATTATTTTTATAATTCAAAGAGATGATAGCAATAAGAAGAATCTAATGTGGAT
GTGATTTTTCTCTTTGAACAATTCTGTTCTGTTGCACTGACCTTAGCACGCACGCACACACACACACACC
ATCGCCACCACTACCATCATCATCTTATCCTCATCTTTGGAAAATAAGTACTGTTGATCCATGTAATCCT
TTGCATAGTCTCATCTTTACTATCTTTCTTCTATTTCGGAACATAAGAAAAATACTCCTAATAACTTGGA
CAATGATACAGAAAGTGACTCTGAGCCTTAGTTACCATCAAAATTACATTCTATCTGCTAAAGCGACATC
AGAAGATAAGGCTTGCAAACCACAGTCCCAACAAGTTGACCTTGTCTCCCTATGATTTCCTTGGAAGATA
AGGCCTTTTTAGTGTTACAGTAGAAAATGGGCCTTTTCATTTATTCATCTGTGAGAAGCTAGAGGTATTT
GTTTCCCTGATAATAACGTGAGCAATAGTCCCTACCTTGAAATCATCCATTAGTCTTGGAGTTTGGAGGG
AGGAATGATTGATTCCTCATTTGTTTTAATCACCTACAAAAGCAGTACTACTGACTTTGCTTCTTACACT
TCACTGAGCCCCAGACTTTAGGGCCCCAATCATTGTCAAGATCATTGTCTAACTGGAACTCTGAAAGGAC
AGTGACAGAACTGAATGAGATGTTTTCCAGTGATTCAGTCACAAAGACAATTTTCTCAGATCTTTTGAAA
GTGATTCTCAAATAAATAAATCAAAATCCAACTCCTTTCCCTCAAAGTTTCTAGGGATCTGAAGGAAAAG
 
GTTTACTGTTGGAGTCCCTTTTTATGTGACCCATGTACCAGTTTTAAAGGCATCATCTATACTTTATCTA
CATGAAGGGATCACCATGACTTTGAGAGAAAAAAGAATCAGATTAGGCCTAACATTGGTTGAAGAAGATT
AAAATAGATTCTTATATTCAAGCAGAAGGGAAAAATGAAGCCAATCTAGAAAAGTAAGCTAAAAATCCTC
TCCTTGGCTTAGGATCATTGGGTCAAACTGGGAGTATATCTGTGTGATTACAACCTGCTGCTATGAGAAA
AAAGAATTGTAACTTGACCATGTTGGCTCACACAGGGCAATGCAAAAGAAGTCAAAACATTCATATCAGG
TCTTGGTATCTGTGTCAGGAGAAACGTGGTGGCTTTCACAGCTTCTTCACCTCTGTCCTCTGTCATGCAA
CTCCATTTTTGGCTAGGTACTATTTCACATGCACACTGTCCTGGGTGCCAGAAATACTTGTGGTGTACTT
GGTTTGAGCAGTACCTCTAGTATGCACCATTCCAGGGAACATGGTTGTCAACATAACAAGTTTAGTGTGT
GTATTTGCAGACAACATAGCCCTGAAAGCATGAAATGTAGAATAGGTAAGGTCTGTCTTAGAAAAGGAGG
AGGAAGTACCAATTTGGTGCAAGAAGATCGTGGTGAGGAAGGACATGGGAGCCACAGTGAAGTGAAAGAG
GCTTTAAGGAATCAGATGCACAGATGGATGCTTTGATTTACACATTTATCTAGAGGAGTGATTCTCAACC
TTGACTGTACATAAGAATCACCTCAGGAAATTTTTAAATTCCCAATTATATTAAAATCATTTGGAGTGGG
AACCAGACACCAATATTTGCGAAAGCCTCCAAGGCGGTTCTAATGTCCAGCCAAAGTTGATTATCACTGC
TTTATGGCCAACTAAGGAAACCTTGTTTGTCTGCCTTGGCTGGTGCTCCAGCCCCAGTTCTGAGTGGTCA
GGGAAGGCAAGATGTAAGATATAGTAATGGTATTTATTCTGTATGTCTCACAGAACAAAAGCATTTGAGA
ATTTCTGACCAGGCAGTGCTCCATCAACTCTTAATAATCATCTTTGTGATATTCTCCTATGTGACTTGTC
CATCAATTATTCCAATTCAAAGGTCTAGGTTTTTTTTCAATTGTCTTACACATGTGACATCACAAGATAC
CAGAAGTAGCACAGTAAATTCAAGAGCATATAAAGCTAAGTGGAAAACTAATTCTTCATATTTAGACAGT
GGAAATTCTCAAAACAATCATTTGCTAAATTAGGTCCAAATTTATTATTCATTCTTCTTCTGGAGGAGAC
ATGGGTACCAGGTACCTGTTCTGCTGTAGGTATATTTCACTGGACTTGGTCTTAGCAAAAAGGAAGAACA
AATCTTCCTTTTTGATTGTTATCTCAATCAAAAGCTCCCAGGGCCACTTGGATTTATTTTCTTAGTCTTT
GGACATGTGTTTAAAGGAAAAGAATACCTGTAACTTCCTATATGACATCCAGTGCCCTGAAAGGGTGCTT
ACACCTTCAAAAAAGGAGAGTCCAGCCTATCAAAGGCAGTAACACATAATGAGAAGAAACTCAGAGTTAT
TGCCTTTCATACTGCTTTCCCTCTGCTGTTGGCCCACCCTTCTTGACTGCAAAGCCAGAAAATAAACTAG
ATGGTGGTTTTGGACAATTGTACACAGGAACAGAATCTCTAATAACCTATAATTTTTAGTACTTAATTAT
GTGATGTTTTATTCTAGGAAATAAGAAACAGATGACATCATTGATGTATCTTTCTTTCATCTTCAGAATT
TTTGCATCTTTTCAGAAAATGACAAATTACTATTTTTCTGTTGCACTCAGCAATTGTGACTATACTTAAC
AAAATTCTTGTAGTCTGTAGAGATATCAATAAAATTGTATATGTTTGTACATAAAAAAAAAAAAAAAAAA
**SEQ ID NO: 34 - mRNA sequence of syntaxin binding protein 5-like transcript 3(taken from NM_001348343 as at the filing date)** 
GTGCAGCGGGGAGAGCGGACGGCGGGCGGGCGAACAGGCGGGAGAGCGCGGCGGCTGCGGGCAGAGT
GTTTAAAATGAAGAAGTTTAATTTCCGAAAAGTTTTGGATGGCTTAACTGCCTCCTCCCCTGGCAGTGGT
AGCAGCAGTGGCAGTAACAGTGGTGGTGGGGCTGGAAGTGGTTCCGTACATCCGGCGGGGACTGCAGGGG
TTCTCAGAGAGGAAATTCAGGAAACTTTGACTTCGGAGTATTTCCAGATTTGCAAGACAGTTCGGCATGG
TTTTCCTCATCAGCCCACAGCATTAGCCTTTGATCCAGTTCAGAAAATCTTGGCTATTGGGACGAGAACA
GGTGCTATACGAATACTCGGGAGACCTGGTGTTGATTGCTATTGCCAACATGAAAGTGGTGCAGCTGTCC
TACAGCTCCAATTTTTGATCAATGAGGGTGCCTTGGTCAGTGCAAGTTCAGATGATACACTTCATTTGTG
GAACCTTAGACAAAAAAGGCCAGCCATACTCCATTCTCTTAAATTTAACCGGGAACGAATTACTTACTGT
CATCTACCTTTCCAGAGTAAATGGCTTTATGTTGGAACAGAAAGAGGAAATACACATATTGTAAATATTG
AATCTTTCATTCTTTCTGGATATGTTATCATGTGGAACAAGGCAATTGAACTATCCACTAAGACTCATCC
AGGTCCAGTTGTACATTTAAGCGATAGCCCAAGAGATGAAGGCAAACTGCTAATAGGTTATGAAAATGGT
ACTGTAGTATTCTGGGACTTGAAATCTAAAAGAGCAGAACTGAGAGTTTATTATGATGAGGCTATTCATT
CAATTGATTGGCATCATGAGGGCAAACAGTTCATGTGCAGCCATTCAGATGGTAGTTTGACTTTATGGAA
CCTGAAAAGCCCAAGTCGCCCTTTCCAGACCACAATTCCACATGGAAAAAGTCAAAGAGAAGGAAGAAAA
TCTGAATCTTGTAAACCAATTCTTAAAGTAGAATACAAGACCTGCAAAAACAGCGAACCATTCATAATAT
TCTCTGGTGGGCTGTCCTATGACAAAGCTTGTAGAAGACCAAGTTTAACCATCATGCATGGAAAAGCAAT
TACAGTACTTGAAATGGATCATCCTATTGTTGAATTTCTAACTTTATGTGAAACGCCCTATCCAAATGAA
TTTCAAGAACCCTATGCTGTCGTGGTACTTCTGGAGAAAGATCTCATTGTAGTTGATCTGACACAAAGCA
ATTTTCCAATCTTTGAAAATCCATATCCCATGGACATTCATGAATCACCAGTTACATGCACAGCATACTT
TGCAGATTGTCCTCCGGATTTGATTCTAGTACTGTATTCTATAGGAGTCAAGCATAAAAAACAAGGATAC
AGTAATAAGGAGTGGCCAATCAGTGGAGGAGCTTGGAACCTTGGAGCACAAACATATCCAGAAATTATTA
TTACTGGTCATGCAGATGGATCAATAAAATTTTGGGATGCTTCTGCAATAACTCTGCAGATGCTGTACAA
GCTAAAAACTTCAAAAGTGTTTGAAAAACAGAAGGTAGGAGAAGGAAAACAAACATGTGAAATTGTAGAG
GAAGACCCATTTGCCATTCAGATGATTTACTGGTGTCCAGAGAGCAGAATATTCTGTGTATCAGGAGTCT
CTGCATATGTCATAATTTATAAATTCAGCAGACATGAAATTACAACAGAAATAGTGTCATTAGAGGTACG
 
ACTTCAGTATGATGTTGAAGATATTATTACCCCTGAACCAGAAACAAGTCCTCCGTTTCCAGATCTCTCA
GCCCAGCTTCCTTCTTCAAGGAGTCTTTCTGGGCACTAACACTGTTGCTAGTGAAGGAGTAACAAAGG
ACAGTATTCCATGCCTCAATGTGAAGACACGGCCAGTGCGAATGCCTCCAGGATATCAAGCAGAACTTGT
TATTCAATTGGTGTGGGTAGATGGTGAACCTCCACAACAGATTACTAGTCTTGCTGTAAGCTCAGCATAT
GGAATAGTTGCATTTGGGAACTGCAATGGGTTGGCTGTGGTGGATTTTATACAGAAGACAGTACTGTTAA
GCATGGGGACCATTGACCTATATAGATCAAGTGACTTATACCAGCGACAACCACGGTCTCCTCGAAAAAA
CAAACAGTTCATTGCAGACAACTTTTGCATGCGTGGCCTGTCTAACTTTTATCCTGATTTAACGAAACGG
ATCCGTACTTCCTATCAGAGTTTAACTGAACTGAATGACAGTCCAGTTCCCCTAGAACTTGAGCGCTGCA
AGTCTCCTACCTCAGACCATGTAAATGGACACTGCACAAGTCCAACTTCTCAGAGTTGCAGTTCTGGAAA
ACGTCTTTCTAGTGCCGATGTTTCAAAAGTAAATCGCTGGGGTCCTGGAAGACCACCATTTCGAAAGGCC
CAGTCAGCAGCCTGCATGGAGATTTCTTTACCAGTTACAACAGAAGAAAACCGAGAAAATTCCTATAATC
GTTCTAGAAGCTCTAGTATCTCCAGTATTGACAAAGATTCTAAAGAAGCAATTACAGCACTATACTTCAT
GGACTCCTTTGCACGGAAAAATGACTCTACCATCTCTCCTTGTCTGTTCGTTGGAACCAGTCTGGGAATG
GTGTTAATCATCTCCTTAAACCTACCATTAGCAGATGAACAAAGGTTTACAGAGCCAGTCATGGTATTGC
CAAGTGGTACATTCCTCTCATTGAAAGGAGCTGTGCTAACATTCTCCTGTATGGACCGAATGGGTGGATT
AATGCAACCGCCATATGAAGTTTGGAGGGATCCAAACAACATAGATGAAAATGAAAAATCTTGGAGAAGG
AAAGTGGTAATGAACTCATCTTCTGCATCCCAAGAAATAGGAGATCATCAGTATACAATAATCTGCTCAG
AAAAACAAGCCAAAGTCTTCTCACTGCCTTCTCAGACTTGCCTTTATGTTCATAACATCACGGAGACATC
TTTTATACTGCAAGCAAATGTGGTGGTCATGTGTAGCAGTGCCTGCTTGGCATGCTTTTGTGCTAACGGA
CATATCATGATAATGAGCCTACCTAGTCTTCGCCCAATGTTGGATGTTAATTATTTGCCACTGACAGACA
TGAGGATAGCACGAACATTTTGTTTTACCAATGAAGGACAGGCATTATACCTCGTCTCTCCTACTGAAAT
TCAGCGGTTAACATACAGCCAAGAAATGTGTGATAATCTACAGGACATGCTAGGAGATTTGTTTACTCCC
ATAGAGACACCAGAAGCTCAAAACAGAGGCTTTCTCAAGGGACTGTTTGGTGGAAGCGGACAAACATTTG
ACAGAGAAGAGCTCTTTGGGGAAGCTTCGGCAGGAAAAGCATCCCGCAGCCTTGCGCAACACATTCCTGG
ACCAGGTAGTATAGAAGGGATGAAAGGCGCTGCTGGAGGGGTGATGGGAGAGCTGACCCGTGCACGGATT
GCACTTGATGAAAGAGGACAGAGGCTAGGAGAGCTGGAAGAGAAAACTGCAGGCATGATGACCAGTGCAG
AAGCATTTTCCAAACATGCACATGAGTTAATGCTGAAATACAAGGATAAGAAATGGTACCAATTCTGACT
TCTAAAGAAGCTGTGACTGCTTTGAGAAACCATATTCAGGGAAACCAAATTTATTCCCAGCATCACTACT
CAACTGCTAGAAGCCAGTTTCTTCTACAAAATGTTCCATTTACAGTCAATCTGAAATTTTCATAAAAGAG
ATGTATCAGAGACACTGTGCAACCCAAAGGCTGGAGCCCTGGTTAAAATCCCAAAATACGGCTGAATTTG
CCTTTTCCCATGTGGAATGGAGCTATCATCTTGGCATGTCATTCGATAAGGATTTATATTTAGTAACTAC
GGGGAGTCCTCTTGATTTGAAAATTCCTGTACAAACAAAAGCATTAATGCACTTAATGAAAAAAAATCTT
TGCATGATAAATTAACATCTTAAGGGGAAAAGAAAAAAAAGCATGTTGTGATAGAAGAAAAAAAGATTCA
TGGTAAACTATTTCTATCAATTGGGCCACACTCAACAATTTAAAAAATCTGCATTTGAAGATGTCAGTGC
ATTTCAAATATATTTGCCATACCCAACTGAAACGGAAAACAAAACAAAACAAGAACCCAGAGTTTTCTCC
TCATCTGTAACTTTCATTGTACTAGAACCTTATTGAATTCTTATGGCAAGAGTCTGATTAATGTTCTCTC
TCCATGTTTTACATCATTCACAGTTGTGTCAAAATCAAATATTGCCAGTATAGTAAATTATTCCCAACAC
AATCAGTTTCATTTCTATCACCTTCAGAATGGGTTGCTGTTTTTAGCACAAACCATGCAGGGTTGGGTCA
CCTCAAAGCATTTTCTTCAGCCTGGCTTGTACTCTATAGGGGAGTTGGTAGTAGGTGTGGGGAATCTATA
GGAACAATAGAGTTAGCTATGAGAACTGACTTCACAAAAAGGGAAATTTCACACACCCCAATTTCAGCTT
CTAAAGTGATATATTTTAAAAGCTTCATTTGTATCCTTGTCTGCCAGTTACACAGTATATTACTGTTCAA
ACCCAGTAGGGTCTGCAATCGAAAGGTGCTCTTGCAGCCTGACATTCACAAACCAAACTGGTCATTCTCT
TTAGTGTGATTCAGTAAAATCTCAGTTAATGTTTTGGGAAAGGGATTCTGATTTATGACATTTCTGATTT
ACAAGGGCTTTCCTTAGAAAATACCTTTCGGTTTGATACTTACCACTGGAAATTGGTTCGATACTTACCA
CTGGAAATCTTTATAAAATATAGTGATCTGTTTGCTATCTTGGTCTCAGTCATCATTATAGACAATAGAA
ATACATAAAGAGTTTAATGATACAAAATCAATGCTTTTGTCCCTAGGTCAAATAATATAAGCCACTAGAT
TATGTCAATAGATGCCAAACACTTGTCCCTTATTAAAGACTCTTTTAAAGCTGGAATAAAGAATATCCTT
AACATGATACAAGGTATCTATAACTCACCAGAAAAGCACCAGAGTCAAAACCAAATAGAGACACCTACTA
CCATTTAACATGATTCTGGAAGTCAAAAGTTATAGGAATGTATATATACACATTCCAGGACCCATCCTGT
AATATGTTGTTTCGTCAAGAAAAGTTTTAATTATGGTTTTGGCTTATTGAATTTTTGTTTATCAAGGTCT
CATTGGATTTACATTTGTGTTTCTTAAGACTTCACAATTTTCCTGGATTTCAGGTTATTTTATTAGCCAA
AAAATCGTATTTTTATTTCCTTTTTAGTCTAATTTTTTGCTAGAAGTTGTACTTTCTAGCAAATGTTTCT
AGTGGTACAATGTTCTCAGTAATTTTGAGGAGAACAAAGATGAGTGAAAGAGTTAGATAATGTACACTAG
GATAGCATTTCTCAGAGTCCTTGAATCCTCTTGTTAAGAAAGCCTCTCTGAATCTCCCAGACTCCTGTGA
ACAGCCTTACGGTAACAGAATCTGGCTTGGAAACACAGACATTACATGACTGTATAATTTATAGAATTAT
AAAATTATATGGTAAAAATATTAAAGAAACTTAAGAGTAGGATCTAGAAAATACTAGTGGCCAAATTGCA
ATGTATTCTTTGTACCATCTGTTTCAAAGACTTAGTTTTCTGATTTTCAGAACAAATACTTTATCGTTTC
TGTATTTTAAAAAAAACTAAAAGAGTAAAATATTAGTTTATTTTAGAAATAATGAAATTCAGTCCAAAGT
AGCAGTACATTTGATGAGTATTTCTGAAGCCTTCAATAAGATTTAACTTGTTTTAACTTGATCTACATCG
 
AGATTTCTCAAAAGCAGCACTATTAGCTGAAAGTCTTTGTTGTGGGGAACTGTCCTGTGTATTGTAGGAT
GTTAGCAACATCCCTGGCCTCTGTCTACTAGATGCCAGTAAGAACCTCCTAGTAATGACAATCAAAATGT
CTCCAGTCATTGCCAAAATTTCTCCCAGTTCACAGCCACTATTCTACATCATACAAAGAGGTAGATTGTG
TGAATATGTAATATATGTTGTTGTATAAAACTGTACAATAAGTATACAATGTCTTAGCAAGATAAATTAT
TATACCCAAAATTACATGTGTGGTTCATATGCAATAAGATACAATTTTGAAGAAAGCTTATAGGAATCAG
TGAAAAATAACAACTACAGAATATTTTAAGGCACACAGAGAAAATATCAAAAAGGACATTATAGCATTTC
ACTGTAGAAGTGCTTAAAACCTTGGCCTCAGTGAATAGATCAAAAGTTTATAATTAGCACACCAATTGGA
AGTAAACAGGTGCTTCTCAAATGCTTGAAAAGAAAAGGCTCTTTGAAGTAGTTAAAACGTGTCTTTCAAA
ATTGCTTTTATAAGCCCTTCCTTAACGTAAGGGTAACTTCATCTCACAAACTCCTGGTTAGTGGAATCCA
AATTAATGAGGTTTTACTGTATCAAAATTATTTTTATAATTCAAAGAGATGATAGCAATAAGAAGAATCT
AATGTGGATGTGATTTTTCTCTTTGAACAATTCTGTTCTGTTGCACTGACCTTAGCACGCACGCACACAC
ACACACACCATCGCCACCACTACCATCATCATCTTATCCTCATCTTTGGAAAATAAGTACTGTTGATCCA
TGTAATCCTTTGCATAGTCTCATCTTTACTATCTTTCTTCTATTTCGGAACATAAGAAAAATACTCCTAA
TAACTTGGACAATGATACAGAAAGTGACTCTGAGCCTTAGTTACCATCAAAATTACATTCTATCTGCTAA
AGCGACATCAGAAGATAAGGCTTGCAAACCACAGTCCCAACAAGTTGACCTTGTCTCCCTATGATTTCCT
TGGAAGATAAGGCCTTTTTAGTGTTACAGTAGAAAATGGGCCTTTTCATTTATTCATCTGTGAGAAGCTA
GAGGTATTTGTTTCCCTGATAATAACGTGAGCAATAGTCCCTACCTTGAAATCATCCATTAGTCTTGGAG
TTTGGAGGGAGGAATGATTGATTCCTCATTTGTTTTAATCACCTACAAAAGCAGTACTACTGACTTTGCT
TCTTACACTTCACTGAGCCCCAGACTTTAGGGCCCCAATCATTGTCAAGATCATTGTCTAACTGGAACTC
TGAAAGGACAGTGACAGAACTGAATGAGATGTTTTCCAGTGATTCAGTCACAAAGACAATTTTCTCAGAT
CTTTTGAAAGTGATTCTCAAATAAATAAATCAAAATCCAACTCCTTTCCCTCAAAGTTTCTAGGGATCTG
AAGGAAAAGGTTTACTGTTGGAGTCCCTTTTTATGTGACCCATGTACCAGTTTTAAAGGCATCATCTATA
CTTTATCTACATGAAGGGATCACCATGACTTTGAGAGAAAAAAGAATCAGATTAGGCCTAACATTGGTTG
AAGAAGATTAAAATAGATTCTTATATTCAAGCAGAAGGGAAAAATGAAGCCAATCTAGAAAAGTAAGCTA
AAAATCCTCTCCTTGGCTTAGGATCATTGGGTCAAACTGGGAGTATATCTGTGTGATTACAACCTGCTGC
TATGAGAAAAAAGAATTGTAACTTGACCATGTTGGCTCACACAGGGCAATGCAAAAGAAGTCAAAACATT
CATATCAGGTCTTGGTATCTGTGTCAGGAGAAACGTGGTGGCTTTCACAGCTTCTTCACCTCTGTCCTCT
GTCATGCAACTCCATTTTTGGCTAGGTACTATTTCACATGCACACTGTCCTGGGTGCCAGAAATACTTGT
GGTGTACTTGGTTTGAGCAGTACCTCTAGTATGCACCATTCCAGGGAACATGGTTGTCAACATAACAAGT
TTAGTGTGTGTATTTGCAGACAACATAGCCCTGAAAGCATGAAATGTAGAATAGGTAAGGTCTGTCTTAG
AAAAGGAGGAGGAAGTACCAATTTGGTGCAAGAAGATCGTGGTGAGGAAGGACATGGGAGCCACAGTGAA
GTGAAAGAGGCTTTAAGGAATCAGATGCACAGATGGATGCTTTGATTTACACATTTATCTAGAGGAGTGA
TTCTCAACCTTGACTGTACATAAGAATCACCTCAGGAAATTTTTAAATTCCCAATTATATTAAAATCATT
TGGAGTGGGAACCAGACACCAATATTTGCGAAAGCCTCCAAGGCGGTTCTAATGTCCAGCCAAAGTTGAT
TATCACTGCTTTATGGCCAACTAAGGAAACCTTGTTTGTCTGCCTTGGCTGGTGCTCCAGCCCCAGTTCT
GAGTGGTCAGGGAAGGCAAGATGTAAGATATAGTAATGGTATTTATTCTGTATGTCTCACAGAACAAAAG
CATTTGAGAATTTCTGACCAGGCAGTGCTCCATCAACTCTTAATAATCATCTTTGTGATATTCTCCTATG
TGACTTGTCCATCAATTATTCCAATTCAAAGGTCTAGGTTTTTTTTCAATTGTCTTACACATGTGACATC
ACAAGATACCAGAAGTAGCACAGTAAATTCAAGAGCATATAAAGCTAAGTGGAAAACTAATTCTTCATAT
TTAGACAGTGGAAATTCTCAAAACAATCATTTGCTAAATTAGGTCCAAATTTATTATTCATTCTTCTTCT
GGAGGAGACATGGGTACCAGGTACCTGTTCTGCTGTAGGTATATTTCACTGGACTTGGTCTTAGCAAAAA
GGAAGAACAAATCTTCCTTTTTGATTGTTATCTCAATCAAAAGCTCCCAGGGCCACTTGGATTTATTTTC
TTAGTCTTTGGACATGTGTTTAAAGGAAAAGAATACCTGTAACTTCCTATATGACATCCAGTGCCCTGAA
AGGGTGCTTACACCTTCAAAAAAGGAGAGTCCAGCCTATCAAAGGCAGTAACACATAATGAGAAGAAACT
CAGAGTTATTGCCTTTCATACTGCTTTCCCTCTGCTGTTGGCCCACCCTTCTTGACTGCAAAGCCAGAAA
ATAAACTAGATGGTGGTTTTGGACAATTGTACACAGGAACAGAATCTCTAATAACCTATAATTTTTAGTA
CTTAATTATGTGATGTTTTATTCTAGGAAATAAGAAACAGATGACATCATTGATGTATCTTTCTTTCATC
TTCAGAATTTTTGCATCTTTTCAGAAAATGACAAATTACTATTTTTCTGTTGCACTCAGCAATTGTGACT
ATACTTAACAAAATTCTTGTAGTCTGTAGAGATATCAATAAAATTGTATATGTTTGTACATA
**SEQ ID NO: 35 - mRNA sequence of syntaxin binding protein 5-like transcript 4(taken from NM_001348344 as at the filing date)** 
GTGCAGCGGGGAGAGCGGACGGCGGGCGGGCGAACAGGCGGGAGAGCGCGGCGGCTGCGGGCAGAGT
GTTTAAAATGAAGAAGTTTAATTTCCGAAAAGTTTTGGATGGCTTAACTGCCTCCTCCCCTGGCAGTGGT
AGCAGCAGTGGCAGTAACAGTGGTGGTGGGGCTGGAAGTGGTTCCGTACATCCGGCGGGGACTGCAGGGG
TTCTCAGAGAGGAAATTCAGGAAACTTTGACTTCGGAGTATTTCCAGATTTGCAAGACAGTTCGGCATGG
TTTTCCTCATCAGCCCACAGCATTAGCCTTTGATCCAGTTCAGAAAATCTTGGCTATTGGGACGAGAACA
 
GGTGCTATACGAATACTCGGGAGACCTGGTGTTGATTGCTATTGCCAACATGAAAGTGGTGCAGCTGTCC
TACAGCTCCAATTTTTGATCAATGAGGGTGCCTTGGTCAGTGCAAGTTCAGATGATACACTTCATTTGTG
GAACCTTAGACAAAAAAGGCCAGCCATACTCCATTCTCTTAAATTTAACCGGGAACGAATTACTTACTGT
CATCTACCTTTCCAGAGTAAATGGCTTTATGTTGGAACAGAAAGAGGAAATACACATATTGTAAATATTG
AATCTTTCATTCTTTCTGGATATGTTATCATGTGGAACAAGGCAATTGAACTATCCACTAAGACTCATCC
AGGTCCAGTTGTACATTTAAGCGATAGCCCAAGAGATGAAGGCAAACTGCTAATAGGTTATGAAAATGGT
ACTGTAGTATTCTGGGACTTGAAATCTAAAAGAGCAGAACTGAGAGTTTATTATGATGAGGCTATTCATT
CAATTGATTGGCATCATGAGGGCAAACAGTTCATGTGCAGCCATTCAGATGGTAGTTTGACTTTATGGAA
CCTGAAAAGCCCAAGTCGCCCTTTCCAGACCACAATTCCACATGGAAAAAGTCAAAGAGAAGGAAGAAAA
TCTGAATCTTGTAAACCAATTCTTAAAGTAGAATACAAGACCTGCAAAAACAGCGAACCATTCATAATAT
TCTCTGGTGGGCTGTCCTATGACAAAGCTTGTAGAAGACCAAGTTTAACCATCATGCATGGAAAAGCAAT
TACAGTACTTGAAATGGATCATCCTATTGTTGAATTTCTAACTTTATGTGAAACGCCCTATCCAAATGAA
TTTCAAGAACCCTATGCTGTCGTGGTACTTCTGGAGAAAGATCTCATTGTAGTTGATCTGACACAAAGCA
ATTTTCCAATCTTTGAAAATCCATATCCCATGGACATTCATGAATCACCAGTTACATGCACAGCATACTT
TGCAGATTGTCCTCCGGATTTGATTCTAGTACTGTATTCTATAGGAGTCAAGCATAAAAAACAAGGATAC
AGTAATAAGGAGTGGCCAATCAGTGGAGGAGCTTGGAACCTTGGAGCACAAACATATCCAGAAATTATTA
TTACTGGTCATGCAGATGGATCAATAAAATTTTGGGATGCTTCTGCAATAACTCTGCAGATGCTGTACAA
GCTAAAAACTTCAAAAGTGTTTGAAAAACAGAAGGTAGGAGAAGGAAAACAAACATGTGAAATTGTAGAG
GAAGACCCATTTGCCATTCAGATGATTTACTGGTGTCCAGAGAGCAGAATATTCTGTGTATCAGGAGTCT
CTGCATATGTCATAATTTATAAATTCAGCAGACATGAAATTACAACAGAAATAGTGTCATTAGAGGTACG
ACTTCAGTATGATGTTGAAGATATTATTACCCCTGAACCAGAAACAAGTCCTCCGTTTCCAGATCTCTCA
GCCCAGCTTCCTTCTTCAAGGAGTCTTTCTGGGAGCACTAACACTGTTGCTAGTGAAGGAGTAACAAAGG
ACAGTATTCCATGCCTCAATGTGAAGACACGGCCAGTGCGAATGCCTCCAGGATATCAAGCAGAACTTGT
TATTCAATTGGTGTGGGTAGATGGTGAACCTCCACAACAGATTACTAGTCTTGCTGTAAGCTCAGCATAT
GCAATAGTTGCATTTGGGAACTGCAATGGGTTGGCTGTGGTGGATTTTATACAGAAGACAGTACTGTTAA
GCATGGGGACCATTGACCTATATAGATCAAGTGACTTATACCAGCGACAACCACGGTCTCCTCGAAAAAA
CAAACAGTTCATTGCAGGTTTAACTGAACTGAATGACAGTCCAGTTCCCCTAGAACTTGAGCGCTGCAAG
TCTCCTACCTCAGACCATGTAAATGGACACTGCACAAGTCCAACTTCTCAGAGTTGCAGTTCTGGAAAAC
GTCTTTCTAGTGCCGATGTTTCAAAAGTAAATCGCTGGGGTCCTGGAAGACCACCATTTCGAAAGGCCCA
GTCAGCAGCCTGCATGGAGATTTCTTTACCAGTTACAACAGAAGAAAACCGAGAAAATTCCTATAATCGT
TCTAGAAGCTCTAGTATCTCCAGTATTGACAAAGATTCTAAAGAAGCAATTACAGCACTATACTTCATGG
ACTCCTTTGCACGGAAAAATGACTCTACCATCTCTCCTTGTCTGTTCGTTGGAACCAGTCTGGGAATGGT
GTTAATCATCTCCTTAAACCTACCATTAGCAGATGAACAAAGGTTTACAGAGCCAGTCATGGTATTGCCA
AGTGGTACATTCCTCTCATTGAAAGGAGCTGTGCTAACATTCTCCTGTATGGACCGAATGGGTGGATTAA
TGCAACCGCCATATGAAGTTTGGAGGGATCCAAACAACATAGATGAAAATGAAAAATCTTGGAGAAGGAA
AGTGGTAATGAACTCATCTTCTGCATCCCAAGAAATAGGAGATCATCAGTATACAATAATCTGCTCAGAA
AAACAAGCCAAAGTCTTCTCACTGCCTTCTCAGACTTGCCTTTATGTTCATAACATCACGGAGACATCTT
TTATACTGCAAGCAAATGTGGTGGTCATGTGTAGCAGTGCCTGCTTGGCATGCTTTTGTGCTAACGGACA
TATCATGATAATGAGCCTACCTAGTCTTCGCCCAATGTTGGATGTTAATTATTTGCCACTGACAGACATG
AGGATAGCACGAACATTTTGTTTTACCAATGAAGGACAGGCATTATACCTCGTCTCTCCTACTGAAATTC
AGCGGTTAACATACAGCCAAGAAATGTGTGATAATCTACAGGACATGCTAGGAGATTTGTTTACTCCCAT
AGAGACACCAGAAGCTCAAAACAGAGGCTTTCTCAAGGGACTGTTTGGTGGAAGCGGACAAACATTTGAC
AGAGAAGAGCTCTTTGGGGAAGCTTCGGCAGGAAAAGCATCCCGCAGCCTTGCGCAACACATTCCTGGAC
CAGGTAGTATAGAAGGGATGAAAGGCGCTGCTGGAGGGGTGATGGGAGAGCTGACCCGTGCACGGATTGC
ACTTGATGAAAGAGGACAGAGGCTAGGAGAGCTGGAAGAGAAAACTGCAGGCATGATGACCAGTGCAGAA
GCATTTTCCAAACATGCACATGAGTTAATGCTGAAATACAAGGATAAGAAATGGTACCAATTCTGACTTC
TAAAGAAGCTGTGACTGCTTTGAGAAACCATATTCAGGGAAACCAAATTTATTCCCAGCATCACTACTCA
ACTGCTAGAAGCCAGTTTCTTCTACAAAATGTTCCATTTACAGTCAATCTGAAATTTTCATAAAAGAGAT
GTATCAGAGACACTGTGCAACCCAAAGGCTGGAGCCCTGGTTAAAATCCCAAAATACGGCTGAATTTGCC
TTTTCCCATGTGGAATGGAGCTATCATCTTGGCATGTCATTCGATAAGGATTTATATTTAGTAACTACGG
GGAGTCCTCTTGATTTGAAAATTCCTGTACAAACAAAAGCATTAATGCACTTAATGAAAAAAAATCTTTG
CATGATAAATTAACATCTTAAGGGGAAAAGAAAAAAAAGCATGTTGTGATAGAAGAAAAAAAGATTCATG
GTAAACTATTTCTATCAATTGGGCCACACTCAACAATTTAAAAAATCTGCATTTGAAGATGTCAGTGCAT
TTCAAATATATTTGCCATACCCAACTGAAACGGAAAACAAAACAAAACAAGAACCCAGAGTTTTCTCCTC
ATCTGTAACTTTCATTGTACTAGAACCTTATTGAATTCTTATGGCAAGAGTCTGATTAATGTTCTCTCTC
CATGTTTTACATCATTCACAGTTGTGTCAAAATCAAATATTGCCAGTATAGTAAATTATTCCCAACACAA
TCAGTTTCATTTCTATCACCTTCAGAATGGGTTGCTGTTTTTAGCACAAACCATGCAGGGTTGGGTCACC
TCAAAGCATTTTCTTCAGCCTGGCTTGTACTCTATAGGGGAGTTGGTAGTAGGTGTGGGGAATCTATAGG
AACAATAGAGTTAGCTATGAGAACTGACTTCACAAAAAGGGAAATTTCACACACCCCAATTTCAGCTTCT
 
AAAGTGATATATTTTAAAAGCTTCATTTGTATCCTTGTCTGCCAGTTACACAGTATATTACTGTTCAAAC
CCAGTAGGGTCTGCAATCGAAAGGTGCTCTTGCAGCCTGACATTCACAAACCAAACTGGTCATTCTCTTT
AGTGTGATTCAGTAAAATCTCAGTTAATGTTTTGGGAAAGGGATTCTGATTTATGACATTTCTGATTTAC
AAGGGCTTTCCTTAGAAAATACCTTTCGGTTTGATACTTACCACTGGAAATTGGTTCGATACTTACCACT
GGAAATCTTTATAAAATATAGTGATCTGTTTGCTATCTTGGTCTCAGTCATCATTATAGACAATAGAAAT
ACATAAAGAGTTTAATGATACAAAATCAATGCTTTTGTCCCTAGGTCAAATAATATAAGCCACTAGATTA
TGTCAATAGATGCCAAACACTTGTCCCTTATTAAAGACTCTTTTAAAGCTGGAATAAAGAATATCCTTAA
CATGATACAAGGTATCTATAACTCACCAGAAAAGCACCAGAGTCAAAACCAAATAGAGACACCTACTACC
ATTTAACATGATTCTGGAAGTCAAAAGTTATAGGAATGTATATATACACATTCCAGGACCCATCCTGTAA
TATGTTGTTTCGTCAAGAAAAGTTTTAATTATGGTTTTGGCTTATTGAATTTTTGTTTATCAAGGTCTCA
TTGGATTTACATTTGTGTTTCTTAAGACTTCACAATTTTCCTGGATTTCAGGTTATTTTATTAGCCAAAA
AATCGTATTTTTATTTCCTTTTTAGTCTAATTTTTTGCTAGAAGTTGTACTTTCTAGCAAATGTTTCTAG
TGGTACAATGTTCTCAGTAATTTTGAGGAGAACAAAGATGAGTGAAAGAGTTAGATAATGTACACTAGGA
TAGCATTTCTCAGAGTCCTTGAATCCTCTTGTTAAGAAAGCCTCTCTGAATCTCCCAGACTCCTGTGAAC
AGCCTTACGGTAACAGAATCTGGCTTGGAAACACAGACATTACATGACTGTATAATTTATAGAATTATAA
AATTATATGGTAAAAATATTAAAGAAACTTAAGAGTAGGATCTAGAAAATACTAGTGGCCAAATTGCAAT
GTATTCTTTGTACCATCTGTTTCAAAGACTTAGTTTTCTGATTTTCAGAACAAATACTTTATCGTTTCTG
TATTTTAAAAAAAACTAAAAGAGTAAAATATTAGTTTATTTTAGAAATAATGAAATTCAGTCCAAAGTAG
CAGTACATTTGATGAGTATTTCTGAAGCCTTCAATAAGATTTAACTTGTTTTAACTTGATCTACATCGAG
ATTTCTCAAAAGCAGCACTATTAGCTGAAAGTCTTTGTTGTGGGGAACTGTCCTGTGTATTGTAGGATGT
TAGCAACATCCCTGGCCTCTGTCTACTAGATGCCAGTAAGAACCTCCTAGTAATGACAATCAAAATGTCT
CCAGTCATTGCCAAAATTTCTCCCAGTTCACAGCCACTATTCTACATCATACAAAGAGGTAGATTGTGTG
AATATGTAATATATGTTGTTGTATAAAACTGTACAATAAGTATACAATGTCTTAGCAAGATAAATTATTA
TACCCAAAATTACATGTGTGGTTCATATGCAATAAGATACAATTTTGAAGAAAGCTTATAGGAATCAGTG
AAAAATAACAACTACAGAATATTTTAAGGCACACAGAGAAAATATCAAAAAGGACATTATAGCATTTCAC
TGTAGAAGTGCTTAAAACCTTGGCCTCAGTGAATAGATCAAAAGTTTATAATTAGCACACCAATTGGAAG
TAAACAGGTGCTTCTCAAATGCTTGAAAAGAAAAGGCTCTTTGAAGTAGTTAAAACGTGTCTTTCAAAAT
TGCTTTTATAAGCCCTTCCTTAACGTAAGGGTAACTTCATCTCACAAACTCCTGGTTAGTGGAATCCAAA
TTAATGAGGTTTTACTGTATCAAAATTATTTTTATAATTCAAAGAGATGATAGCAATAAGAAGAATCTAA
TGTGGATGTGATTTTTCTCTTTGAACAATTCTGTTCTGTTGCACTGACCTTAGCACGCACGCACACACAC
ACACACCATCGCCACCACTACCATCATCATCTTATCCTCATCTTTGGAAAATAAGTACTGTTGATCCATG
TAATCCTTTGCATAGTCTCATCTTTACTATCTTTCTTCTATTTCGGAACATAAGAAAAATACTCCTAATA
ACTTGGACAATGATACAGAAAGTGACTCTGAGCCTTAGTTACCATCAAAATTACATTCTATCTGCTAAAG
CGACATCAGAAGATAAGGCTTGCAAACCACAGTCCCAACAAGTTGACCTTGTCTCCCTATGATTTCCTTG
GAAGATAAGGCCTTTTTAGTGTTACAGTAGAAAATGGGCCTTTTCATTTATTCATCTGTGAGAAGCTAGA
GGTATTTGTTTCCCTGATAATAACGTGAGCAATAGTCCCTACCTTGAAATCATCCATTAGTCTTGGAGTT
TGGAGGGAGGAATGATTGATTCCTCATTTGTTTTAATCACCTACAAAAGCAGTACTACTGACTTTGCTTC
TTACACTTCACTGAGCCCCAGACTTTAGGGCCCCAATCATTGTCAAGATCATTGTCTAACTGGAACTCTG
AAAGGACAGTGACAGAACTGAATGAGATGTTTTCCAGTGATTCAGTCACAAAGACAATTTTCTCAGATCT
TTTGAAAGTGATTCTCAAATAAATAAATCAAAATCCAACTCCTTTCCCTCAAAGTTTCTAGGGATCTGAA
GGAAAAGGTTTACTGTTGGAGTCCCTTTTTATGTGACCCATGTACCAGTTTTAAAGGCATCATCTATACT
TTATCTACATGAAGGGATCACCATGACTTTGAGAGAAAAAAGAATCAGATTAGGCCTAACATTGGTTGAA
GAAGATTAAAATAGATTCTTATATTCAAGCAGAAGGGAAAAATGAAGCCAATCTAGAAAAGTAAGCTAAA
AATCCTCTCCTTGGCTTAGGATCATTGGGTCAAACTGGGAGTATATCTGTGTGATTACAACCTGCTGCTA
TGAGAAAAAAGAATTGTAACTTGACCATGTTGGCTCACACAGGGCAATGCAAAAGAAGTCAAAACATTCA
TATCAGGTCTTGGTATCTGTGTCAGGAGAAACGTGGTGGCTTTCACAGCTTCTTCACCTCTGTCCTCTGT
CATGCAACTCCATTTTTGGCTAGGTACTATTTCACATGCACACTGTCCTGGGTGCCAGAAATACTTGTGG
TGTACTTGGTTTGAGCAGTACCTCTAGTATGCACCATTCCAGGGAACATGGTTGTCAACATAACAAGTTT
AGTGTGTGTATTTGCAGACAACATAGCCCTGAAAGCATGAAATGTAGAATAGGTAAGGTCTGTCTTAGAA
AAGGAGGAGGAAGTACCAATTTGGTGCAAGAAGATCGTGGTGAGGAAGGACATGGGAGCCACAGTGAAGT
GAAAGAGGCTTTAAGGAATCAGATGCACAGATGGATGCTTTGATTTACACATTTATCTAGAGGAGTGATT
CTCAACCTTGACTGTACATAAGAATCACCTCAGGAAATTTTTAAATTCCCAATTATATTAAAATCATTTG
GAGTGGGAACCAGACACCAATATTTGCGAAAGCCTCCAAGGCGGTTCTAATGTCCAGCCAAAGTTGATTA
TCACTGCTTTATGGCCAACTAAGGAAACCTTGTTTGTCTGCCTTGGCTGGTGCTCCAGCCCCAGTTCTGA
GTGGTCAGGGAAGGCAAGATGTAAGATATAGTAATGGTATTTATTCTGTATGTCTCACAGAACAAAAGCA
TTTGAGAATTTCTGACCAGGCAGTGCTCCATCAACTCTTAATAATCATCTTTGTGATATTCTCCTATGTG
ACTTGTCCATCAATTATTCCAATTCAAAGGTCTAGGTTTTTTTTCAATTGTCTTACACATGTGACATCAC
AAGATACCAGAAGTAGCACAGTAAATTCAAGAGCATATAAAGCTAAGTGGAAAACTAATTCTTCATATTT
AGACAGTGGAAATTCTCAAAACAATCATTTGCTAAATTAGGTCCAAATTTATTATTCATTCTTCTTCTGG
 
AGGAGACATGGGTACCAGGTACCTGTTCTGCTGTAGGTATATTTCACTGGACTTGGTCTTAGCAAAAAGG
AAGAACAAATCTTCCTTTTTGATTGTTATCTCAATCAAAAGCTCCCAGGGCCACTTGGATTTATTTTCTT
AGTCTTTGGACATGTGTTTAAAGGAAAAGAATACCTGTAACTTCCTATATGACATCCAGTGCCCTGAAAG
GGTGCTTACACCTTCAAAAAAGGAGAGTCCAGCCTATCAAAGGCAGTAACACATAATGAGAAGAAACTCA
GAGTTATTGCCTTTCATACTGCTTTCCCTCTGCTGTTGGCCCACCCTTCTTGACTGCAAAGCCAGAAAAT
AAACTAGATGGTGGTTTTGGACAATTGTACACAGGAACAGAATCTCTAATAACCTATAATTTTTAGTACT
TAATTATGTGATGTTTTATTCTAGGAAATAAGAAACAGATGACATCATTGATGTATCTTTCTTTCATCTT
CAGAATTTTTGCATCTTTTCAGAAAATGACAAATTACTATTTTTCTGTTGCACTCAGCAATTGTGACTAT
ACTTAACAAAATTCTTGTAGTCTGTAGAGATATCAATAAAATTGTATATGTTTGTACATA
**SEQ ID NO: 36 - mRNA sequence of syntaxin binding protein 5-like transcript 5(taken from NM_001348345 as at the filing date)** 
CCAACGTCGTTTTTCTGAAAATAAAATACTCTGAGCCGAACGTTTCGAAGAAGGAAAGTCCGCCCTTAAG
AGAGAGACAGAGTCTTGTGTTTAAAATGAAGAAGTTTAATTTCCGAAAAGTTTTGGATGGCTTAACTGCC
TCCTCCCCTGGCAGTGGTAGCAGCAGTGGCAGTAACAGTGGTGGTGGGGCTGGAAGTGGTTCCGTACATC
CGGCGGGGACTGCAGGGGTTCTCAGAGAGGAAATTCAGGAAACTTTGACTTCGGAGTATTTCCAGATTTG
CAAGACAGTTCGGCATGGTTTTCCTCATCAGCCCACAGCATTAGCCTTTGATCCAGTTCAGAAAATCTTG
GCTATTGGGACGAGAACAGGTGCTATACGAATACTCGGGAGACCTGGTGTTGATTGCTATTGCCAACATG
AAAGTGGTGCAGCTGTCCTACAGCTCCAATTTTTGATCAATGAGGGTGCCTTGGTCAGTGCAAGTTCAGA
TGATACACTTCATTTGTGGAACCTTAGACAAAAAAGGCCAGCCATACTCCATTCTCTTAAATTTAACCGG
GAACGAATTACTTACTGTCATCTACCTTTCCAGAGTAAATGGCTTTATGTTGGAACAGAAAGAGGAAATA
CACATATTGTAAATATTGAATCTTTCATTCTTTCTGGATATGTTATCATGTGGAACAAGGCAATTGAACT
ATCCACTAAGACTCATCCAGGTCCAGTTGTACATTTAAGCGATAGCCCAAGAGATGAAGGCAAACTGCTA
ATAGGTTATGAAAATGGTACTGTAGTATTCTGGGACTTGAAATCTAAAAGAGCAGAACTGAGAGTTTATT
ATGATGAGGCTATTCATTCAATTGATTGGCATCATGAGGGCAAACAGTTCATGTGCAGCCATTCAGATGG
TAGTTTGACTTTATGGAACCTGAAAAGCCCAAGTCGCCCTTTCCAGACCACAATTCCACATGGAAAAAGT
CAAAGAGAAGGAAGAAAATCTGAATCTTGTAAACCAATTCTTAAAGTAGAATACAAGACCTGCAAAAACA
GCGAACCATTCATAATATTCTCTGGTGGGCTGTCCTATGACAAAGCTTGTAGAAGACCAAGTTTAACCAT
CATGCATGGAAAAGCAATTACAGTACTTGAAATGGATCATCCTATTGTTGAATTTCTAACTTTATGTGAA
ACGCCCTATCCAAATGAATTTCAAGAACCCTATGCTGTCGTGGTACTTCTGGAGAAAGATCTCATTGTAG
TTGATCTGACACAAAGCAATTTTCCAATCTTTGAAAATCCATATCCCATGGACATTCATGAATCACCAGT
TACATGCACAGCATACTTTGCAGATTGTCCTCCGGATTTGATTCTAGTACTGTATTCTATAGGAGTCAAG
CATAAAAAACAAGGATACAGTAATAAGGAGTGGCCAATCAGTGGAGGAGCTTGGAACCTTGGAGCACAAA
CATATCCAGAAATTATTATTACTGGTCATGCAGATGGATCAATAAAATTTTGGGATGCTTCTGCAATAAC
TCTGCAGATGCTGTACAAGCTAAAAACTTCAAAAGTGTTTGAAAAACAGAAGGTAGGAGAAGGAAAACAA
ACATGTGAAATTGTAGAGGAAGACCCATTTGCCATTCAGATGATTTACTGGTGTCCAGAGAGCAGAATAT
TCTGTGTATCAGGAGTCTCTGCATATGTCATAATTTATAAATTCAGCAGACATGAAATTACAACAGAAAT
AGTGTCATTAGAGGTACGACTTCAGTATGATGTTGAAGATATTATTACCCCTGAACCAGAAACAAGTCCT
CCGTTTCCAGATCTCTCAGCCCAGCTTCCTTCTTCAAGGAGTCTTTCTGGGAGCACTAACACTGTTGCTA
GTGAAGGAGTAACAAAGGACAGTATTCCATGCCTCAATGTGAAGACACGGCCAGTGCGAATGCCTCCAGG
ATATCAAGCAGAACTTGTTATTCAATTGGTGTGGGTAGATGGTGAACCTCCACAACAGATTACTAGTCTT
GCTGTAAGCTCAGCATATGGAATAGTTGCATTTGGGAACTGCAATGGGTTGGCTGTGGTGGATTTTATAC
AGAAGACAGTACTGTTAAGCATGGGGACCATTGACCTATATAGATCAAGTGACTTATACCAGCGACAACC
ACGGTCTCCTCGAAAAAACAAACAGTTCATTGCAGGTTTAACTGAACTGAATGACAGTCCAGTTCCCCTA
GAACTTGAGCGCTGCAAGTCTCCTACCTCAGACCATGTAAATGGACACTGCACAAGTCCAACTTCTCAGA
 
GTTGCAGTTCTGGAAAACGTCTTTCTAGTGCCGATGTTTCAAAAGTAAATCGCTGGGGTCCTGGAAGACC
ACCATTTCGAAAGGCCCAGTCAGCAGCCTGCATGGAGATTTCTTTACCAGTTACAACAGAAGAAAACCGA
GAAAATTCCTATAATCGTTCTAGAAGCTCTAGTATCTCCAGTATTGACAAAGATTCTAAAGAAGCAATTA
CAGCACTATACTTCATGGACTCCTTTGCACGGAAAAATGACTCTACCATCTCTCCTTGTCTGTTCGTTGG
AACCAGTCTGGGAATGGTGTTAATCATCTCCTTAAACCTACCATTAGCAGATGAACAAAGGTTTACAGAG
CCAGTCATGGTATTGCCAAGTGGTACATTCCTCTCATTGAAAGGAGCTGTGCTAACATTCTCCTGTATGG
ACCGAATGGGTGGATTAATGCAACCGCCATATGAAGTTTGGAGGGATCCAAACAACATAGATGAAAATGA
AAAATCTTGGAGAAGGAAAGTGGTAATGAACTCATCTTCTGCATCCCAAGAAATAGGAGATCATCAGTAT
ACAATAATCTGCTCAGAAAAACAAGCCAAAGTCTTCTCACTGCCTTCTCAGACTTGCCTTTATGTTCATA
ACATCACGGAGACATCTTTTATACTGCAAGCAAATGTGGTGGTCATGTGTAGCAGTGCCTGCTTGGCATG
CTTTTGTGCTAACGGACATATCATGATAATGAGCCTACCTAGTCTTCGCCCAATGTTGGATGTTAATTAT
TTGCCACTGACAGACATGAGGATAGCACGAACATTTTGTTTTACCAATGAAGGACAGGCATTATACCTCG
TCTCTCCTACTGAAATTCAGCGGTTAACATACAGCCAAGAAATGTGTGATAATCTACAGGACATGCTAGG
AGATTTGTTTACTCCCATAGAGACACCAGAAGCTCAAAACAGAGGCTTTCTCAAGGGACTGTTTGGTGGA
AGCGGACAAACATTTGACAGAGAAGAGCTCTTTGGGGAAGCTTCGGCAGGAAAAGCATCCCGCAGCCTTG
CGCAACACATTCCTGGACCAGGTAGTATAGAAGGGATGAAAGGCGCTGCTGGAGGGGTGATGGGAGAGCT
GACCCGTGCACGGATTGCACTTGATGAAAGAGGACAGAGGCTAGGAGAGCTGGAAGAGAAAACTGCAGGC
ATGATGACCAGTGCAGAAGCATTTTCCAAACATGCACATGAGTTAATGCTGAAATACAAGGATAAGAAAT
GGTACCAATTCTGACTTCTAAAGAAGCTGTGACTGCTTTGAGAAACCATATTCAGGGAAACCAAATTTAT
TCCCAGCATCACTACTCAACTGCTAGAAGCCAGTTTCTTCTACAAAATGTTCCATTTACAGTCAATCTGA
AATTTTCATAAAAGAGATGTATCAGAGACACTGTGCAACCCAAAGGCTGGAGCCCTGGTTAAAATCCCAA
AATACGGCTGAATTTGCCTTTTCCCATGTGGAATGGAGCTATCATCTTGGCATGTCATTCGATAAGGATT
TATATTTAGTAACTACGGGGAGTCCTCTTGATTTGAAAATTCCTGTACAAACAAAAGCATTAATGCACTT
AATGAAAAAAAATCTTTGCATGATAAATTAACATCTTAAGGGGAAAAGAAAAAAAAGCATGTTGTGATAG
AAGAAAAAAAGATTCATGGTAAACTATTTCTATCAATTGGGCCACACTCAACAATTTAAAAAATCTGCAT
TTGAAGATGTCAGTGCATTTCAAATATATTTGCCATACCCAACTGAAACGGAAAACAAAACAAAACAAGA
ACCCAGAGTTTTCTCCTCATCTGTAACTTTCATTGTACTAGAACCTTATTGAATTCTTATGGCAAGAGTC
TGATTAATGTTCTCTCTCCATGTTTTACATCATTCACAGTTGTGTCAAAATCAAATATTGCCAGTATAGT
AAATTATTCCCAACACAATCAGTTTCATTTCTATCACCTTCAGAATGGGTTGCTGTTTTTAGCACAAACC
ATGCAGGGTTGGGTCACCTCAAAGCATTTTCTTCAGCCTGGCTTGTACTCTATAGGGGAGTTGGTAGTAG
GTGTGGGGAATCTATAGGAACAATAGAGTTAGCTATGAGAACTGACTTCACAAAAAGGGAAATTTCACAC
ACCCCAATTTCAGCTTCTAAAGTGATATATTTTAAAAGCTTCATTTGTATCCTTGTCTGCCAGTTACACA
GTATATTACTGTTCAAACCCAGTAGGGTCTGCAATCGAAAGGTGCTCTTGCAGCCTGACATTCACAAACC
AAACTGGTCATTCTCTTTAGTGTGATTCAGTAAAATCTCAGTTAATGTTTTGGGAAAGGGATTCTGATTT
ATGACATTTCTGATTTACAAGGGCTTTCCTTAGAAAATACCTTTCGGTTTGATACTTACCACTGGAAATT
GGTTCGATACTTACCACTGGAAATCTTTATAAAATATAGTGATCTGTTTGCTATCTTGGTCTCAGTCATC
ATTATAGACAATAGAAATACATAAAGAGTTTAATGATACAAAATCAATGCTTTTGTCCCTAGGTCAAATA
ATATAAGCCACTAGATTATGTCAATAGATGCCAAACACTTGTCCCTTATTAAAGACTCTTTTAAAGCTGG
AATAAAGAATATCCTTAACATGATACAAGGTATCTATAACTCACCAGAAAAGCACCAGAGTCAAAACCAA
ATAGAGACACCTACTACCATTTAACATGATTCTGGAAGTCAAAAGTTATAGGAATGTATATATACACATT
CCAGGACCCATCCTGTAATATGTTGTTTCGTCAAGAAAAGTTTTAATTATGGTTTTGGCTTATTGAATTT
TTGTTTATCAAGGTCTCATTGGATTTACATTTGTGTTTCTTAAGACTTCACAATTTTCCTGGATTTCAGG
TTATTTTATTAGCCAAAAAATCGTATTTTTATTTCCTTTTTAGTCTAATTTTTTGCTAGAAGTTGTACTT
 
TCTAGCAAATGTTTCTAGTGGTACAATGTTCTCAGTAATTTTGAGGAGAACAAAGATGAGTGAAAGAGTT
AGATAATGTACACTAGGATAGCATTTCTCAGAGTCCTTGAATCCTCTTGTTAAGAAAGCCTCTCTGAATC
TCCCAGACTCCTGTGAACAGCCTTACGGTAACAGAATCTGGCTTGGAAACACAGACATTACATGACTGTA
TAATTTATAGAATTATAAAATTATATGGTAAAAATATTAAAGAAACTTAAGAGTAGGATCTAGAAAATAC
TAGTGGCCAAATTGCAATGTATTCTTTGTACCATCTGTTTCAAAGACTTAGTTTTCTGATTTTCAGAACA
AATACTTTATCGTTTCTGTATTTTAAAAAAAACTAAAAGAGTAAAATATTAGTTTATTTTAGAAATAATG
AAATTCAGTCCAAAGTAGCAGTACATTTGATGAGTATTTCTGAAGCCTTCAATAAGATTTAACTTGTTTT
AACTTGATCTACATCGAGATTTCTCAAAAGCAGCACTATTAGCTGAAAGTCTTTGTTGTGGGGAACTGTC
CTGTGTATTGTAGGATGTTAGCAACATCCCTGGCCTCTGTCTACTAGATGCCAGTAAGAACCTCCTAGTA
ATGACAATCAAAATGTCTCCAGTCATTGCCAAAATTTCTCCCAGTTCACAGCCACTATTCTACATCATAC
AAAGAGGTAGATTGTGTGAATATGTAATATATGTTGTTGTATAAAACTGTACAATAAGTATACAATGTCT
TAGCAAGATAAATTATTATACCCAAAATTACATGTGTGGTTCATATGCAATAAGATACAATTTTGAAGAA
AGCTTATAGGAATCAGTGAAAAATAACAACTACAGAATATTTTAAGGCACACAGAGAAAATATCAAAAAG
GACATTATAGCATTTCACTGTAGAAGTGCTTAAAACCTTGGCCTCAGTGAATAGATCAAAAGTTTATAAT
TAGCACACCAATTGGAAGTAAACAGGTGCTTCTCAAATGCTTGAAAAGAAAAGGCTCTTTGAAGTAGTTA
AAACGTGTCTTTCAAAATTGCTTTTATAAGCCCTTCCTTAACGTAAGGGTAACTTCATCTCACAAACTCC
TGGTTAGTGGAATCCAAATTAATGAGGTTTTACTGTATCAAAATTATTTTTATAATTCAAAGAGATGATA
GCAATAAGAAGAATCTAATGTGGATGTGATTTTTCTCTTTGAACAATTCTGTTCTGTTGCACTGACCTTA
GCACGCACGCACACACACACACACCATCGCCACCACTACCATCATCATCTTATCCTCATCTTTGGAAAAT
AAGTACTGTTGATCCATGTAATCCTTTGCATAGTCTCATCTTTACTATCTTTCTTCTATTTCGGAACATA
AGAAAAATACTCCTAATAACTTGGACAATGATACAGAAAGTGACTCTGAGCCTTAGTTACCATCAAAATT
ACATTCTATCTGCTAAAGCGACATCAGAAGATAAGGCTTGCAAACCACAGTCCCAACAAGTTGACCTTGT
CTCCCTATGATTTCCTTGGAAGATAAGGCCTTTTTAGTGTTACAGTAGAAAATGGGCCTTTTCATTTATT
CATCTGTGAGAAGCTAGAGGTATTTGTTTCCCTGATAATAACGTGAGCAATAGTCCCTACCTTGAAATCA
TCCATTAGTCTTGGAGTTTGGAGGGAGGAATGATTGATTCCTCATTTGTTTTAATCACCTACAAAAGCAG
TACTACTGACTTTGCTTCTTACACTTCACTGAGCCCCAGACTTTAGGGCCCCAATCATTGTCAAGATCAT
TGTCTAACTGGAACTCTGAAAGGACAGTGACAGAACTGAATGAGATGTTTTCCAGTGATTCAGTCACAAA
GACAATTTTCTCAGATCTTTTGAAAGTGATTCTCAAATAAATAAATCAAAATCCAACTCCTTTCCCTCAA
AGTTTCTAGGGATCTGAAGGAAAAGGTTTACTGTTGGAGTCCCTTTTTATGTGACCCATGTACCAGTTTT
AAAGGCATCATCTATACTTTATCTACATGAAGGGATCACCATGACTTTGAGAGAAAAAAGAATCAGATTA
GGCCTAACATTGGTTGAAGAAGATTAAAATAGATTCTTATATTCAAGCAGAAGGGAAAAATGAAGCCAAT
CTAGAAAAGTAAGCTAAAAATCCTCTCCTTGGCTTAGGATCATTGGGTCAAACTGGGAGTATATCTGTGT
GATTACAACCTGCTGCTATGAGAAAAAAGAATTGTAACTTGACCATGTTGGCTCACACAGGGCAATGCAA
AAGAAGTCAAAACATTCATATCAGGTCTTGGTATCTGTGTCAGGAGAAACGTGGTGGCTTTCACAGCTTC
TTCACCTCTGTCCTCTGTCATGCAACTCCATTTTTGGCTAGGTACTATTTCACATGCACACTGTCCTGGG
TGCCAGAAATACTTGTGGTGTACTTGGTTTGAGCAGTACCTCTAGTATGCACCATTCCAGGGAACATGGT
TGTCAACATAACAAGTTTAGTGTGTGTATTTGCAGACAACATAGCCCTGAAAGCATGAAATGTAGAATAG
GTAAGGTCTGTCTTAGAAAAGGAGGAGGAAGTACCAATTTGGTGCAAGAAGATCGTGGTGAGGAAGGACA
TGGGAGCCACAGTGAAGTGAAAGAGGCTTTAAGGAATCAGATGCACAGATGGATGCTTTGATTTACACAT
TTATCTAGAGGAGTGATTCTCAACCTTGACTGTACATAAGAATCACCTCAGGAAATTTTTAAATTCCCAA
TTATATTAAAATCATTTGGAGTGGGAACCAGACACCAATATTTGCGAAAGCCTCCAAGGCGGTTCTAATG
TCCAGCCAAAGTTGATTATCACTGCTTTATGGCCAACTAAGGAAACCTTGTTTGTCTGCCTTGGCTGGTG
CTCCAGCCCCAGTTCTGAGTGGTCAGGGAAGGCAAGATGTAAGATATAGTAATGGTATTTATTCTGTATG
 
TCTCACAGAACAAAAGCATTTGAGAATTTCTGACCAGGCAGTGCTCCATCAACTCTTAATAATCATCTTT
GTGATATTCTCCTATGTGACTTGTCCATCAATTATTCCAATTCAAAGGTCTAGGTTTTTTTTCAATTGTC
TTACACATGTGACATCACAAGATACCAGAAGTAGCACAGTAAATTCAAGAGCATATAAAGCTAAGTGGAA
AACTAATTCTTCATATTTAGACAGTGGAAATTCTCAAAACAATCATTTGCTAAATTAGGTCCAAATTTAT
TATTCATTCTTCTTCTGGAGGAGACATGGGTACCAGGTACCTGTTCTGCTGTAGGTATATTTCACTGGAC
TTGGTCTTAGCAAAAAGGAAGAACAAATCTTCCTTTTTGATTGTTATCTCAATCAAAAGCTCCCAGGGCC
ACTTGGATTTATTTTCTTAGTCTTTGGACATGTGTTTAAAGGAAAAGAATACCTGTAACTTCCTATATGA
CATCCAGTGCCCTGAAAGGGTGCTTACACCTTCAAAAAAGGAGAGTCCAGCCTATCAAAGGCAGTAACAC
ATAATGAGAAGAAACTCAGAGTTATTGCCTTTCATACTGCTTTCCCTCTGCTGTTGGCCCACCCTTCTTG
ACTGCAAAGCCAGAAAATAAACTAGATGGTGGTTTTGGACAATTGTACACAGGAACAGAATCTCTAATAA
CCTATAATTTTTAGTACTTAATTATGTGATGTTTTATTCTAGGAAATAAGAAACAGATGACATCATTGAT
GTATCTTTCTTTCATCTTCAGAATTTTTGCATCTTTTCAGAAAATGACAAATTACTATTTTTCTGTTGCA
CTCAGCAATTGTGACTATACTTAACAAAATTCTTGTAGTCTGTAGAGATATCAATAAAATTGTATATGTT
TGTACATA
**SEQ ID NO: 37 - Sequence of human PC4 and SFRS1-interacting protein isoform 1(taken from UniProt ID 075475 as at the filing date)** 
MTRDFKPGDL IFAKMKGYPH WPARVDEVPD GAVKPPTNKL PIFFFGTHET
AFLGPKDIFP YSENKEKYGK PNKRKGFNEG LWEIDNNPKV KFSSQQAATK
QSNASSDVEV EEKETSVSKE DTDHEEKASN EDVTKAVDIT TPKAARRGRK
RKAEKQVETE EAGVVTTATA SVNLKVSPKR GRPAATEVKI PKPRGRPKMV
KQPCPSESDI ITEEDKSKKK GQEEKQPKKQ PKKDEEGQKE EDKPRKEPDK
KEGKKEVESK RKNLAKTGVT STSDSEEEGD DQEGEKKRKG GRNFQTAHRR
NMLKGQHEKE AADRKRKQEE QMETEQQNKD EGKKPEVKKV EKKRETSMDS
RLQRIHAEIK NSLKIDNLDV NRCIEALDEL ASLQVTMQQA QKHTEMITTL
KKIRRFKVSQ VIMEKSTMLY NKFKNMFLVG EGDSVITQVL NKSLAEQRQH
EEANKTKDQG KKGPNKKLEK EQTGSKTLNG GSDAQDGNQP QHNGESNEDS
KDNHEASTKK KPSSEERETE ISLKDSTLDN
**SEQ ID NO: 38 - Sequence of human PC4 and SFRS1-interacting protein isoform 2(taken from UniProt ID 075475 as at the filing date)** 
MTRDFKPGDL IFAKMKGYPH WPARVDEVPD GAVKPPTNKL PIFFFGTHET
AFLGPKDIFP YSENKEKYGK PNKRKGFNEG LWEIDNNPKV KFSSQQAATK
QSNASSDVEV EEKETSVSKE DTDHEEKASN EDVTKAVDIT TPKAARRGRK
RKAEKQVETE EAGVVTTATA SVNLKVSPKR GRPAATEVKI PKPRGRPKMV
KQPCPSESDI ITEEDKSKKK GQEEKQPKKQ PKKDEEGQKE EDKPRKEPDK
KEGKKEVESK RKNLAKTGVT STSDSEEEGD DQEGEKKRKG GRNFQTAHRR
NMLKGQHEKE AADRKRKQEE QMETEHQTTC NLQ
**SEQ ID NO: 39 - Sequence of human PC4 and SFRS1-interacting protein isoform 3(taken from UniProt ID 075475 as at the filing date)** 
MTRDFKPGDL IFAKMKGYPH WPARVDEVPD GAVKPPTNKL PIFFFGTHET
AFLGPKDIFP YSENKEKYGK PNKRKGFNEG LWEIDNNPKV KFSSQQAATK
QSNASSDVEV EEKETSVSKE DTDHEEKASN EDVTKAVDIT TPKAARRGRK
RKAEKQVETE EAGVVTTATA SVNLKVSPKR GRPAATEVKI PKPRGRPKMV
KQPCPSESDI ITEEDKSKKK GQEEKQPKKQ PKKDEEGQKE EDKPRKEPDK
KEGKKEVESK RKNLAKTGVT STSDSEEEGD DQEGEKKRKG GRNFQTAHRR
NMLKGQHEKE AADRKRKQEE QMETEHFAL
**SEQ ID NO: 40 - mRNA sequence of human PC4 and SFRS1-interacting protein transcript 1 (taken from NM_021144 as at the filing date)** 
CGGGCCCGGTAGCTGGGCCCGCGTCCGCCCGCATCCCCGCGCCGCCGCATCTCCTCGCCGCCTCCCG
GGCTTCGGACCCCCGGTCTCGCCCCCGAAACATGACTCGCGATTTCAAACCTGGAGACCTCATCTTCGCC
AAGATGAAAGGTTATCCCCATTGGCCAGCTCGAGTAGACGAAGTTCCTGATGGAGCTGTAAAGCCACCCA
CAAACAAACTACCCATTTTCTTTTTTGGAACTCATGAGACTGCTTTTTTAGGACCAAAGGATATATTTCC
TTACTCAGAAAATAAGGAAAAGTATGGCAAACCAAATAAAAGAAAAGGTTTTAATGAAGGTTTATGGGAG
ATAGATAACAATCCAAAAGTGAAATTTTCAAGTCAACAGGCAGCAACTAAACAATCAAATGCATCATCTG
ATGTTGAAGTTGAAGAAAAGGAAACTAGTGTTTCAAAGGAAGATACCGACCATGAAGAAAAAGCCAGCAA
TGAGGATGTGACTAAAGCAGTTGACATAACTACTCCAAAAGCTGCCAGAAGGGGGAGAAAGAGAAAGGCA
GAAAAACAAGTAGAAACTGAGGAGGCAGGAGTAGTGACAACAGCAACAGCATCTGTTAATCTAAAAGTGA
GTCCTAAAAGAGGACGACCTGCAGCTACAGAAGTCAAGATTCCAAAACCAAGAGGCAGACCCAAAATGGT
AAAACAGCCCTGTCCTTCAGAGAGTGACATCATTACTGAAGAGGACAAAAGTAAGAAAAAGGGGCAAGAG
GAAAAACAACCTAAAAAGCAGCCTAAGAAGGATGAAGAGGGCCAGAAGGAAGAAGATAAGCCAAGAAAAG
AGCCGGATAAAAAAGAGGGGAAGAAAGAAGTTGAATCAAAAAGGAAAAATTTAGCTAAAACAGGGGTTAC
TTCAACCTCCGATTCTGAAGAAGAAGGAGATGATCAAGAAGGTGAAAAGAAGAGAAAAGGTGGGAGGAAC
TTTCAGACTGCTCACAGAAGGAATATGCTGAAAGGCCAACATGAGAAAGAAGCAGCAGATCGAAAACGCA
AGCAAGAGGAACAAATGGAAACTGAGCACCAAACAACATGTAATCTACAGTAATAAAAAATATATCTCAT
TTTGGGCTCAAAGCATTAATCCAGTTACTGAAAAGAGAATACAAGTGGAGCAAACAAGAGATGAAGATCT
TGATACAGACTCATTGGACTGAATTTCCCCCTTCCCCCCATTGATGGAAGAATGTTCCAGATTCTAAATT
GAGGACTTCATTATTAATGGCATTACTGTGTTATGATTAACAAATTTCCTGTAAGGTACACACTACATAC
TAAGGTCGGCCATCATTCCTGTTTTTTTTTTTTTTTTTTTTTTTTAACCAAGCTTAAAATGAAGCTTTGT
GTTTGAAAGTAATAACAAGCTCAGACGAAGATGGTGGTTGTACATTATTCATCTAGAAAATATAAAAATT
CATTTTGTTTTGAAGCTAGTTATTAAACTGGAATAGCAGTTATATCCCTGAGAATGGGGCCCTTCTCTTG
ACATTCCTTTGTTGTTTAATTCTTTAGAATCTTAATAAATGTTTTTTTAATCCTGAGAGATTAAACAGTA
GTAGACTTGTTAAGAATGAAACTGTAACCAAAATTTTAAAATAAAGTTTTTTTTAAAAAAAAAAAAAAAA
AAAAAAAAAA
**SEQ ID NO: 41 - mRNA sequence of human PC4 and SFRS1-interacting protein transcript 2 (taken from NM_033222 as at the filing date)** 
AGTTGGTCGCGCCCCAGTGCTAGCGGGCGCCGAGCGGGAGCCGCGCGGGAGCAGCGCAGCTACGGCGGCG
GCAGCGGCGGCGCGGTTGCGATTCCGAGCCGTTGAGACGCCTCTGCGGCAGCTGGTGGCGCAGGTGGCTT
GCGTGGACGCGGGTAGAGGCGACCGGCCAGCAACCGCAGCGTCGGCGCCCGCGGCCCCGGCAGCAGGCGC
GTCGGGACGCCCCGAGGCATCCTCCCCCGCCCGCGGGCCCGGTAGCTGGGCCCGCGTCCGCCGCCCGCAT
CCCCGCGCCGCCGCATCTCCTCGCCGCCTCCCGGGCTTCGGACCCCCGGTCTCGCCCCCGAAACATGACT
CGCGATTTCAAACCTGGAGACCTCATCTTCGCCAAGATGAAAGGTTATCCCCATTGGCCAGCTCGAGTAG
ACGAAGTTCCTGATGGAGCTGTAAAGCCACCCACAAACAAACTACCCATTTTCTTTTTTGGAACTCATGA
GACTGCTTTTTTAGGACCAAAGGATATATTTCCTTACTCAGAAAATAAGGAAAAGTATGGCAAACCAAAT
AAAAGAAAAGGTTTTAATGAAGGTTTATGGGAGATAGATAACAATCCAAAAGTGAAATTTTCAAGTCAAC
AGGCAGCAACTAAACAATCAAATGCATCATCTGATGTTGAAGTTGAAGAAAAGGAAACTAGTGTTTCAAA
GGAAGATACCGACCATGAAGAAAAAGCCAGCAATGAGGATGTGACTAAAGCAGTTGACATAACTACTCCA
AAAGCTGCCAGAAGGGGGAGAAAGAGAAAGGCAGAAAAACAAGTAGAAACTGAGGAGGCAGGAGTAGTGA
CAACAGCAACAGCATCTGTTAATCTAAAAGTGAGTCCTAAAAGAGGACGACCTGCAGCTACAGAAGTCAA
GATTCCAAAACCAAGAGGCAGACCCAAAATGGTAAAACAGCCCTGTCCTTCAGAGAGTGACATCATTACT
GAAGAGGACAAAAGTAAGAAAAAGGGGCAAGAGGAAAAACAACCTAAAAAGCAGCCTAAGAAGGATGAAG
AGGGCCAGAAGGAAGAAGATAAGCCAAGAAAAGAGCCGGATAAAAAAGAGGGGAAGAAAGAAGTTGAATC
AAAAAGGAAAAATTTAGCTAAAACAGGGGTTACTTCAACCTCCGATTCTGAAGAAGAAGGAGATGATCAA
GAAGGTGAAAAGAAGAGAAAAGGTGGGAGGAACTTTCAGACTGCTCACAGAAGGAATATGCTGAAAGGCC
AACATGAGAAAGAAGCAGCAGATCGAAAACGCAAGCAAGAGGAACAAATGGAAACTGAGCAGCAGAATAA
AGATGAAGGAAAGAAGCCAGAAGTTAAGAAAGTGGAGAAGAAGCGAGAAACATCAATGGATTCTCGACTT
CAAAGGATACATGCTGAGATTAAAAATTCACTCAAAATTGATAATCTTGATGTGAACAGATGCATTGAGG
CCTTGGATGAACTTGCTTCACTTCAGGTCACAATGCAACAAGCTCAGAAACACACAGAGATGATTACTAC
ACTGAAAAAAATACGGCGATTCAAAGTTAGTCAGGTAATCATGGAAAAGTCTACAATGTTGTATAACAAG
TTTAAGAACATGTTCTTGGTTGGTGAAGGAGATTCCGTGATCACCCAAGTGCTGAATAAATCTCTTGCTG
AACAAAGACAGCATGAGGAAGCGAATAAAACCAAAGATCAAGGGAAGAAAGGGCCAAACAAAAAGCTAGA
 
GAAGGAACAAACAGGGTCAAAGACTCTAAATGGAGGATCTGATGCTCAAGATGGTAATCAGCCACAACAT
AACGGGGAGAGCAATGAAGACAGCAAAGACAACCATGAAGCCAGCACGAAGAAAAAGCCATCCAGTGAAG
AGAGAGAGACTGAAATATCTCTGAAGGATTCTACACTAGATAACTAGGTTGACATACCTGGAATATAGAG
AACACTTGAGAAGTTTGTAATGGTTTTCATTTGAAATAGACTGCTGAAAGTTTTAAATTTTTATAAGCAT
AGGTTTGATGTTGAAAACTTGTTTTGAGGGAGAAAATCCCTTTGTTTTAAAGTAAAGTAAACATTATCGC
TAAGTGTACTTGTGCAGTATTAACAGCTACATTATACAGTAAATGTGGGATAAAATCCATTTAGAAAATG
TTAAACTGCTTTTCCAGACATGGTTGTAGCATATTTTCAATTAGTGTGTGTATGTTAATGTGTAATTGAT
AGTAGAACAAAGTTACATTTTTAAAACTGCTACTTGTATAAACCTTGCCTCTTTTCCCAAATACTGTGGG
TTTTGTGCATAGTTTTTACAAACCTTGGATTTACCAGACTGTCTTTTCACTGTTTGTGGGTTTTGTAGAA
GTTACACATTTTTATGGTAGATAAAATGTTACTTCTATACAAGTACTCACTCCCTTTTTATCAAAAGTTA
ATTTTAATCTCACAGTCTACATTGTGCTACATTATCCAGCTTCTTTGGAACAATGTGTGCTCTGTATGGT
TTTTTTTGGTATGACAACTAATTAAGCAACTGACATGGAACTGAGAATTCTACAAACTATAAAACATTAA
TTTTTGAAGGTAATTTAGTTTTGTGGCTGGGCATTCAGTGAAGTCTTAGGACTTCTTTGCAGACAACTGA
CTGGGTATATATAGGAATGAATCTGGCTTTAGGGTTAAATCATTTAAGGTCCTTTTATAGGCAGGCACTA
GTAACTAAAACTGAAAACTAAGTAAGTTTATTTTTGAGGAATGTTGTTAAAAATGTCTTTAGGAAGTCAC
TAAAACTTAATTGGAAGAAAAAATCATGATGCTTATACAATAAATATGAATAAATGTTATATAAGGAAAC
TCACCTATTTGAAATCATGGCTATATTGTTTTTATTTTCTAGATTCCAAAAATACAAACACTAGTTGTTC
CAGCATTGTACTTTGATAAGTCTGTACATTGACGTGTATGGACTAAATCCAGGGTAAAATCAATGTTACA
AAATTTAAGGGTATGTTAACTAAAGGATAGCATTTCTAAGATATTTTGAATATTAGGGTCATTTGGCACT
TCTCAGCAAGTAGGATACTTCTCATGTTTTGAAATTATATGAATATGGAAAAAAATGGCTTAAGACCAGC
GTCTCTGTATGACATTGTGTGGTTGACCCTCTGAGATAACTGTTTTCATCTACAGAATTGCATTTTTGCT
TTTAAAGAGGTCTTATAATGGAACTAGGAATCACCGTTTTGAGAGAACCTGCATATATACCAGTCATTAT
CTGTTTGGTCCTTATACAGTTTTAACTTACTTAGATTTATTCTAGTTAAGCCATAAGTTCAACGTGTAAA
CTTGTTTTCATTAAAGAATTTTTCTATCAAAAAAAAAAAAAAAAAAAA
**SEQ ID NO: 42 - mRNA sequence of human PC4 and SFRS1-interacting protein transcript 3 (taken from NM_001128217 as at the filing date)** 
AGTTGGTCGCGCCCCAGTGCTAGCGGGCGCCGAGCGGGAGCCGCGCGGGAGCAGCGCAGCTACGGCGGCG
GCAGCGGCGGCGCGGTTGCGATTCCGAGCCGTTGAGACGCCTCTGCGGCAGCTGGTGGCGCAGGTGGCTT
GCGTGGACGCGGGTAGAGGCGACCGGCCAGCAACCGCAGCGTCGGCGCCCGCGGCCCCGGCAGGCGCGTC
GGGACGCCCCGAGGCATCCTCCCCCGCCCGCGGGCCCGGTAGCTGGGCCCGCGTCCGCCGCCCGCATCCC
CGCGCCGCCGCATCTCCTCGCCGCCTCCCGGGCTTCGGACCCCCGGTCTCGCCCCCGAAACATGACTCGC
GATTTCAAACCTGGAGACCTCATCTTCGCCAAGATGAAAGGTTATCCCCATTGGCCAGCTCGAGTAGACG
AAGTTCCTGATGGAGCTGTAAAGCCACCCACAAACAAACTACCCATTTTCTTTTTTGGAACTCATGAGAC
TGCTTTTTTAGGACCAAAGGATATATTTCCTTACTCAGAAAATAAGGAAAAGTATGGCAAACCAAATAAA
AGAAAAGGTTTTAATGAAGGTTTATGGGAGATAGATAACAATCCAAAAGTGAAATTTTCAAGTCAACAGG
CAGCAACTAAACAATCAAATGCATCATCTGATGTTGAAGTTGAAGAAAAGGAAACTAGTGTTTCAAAGGA
AGATACCGACCATGAAGAAAAAGCCAGCAATGAGGATGTGACTAAAGCAGTTGACATAACTACTCCAAAA
GCTGCCAGAAGGGGGAGAAAGAGAAAGGCAGAAAAACAAGTAGAAACTGAGGAGGCAGGAGTAGTGACAA
CAGCAACAGCATCTGTTAATCTAAAAGTGAGTCCTAAAAGAGGACGACCTGCAGCTACAGAAGTCAAGAT
TCCAAAACCAAGAGGCAGACCCAAAATGGTAAAACAGCCCTGTCCTTCAGAGAGTGACATCATTACTGAA
GAGGACAAAAGTAAGAAAAAGGGGCAAGAGGAAAAACAACCTAAAAAGCAGCCTAAGAAGGATGAAGAGG
GCCAGAAGGAAGAAGATAAGCCAAGAAAAGAGCCGGATAAAAAAGAGGGGAAGAAAGAAGTTGAATCAAA
AAGGAAAAATTTAGCTAAAACAGGGGTTACTTCAACCTCCGATTCTGAAGAAGAAGGAGATGATCAAGAA
GGTGAAAAGAAGAGAAAAGGTGGGAGGAACTTTCAGACTGCTCACAGAAGGAATATGCTGAAAGGCCAAC
ATGAGAAAGAAGCAGCAGATCGAAAACGCAAGCAAGAGGAACAAATGGAAACTGAGCAGCAGAATAAAGA
TGAAGGAAAGAAGCCAGAAGTTAAGAAAGTGGAGAAGAAGCGAGAAACATCAATGGATTCTCGACTTCAA
AGGATACATGCTGAGATTAAAAATTCACTCAAAATTGATAATCTTGATGTGAACAGATGCATTGAGGCCT
TGGATGAACTTGCTTCACTTCAGGTCACAATGCAACAAGCTCAGAAACACACAGAGATGATTACTACACT
GAAAAAAATACGGCGATTCAAAGTTAGTCAGGTAATCATGGAAAAGTCTACAATGTTGTATAACAAGTTT
AAGAACATGTTCTTGGTTGGTGAAGGAGATTCCGTGATCACCCAAGTGCTGAATAAATCTCTTGCTGAAC
AAAGACAGCATGAGGAAGCGAATAAAACCAAAGATCAAGGGAAGAAAGGGCCAAACAAAAAGCTAGAGAA
GGAACAAACAGGGTCAAAGACTCTAAATGGAGGATCTGATGCTCAAGATGGTAATCAGCCACAACATAAC
GGGGAGAGCAATGAAGACAGCAAAGACAACCATGAAGCCAGCACGAAGAAAAAGCCATCCAGTGAAGAGA
GAGAGACTGAAATATCTCTGAAGGATTCTACACTAGATAACTAGGTTGACATACCTGGAATATAGAGAAC
ACTTGAGAAGTTTGTAATGGTTTTCATTTGAAATAGACTGCTGAAAGTTTTAAATTTTTATAAGCATAGG
 
TTTGATGTTGAAAACTTGTTTTGAGGGAGAAAATCCCTTTGTTTTAAAGTAAAGTAAACATTATCGCTAA
GTGTACTTGTGCAGTATTAACAGCTACATTATACAGTAAATGTGGGATAAAATCCATTTAGAAAATGTTA
AACTGCTTTTCCAGACATGGTTGTAGCATATTTTCAATTAGTGTGTGTATGTTAATGTGTAATTGATAGT
AGAACAAAGTTACATTTTTAAAACTGCTACTTGTATAAACCTTGCCTCTTTTCCCAAATACTGTGGGTTT
TGTGCATAGTTTTTACAAACCTTGGATTTACCAGACTGTCTTTTCACTGTTTGTGGGTTTTGTAGAAGTT
ACACATTTTTATGGTAGATAAAATGTTACTTCTATACAAGTACTCACTCCCTTTTTATCAAAAGTTAATT
TTAATCTCACAGTCTACATTGTGCTACATTATCCAGCTTCTTTGGAACAATGTGTGCTCTGTATGGTTTT
TTTTGGTATGACAACTAATTAAGCAACTGACATGGAACTGAGAATTCTACAAACTATAAAACATTAATTT
TTGAAGGTAATTTAGTTTTGTGGCTGGGCATTCAGTGAAGTCTTAGGACTTCTTTGCAGACAACTGACTG
GGTATATATAGGAATGAATCTGGCTTTAGGGTTAAATCATTTAAGGTCCTTTTATAGGCAGGCACTAGTA
ACTAAAACTGAAAACTAAGTAAGTTTATTTTTGAGGAATGTTGTTAAAAATGTCTTTAGGAAGTCACTAA
AACTTAATTGGAAGAAAAAATCATGATGCTTATACAATAAATATGAATAAATGTTATATAAGGAAACTCA
CCTATTTGAAATCATGGCTATATTGTTTTTATTTTCTAGATTCCAAAAATACAAACACTAGTTGTTCCAG
CATTGTACTTTGATAAGTCTGTACATTGACGTGTATGGACTAAATCCAGGGTAAAATCAATGTTACAAAA
TTTAAGGGTATGTTAACTAAAGGATAGCATTTCTAAGATATTTTGAATATTAGGGTCATTTGGCACTTCT
CAGCAAGTAGGATACTTCTCATGTTTTGAAATTATATGAATATGGAAAAAAATGGCTTAAGACCAGCGTC
TCTGTATGACATTGTGTGGTTGACCCTCTGAGATAACTGTTTTCATCTACAGAATTGCATTTTTGCTTTT
AAAGAGGTCTTATAATGGAACTAGGAATCACCGTTTTGAGAGAACCTGCATATATACCAGTCATTATCTG
TTTGGTCCTTATACAGTTTTAACTTACTTAGATTTATTCTAGTTAAGCCATAAGTTCAACGTGTAAACTT
GTTTTCATTAAAGAATTTTTCTATCAAAAAAAAAAAAAAAAAAAA
**SEQ ID NO: 43 - mRNA sequence of human PC4 and SFRS1-interacting protein transcript 4 (taken from NM_001317898 as at the filing date)** 
AGTTGGTCGCGCCCCAGTGCTAGCGGGCGCCGAGCGGGAGCCGCGCGGGAGCAGCGCAGCTACGGCGGCG
GCAGCGGCGGCGCGGTTGCGATTCCGAGCCGTTGAGACGCCTCTGCGGCAGCTGGTGGCGCAGGTGGCTT
GCGTGGACGCGGGTAGAGGCGACCGGCCAGCAACCGCAGCGTCGGCGCCCGCGGCCCCGGCAGGCGCGTC
GGGACGCCCCGAGGCATCCTCCCCCGCCCGCGGGCCCGGTAGCTGGGCCCGCGTCCGCCGCCCGCATCCC
CGCGCCGCCGCATCTCCTCGCCGCCTCCCGGGCTTCGGACCCCCGGTCTCGCCCCCGAAACATGACTCGC
GATTTCAAACCTGGAGACCTCATCTTCGCCAAGATGAAAGGTTATCCCCATTGGCCAGCTCGAGTAGACG
AAGTTCCTGATGGAGCTGTAAAGCCACCCACAAACAAACTACCCATTTTCTTTTTTGGAACTCATGAGAC
TGCTTTTTTAGGACCAAAGGATATATTTCCTTACTCAGAAAATAAGGAAAAGTATGGCAAACCAAATAAA
AGAAAAGGTTTTAATGAAGGTTTATGGGAGATAGATAACAATCCAAAAGTGAAATTTTCAAGTCAACAGG
CAGCAACTAAACAATCAAATGCATCATCTGATGTTGAAGTTGAAGAAAAGGAAACTAGTGTTTCAAAGGA
AGATACCGACCATGAAGAAAAAGCCAGCAATGAGGATGTGACTAAAGCAGTTGACATAACTACTCCAAAA
GCTGCCAGAAGGGGGAGAAAGAGAAAGGCAGAAAAACAAGTAGAAACTGAGGAGGCAGGAGTAGTGACAA
CAGCAACAGCATCTGTTAATCTAAAAGTGAGTCCTAAAAGAGGACGACCTGCAGCTACAGAAGTCAAGAT
TCCAAAACCAAGAGGCAGACCCAAAATGGTAAAACAGCCCTGTCCTTCAGAGAGTGACATCATTACTGAA
GAGGACAAAAGTAAGAAAAAGGGGCAAGAGGAAAAACAACCTAAAAAGCAGCCTAAGAAGGATGAAGAGG
GCCAGAAGGAAGAAGATAAGCCAAGAAAAGAGCCGGATAAAAAAGAGGGGAAGAAAGAAGTTGAATCAAA
AAGGAAAAATTTAGCTAAAACAGGGGTTACTTCAACCTCCGATTCTGAAGAAGAAGGAGATGATCAAGAA
GGTGAAAAGAAGAGAAAAGGTGGGAGGAACTTTCAGACTGCTCACAGAAGGAATATGCTGAAAGGCCAAC
ATGAGAAAGAAGCAGCAGATCGAAAACGCAAGCAAGAGGAACAAATGGAAACTGAGCATTTTGCTCTGTA
AAGCATTTCAGATGCAGCTTGTGTGAAATGCATTATGCAAAATAAAGTTTATTGTATTGTATTCCCAGCC
AAACAACATGTAATCTACAGTAATAAAAAATATATCTCATTTTGGGCTCAAAGCATTAATCCAGTTACTG
AAAAGAGAATACAAGTGGAGCAAACAAGAGATGAAGATCTTGATACAGACTCATTGGACTGAATTTCCCC
CTTCCCCCCATTGATGGAAGAATGTTCCAGATTCTAAATTGAGGACTTCATTATTAATGGCATTACTGTG
TTATGATTAACAAATTTCCTGTAAGGTACACACTACATACTAAGGTCGGCCATCATTCCTGTTTTTTTTT
TTTTTTTTTTTTTTTAACCAAGCTTAAAATGAAGCTTTGTGTTTGAAAGTAATAACAAGCTCAGACGAAG
ATGGTGGTTGTACATTATTCATCTAGAAAATATAAAAATTCATTTTGTTTTGAAGCTAGTTATTAAACTG
GAATAGCAGTTATATCCCTGAGAATGGGGCCCTTCTCTTGACATTCCTTTGTTGTTTAATTCTTTAGAAT
CTTAATAAATGTTTTTTTAATCCTGAGAGATTAAACAGTAGTAGACTTGTTAAGAATGAAACTGTAACCA
AAATTTTAAAATAAAGTTTTTTTTAAAAAAAAAAAAAAAAAAAAAAAAAAAAA
**SEQ ID NO: 44 - mRNA sequence of human PC4 and SFRS1-interacting protein transcript 5 (taken from NM_001317900 as at the filing date)** AGTTGGTCGCGCCCCAGTGCTAGCGGGCGCCGAGCGGGAGCCGCGCGGGAGCAGCGCAGCTACGGCGGCG 
GCAGCGGCGGCGCGGTTGCGATTCCGAGCCGTTGAGACGCCTCTGCGGCAGCTGGTGGCGCAGGTGGCTT
GCGTGGACGCGGGTAGAGGCGACCGGCCAGCAACCGCAGCGTCGGCGCCCGCGGCCCCGGCAGGCGCGTC
GGGACGCCCCGAGGCATCCTCCCCCGCCCGCGGGCCCGGTAGCTGGGCCCGCGTCCGCCGCCCGCATCCC
CGCGCCGCCGCATCTCCTCGCCGCCTCCCGGGCTTCGGACCCCCGGTCTCGCCCCCGAAACATGACTCGC
GATTTCAAACCTGGAGACCTCATCTTCGCCAAGATGAAAGGTTATCCCCATTGGCCAGCTCGAGTAGACG
AAGTTCCTGATGGAGCTGTAAAGCCACCCACAAACAAACTACCCATTTTCTTTTTTGGAACTCATGAGAC
TGCTTTTTTAGGACCAAAGGATATATTTCCTTACTCAGAAAATAAGGAAAAGTATGGCAAACCAAATAAA
AGAAAAGGTTTTAATGAAGGTTTATGGGAGATAGATAACAATCCAAAAGTGAAATTTTCAAGTCAACAGG
CAGCAACTAAACAATCAAATGCATCATCTGATGTTGAAGTTGAAGAAAAGGAAACTAGTGTTTCAAAGGA
AGATACCGACCATGAAGAAAAAGCCAGCAATGAGGATGTGACTAAAGCAGTTGACATAACTACTCCAAAA
GCTGCCAGAAGGGGGAGAAAGAGAAAGGCAGGAGTAGTGACAACAGCAACAGCATCTGTTAATCTAAAAG
TGAGTCCTAAAAGAGGACGACCTGCAGCTACAGAAGTCAAGATTCCAAAACCAAGAGGCAGACCCAAAAT
GGTAAAACAGCCCTGTCCTTCAGAGAGTGACATCATTACTGAAGAGGACAAAAGTAAGAAAAAGGGGCAA
GAGGAAAAACAACCTAAAAAGCAGCCTAAGAAGGATGAAGAGGGCCAGAAGGAAGAAGATAAGCCAAGAA
AAGAGCCGGATAAAAAAGAGGGGAAGAAAGAAGTTGAATCAAAAAGGAAAAATTTAGCTAAAACAGGGGT
TACTTCAACCTCCGATTCTGAAGAAGAAGGAGATGATCAAGAAGGTGAAAAGAAGAGAAAAGGTGGGAGG
AACTTTCAGACTGCTCACAGAAGGAATATGCTGAAAGGCCAACATGAGAAAGAAGCAGCAGATCGAAAAC
GCAAGCAAGAGGAACAAATGGAAACTGAGCACCAAACAACATGTAATCTACAGTAATAAAAAATATATCT
CATTTTGGGCTCAAAGCATTAATCCAGTTACTGAAAAGAGAATACAAGTGGAGCAAACAAGAGATGAAGA
TCTTGATACAGACTCATTGGACTGAATTTCCCCCTTCCCCCCATTGATGGAAGAATGTTCCAGATTCTAA
ATTGAGGACTTCATTATTAATGGCATTACTGTGTTATGATTAACAAATTTCCTGTAAGGTACACACTACA
TACTAAGGTCGGCCATCATTCCTGTTTTTTTTTTTTTTTTTTTTTTTTAACCAAGCTTAAAATGAAGCTT
TGTGTTTGAAAGTAATAACAAGCTCAGACGAAGATGGTGGTTGTACATTATTCATCTAGAAAATATAAAA
ATTCATTTTGTTTTGAAGCTAGTTATTAAACTGGAATAGCAGTTATATCCCTGAGAATGGGGCCCTTCTC
TTGACATTCCTTTGTTGTTTAATTCTTTAGAATCTTAATAAATGTTTTTTTAATCCTGAGAGATTAAACA
GTAGTAGACTTGTTAAGAATGAAACTGTAACCAAAATTTTAAAATAAAGTTTTTTTTAAAAAAAAAAAAA
AAAAAAAAAAAAA
**SEQ ID NO: 45 - Protein sequence of human neuronal tyrosine-phosphorylated phosphoinositide-3-kinase adaptor 2 isoform 1 (UniProt ID Q9P242 as at the filing date)** 
MISSKMMSSN PEEDPLDTFL QYIEDMGMKA YDGLVIQNAS DIARENDRLR
NETNLAYLKE KNEKRRRQEE AIKRIGGEVG RGHEGSYVGK HFRMGFMTMP
APQDRLPHPC SSGFSVRSQS LHSVGGTDDD SSCGSRRQPP PKPKRDPSTK
LSTSSETVSS TAASKSGKTP ERTEASAKPR PHSDEYSKKI PPPKPKRNPN
TQLSTSFDET YIKKHGPRRT SLPRDSSLSQ MGSPAGDPEE EEPVYIEMVG
NILRDFRKED DDQSEAVYEE MKYPIFDDLG QDAKCDFDHH SCSSQCATPT
VPDLDFAKAS VPCPPKGLLC DIPPPFPNLL SHRPPLLVFP PAPVHCSPNS
DESPLTPLEV TKLPVLENVS YMKQPAGASP STLPSHVPGH AKLEKEQAAA
LGPASATPAL SSSPPPPSTL YRTQSPHGYP KSHSTSPSPV SMGRSLTPLS
LKRPPPYDAV HSGSLSRSSP SVPHSTPRPV SQDGAKMVNA AVNTYGAAPG
GSRSRTPTSP LEELTSLFSS GRSLLRKSSS GRRSKEPAEK STEELKVRSH
STEPLPKLDN KERGHHGASS SREPVKAQEW DGTPGTPVVT SRLGRCSVSP
TLLAGNHSSE PKVSCKLGRS ASTSGVPPPS VTPLRQSSDL QQSQVPSSLA
NRD
**SEQ ID NO: 46 - Protein sequence of human neuronal tyrosine-phosphorylated phosphoinositide-3-kinase adaptor 2 isoform 2 (UniProt ID Q9P242 as at the filing date)** 
MISSKMMSSN PEEDPLDTFL QYIEDMGMKA YDGLVIQNAS DIARENDRLR
NETNLAYLKE KNEKRRRQEE AIKRINRGTE SPKPQHGAIT KVGQQGKRPP
WGVFLQRACQ SSGMGWNTRA TCGHQSTRKM LCEPHLVSGK PQFRA
**SEQ ID NO: 47 - Protein sequence of human neuronal tyrosine-phosphorylated phosphoinositide-3-kinase adaptor 2 isoform 3 (UniProt ID Q9P242 as at the filing date)** 
MVNAAVNTYG AAPGGSRSRT PTSPLEELTS LFSSGRSLLR KSSSGRRSKE
PAEKSTEELK VRSHSTEPLP KLDNKERGHH GASSSREPVK AQEWDGTPGT
PVVTSRLGRC SVSPTLLAGN HSSEPKVSCK LGRSASTSGV PPPSVTPLRQ
SSDLQQSQI
**SEQ ID NO: 48 - mRNA sequence of human neuronal tyrosine-phosphorylated phosphoinositide-3-kinase adaptor 2 (NM_020864 as at the filing date)** 
TAATTGAAGAAACACTGAGCTGAACTACAAGTCTATTTCTTATGGTGCTGGATTACTCCTACAGAACTGC
CCTGAGATCAACCGTGAGAGAGGGCTCTGACAGACACAAGTCACCTTCTGGTTATTGCACTTAGCTCTCC
CTGGGGACTTAAATTTTGGCAGTGTTCCTCTTTACATGATATCCTCCAAGATGATGAGTTCTAATCCTGA
GGAAGACCCTTTGGACACATTTCTCCAGTACATTGAGGATATGGGGATGAAGGCCTATGATGGCTTGGTT
ATTCAGAATGCGTCAGATATTGCTCGAGAGAATGATCGCTTGAGAAATGAAACTAACCTAGCCTATTTGA
AAGAGAAGAATGAAAAACGCCGAAGACAAGAAGAAGCAATAAAGCGCATAGGTGGAGAAGTAGGGCGAGG
CCACGAAGGAAGTTACGTGGGCAAACATTTCCGCATGGGATTCATGACAATGCCTGCTCCTCAGGACAGA
CTTCCCCATCCTTGCTCCAGTGGCTTTTCTGTGAGATCACAGTCCCTGCACTCGGTTGGGGGCACAGACG
ATGACAGCAGCTGTGGCTCACGGAGACAACCACCAGCCAAACCCAAGAGGGACCCCAGCACCAAGCTGAG
CACCTCATCAGAGACAGTCAGCAGCACTGCAGCCAGTAAGAGCGGAAAAACCCCTGAGAGGACTGAAGCG
TCAGCTAAACCAAGACCCCACAGCGATGAATATTCCAAGAAGATTCCTCCTCCCAAACCGAAGCGAAATC
CGAACACTCAGCTGAGCACATCTTTCGATGAAACGTACATCAAAAAGCATGGGCCCCGGAGGACGTCGCT
GCCGCGGGACTCCTCCTTGTCCCAGATGGGCAGCCCCGCGGGAGACCCCGAGGAAGAGGAGCCCGTGTAC
ATCGAGATGGTGGGGAACATTCTCAGAGACTTCAGGAAGGAGGACGATGACCAGAGCGAGGCCGTCTACG
AGGAAATGAAGTACCCTATCTTTGACGACTTGGGCCAAGACGCCAAATGTGACTTCGACCATCACAGCTG
TTCTTCGCAGTGTGCTACTCCCACGGTGCCTGACTTGGACTTCGCCAAGGCCTCAGTGCCATGCCCCCCC
AAGGGGCTGCTTTGCGACATCCCTCCGCCCTTCCCCAACCTGCTTTCTCACAGACCCCCGCTGCTGGTAT
TTCCCCCCGCCCCCGTGCATTGCTCCCCCAACTCCGACGAGTCCCCGCTTACCCCTCTGGAGGTCACGAA
GCTTCCCGTGCTGGAAAACGTGTCTTACATGAAACAGCCAGCCGGGGCGTCGCCCTCCACGCTGCCGTCC
CACGTCCCCGGCCATGCGAAACTGGAGAAAGAGCAGGCCGCGGCCCTGGGACCTGCCTCTGCCACCCCTG
CGCTCTCCTCGTCGCCCCCACCCCCGTCTACGCTGTACCGAACCCAGTCTCCCCATGGCTACCCTAAAAG
TCACTCCACCTCTCCCTCCCCCGTCAGCATGGGGAGGTCCCTGACTCCCCTGAGCCTCAAAAGGCCTCCC
CCTTACGACGCTGTGCATTCGGGCAGCCTCTCAAGGAGCTCTCCTTCAGTGCCTCACTCGACCCCCAGAC
CCGTGTCGCAAGATGGGGCCAAGATGGTCAACGCCGCGGTGAACACCTACGGGGCAGCCCCGGGTGGCTC
CCGGTCCCGGACACCCACGAGCCCGCTGGAGGAGCTGACCAGCCTCTTCTCCTCCGGCCGCAGCCTGCTG
CGCAAGTCGTCCAGTGGCCGGCGCTCCAAAGAGCCTGCAGAGAAATCAACAGAGGAACTGAAAGTCCGAA
GCCACAGCACGGAGCCATTACCAAAGTTGGACAACAAGGAAAGAGGCCACCATGGGGCGTCTTCCTCCAG
AGAGCCTGTCAAAGCTCAGGAATGGGATGGAACACCAGGGACACCTGTGGTCACCAGTCGACTAGGAAGA
TGCTCTGTGAGCCCCACCTTGTTAGCGGGAAACCACAGTTCAGAGCCTAAAGTAAGCTGCAAATTAGGCC
GGTCTGCGTCGACGTCAGGTGTGCCTCCTCCATCAGTCACTCCCCTCAGGCAAAGCAGTGACCTGCAACA
GAGCCAGGTACCATCATCGTTAGCCAATCGTGATTGACTTCCTGTGATACAACTTGCCAAATGCTTCCCA
CCTCTGTCTGTCCTGTTGCTGTAGACAACTTTCGCATTTGCTTTTATTTTTCTATGTGTGTATGGGTTAG
GGGATGCGGGGGATGAGTTCTGGCAGTCTGTGTTTTCATTTGAAAAAGAATATCTTTCTTCCTTGTGATT
GGTGGTGAAACTTTCTTTGCTGTTTGTTACCAAATCGTTTTTGTCTCTGGTTTCCATCATTCTGTAATAT
AAATGTAGTAAACTTGTACTATATGTATTGGCTTAGTGGTTCTTTTTTAAATTCTTTCTCTCTTTCATGT
TTTGTGTACTTTTATACTGTCTCTGAAAATTTATCAATATTTGATAAATTTATCTACTTTGTTTTATGTA
GATTTCTTTTTAAATGTTTTGTCCAGAACACTCGCACAGATGTTGTCAATGAATTTGTACATATTTCTTA
GCTCTTATCCTATTATACTGTAATATTTCTGGTGGTTTTATTTTTATTTAGCTTGGAGCATGACTGTAAG
ACACTGTTGAATATTGATGTCCTTATAAATATTCATATCCCGATTCATTTGGATTGAGTATGGCAGCTAG
TCTTTCTTCTTTCTTAGGCTATTGACTGGCCTAAGACAATTTGACTGGCCAGACAAATTGACTGGCCAGA
TAATCTAGATATTTAACAAAAACTGCAGATTAATAAGGCAACCTTTAAATGAATGACTTTTCTCTCTTAT
ACCAACAATATCAGAAATGTTCTCAGAAAGGGAATGTAAGTGTTCATGCATGGTAAATGAGATCTCAATT
ATCACTTGGAGAAAAGAGACAAGAAATAAAGGCATAAACTGAAATATCATTTAATCCTTTACAGCATAAT
ATGTTGCTCTGATGTTCGTTTGGGTACGTGGTTGTGGATGGGGAATTAGTATGGGGAAAAATCACTACAC
ATAAATGTCCTATCTTTAGCTCACCCAATAGGAATTCAATACATTGACTTAATTTGTGAGGCTTAATTGT
CGTTACTGTTAAGTATTATAGGTGTTAAGTAGGGTGGTGTCATTCTGGAATGTTTTCTCTCTGCTTCCTA
GCTTCAATCTTTGCATTCATGAAACTCTTCTGAAATAGCAACTTATAAAACACTGATGATACCTCCAAGG
GAACTGCCCATTACTGATGAGAAAATTACATATTCATCTATTATTTTAAATGTCAGGCTATTTTAAAAAC
ATAACTAAGTAGAATAATTGCGTTTTCTTCTAATGAGAGACATTGTGCCTCTTAGTGTTTTTGTCTGACT
TAAATATGCAAAATAGTTGATTTATAAATATATGAGGTATCTGCAAATACAAGAAATGAGAGGCTTCTCT
CAAGGGTATCTCAAGTACCATTTAGAATTTCTTGTGTCTTAATTTAAAATTTAAATGCCTTTATATAAAT
 
GTTAAATGCCTTTATAACTAAATGTACCAACTCAAACACTTTTTGGATATAAAAGAAGTAGAAACAGTAA
GACACTGAATAAAATAAATAAGATAAACTGCCAACTTAGCTAATTAAAGCTATTCCAAAAATATTGTACT
TACCAACATTTAAAGCTTAAAAACATTGGGTACTGAAAGAAGAGAAGTTTAGCTAATTGGCAGAGGATTG
CACTAATACAATCAAGTTTTCAAGTTTATGACCCTTGCTAGTATATTACCTTCAATATCTCAGAGATGTT
TTGTATTATTTGTTTTGTTTTGCTTTTTTCCTAGTTGTCTTTATAGCTGTTTCACCCTAAGCCCCTTCAA
ACTCTCAATGAAAGCAGGTTCTTGGGATAAACTTCCAGAATAGAGACAAGGTATACCCTTTGTGCCTTTG
CATTATCAACTCTTTGTTCACCTGATGGGAAGTTCTTCGTTTTTCAAAATGTAGCAAGGGAGAAAGCCCA
GGACGCCTTTATATGCTGTTAGTTTCCTTACCTGCTGATAGAGATTCTGACACACAGTCAAATCATACAT
GGGCTGTCAGAGCTATAAATTAGAAGGCTGGCCTCTAGGCTTCTCCTCTGTGGCTTATAGCCAGTTGTAA
TATACATGCATTCCTATACTCTAGAGATGAAGTGGTAAGCATAGCTCATATGAACACTGCTCTGAACTCC
TCTGACTTAGCATTCAACTTAAGTCAAGAAATACTTATTGGCTGGGCGTGGTGGCTCACGCCTGTAATCC
CAGCACTCTGGGAGGCAGAGGTGGGTGGATCACAAGGTCAGGAGATTGAGACCATCCTGGCTAACACGGT
GAGACCCCATCTCTACTAAAAATACAAAAAATTAGCCAGGTGTGGTGGCGGGCGCCTGTAGTCCCAGCTA
CTTGGGAGGCTGAGGCAGGAGAATGTGGTGAACCTGGGAGGTGGAGCTTGCAGTGAGCTGAGATCGCACC
ACTGCACTCCAGCCTGGGTGACAGAGCGAGACTCCATCTC
**SEQ ID NO: 49 - Protein sequence of human catenin delta-2 isoform 1 (UniProt ID Q9UQB3 as at the filing date)**
**SEQ ID NO: 50 - Protein sequence of human catenin delta-2 isoform 2 (UniProt ID Q9UQB3 as at the filing date)** 
MFARKPPGAA PLGAMPVPDQ PSSASEKTSS LSPGLNTSNG DGSETETTSA
ILASVKEQEL QFERLTRELE AERQIVASQL ERCKLGSETG SMSSMSSAEE
QFQWQSQDGQ KDIEDELTTG LELVDSCIRS LQESGILDPQ DYSTGERPSL
LSQSALQLNS KPEGSFQYPA SYHSNQTLAL GETTPSQLPA RGTQARATGQ
SFSQGTTSRA GHLAGPEPAP PPPPPPREPF APSLGSAFHL PDAPPAAAAA
ALYYSSSTLP APPRGGSPLA APQGGSPTKL QRGGSAPEGA TYAAPRGSSP
KQSPSRLAKS YSTSSPINIV VSSAGLSPIR VTSPPTVQST ISSSPIHQLS
STIGTYATLS PTKRLVHASE QYSKHSQELY ATATLQRPGS LAAGSRASYS
SQHGHLGPEL RALQSPEHHI DPIYEDRVYQ KPPMRSLSQS QGDPLPPAHT
GTYRTSTAPS SPGVDSVPLQ RTGSQHGPQN AAAATFQRAS YAAGPASNYA
DPYRQLQYCP SVESPYSKSG PALPPEGTLA RSPSIDSIQK DPREFGWRDP
 
ELPEVIQMLQ HQFPSVQSNA AAYLQHLCFG DNKIKAEIRR QGGIQLLVDL
LDHRMTEVHR SACGALRNLV YGKANDDNKI ALKNCGGIPA LVRLLRKTTD
LEIRELVTGV LWNLSSCDAL KMPIIQDALA VLTNAVIIPH SGWENSPLQD
DRKIQLHSSQ VLRNATGCLR NVSSAGEEAR RRMRECDGLT DALLYVIQSA
LGSSEIDSKT VENCVCILRN LSYRLAAETS QGQHMGTDEL DGLLCGEANG
KDAESSGCWG KKKKKKKSQD QWSVYIRAAV RKEKGLPILV ELLRIDNDRV
VCAVATALRN MALDVRNKEL IGKYAMRDLV HRLPGGNNSN NTASKAMSDD
TVTAVCCTLH EVITKNMENA KALRDAGGIE KLVGISKSKG DKHSPKVVKA
ASQVLNSMWQ YRDLRSLYKK DGWSQYHFVA SSSTIERDRQ RPYSSSRTPS
ISPVRVSPNN RSASAPASPR EMISLKERKT DYECTGSNAT YHGAKGEHTS
RKDAMTAQNT GISTLYRNSY GAPAEDIKHN QVSAQPVPQE PSRKDYETYQ
PFQNSTRNYD ESFFEDQVHH RPPASEYTMH LGLKSTGNYV DFYSAARPYS
ELNYETSHYP ASPDSWV
**SEQ ID NO: 51 - mRNA. sequence of human catenin delta-2 transcript 1 (NM_001332 as at the filing date)** 
GCGTCGGGAGCCGCCTCTCGCCCGCGGCGCTCGCCCCTGCCGCCCGCCAGCATCCCTTGTCCCGCGGCCG
CGCTCAGACAACAAAAGCGGAAGATGCTGCAGTTGGGCAAGGTCAGGACCTTGCCCTGAAGCCGGGCGGC
GCCGCGCACGCCTCTTCCCGGACTGAGGAGCTGTCGCCGGCGGAGGGTGCATGTTTGCGAGGAAGCCGCC
GGGCGCCGCGCCTTTGGGAGCTATGCCTGTTCCAGACCAGCCTTCATCAGCCTCAGAGAAGACGAGTTCC
CTGAGCCCCGGCTTAAACACCTCCAACGGGGATGGCTCTGAAACAGAAACCACCTCTGCCATCCTCGCCT
CAGTCAAAGAACAGGAATTACAGTTTGAAAGGCTGACCCGAGAGCTGGAGGCTGAACGGCAGATCGTAGC
CAGCCAGCTGGAGCGATGCAAGCTCGGATCCGAGACTGGCAGCATGAGCAGCATGAGTTCAGCAGAAGAG
CAGTTTCAGTGGCAGTCACAAGATGGTCAAAAAGATATCGAAGATGAGCTTACAACAGGTCTCGAGCTGG
TGGACTCCTGTATTAGGTCACTACAGGAATCAGGAATACTTGACCCACAGGATTATTCTACAGGTGAAAG
GCCCAGCCTGCTCTCCCAGAGTGCACTTCAGCTCAATTCCAAACCTGAAGGGTCTTTCCAGTATCCGGCC
AGCTACCATAGCAACCAGACCCTGGCCCTGGGGGAAACCACCCCTTCACAGCTCCCGGCCCGAGGCACAC
AAGCCCGAGCTACGGGCCAGAGCTTCAGCCAGGGCACGACCAGCCGCGCCGGCCACCTGGCGGGGCCCGA
GCCCGCGCCGCCGCCGCCGCCGCCGCCGCGGGAGCCGTTCGCGCCCAGCCTGGGCAGCGCCTTCCACCTG
CCCGACGCGCCGCCCGCCGCCGCCGCCGCGCTCTACTACTCCAGCTCCACGCTGCCCGCGCCGCCGC
GCGGGGGCTCCCCGCTGGCCGCGCCCCAGGGCGGTTCGCCCACCAAGCTGCAGCGCGGCGGCTCGGCCCC
CGAGGGCGCCACCTACGCCGCGCCGCGCGGCTCCTCGCCCAAGCAGTCGCCCAGCCGCCTGGCCAAGTCC
TACAGCACCAGCTCGCCCATCAACATCGTCGTGTCCTCGGCCGGCCTGTCCCCGATCCGCGTGACCTCGC
CCCCCACCGTGCAGTCCACCATCTCCTCCTCGCCCATCCACCAGCTGAGCTCCACCATCGGCACGTACGC
CACCCTGTCGCCCACCAAGCGCCTGGTCCACGCGTCCGAGCAGTACAGCAAGCACTCGCAGGAGCTGTAT
GCCACGGCCACCCTCCAGAGGCCGGGCAGCCTGGCAGCTGGTTCCCGAGCCTCATACAGCAGCCAGCATG
GGCACCTGGGCCCAGAGTTGCGGGCCCTGCAGTCCCCAGAACACCACATAGATCCCATCTATGAAGACCG
CGTCTATCAGAAGCCCCCTATGAGGAGTCTCAGCCAGAGCCAGGGGGACCCTCTGCCGCCAGCACACACC
GGCACCTACCGCACGAGCACAGCCCCATCTTCCCCTGGTGTCGACTCCGTCCCCTTGCAGCGCACAGGCA
GCCAGCACGGCCCACAGAATGCCGCCGCGGCCACCTTCCAGAGGGCCAGCTATGCCGCCGGCCCAGCCTC
CAATTACGCGGACCCCTACCGACAGCTGCAGTATTGTCCCTCTGTTGAGTCTCCATACAGCAAATCCGGC
CCTGCTCTCCCGCCTGAAGGCACCTTGGCCAGGTCCCCGTCCATTGATAGCATTCAGAAAGATCCCAGAG
AATTTGGATGGAGAGACCCGGAACTGCCGGAAGTGATTCAGATGTTGCAGCACCAGTTTCCCTCGGTCCA
GTCTAACGCGGCAGCCTACTTGCAACACCTCTGTTTTGGAGACAACAAAATTAAAGCCGAGATAAGGAGA
CAAGGAGGCATCCAGCTCCTGGTGGACCTGTTGGATCATCGGATGACCGAAGTCCACCGTAGTGCCTGTG
GAGCTCTGAGAAACCTGGTGTATGGGAAGGCCAACGATGATAACAAAATTGCCCTGAAAAACTGTGGTGG
CATCCCAGCACTGGTGAGGTTACTCCGCAAGACGACTGACCTGGAGATCCGGGAGCTGGTCACAGGAGTC
CTTTGGAACCTCTCCTCATGCGATGCACTCAAAATGCCAATCATCCAGGATGCCCTAGCAGTACTGACCA
ACGCGGTGATTATCCCCCACTCAGGCTGGGAAAATTCGCCTCTTCAGGATGATCGGAAAATACAGCTGCA
TTCATCACAGGTGCTGCGTAACGCCACCGGGTGCCTAAGGAATGTTAGTTCGGCCGGAGAGGAGGCCCGC
AGAAGGATGAGAGAGTGTGATGGGCTTACGGATGCCTTGCTGTACGTGATCCAGTCTGCGCTGGGGAGCA
GTGAGATCGATAGCAAGACCGTTGAAAACTGTGTGTGCATTTTAAGGAACCTCTCGTACCGGCTGGCGGC
AGAAACGTCTCAGGGACAGCACATGGGCACGGACGAGCTGGACGGGCTACTCTGTGGCGAGGCCAATGGC
AAGGATGCTGAGAGCTCTGGGTGCTGGGGCAAGAAGAAGAAGAAAAAGAAATCCCAAGATCAGTGGGATG
GAGTAGGACCTCTTCCAGACTGTGCTGAACCACCAAAAGGGATCCAGATGCTGTGGCACCCATCAATAGT
CAAACCCTACCTCACACTGCTCTCTGAGTGCTCAAATCCAGACACGCTGGAAGGGGCGGCAGGCGCCCTG
CAGAACTTGGCTGCAGGGAGCTGGAAGTGGTCAGTATATATCCGAGCCGCTGTCCGAAAAGAGAAAGGCC
 
TGCCCATCCTCGTGGAGCTGCTCCGAATAGACAATGACCGTGTGGTGTGCGCGGTGGCCACTGCGCTGCG
GAACATGGCCTTGGACGTCAGAAATAAGGAGCTCATCGGCAAATACGCCATGCGAGACCTAGTCCACAGG
CTTCCAGGAGGGAACAACAGCAACAACACTGCAAGCAAGGCCATGTCGGATGACACAGTGACAGCTGTCT
GCTGCACACTGCACGAAGTGATTACCAAGAACATGGAGAACGCCAAGGCCTTACGGGATGCCGGTGGCAT
CGAGAAGTTGGTCGGCATCTCCAAAAGCAAAGGAGATAAACACTCTCCAAAAGTGGTCAAGGCTGCATCT
CAGGTCCTCAACAGCATGTGGCAGTACCGAGATCTGAGGAGTCTCTACAAAAAGGATGGATGGTCACAAT
ACCACTTTGTAGCCTCGTCTTCAACCATCGAGAGGGACCGGCAAAGGCCCTACTCCTCCCGCACGCC
CTCCATCTCCCCTGTGCGCGTGTCTCCCAACAACCGCTCAGCAAGTGCCCCAGCTTCACCTCGGGAAATG
ATCAGCCTCAAAGAAAGGAAAACAGACTACGAGTGCACCGGCAGCAACGCCACCTACCACGGAGCTAAAG
GCGAACACACTTCCAGGAAAGATGCCATGACAGCTCAAAACACTGGAATTTCAACTTTGTATAGGAATTC
TTATGGTGCGCCCGCTGAAGACATCAAACACAACCAGGTTTCAGCACAGCCAGTCCCACAGGAGCCCAGC
AGAAAAGATTACGAGACCTACCAGCCATTTCAGAATTCCACAAGAAATTACGATGAGTCCTTCTTCGAGG
ACCAGGTCCACCATCGCCCTCCCGCCAGCGAGTACACCATGCACCTGGGTCTCAAGTCCACCGGCAACTA
CGTTGACTTCTACTCAGCTGCCCGTCCCTACAGTGAACTGAACTATGAAACGAGCCACTACCCGGCCTCC
CCCGACTCCTGGGTGTGAGGAGCAGGGCACAGGCGCTCCGGGAACAGTGCATGTGCATGCATACCACAAG
ACATTTCTTTCTGTTTTGTTTTTTTCTCCTGCAAATTTAGTTTGTTAAAGCCTGTTCCATAGGAAGGCTG
TGATAACCAGTAAGGAAATATTAAGAGCTATTTTAGAAAGCTAAATGAATCGCAAGTTAACTTGGAAATC
AGTAGAAAGCTAAAGTGATCCTAAATATGACAGTGGGCAGCACCTTTCTAGCGTGAGCTGTAGAGTAACG
AGAAGTGCTTTATACTGAACGTGGTTGATGGGAGGAGAGACGAGGCATTCGGGCCGGTGGGGCGTAAGGG
TTATCGTTAAGCACAAGACACAGAATAGTTTACACACTGTGTGGGGGACGGCTTCTCACGCTTTGTTTAC
TCTCTTCATCCGTTGTGACTCTAGGCTTCAGGTTGCATTGGGGTTCCTCTGTACAGCAAGATGTTTCTTG
CCTTTTGTTAATGCATTGTTGTAAAGTATTTGATGTACATTACAGATTAAAGAAGAAAAGCGCGTTGTGT
ATATTACACCAATGCCGCCGTGTTTCCTCATCTATGGTTCTAAATATTGCTTCAATTTCAAACTTTTGAA
AGATGTATGGATTTCCAGTTTTTCTTTACTTTCTCCCAGTATGTTTTAACAAAAAAAAAAAAAAGCAGGA
AAAAAGGAATATTTAGCAGTATTGTTCGTTCTGATATGTGAATTTGTTTGTGACAACTAAACAAGGCATT
CAGCAGTTTCTGACAATTAACATACATCATTCCACACTCCTTGTCAACAAAGTGCTTTTTCACTGCCTAA
AATTTTAGATGTAGATATTTGAAATAGATTTTTTCATTTATACCAGTTTTCTTTATGATGATACAGTGTT
AAAAGAAAATAAATTACAATTGATCTGTCATCCATATTTGCTAAGAATGTCTATTTCCAAGAACCTATAT
CTTACAAAATACACATTCTTGCTTGTGTGTCTGTGTGTGTGGGTGTATGTATGTCCACGTACTAGAAAAA
TTACCTGTGTGAGATTTTATTTTGACAGAAGCTATTTCATTTGCTGTTCATCTTGTACAAACTTTGTTTT
TGGTTTTTTAGTATAAATGTACATCAGTTTGTTCCTTTTGTACTGTATCTATTCTTCTGACCATCTAGTG
ACTCAGGATATTAGGCCAGTTGAAGTGGAGTTATAGGATTTTCACACATTCACGCATACTTGGATATCAA
CCCTCTCTACAGGTCCGTTTCCTTACACATTTTATCCCCTCAGAACACGATAAACCATGGCCAATTCAGT
TTCACTTTGGGGCCAATTACTTGAAGTATCCACAGAAAATGTAAACTGCTCGCCTTATTCCCCCAAACTT
TTTGTTCATGTGGCAGGGAGCAGTACACAACTATGGAATAGATTATTAAAACTGAACTTGAAGCTAACGT
TAGAATAGTGGTTAACAGAGCTAAGAAATAAGGCTAACCATCGAAAAGAACATGGGCCGCGGGTAAACTA
GCTTTGCTCTTTAGATGCAAGTGGCTGAGGCCAATAGGTGCCTAGTAAATGTTAACTGTTCTTGGAAAAA
ATAAAAACATCAGTAACAAAA
**SEQ ID NO: 52 - mRNA sequence of human catenin delta-2 transcript 2 (NM_001288715 as at the filing date)** 
GTTCCTTTTAGCCGTGGTGCAGGGAGGGAGGAGGGAGGTGGGTAGGATGCCAGGAACCGCCTGCTCCACA
GCCTGGGGAAGGAGGGAAGAGTAACCCTTCATCTTTGGGACTAGTCGTCTTGTTTGCCTAGACTACAGTT
TTCAAGTATTTAAGGACCATTTATTTCAAAAGGAATTACAGTTTGAAAGGCTGACCCGAGAGCTGGAGGC
TGAACGGCAGATCGTAGCCAGCCAGCTGGAGCGATGCAAGCTCGGATCCGAGACTGGCAGCATGAGCAGC
ATGAGTTCAGCAGAAGAGCAGTTTCAGTGGCAGTCACAAGATGGTCAAAAAGATATCGAAGATGAGCTTA
CAACAGGTCTCGAGCTGGTGGACTCCTGTATTAGGTCACTACAGGAATCAGGAATACTTGACCCACAGGA
TTATTCTACAGGTGAAAGGCCCAGCCTGCTCTCCCAGAGTGCACTTCAGCTCAATTCCAAACCTGAAGGG
TCTTTCCAGTATCCGGCCAGCTACCATAGCAACCAGACCCTGGCCCTGGGGGAAACCACCCCTTCACAGC
TCCCGGCCCGAGGCACACAAGCCCGAGCTACGGGCCAGAGCTTCAGCCAGGGCACGACCAGCCGCGCCGG
CCACCTGGCGGGGCCCGAGCCCGCGCCGCCGCCGCCGCCGCCGCCGCGGGAGCCGTTCGCGCCCAGCCTG
GGCAGCGCCTTCCACCTGCCCGACGCGCCGCCCGCCGCCGCCGCCGCCGCGCTCTACTACTCCAGCTCCA
CGCTGCCCGCGCCGCCGCGCGGGGGCTCCCCGCTGGCCGCGCCCCAGGGCGGTTCGCCCACCAAGCTGCA
GCGCGGCGGCTCGGCCCCCGAGGGCGCCACCTACGCCGCGCCGCGCGGCTCCTCGCCCAAGCAGTCGCCC
AGCCGCCTGGCCAAGTCCTACAGCACCAGCTCGCCCATCAACATCGTCGTGTCCTCGGCCGGCCTGTCCC
CGATCCGCGTGACCTCGCCCCCCACCGTGCAGTCCACCATCTCCTCCTCGCCCATCCACCAGCTGAGCTC
CACCATCGGCACGTACGCCACCCTGTCGCCCACCAAGCGCCTGGTCCACGCGTCCGAGCAGTACAGCAAG
 
CACTCGCAGGAGCTGTATGCCACGGCCACCCTCCAGAGGCCGGGCAGCCTGGCAGCTGGTTCCCGAGCCT
CATACAGCAGCCAGCATGGGCACCTGGGCCCAGAGTTGCGGGCCCTGCAGTCCCCAGAACACCACATAGA
TCCCATCTATGAAGACCGCGTCTATCAGAAGCCCCCTATGAGGAGTCTCAGCCAGAGCCAGGGGGACCCT
CTGCCGCCAGCACACACCGGCACCTACCGCACGAGCACAGCCCCATCTTCCCCTGGTGTCGACTCCGTCC
CCTTGCAGCGCACAGGCAGCCAGCACGGCCCACAGAATGCCGCCGCGGCCACCTTCCAGAGGGCCAGCTA
TGCCGCCGGCCCAGCCTCCAATTACGCGGACCCCTACCGACAGCTGCAGTATTGTCCCTCTGTTGAGTCT
CCATACAGCAAATCCGGCCCTGCTCTCCCGCCTGAAGGCACCTTGGCCAGGTCCCCGTCCATTGATAGCA
TTCAGAAAGATCCCAGAGAATTTGGATGGAGAGACCCGGAACTGCCGGAAGTGATTCAGATGTTGCAGCA
CCAGTTTCCCTCGGTCCAGTCTAACGCGGCAGCCTACTTGCAACACCTCTGTTTTGGAGACAACAAAATT
AAAGCCGAGATAAGGAGACAAGGAGGCATCCAGCTCCTGGTGGACCTGTTGGATCATCGGATGACCGAAG
TCCACCGTAGTGCCTGTGGAGCTCTGAGAAACCTGGTGTATGGGAAGGCCAACGATGATAACAAAATTGC
CCTGAAAAACTGTGGTGGCATCCCAGCACTGGTGAGGTTACTCCGCAAGACGACTGACCTGGAGATCCGG
GAGCTGGTCACAGGAGTCCTTTGGAACCTCTCCTCATGCGATGCACTCAAAATGCCAATCATCCAGGATG
CCCTAGCAGTACTGACCAACGCGGTGATTATCCCCCACTCAGGCTGGGAAAATTCGCCTCTTCAGGATGA
TCGGAAAATACAGCTGCATTCATCACAGGTGCTGCGTAACGCCACCGGGTGCCTAAGGAATGTTAGTTCG
GCCGGAGAGGAGGCCCGCAGAAGGATGAGAGAGTGTGATGGGCTTACGGATGCCTTGCTGTACGTGATCC
AGTCTGCGCTGGGGAGCAGTGAGATCGATAGCAAGACCGTTGAAAACTGTGTGTGCATTTTAAGGAACCT
CTCGTACCGGCTGGCGGCAGAAACGTCTCAGGGACAGCACATGGGCACGGACGAGCTGGACGGGCTACTC
TGTGGCGAGGCCAATGGCAAGGATGCTGAGAGCTCTGGGTGCTGGGGCAAGAAGAAGAAGAAAAAGAAAT
CCCAAGATCAGTGGGATGGAGTAGGACCTCTTCCAGACTGTGCTGAACCACCAAAAGGGATCCAGATGCT
GTGGCACCCATCAATAGTCAAACCCTACCTCACACTGCTCTCTGAGTGCTCAAATCCAGACACGCTGGAA
GGGGCGGCAGGCGCCCTGCAGAACTTGGCTGCAGGGAGCTGGAAGTGGTCAGTATATATCCGAGCCGCTG
TCCGAAAAGAGAAAGGCCTGCCCATCCTCGTGGAGCTGCTCCGAATAGACAATGACCGTGTGGTGTGCGC
GGTGGCCACTGCGCTGCGGAACATGGCCTTGGACGTCAGAAATAAGGAGCTCATCGGCAAATACGCCATG
CGAGACCTAGTCCACAGGCTTCCAGGAGGGAACAACAGCAACAACACTGCAAGCAAGGCCATGTCGGATG
ACACAGTGACAGCTGTCTGCTGCACACTGCACGAAGTGATTACCAAGAACATGGAGAACGCCAAGGCCTT
ACGGGATGCCGGTGGCATCGAGAAGTTGGTCGGCATCTCCAAAAGCAAAGGAGATAAACACTCTCCAAAA
GTGGTCAAGGCTGCATCTCAGGTCCTCAACAGCATGTGGCAGTACCGAGATCTGAGGAGTCTCTACAAAA
AGGATGGATGGTCACAATACCACTTTGTAGCCTCGTCTTCAACCATCGAGAGGGACCGGCAAAGGCCCTA
CTCCTCCTCCCGCACGCCCTCCATCTCCCCTGTGCGCGTGTCTCCCAACAACCGCTCAGCAAGTGCCCCA
GCTTCACCTCGGGAAATGATCAGCCTCAAAGAAAGGAAAACAGACTACGAGTGCACCGGCAGCAACGCCA
CCTACCACGGAGCTAAAGGCGAACACACTTCCAGGAAAGATGCCATGACAGCTCAAAACACTGGAATTTC
AACTTTGTATAGGAATTCTTATGGTGCGCCCGCTGAAGACATCAAACACAACCAGGTTTCAGCACAGCCA
GTCCCACAGGAGCCCAGCAGAAAAGATTACGAGACCTACCAGCCATTTCAGAATTCCACAAGAAATTACG
ATGAGTCCTTCTTCGAGGACCAGGTCCACCATCGCCCTCCCGCCAGCGAGTACACCATGCACCTGGGTCT
CAAGTCCACCGGCAACTACGTTGACTTCTACTCAGCTGCCCGTCCCTACAGTGAACTGAACTATGAAACG
AGCCACTACCCGGCCTCCCCCGACTCCTGGGTGTGAGGAGCAGGGCACAGGCGCTCCGGGAACAGTGCAT
GTGCATGCATACCACAAGACATTTCTTTCTGTTTTGTTTTTTTCTCCTGCAAATTTAGTTTGTTAAAGCC
TGTTCCATAGGAAGGCTGTGATAACCAGTAAGGAAATATTAAGAGCTATTTTAGAAAGCTAAATGAATCG
CAAGTTAACTTGGAAATCAGTAGAAAGCTAAAGTGATCCTAAATATGACAGTGGGCAGCACCTTTCTAGC
GTGAGCTGTAGAGTAACGAGAAGTGCTTTATACTGAACGTGGTTGATGGGAGGAGAGACGAGGCATTCGG
GCCGGTGGGGCGTAAGGGTTATCGTTAAGCACAAGACACAGAATAGTTTACACACTGTGTGGGGGACGGC
TTCTCACGCTTTGTTTACTCTCTTCATCCGTTGTGACTCTAGGCTTCAGGTTGCATTGGGGTTCCTCTGT
ACAGCAAGATGTTTCTTGCCTTTTGTTAATGCATTGTTGTAAAGTATTTGATGTACATTACAGATTAAAG
AAGAAAAGCGCGTTGTGTATATTACACCAATGCCGCCGTGTTTCCTCATCTATGGTTCTAAATATTGCTT
CAATTTCAAACTTTTGAAAGATGTATGGATTTCCAGTTTTTCTTTACTTTCTCCCAGTATGTTTTAACAA
AAAAAAAAAAAAGCAGGAAAAAAGGAATATTTAGCAGTATTGTTCGTTCTGATATGTGAATTTGTTTGTG
ACAACTAAACAAGGCATTCAGCAGTTTCTGACAATTAACATACATCATTCCACACTCCTTGTCAACAAAG
TGCTTTTTCACTGCCTAAAATTTTAGATGTAGATATTTGAAATAGATTTTTTCATTTATACCAGTTTTCT
TTATGATGATACAGTGTTAAAAGAAAATAAATTACAATTGATCTGTCATCCATATTTGCTAAGAATGTCT
ATTTCCAAGAACCTATATCTTACAAAATACACATTCTTGCTTGTGTGTCTGTGTGTGTGGGTGTATGTAT
GTCCACGTACTAGAAAAATTACCTGTGTGAGATTTTATTTTGACAGAAGCTATTTCATTTGCTGTTCATC
TTGTACAAACTTTGTTTTTGGTTTTTTAGTATAAATGTACATCAGTTTGTTCCTTTTGTACTGTATCTAT
TCTTCTGACCATCTAGTGACTCAGGATATTAGGCCAGTTGAAGTGGAGTTATAGGATTTTCACACATTCA
CGCATACTTGGATATCAACCCTCTCTACAGGTCCGTTTCCTTACACATTTTATCCCCTCAGAACACGATA
AACCATGGCCAATTCAGTTTCACTTTGGGGCCAATTACTTGAAGTATCCACAGAAAATGTAAACTGCTCG
CCTTATTCCCCCAAACTTTTTGTTCATGTGGCAGGGAGCAGTACACAACTATGGAATAGATTATTAAAAC
TGAACTTGAAGCTAACGTTAGAATAGTGGTTAACAGAGCTAAGAAATAAGGCTAACCATCGAAAAGAACA
TGGGCCGCGGGTAAACTAGCTTTGCTCTTTAGATGCAAGTGGCTGAGGCCAATAGGTGCCTAGTAAATGT
 TAACTGTTCTTGGAAAAAATAAAAACATCAGTAACAAAA
**SEQ ID NO: 53 - mRNA sequence of human catenin delta-2 transcript 3 (NM_001288716 as at the filing date)** 
GTTCCTTTTAGCCGTGGTGCAGGGAGGGAGGAGGGAGGTGGGTAGGATGCCAGGAACCGCCTGCTCCACA
GCCTGGGGAAGGAGGGAAGAGTAACCCTTCATCTTTGGGACTAGTCGTCTTGTTTGCCTAGACTACAGTT
TTCAAGTATTTAAGGACCATTTATTTCAAAAGGAATTACAGTTTGAAAGGCTGACCCGAGAGCTGGAGGC
TGAACGGCAGATCGTAGCCAGCCAGCTGGAGCGATGCAAGCTCGGATCCGAGACTGGCAGCATGAGCAGC
ATGAGTTCAGCAGAAGAGCAGTTTCAGTGGCAGTCACAAGATGGTCAAAAAGATATCGAAGATGAGCTTA
CAACAGGTCTCGAGCTGGTGGACTCCTGTATTAGGTCACTACAGGAATCAGGAATACTTGACCCACAGGA
TTATTCTACAGGTGAAACTGGTTCCCGAGCCTCATACAGCAGCCAGCATGGGCACCTGGGCCCAGAGTTG
CGGGCCCTGCAGTCCCCAGAACACCACATAGATCCCATCTATGAAGACCGCGTCTATCAGAAGCCCCCTA
TGAGGAGTCTCAGCCAGAGCCAGGGGGACCCTCTGCCGCCAGCACACACCGGCACCTACCGCACGAGCAC
AGCCCCATCTTCCCCTGGTGTCGACTCCGTCCCCTTGCAGCGCACAGGCAGCCAGCACGGCCCACAGAAT
GCCGCCGCGGCCACCTTCCAGAGGGCCAGCTATGCCGCCGGCCCAGCCTCCAATTACGCGGACCCCTACC
GACAGCTGCAGTATTGTCCCTCTGTTGAGTCTCCATACAGCAAATCCGGCCCTGCTCTCCCGCCTGAAGG
CACCTTGGCCAGGTCCCCGTCCATTGATAGCATTCAGAAAGATCCCAGAGAATTTGGATGGAGAGACCCG
GAACTGCCGGAAGTGATTCAGATGTTGCAGCACCAGTTTCCCTCGGTCCAGTCTAACGCGGCAGCCTACT
TGCAACACCTCTGTTTTGGAGACAACAAAATTAAAGCCGAGATAAGGAGACAAGGAGGCATCCAGCTCCT
GGTGGACCTGTTGGATCATCGGATGACCGAAGTCCACCGTAGTGCCTGTGGAGCTCTGAGAAACCTGGTG
TATGGGAAGGCCAACGATGATAACAAAATTGCCCTGAAAAACTGTGGTGGCATCCCAGCACTGGTGAGGT
TACTCCGCAAGACGACTGACCTGGAGATCCGGGAGCTGGTCACAGGAGTCCTTTGGAACCTCTCCTCATG
CGATGCACTCAAAATGCCAATCATCCAGGATGCCCTAGCAGTACTGACCAACGCGGTGATTATCCCCCAC
TCAGGCTGGGAAAATTCGCCTCTTCAGGATGATCGGAAAATACAGCTGCATTCATCACAGGTGCTGCGTA
ACGCCACCGGGTGCCTAAGGAATGTTAGTTCGGCCGGAGAGGAGGCCCGCAGAAGGATGAGAGAGTGTGA
TGGGCTTACGGATGCCTTGCTGTACGTGATCCAGTCTGCGCTGGGGAGCAGTGAGATCGATAGCAAGACC
GTTGAAAACTGTGTGTGCATTTTAAGGAACCTCTCGTACCGGCTGGCGGCAGAAACGTCTCAGGGACAGC
ACATGGGCACGGACGAGCTGGACGGGCTACTCTGTGGCGAGGCCAATGGCAAGGATGCTGAGAGCTCTGG
GTGCTGGGGCAAGAAGAAGAAGAAAAAGAAATCCCAAGATCAGTGGGATGGAGTAGGACCTCTTCCAGAC
TGTGCTGAACCACCAAAAGGGATCCAGATGCTGTGGCACCCATCAATAGTCAAACCCTACCTCACACTGC
TCTCTGAGTGCTCAAATCCAGACACGCTGGAAGGGGCGGCAGGCGCCCTGCAGAACTTGGCTGCAGGGAG
CTGGAAGTGGTCAGTATATATCCGAGCCGCTGTCCGAAAAGAGAAAGGCCTGCCCATCCTCGTGGAGCTG
CTCCGAATAGACAATGACCGTGTGGTGTGCGCGGTGGCCACTGCGCTGCGGAACATGGCCTTGGACGTCA
GAAATAAGGAGCTCATCGGCAAATACGCCATGCGAGACCTAGTCCACAGGCTTCCAGGAGGGAACAACAG
CAACAACACTGCAAGCAAGGCCATGTCGGATGACACAGTGACAGCTGTCTGCTGCACACTGCACGAAGTG
ATTACCAAGAACATGGAGAACGCCAAGGCCTTACGGGATGCCGGTGGCATCGAGAAGTTGGTCGGCATCT
CCAAAAGCAAAGGAGATAAACACTCTCCAAAAGTGGTCAAGGCTGCATCTCAGGTCCTCAACAGCATGTG
GCAGTACCGAGATCTGAGGAGTCTCTACAAAAAGGATGGATGGTCACAATACCACTTTGTAGCCTCGTCT
TCAACCATCGAGAGGGACCGGCAAAGGCCCTACTCCTCCTCCCGCACGCCCTCCATCTCCCCTGTGCGCG
TGTCTCCCAACAACCGCTCAGCAAGTGCCCCAGCTTCACCTCGGGAAATGATCAGCCTCAAAGAAAGGAA
AACAGACTACGAGTGCACCGGCAGCAACGCCACCTACCACGGAGCTAAAGGCGAACACACTTCCAGGAAA
GATGCCATGACAGCTCAAAACACTGGAATTTCAACTTTGTATAGGAATTCTTATGGTGCGCCCGCTGAAG
ACATCAAACACAACCAGGTTTCAGCACAGCCAGTCCCACAGGAGCCCAGCAGAAAAGATTACGAGACCTA
CCAGCCATTTCAGAATTCCACAAGAAATTACGATGAGTCCTTCTTCGAGGACCAGGTCCACCATCGCCCT
CCCGCCAGCGAGTACACCATGCACCTGGGTCTCAAGTCCACCGGCAACTACGTTGACTTCTACTCAGCTG
CCCGTCCCTACAGTGAACTGAACTATGAAACGAGCCACTACCCGGCCTCCCCCGACTCCTGGGTGTGAGG
AGCAGGGCACAGGCGCTCCGGGAACAGTGCATGTGCATGCATACCACAAGACATTTCTTTCTGTTTTGTT
TTTTTCTCCTGCAAATTTAGTTTGTTAAAGCCTGTTCCATAGGAAGGCTGTGATAACCAGTAAGGAAATA
TTAAGAGCTATTTTAGAAAGCTAAATGAATCGCAAGTTAACTTGGAAATCAGTAGAAAGCTAAAGTGATC
CTAAATATGACAGTGGGCAGCACCTTTCTAGCGTGAGCTGTAGAGTAACGAGAAGTGCTTTATACTGAAC
GTGGTTGATGGGAGGAGAGACGAGGCATTCGGGCCGGTGGGGCGTAAGGGTTATCGTTAAGCACAAGACA
CAGAATAGTTTACACACTGTGTGGGGGACGGCTTCTCACGCTTTGTTTACTCTCTTCATCCGTTGTGACT
CTAGGCTTCAGGTTGCATTGGGGTTCCTCTGTACAGCAAGATGTTTCTTGCCTTTTGTTAATGCATTGTT
GTAAAGTATTTGATGTACATTACAGATTAAAGAAGAAAAGCGCGTTGTGTATATTACACCAATGCCGCCG
TGTTTCCTCATCTATGGTTCTAAATATTGCTTCAATTTCAAACTTTTGAAAGATGTATGGATTTCCAGTT
TTTCTTTACTTTCTCCCAGTATGTTTTAACAAAAAAAAAAAAAAGCAGGAAAAAAGGAATATTTAGCAGT
ATTGTTCGTTCTGATATGTGAATTTGTTTGTGACAACTAAACAAGGCATTCAGCAGTTTCTGACAATTAA
CATACATCATTCCACACTCCTTGTCAACAAAGTGCTTTTTCACTGCCTAAAATTTTAGATGTAGATATTT
 
GAAATAGATTTTTTCATTTATACCAGTTTTCTTTATGATGATACAGTGTTAAAAGAAAATAAATTACAAT
TGATCTGTCATCCATATTTGCTAAGAATGTCTATTTCCAAGAACCTATATCTTACAAAATACACATTCTT
GCTTGTGTGTCTGTGTGTGTGGGTGTATGTATGTCCACGTACTAGAAAAATTACCTGTGTGAGATTTTAT
TTTGACAGAAGCTATTTCATTTGCTGTTCATCTTGTACAAACTTTGTTTTTGGTTTTTTAGTATAAATGT
ACATCAGTTTGTTCCTTTTGTACTGTATCTATTCTTCTGACCATCTAGTGACTCAGGATATTAGGCCAGT
TGAAGTGGAGTTATAGGATTTTCACACATTCACGCATACTTGGATATCAACCCTCTCTACAGGTCCGTTT
CCTTACACATTTTATCCCCTCAGAACACGATAAACCATGGCCAATTCAGTTTCACTTTGGGGCCAATTAC
TTGAAGTATCCACAGAAAATGTAAACTGCTCGCCTTATTCCCCCAAACTTTTTGTTCATGTGGCAGGGAG
CAGTACACAACTATGGAATAGATTATTAAAACTGAACTTGAAGCTAACGTTAGAATAGTGGTTAACAGAG
CTAAGAAATAAGGCTAACCATCGAAAAGAACATGGGCCGCGGGTAAACTAGCTTTGCTCTTTAGATGCAA
GTGGCTGAGGCCAATAGGTGCCTAGTAAATGTTAACTGTTCTTGGAAAAAATAAAAACATCAGTAACAAA
A
**SEQ ID NO: 54 - mRNA sequence of human catenin delta-2 transcript 4 (NM_001288717 as at the filing date)** 
GCGTCGGGAGCCGCCTCTCGCCCGCGGCGCTCGCCCCTGCCGCCCGCCAGCATCCCTTGTCCCGCGGCCG
CGCTCAGACAACAAAAGCGGAAGATGCTGCAGTTGGGCAAGGTCAGGACCTTGCCCTGAAGCCGGGCGGC
GCCGCGCACGCCTCTTCCCGGACTGAGGAGCTGTCGCCGGCGGAGGGTGCATGTTTGCGAGGAAGCCGCC
GGGCGCCGCGCCTTTGGGAGCTATGCCTGTTCCAGACCAGCCTTCATCAGCCTCAGAGAAGACGAGTTCC
CTGAGCCCCGGCTTAAACACCTCCAACGGGGATGGCTCTGAAACAGAAACCACCTCTGCCATCCTCGCCT
CAGTCAAAGAACAGGAATTACAGTTTGAAAGGCTGACCCGAGAGCTGGAGGCTGAACGGCAGATCGTAGC
CAGCCAGCTGGAGCGATGCAAGCTCGGATCCGAGACTGGCAGCATGAGCAGCATGAGTTCAGCAGAAGAG
CAGTTTCAGTGGCAGTCACAAGATGGTCAAAAAGATATCGAAGATGAGCTTACAACAGGTCTCGAGCTGG
TGGACTCCTGTATTAGGTCACTACAGGAATCAGGAATACTTGACCCACAGGATTATTCTACAGGTGAAAG
GCCCAGCCTGCTCTCCCAGAGTGCACTTCAGCTCAATTCCAAACCTGAAGGGTCTTTCCAGTATCCGGCC
AGCTACCATAGCAACCAGACCCTGGCCCTGGGGGAAACCACCCCTTCACAGCTCCCGGCCCGAGGCACAC
AAGCCCGAGCTACGGGCCAGAGCTTCAGCCAGCTGGTTCCCGAGCCTCATACAGCAGCCAGCATGGGCAC
CTGGGCCCAGAGTTGCGGGCCCTGCAGTCCCCAGAACACCACATAGATCCCATCTATGAAGACCGCGTCT
ATCAGAAGCCCCCTATGAGGAGTCTCAGCCAGAGCCAGGGGGACCCTCTGCCGCCAGCACACACCGGCAC
CTACCGCACGAGCACAGCCCCATCTTCCCCTGGTGTCGACTCCGTCCCCTTGCAGCGCACAGGCAGCCAG
CACGGCCCACAGAATGCCGCCGCGGCCACCTTCCAGAGGGCCAGCTATGCCGCCGGCCCAGCCTCCAATT
ACGCGGACCCCTACCGACAGCTGCAGTATTGTCCCTCTGTTGAGTCTCCATACAGCAAATCCGGCCCTGC
TCTCCCGCCTGAAGGCACCTTGGCCAGGTCCCCGTCCATTGATAGCATTCAGAAAGATCCCAGAGAATTT
GGATGGAGAGACCCGGAACTGCCGGAAGTGATTCAGATGTTGCAGCACCAGTTTCCCTCGGTCCAGTCTA
ACGCGGCAGCCTACTTGCAACACCTCTGTTTTGGAGACAACAAAATTAAAGCCGAGATAAGGAGACAAGG
AGGCATCCAGCTCCTGGTGGACCTGTTGGATCATCGGATGACCGAAGTCCACCGTAGTGCCTGTGGAGCT
CTGAGAAACCTGGTGTATGGGAAGGCCAACGATGATAACAAAATTGCCCTGAAAAACTGTGGTGGCATCC
CAGCACTGGTGAGGTTACTCCGCAAGACGACTGACCTGGAGATCCGGGAGCTGGTCACAGGAGTCCTTTG
GAACCTCTCCTCATGCGATGCACTCAAAATGCCAATCATCCAGGATGCCCTAGCAGTACTGACCAACGCG
GTGATTATCCCCCACTCAGGCTGGGAAAATTCGCCTCTTCAGGATGATCGGAAAATACAGCTGCATTCAT
CACAGGTGCTGCGTAACGCCACCGGGTGCCTAAGGAATGTTAGTTCGGCCGGAGAGGAGGCCCGCAGAAG
GATGAGAGAGTGTGATGGGCTTACGGATGCCTTGCTGTACGTGATCCAGTCTGCGCTGGGGAGCAGTGAG
ATCGATAGCAAGACCGTTGAAAACTGTGTGTGCATTTTAAGGAACCTCTCGTACCGGCTGGCGGCAGAAA
CGTCTCAGGGACAGCACATGGGCACGGACGAGCTGGACGGGCTACTCTGTGGCGAGGCCAATGGCAAGGA
TGCTGAGAGCTCTGGGTGCTGGGGCAAGAAGAAGAAAAAGAAATCCCAAGATCAGTGGGATGGAGTA
GGACCTCTTCCAGACTGTGCTGAACCACCAAAAGGGATCCAGATGCTGTGGCACCCATCAATAGTCAAAC
CCTACCTCACACTGCTCTCTGAGTGCTCAAATCCAGACACGCTGGAAGGGGCGGCAGGCGCCCTGCAGAA
CTTGGCTGCAGGGAGCTGGAAGTGGTCAGTATATATCCGAGCCGCTGTCCGAAAAGAGAAAGGCCTGCCC
ATCCTCGTGGAGCTGCTCCGAATAGACAATGACCGTGTGGTGTGCGCGGTGGCCACTGCGCTGCGGAACA
TGGCCTTGGACGTCAGAAATAAGGAGCTCATCGGCAAATACGCCATGCGAGACCTAGTCCACAGGCTTCC
AGGAGGGAACAACAGCAACAACACTGCAAGCAAGGCCATGTCGGATGACACAGTGACAGCTGTCTGCTGC
ACACTGCACGAAGTGATTACCAAGAACATGGAGAACGCCAAGGCCTTACGGGATGCCGGTGGCATCGAGA
AGTTGGTCGGCATCTCCAAAAGCAAAGGAGATAAACACTCTCCAAAAGTGGTCAAGGCTGCATCTCAGGT
CCTCAACAGCATGTGGCAGTACCGAGATCTGAGGAGTCTCTACAAAAAGGATGGATGGTCACAATACCAC
TTTGTAGCCTCGTCTTCAACCATCGAGAGGGACCGGCAAAGGCCCTACTCCTCCTCCCGCACGCCCTCCA
TCTCCCCTGTGCGCGTGTCTCCCAACAACCGCTCAGCAAGTGCCCCAGCTTCACCTCGGGAAATGATCAG
CCTCAAAGAAAGGAAAACAGACTACGAGTGCACCGGCAGCAACGCCACCTACCACGGAGCTAAAGGCGAA
CACACTTCCAGGAAAGATGCCATGACAGCTCAAAACACTGGAATTTCAACTTTGTATAGGAATTCTTATG
 
GTGCGCCCGCTGAAGACATCAAACACAACCAGGTTTCAGCACAGCCAGTCCCACAGGAGCCCAGCAGAAA
AGATTACGAGACCTACCAGCCATTTCAGAATTCCACAAGAAATTACGATGAGTCCTTCTTCGAGGACCAG
GTCCACCATCGCCCTCCCGCCAGCGAGTACACCATGCACCTGGGTCTCAAGTCCACCGGCAACTACGTTG
ACTTCTACTCAGCTGCCCGTCCCTACAGTGAACTGAACTATGAAACGAGCCACTACCCGGCCTCCCCCGA
CTCCTGGGTGTGAGGAGCAGGGCACAGGCGCTCCGGGAACAGTGCATGTGCATGCATACCACAAGACATT
TCTTTCTGTTTTGTTTTTTTCTCCTGCAAATTTAGTTTGTTAAAGCCTGTTCCATAGGAAGGCTGTGATA
ACCAGTAAGGAAATATTAAGAGCTATTTTAGAAAGCTAAATGAATCGCAAGTTAACTTGGAAATCAGTAG
AAAGCTAAAGTGATCCTAAATATGACAGTGGGCAGCACCTTTCTAGCGTGAGCTGTAGAGTAACGAGAAG
TGCTTTATACTGAACGTGGTTGATGGGAGGAGAGACGAGGCATTCGGGCCGGTGGGGCGTAAGGGTTATC
GTTAAGCACAAGACACAGAATAGTTTACACACTGTGTGGGGGACGGCTTCTCACGCTTTGTTTACTCTCT
TCATCCGTTGTGACTCTAGGCTTCAGGTTGCATTGGGGTTCCTCTGTACAGCAAGATGTTTCTTGCCTTT
TGTTAATGCATTGTTGTAAAGTATTTGATGTACATTACAGATTAAAGAAGAAAAGCGCGTTGTGTATATT
ACACCAATGCCGCCGTGTTTCCTCATCTATGGTTCTAAATATTGCTTCAATTTCAAACTTTTGAAAGATG
TATGGATTTCCAGTTTTTCTTTACTTTCTCCCAGTATGTTTTAACAAAAAAAAAAAAAAGCAGGAAAAAA
GGAATATTTAGCAGTATTGTTCGTTCTGATATGTGAATTTGTTTGTGACAACTAAACAAGGCATTCAGCA
GTTTCTGACAATTAACATACATCATTCCACACTCCTTGTCAACAAAGTGCTTTTTCACTGCCTAAAATTT
TAGATGTAGATATTTGAAATAGATTTTTTCATTTATACCAGTTTTCTTTATGATGATACAGTGTTAAAAG
AAAATAAATTACAATTGATCTGTCATCCATATTTGCTAAGAATGTCTATTTCCAAGAACCTATATCTTAC
AAAATACACATTCTTGCTTGTGTGTCTGTGTGTGTGGGTGTATGTATGTCCACGTACTAGAAAAATTACC
TGTGTGAGATTTTATTTTGACAGAAGCTATTTCATTTGCTGTTCATCTTGTACAAACTTTGTTTTTGGTT
TTTTAGTATAAATGTACATCAGTTTGTTCCTTTTGTACTGTATCTATTCTTCTGACCATCTAGTGACTCA
GGATATTAGGCCAGTTGAAGTGGAGTTATAGGATTTTCACACATTCACGCATACTTGGATATCAACCCTC
TCTACAGGTCCGTTTCCTTACACATTTTATCCCCTCAGAACACGATAAACCATGGCCAATTCAGTTTCAC
TTTGGGGCCAATTACTTGAAGTATCCACAGAAAATGTAAACTGCTCGCCTTATTCCCCCAAACTTTTTGT
TCATGTGGCAGGGAGCAGTACACAACTATGGAATAGATTATTAAAACTGAACTTGAAGCTAACGTTAGAA
TAGTGGTTAACAGAGCTAAGAAATAAGGCTAACCATCGAAAAGAACATGGGCCGCGGGTAAACTAGCTTT
GCTCTTTAGATGCAAGTGGCTGAGGCCAATAGGTGCCTAGTAAATGTTAACTGTTCTTGGAAAAAATAAA
AACATCAGTAACAAAA
**SEQ ID NO: 55 - Protein sequence of human PERM and PDZ domain-containing protein 4 (UniProt ID Q14CM0 as at the filing date)**
**SEQ ID NO: 56 - mRNA sequence of human FERM and PDZ domain-containing protein 4 (NM_014728 as at the filing date)** 
GGGAGGCAGCTGCAGTGCTGCGAGGGGTGCGTGCGCGTGTGCCCAGCTCGCCTGCCGTCTCCCGGCTCCG
TCTGCGCTCCTCGGTGCGTGGGGCACGAGGGTCCGAGGGCCGGGAAGCCAGAGCAGCGCCTCTCGCCCAG
GCCTCGCTTTCTCTGGACGCCCGGCAGTCCCCGGGGCTAGAGCGCACGGGGCGGGGCGCGAGACCCGGGC
CGCGCTCCCCGCCCCCGCCTCTTGCGCCCTGCCTGGCTCCCTCTCCATTGAGTTTTCCTGCCTCGGGTGC
CTATCAATGGTCCTGCTCGGGGATGCACACGCAGCTCGCGGCCGGAGGGGGGTAGCAGCCGCCGCCTCCA
GGTGCAGGTTCTCTCCGGCCGCCGCCGCCACCCGCTGTCGCCGCGACTCGAGCCCCGGGCGCACTGAGGT
CTTGGCCATGGGGCTGCGGAGGCTGCTGTTGCTGGCGTGAGAAGGGGCGACGGAGTTGTCGCGCTCGGGG
GTCCCCAGCTGCCTGCATGGATGTCTTCAGCTTTGTGAAGATTGCAAAGCTTTCGAGCCACAGGACGAAG
TCTTCAGGCTGGCCGCCTCCCTCGGGAACCTGGGGCTTGAGCCAGGTGCCGCCCTATGGATGGGAGATGA
CGGCAAACCGAGATGGGCGAGACTACTTCATCAATCACATGACACAGGCAATCCCTTTTGACGACCCTCG
GTTAGAGAGCTGCCAAATCATCCCTCCGGCTCCTCGGAAGGTGGAGATGAGAAGGGACCCCGTGCTGGGA
TTTGGTTTTGTGGCAGGCAGTGAAAAGCCAGTGGTCGTTCGCTCAGTAACACCAGGTGGCCCCTCTGAAG
GCAAGCTGATCCCGGGAGATCAGATTGTAATGATTAATGATGAACCGGTCAGCGCTGCACCCAGAGAGCG
GGTCATCGATCTGGTCAGAAGCTGCAAAGAATCGATACTCCTCACTGTCATTCAGCCTTACCCTTCTCCC
AAATCAGCATTTATTAGTGCTGCAAAAAAGGCAAGATTAAAGTCCAATCCTGTCAAAGTACGCTTCTCTG
AGGAGGTCATCATCAACGGCCAAGTGTCGGAAACTGTTAAGGACAACTCACTTCTTTTTATGCCAAATGT
TTTGAAAGTCTATCTGGAAAATGGGCAGACCAAATCATTTCGTTTTGACTGCAGCACTTCCATTAAGGAT
GTCATCTTAACCCTTCAAGAGAAGCTCTCCATCAAAGGCATTGAACACTTCTCTCTCATGCTGGAGCAGA
GGACAGAAGGGGCTGGAACGAAGCTGCTCTTGCTTCATGAACAGGAGACTCTAACTCAGGTGACACAGAG
GCCCAGCTCCCATAAGATGAGATGTCTTTTCCGAATTAGCTTCGTCCCAAAAGATCCAATTGACCTTTTA
AGGAGAGATCCAGTTGCTTTCGAGTATCTCTATGTTCAGAGTTGTAACGATGTGGTTCAGGAGCGATTTG
GGCCGGAGCTGAAATATGACATAGCCCTGCGGCTGGCCGCATTACAAATGTACATTGCAACCGTTACCAC
CAAGCAAACGCAGAAAATCTCCCTCAAATACATCGAAAAAGAATGGGGATTAGAGACTTTTCTTCCCTCT
GCTGTGCTGCAAAGCATGAAAGAGAAGAACATAAAGAAAGCACTTTCACACCTTGTCAAAGCAAATCAAA
ACTTGGTACCACCGGGTAAAAAGCTCTCTGCACTACAAGCCAAGGTCCATTATCTCAAGTTCCTCAGTGA
CCTACGATTGTATGGGGGCCGTGTGTTCAAGGCAACATTAGTGCAGGCAGAAAAGCGCTCGGAAGTGACT
CTCCTGGTTGGGCCCCGGTATGGCATAAGCCATGTCATCAACACCAAAACCAATCTGGTGGCTCTTTTAG
CCGACTTTAGCCACGTCAACAGGATCGAAATGTTTTCCGAGGAGGAGAGCTTGGTGCGGGTAGAACTCCA
CGTGCTAGATGTGAAGCCTATCACGCTTCTGATGGAATCCTCAGATGCCATGAACCTGGCCTGCTTGACG
GCTGGATACTACCGGCTGCTTGTTGATTCCAGGAGGTCGATATTTAACATGGCCAACAAGAAAAACACAG
CGACCCAGGAAACAGGACCTGAAAACAAGGGGAAGCATAACCTCCTTGGCCCAGATTGGAACTGTATACC
CCAAATGACCACCTTTATTGGCGAAGGGGAACAAGAAGCCCAGATAACATACATAGATTCAAAGCAGAAG
ACGGTGGAGATCACAGACAGCACCATGTGTCCAAAAGAGCACCGGCACTTGTACATAGACAATGCCTATA
GTTCAGATGGACTTAACCAGCAGCTGAGCCAGCCCGGGGAGGCCCCCTGTGAGGCAGACTACAGAAGTCT
AGCTCAGCGGTCCCTATTGACCCTCTCAGGACCAGAAACTCTGAAGAAAGCACAGGAATCTCCGAGAGGA
GCTAAAGTGTCCTTTATTTTTGGAGACTTCGCCTTGGATGATGGTATTAGTCCCCCAACCCTTGGCTATG
AAACGCTACTAGATGAGGGTCCTGAAATGCTGGAGAAGCAGAGAAATCTCTACATTGGCAGTGCCAATGA
CATGAAGGGCCTGGATCTCACTCCAGAGGCAGAGGGCATCCAGTTTGTGGAAAATTCTGTTTATGCAAAC
ATAGGCGATGTGAAGAGCTTCCAGGCCGCGGAGGGGATCGAGGAACCCCTCTTGCATGACATCTGTTATG
CAGAAAACACTGATGACGCGGAGGACGAGGACGAGGTGAGCTGCGAGGAGGACCTCGTGGTGGGGGAGAT
GAACCAGCCGGCCATCCTCAACCTGTCTGGGTCAAGCGATGACATCATTGACCTCACATCCCTGCCCCCT
CCAGAAGGTGATGACAATGAGGATGACTTCCTGTTGCGTTCCTTGAACATGGCCATTGCCGCACCCCCAC
CTGGCTTTAGAGACAGTTCAGATGAAGAGGACTCTCAGAGCCAGGCAGCTTCCTTCCCCGAGGACAAGGA
GAAAGGCAGCAGCCTGCAAAATGATGAGATCCCCGTGTCCCTCATTGACGCTGTGCCCACCAGCGCCGAA
GGCAAGTGTGAGAAGGGACTGGATAATGCCGTCGTCTCCACGCTGGGAGCTCTAGAGGCTCTATCCGTGT
CAGAAGAACAGCAGACCAGTGACAATTCAGGTGTAGCCATCTTGCGGGCTTATAGTCCTGAGTCTTCGTC
AGACTCGGGCAATGAAACTAACTCTTCTGAAATGACTGAGAGTTCTGAACTGGCCACAGCACAAAAACAG
TCAGAAAACCTCTCCCGCATGTTCTTGGCCACTCACGAAGGCTACCACCCCCTTGCAGAAGAGCAGACCG
AGTTCCCGGCCTCCAAGACCCCCGCTGGGGGCTTGCCTCCAAAGTCCTCGCACGCCCTGGCTGCTAGGCC
AGCAACCGACCTCCCGCCCAAAGTTGTGCCTTCCAAGCAGTTACTTCACTCAGACCACATGGAGATGGAG
CCTGAAACTATGGAGACTAAGTCGGTCACTGACTATTTTAGCAAACTGCACATGGGGTCGGTGGCATACT
CCTGCACTAGCAAAAGGAAAAGCAAGCTGGCCGATGGTGAGGGGAAGGCACCCCCTAATGGGAACACAAC
AGGAAAAAAACAGCAGGGGACCAAAACGGCAGAGATGGAGGAGGAGGCCAGTGGTAAATTTGGTACTGTG
TCTTCACGAGACAGTCAACACCTGAGCACTTTTAATCTGGAGAGAACTGCCTTTCGCAAGGACAGTCAAA
GATGGTATGTGGCCACTGAAGGTGGGATGGCTGAAAAAAGTGGATTAGAAGCAGCAACAGGGAAAACCTT
 
TCCAAGAGCTTCTGGTCTTGGGGCAAGGGAGGCCGAAGGGAAGGAAGAAGGAGCTCCTGATGGAGAAACC
AGTGATGGCTCAGGACTTGGTCAAGGGGACCGCTTCTTAACTGACGTGACCTGTGCATCTTCAGCCAAAG
ACTTAGATAACCCAGAGGACGCTGACTCGTCCACCTGCGACCATCCTTCCAAGCTTCCTGAGGCTGATGA
GAGTGTGGCCCGCCTTTGTGACTACCACTTGGCCAAGCGGATGTCATCACTGCAAAGCGAGGGCCATTTT
TCTCTGCAGAGCTCCCAAGGCTCTTCAGTGGATGCAGGCTGTGGCACAGGCAGCAGTGGCAGTGCCTGTG
CCACACCCGTGGAGTCGCCGCTCTGCCCCTCCCTGGGGAAGCACTTGATTCCTGACGCTTCTGGGAAAGG
CGTGAATTACATTCCTTCAGAGGAGAGAGCCCCTGGGCTTCCCAACCACGGAGCCACCTTTAAGGAACTG
CACCCACAGACAGAAGGGATGTGTCCACGGATGACAGTGCCTGCTCTGCACACAGCCATTAACACCGAAC
CCCTGTTTGGCACATTGAGAGATGGATGCCATCGGCTCCCCAAGATTAAGGAAACCACAGTGTAGCTTTG
ACAGAGCCTGGGAAGGAGAGACGAGGAGGCATGCCTTCAGCTTGGTCTCAACATCCTGAAGCTGATCCCA
TCCTGCTACCATCAAACATTCACTCGGAATCAAAGGTGCCAATTCCAAATCAAGACCCTAATGATTTCTC
CCAAGCAAATCAGGCATACGGAGAGGCTGTGAGCTGGCGGCCACCGGATCTGAGAGGGGGGAGCCTCAGG
ACACCTCCCAGCCAGAAGGCTCTGAGACATAGCAGCAGTATCCTCTCCGGATCTGTCGATTTGGAGACCT
TCCGAGAGAGAACCAAGGGTGCAGTCAGCTTAAAGTGTCCAGGCATCACAGAAGCACAGGAGGCCAGTTC
TGAAAGGCGAGCAGAACTCCCCCTGGGGAGGAAGCTCACCAAAAGTTTTTCCCAAAGCTCAATGCACTTG
AGCTCTGAGGGGAGGTTTCACAAAAGGTCCCCAGTGGCTCATAAAGACTCAAAGCTGTATAGGACATTAC
CCTTGCGGAAGCTGGAGGGCAGCAATTGGAGATGCCGGGGACCCTTCAGCTATTGCTTCCTGAACCGAGG
GCAGGATGAAGATGGTGAGGAAGAAGAGGAGAGGGGAGAGGCCACCGTCCAGGTCTCTTGCCTCTATAGA
CCACAGATGACTCAAGCCATGCCAGAACCAAGCAGCCCATGCCTGGCTGTGGCGATTCAGAAGCAACGAG
GGGAGCTATCCAGAGGGTCAGTGCTGAAGGTCTGGGCAGAAGACCTGCGAGACCCAGATGACTTGGACTT
CAGCAACCTGGCTTTTGATGCCCGGATTGCAAGAATAAATGCCCTAAAGGAGAGCACATATGCAATGCCT
GATGGGTTCCTTGCAGCCCAAAATGATGCCAATGAGCTGCTCTGTCTCGTCAGGGCAACCAAGGAGAAGA
GGGAGGAGTCACGCCCTGAAGCGTACGACCTTACACTTTCTCAGTACAAGCAACTGTTATCCATTGAGTC
CAGACAGTTGGGAAGTGCCTGTAGGAAAATGGCGATGGCTGAGAAAAGCCCGGAGGAGATGCTCCTAGCT
ATGACTTCCAGCTTTCAAGTGCTCTGTTGCCTAACAGAAGCTTGCATGCGATTAGTTAAAGTCGTGAACT
CAGAAACACAGCGGCAGGAAATTGTAGGGAAGATCGATGAAGTGGTCATAAATTACATTTGTCTACTGAA
AGCTGCCGAAGCAGCCACTGGAAAGAACCCTGGGGACCCTAATGTTGGACTCTCGGCGCGACACTCAACC
ACCATGGCCGCTCTCGTAAGCACACTGACACGTTCTCTCAAGAGGCTTTTAAACAAATAAATATGGAAGT
CACGTCATAATCTACCTTTGCAAAGCCATACATGAACTTTTATTTACTTTGTGTGTATGATGAACAGATG
TCTCCTTTCTTCTCTCTGTATATTTTGTTATTTTATATAAAATAGGAGATAAAAGTCACATTGATGAAAT
GTTGAAATGTACTAATCAGATGTATTCTGTTTATATTATACATATATATACACGTAAAAGAAATATCCAA
GAAAGTGATGACATTTGGCTATTTTTCATATAGTTAAAACTCCAGGTATATGATGTGAAATTTTAAATTC
TACCATGTTAGAGCAAAACAATGAATCCTATCCCCTTTCTTTCCAAGTAGCTACTTGGAAACCATATCAT
TCATATTTAGAAGTAAAACACAAAACAAAAAAGAGAGAGAAAAGAAAAGAAATCACAATGTATATAAAAC
AGTACTTATGTTTTAAAATTATGATTTTTAAGCATTGGAAATAGCAAAAAGACATTTAAAATTCAAGAAG
CTATTATGAATTACTAGAGAATATATCTGTAATAAATTAATTTTTTGCTCATAGTATTTGGTTACTGGAT
GCTTTCTTCCAAGAATCCCACATATTTAATTTGGGTTTTTGCTACTGGGGCTACAAATTGGTGGGGATGG
ATTCTACTGTGTCAGCACAAATGCTCTTCACAGTGGTTCTAGCATTTAAAAAACTTCCCGGGGAGAAGAA
CAGAGGGGATGATGGGCAGTTTCCTAGGTAACACCTAGAGTTATAGAATATCTCATTACATAAAATGTAT
GGAATTAATAATACCAAAATTAATTATTTGATGGAAAGATCTGCTTTGACTAAATGTCAAAAATCTGCAA
ACCAAAGACATTATCTTCCCCTCATCCCAACTCAACTACGAAACTTAAAATTCCCTTTAGAGTGATAGGA
CATTTAGTAAAGTATTTGCAAACTTAAAAAAAGGAACATTTAATGATCATCAAAATTAAGTACAGATTCA
GTAATGTAGACCAGACCACACACCAGCACCTGTGAGTCTCATCTCAGATCACAGCTCTCAGCATAGGGCT
TCATGCATCACCGCCTCTACAGAGGCTAAGGCTGCCAGTCAAATTTGGAATTATAGCGTAGTACTGGGAC
AAAATCTCAAATCTTGGATGTTCCAGAAAATCAGGGAGAGATGGCTACTGTAATCATGGGAGCCATGAGT
AAATAGTTAAGTATTTATTAAATAAATACTTAATCTGGATTGGCTGATAAAAATATGAAATCTGAAAAAA
AAAAGAAAGAAATGGAAGTGGTATATTTGACCAGTTCAAGTGAAACCAATGGAATGCAAAGAACTTTCCA
GGACTGTCACATCTCTAAAAGTACCCTGCAGTCATAAAATAAAATCGATATGTATATATGTATTTCCTGG
GTCATTCAGTGCTCAAGTTACATGCACTCCTACTTAGACAGGTGGCTCCTTAAGAACATGATGGGAGTAC
ACCAGGTGCAAATATTTCTACTTTTGTAGCCTGCTGAGAAGGAGTCATAACACAGTCCACAATTGCAATC
TCTCTCTAGTCAGCCATTCCACAGGGACTCAACAACAAAAGCCGCACGTGGTTATGGATGGCAGCAAAGG
AAAGGAGTTTAGGTGTGTGTTATTATAAATAATGTTTAACTAAATAATAAAACATTATTTCATTACAATT
ATAATTATAAATATTGGTGTTTTGATATTGAGAGATATTAGTGGTGTTTACTGTGGAAAATCTTAGTAAA
TATCATTCTGTGTACTAAAATCCTAACCTATTAATAGGATGTGTGCCCTATGTCAGACAGTGTGCTAAGC
ATATTAACTATATTATCTCATTTAATCATCACAACAATCTGCTGGTTTGGACACTATTCTCTCTGGAGGC
AATATGGATGAGTTCTTACGGATATGGGGTTTGGGGTCAGAAACATCTTGATTAAAATTTTGACTCTGCC
ATTTTCTAGCCCTTTTATGTTGGGAAAGCTACTTTTTCTAGTCTCAATTTCCTCATAATAAAATGAAAAT
CAATTATAAGATCGATTTCCATAGGGTTGTCATGAGTTTTGAGTAAAAATGTGTATGTAAAGTTCTTACC
ACAATACCTGGCAGAGAGTAAGTATTCAATTAATGTTAGCTATTATTATCATCATAATCGTTGTGTTAAA
 
TATATTGCCCAAGATAATATGTGGGGGAATGTTTGTTTTATATGCATATTGTATCCTGACTGTGTGCAGA
ACACATTATGTGTATTGCATATATGTAAAAGAAGTGTACAAGGAAGTGGCCATCCTTGTTCAAGTTACGT
ATCTAGTTAGAACAACTTCCCAGTCCATTGGAGTTCTTTGAGAAGCCTCAGGGAAGTAGCTCTGGGTCCT
TCTAATCAACCTCCCATTACTGCGCCAGTAAGTTTCTGTTTCTTATAAATAAACTCTTTGCTATTCAGAA
AACCTCAAGGGGCCCCCACATTCACTAAGCATGGGGCTCCTAACTGGGGTCCAGGTCCATAGAGTGTAGG
AAGAACATATTAGAACAATACTTGTGCTTATTTTTATCTAGCTTTTTTATGTTTGTGTATGTTTTATAAT
GGCATAATAGTACAATGGTACATGTACATAATTTTAAATAAATAAATGAATGAATGAATAAATAAAAAAA
AAAAAAA
**SEQ ID NO: 57 - Human extended synaptotagain-like protein 3 isoform 1 (UniProt ID A0FGR9 as at the filing date)** 
MRAEEPCAPG APSALGAQRT PGPELRLSSQ LLPELCTFVV RVLFYLGPVY
LAGYLGLSIT WLLLGALLWM WWRRNRRGKL GRLAAAFEFL DNEREFISRE
LRGQHLPAWI HFPDVERVEW ANKIISQTWP YLSMIMESKF REKLEPKIRE
KSIHLRTFTF TKLYFGQKCP RVNGVKAHTN TCNRRRVTVD LQICYIGDCE
ISVELQKIQA GVNGIQLQGT LRVILEPLLV DKPFVGAVTV FFLQKPHLQI
NWTGLTNLLD APGINDVSDS LLEDLIATHL VLPNRVTVPV KKGLDLTNLR
FPLPCGVIRV HLLEAEQLAQ KDNFLGLRGK SDPYAKVSIG LQHFRSRTIY
RNLNPTWNEV FEFMVYEVPG QDLEVDLYDE DTDRDDFLGS LQICLGDVMT
NRVVDEWFVL NDTTSGRLHL RLEWLSLLTD QEVLTEDHGG LSTAILVVFL
ESACNLPRNP FDYLNGEYRA KKLSRFARNK VSKDPSSYVK LSVGKKTHTS
KTCPHNKDPV WSQVFSFFVH NVATERLHLK VLDDDQECAL GMLEVPLCQI
LPYADLTLEQ RFQLDHSGLD SLISMRLVLR FLQVEERELG SPYTGPEALK
KGPLLIKKVA TNQGPKAQPQ EEGPTDLPCP PDPASDTKDV SRSTTTTTSA
TTVATEPTSQ ETGPEPKGKD SAKRFCEPIG EKKSPATIFL TVPGPHSPGP
IKSPRPMKCP ASPFAWPPKR LAPSMSSLNS LASSCFDLAD ISLNIEGGDL
RRRQLGEIQL TVRYVCLRRC LSVLINGCRN LTPCTSSGAD PYVRVYLLPE
RKWACRKKTS VKRKTLEPLF DETFEFFVPM EEVKKRSLDV AVKNSRPLGS
HRRKELGKVL IDLSKEDLIK GFSQWYELTP NGQPRS
**SEQ ID NO: 58 - Human extended synaptotagmin-like protein 3 isoform 2 (UniProt ID A0FGR9 as at the filing date)** 
MRAEEPCAPG APSALGAQRT PGPELRLSSQ LLPELCTFVV RVLFYLGPVY
LAGYLGLSIT WLLLGALLWM WWRRNRRGKL GRLAAAFEFL DNEREFISRE
LRGQHLPAWI HFPDVERVEW ANKIISQTWP YLSMIMESKF REKLEPKIRE
KSIHLRTFTF TKLYFGQKCP RVNGVKAHTN TCNRRRVTVD LQICYIGDCE
ISVELQKIQA GVNGIQLQGT LRVILEPLLV DKPFVGAVTV FFLQKPHLQI
NWTGLTNLLD APGINDVSDS LLEDLIATHL VLPNRVTVPV KKGLDLTNLR
FPLPCGVIRV HLLEAEQLAQ KDNFLGLRGK SDPYAKVSIG LQHFRSRTIY
RNLNPTWNEV FEFMVYEVPG QDLEVDLYDE DTDRDDFLGS LQICLGDVMT
NRVVDEWFVL NDTTSGRLHL RLEWLSLLTD QEVLTEDHGG LSTAILVVFL
ESACNLPRNP FDYLNGEYRA KKLSRFARVK QGQQRPFFLC QTICRQEDTY
K
**SEQ ID NO: 59 - Human extended synaptotagmin-like protein 3 transcript 1** (NM_031913 **as at the** filing **date)** 
ACTCACTGGAGCCGAGAACTGGGGCGGCGCGGCGCGGCGCGGTGCATTTCCAGGCGCTGCTCTCCGTCGC
AGAGAACCCTGAGCTCGGCGCGCCGAGAGTCCCAGCAGGGCAAGGGGGCGCGGCGTCCTGGTCCTCGAGC
TTGGGAGACAGATGCGCATGGGCGTGGGGGCATGCGGACCTAAGCTCGGGTGAAGCTCTCGGGAAGGGCA
AGACTGCGGCGACGAGATGCGAGCAGAGGAGCCCTGCGCCCCCGGGGCCCCCAGCGCCCTGGGAGCCCAG
CGCACGCCGGGCCCCGAGCTGCGCCTGTCCAGCCAGCTGCTGCCCGAGCTCTGTACCTTCGTGGTGCGCG
TGCTGTTCTACCTGGGGCCTGTCTACCTAGCTGGCTACCTGGGGCTCAGCATAACCTGGTTGCTGCTCGG
CGCCCTGCTGTGGATGTGGTGGCGCAGGAACCGCCGCGGGAAGCTTGGGCGCCTGGCCGCCGCCTTCGAA
TTCCTTGACAATGAACGCGAGTTCATCAGCCGCGAGCTGCGGGGCCAGCACCTGCCAGCCTGGATCCACT
TCCCGGACGTGGAGCGGGTCGAGTGGGCCAACAAGATCATCTCTCAGACCTGGCCCTACCTAAGCATGAT
CATGGAAAGCAAGTTCCGGGAGAAACTTGAGCCCAAGATCCGAGAGAAGAGCATCCACCTGAGGACCTTT
 
ACCTTTACCAAGCTCTACTTTGGACAGAAGTGTCCCAGGGTCAACGGTGTCAAGGCACACACTAATACGT
GCAACCGAAGACGTGTGACTGTGGACCTGCAGATCTGCTACATCGGGGACTGTGAGATCAGTGTGGAGCT
GCAGAAGATTCAGGCTGGTGTGAACGGGATCCAGTTGCAGGGCACCCTGCGGGTCATCCTGGAGCCCCTC
CTAGTGGACAAGCCCTTTGTGGGAGCCGTGACTGTGTTCTTCCTTCAGAAGCCGCACCTACAGATCAACT
GGACTGGCCTGACCAACCTGCTGGATGCGCCGGGAATCAATGATGTGTCAGACAGCTTACTGGAGGACCT
CATTGCCACCCACCTGGTGCTGCCCAACCGTGTGACTGTGCCTGTGAAGAAGGGGCTGGATCTGACCAAC
CTGCGCTTCCCTCTGCCCTGTGGGGTGATCAGAGTGCACTTGCTGGAGGCAGAGCAGCTGGCCCAGAAGG
ACAACTTTCTGGGGCTCCGAGGCAAGTCAGATCCCTACGCCAAGGTGAGCATCGGCCTACAGCATTTCCG
GAGTAGGACCATCTACAGGAACCTGAACCCCACCTGGAACGAAGTGTTTGAGTTCATGGTGTACGAAGTC
CCTGGACAGGACCTGGAGGTAGACCTGTATGATGAGGATACCGACAGGGATGACTTCCTGGGCAGCCTGC
AGATCTGCCTTGGAGATGTCATGACCAACAGAGTGGTGGATGAGTGGTTTGTCCTGAATGACACAACCAG
CGGGCGGCTGCACCTGCGGCTGGAGTGGCTTTCATTGCTTACTGACCAAGAAGTTCTGACTGAGGACCAT
GGTGGCCTTTCCACTGCCATTCTCGTGGTCTTCTTGGAGAGTGCCTGCAACTTGCCGAGAAACCCTTTTG
ACTACCTGAATGGTGAATATCGAGCCAAAAAACTCTCCAGGTTTGCCAGAAACAAGGTCAGCAAAGACCC
TTCTTCCTATGTCAAACTATCTGTAGGCAAGAAGACACATACAAGTAAGACCTGTCCCCACAACAAGGAC
CCTGTGTGGAGCCAGGTGTTCTCCTTCTTTGTGCACAATGTGGCCACTGAGCGGCTCCATCTGAAGGTGC
TTGATGATGACCAGGAGTGTGCTCTGGGAATGCTGGAGGTCCCCCTGTGCCAGATCCTCCCCTATGCTGA
CCTCACTCTTGAGCAGCGCTTTCAGCTGGACCACTCAGGCCTGGACAGCCTCATCTCCATGAGGCTGGTG
CTTCGGTTCCTGCAAGTGGAGGAACGAGAGCTGGGGAGCCCATACACAGGACCTGAAGCCCTAAAGAAAG
GCCCTCTGCTCATCAAGAAAGTGGCTACCAACCAGGGTCCCAAAGCCCAACCTCAGGAAGAAGGCCCTAC
AGATTTGCCATGTCCCCCAGACCCTGCTTCTGATACTAAGGACGTATCCAGGAGTACCACAACCACCACC
AGTGCTACCACCGTTGCCACTGAGCCCACATCCCAAGAGACAGGCCCAGAGCCTAAAGGCAAGGACAGTG
CCAAAAGGTTCTGTGAGCCCATCGGGGAGAAGAAGAGTCCAGCCACCATCTTCCTGACTGTCCCAGGTCC
CCACTCTCCAGGGCCCATCAAGTCACCCAGACCCATGAAATGCCCTGCCTCCCCATTCGCATGGCCGCCC
AAGAGGCTGGCTCCCAGCATGTCCTCGCTCAACTCCTTGGCCTCTTCTTGCTTTGACCTGGCAGATATCA
GCCTCAACATTGAAGGTGGGGACCTCAGGCGACGGCAGCTGGGTGAGATTCAGCTCACAGTGCGCTATGT
GTGTCTGCGGCGCTGCCTCAGCGTGCTAATCAATGGCTGCAGAAACCTAACACCATGTACCAGCAGTGGA
GCTGATCCCTACGTCCGTGTCTACTTGTTGCCAGAAAGGAAGTGGGCATGTCGTAAGAAGACTTCAGTGA
AGCGGAAGACCTTGGAACCCCTGTTTGATGAGACATTTGAATTTTTTGTTCCCATGGAAGAAGTAAAGAA
GAGGTCACTAGATGTTGCAGTGAAAAATAGTAGGCCACTTGGCTCACACAGAAGAAAGGAGTTAGGAAAA
GTACTGATTGACTTATCAAAAGAAGATCTGATTAAGGGCTTTTCACAATGGTATGAGCTGACTCCAAATG
GACAGCCCAGAAGCTGATGATGAGAATTCTTATCACTCACCTTTATATTAAAATGTATATATATGTATAT
ATTTTTTCCTTTGGATCACTTACATCCAATATATGTATATTTTGTCATTTAAATCAGAACAACCACTTGA
AATTATATACATACAATTCTTGTGTGGAATTTAACTCCATGACTGAATAGCATAAGGAAGAGGTTATTTA
AAAGCAAGAACTACTTTTTTTGGTTGGATTTTTTTGTTCAAGTCCAGAAAGAAATGTTATATTTGTGCCT
ACTAAATTATCCAAACCTCAGTGTTTATATACTTAAACAAGTGCCACTTTTTAGTAGGTAATTATATACC
ATCTGATTTAGGACTTACCTGAATGTATGTACCAGAAAGTGTCCTGCCTCTGGCATAGGGGCTGGGGGAG
GTCTGTTTCTGGAGCCACTACCAGCATTCCTGGGCAGTGACTGGCCAAAGGGAAGTCACTTTTTTATGGA
AATAGAAAGTGACCCAACTCAAAACTGCCAAGAGCTAACACTGCCAAAGCCCTTCTGGCTGCCACCTGTG
GTACTTGTATGCCTGGATATTTGTTCTTTTATTTGACCTGGAGCTAAAAATGTATTTCCCTACTGGCAAA
AATGTTATACTGATATACTTAAATACCTTGAGTTAAATACTCTTGTGTAGTCTAAGGGCCAAACTCCAGC
GTGTAGCATTATTTCCATAGGAAAATGCAAACCAAGTTCCAAGCCCAGGTCTATGTCTGACTCTCATAGC
AAAGCACTGGAGACTGCTGAAGAGTGTCACCATCAGAGAAAAGGAAGCTTTCAGATAAGTTTAGGCTATT
CAACTTTTCTAAAGAGATGGTTGGTTCACCTAAATAACATGTTAAAAGCAAAGTATTAAATTATATGCTT
AAACAAGCGCCTCTTTTACATTTCAGACTTTACAAGACCAAATATACTTCTCATCCCCTCCAACATTCCT
GGACCTCCTCTTCTCTCTTACCAACACCAACACTACCTGCCGTCAGTTTTCTGTCTCTGCCTCTGCCTTC
ACTGCCACTTTTCTGAAGCAGGGCAACATTCGAGAACAAAGTTTTGTCATGTTACTCTTATTCCAGTTTC
CTGCCATCTGATTCACATGCAGATGTTCTATTCCAGAGTAGTCCAAATACTATGCTCTGCTGACTCAAGT
CATGTCTCAAACCTATTTCCTCTATTAAGTAAGTCAGTCAATCCATAGTGAAATGGACTCTTCCTACCGC
CTGTAGCATTTACTGTTGATACTGAGTATTTTCCATTTTAATAGTTCCCAGCCTTTATAACACTAAGTTT
TAAGAAATGTTGATTAGGCATCTGCTAAGGTTACATGCTAAGCAGCTACTTATATTGTATTTGGTATTTA
ATTTTGTTTATTCTCCAGTTGAACAGTAGGGAACAATTCAAGCTACTTGAATTGTTTTTCACCCTCACCA
TCCAAGACCGTGTTAAGTACATAGAAGATGCATGGTGAAATTCGTTTCTGCTGAGGTTTCTATTTTAAAT
GGCAAATTCTCATAATAAAATGGAGCATTGATGCCTACCCTGTCTACCTTATGATGGCATTCTAATTGCC
CCCTAAATAGACAAGTGCCTTGAAAATGGAGAAGCACCAGAGAAATAAGGGAATGTATCATATGTACATC
GAAACATTAGCAACCCTAAAACAGCTAATCAAAATGGCAGATAAGTGAAAACCTACCCCTCTTCTTCCAA
ACTTAAACCACAAACATTTATTTTAAACGTGGTCAATTGAACCGTTTTTACCTGTGTCAGGCAACACAGG
TAAATACCTGGTGATGGTCTGAAGAGGAGTTAAAATGTTGAAGGGAACCAGAATTTTAGTACACAGCAGA
CAGGAGGCAAGGTTATATAGGCGCTGTGTGAGGGGCAGGGAGCCTGAAAAGTTGTTAATACCTTGGCTGC
 
CACTGAGGACAAGAATTGGTTTTCAGCAGTGGGTGGAAACAATCAGCTAATATGCAGGAGCATAGGGTCT
GAAGTTAACATCATCTTGGTACAGAAACCCCCAAACCTAAGACGGAATGTGAATATTGGTTTAGAACCTG
GAAGCCTGTGAAGCCTTGATGGAGGCAACCATAAAATTGGCCCCACAGGGTTGCCACTCCCATCCAGGGC
ACATATGCGACAACTGCAGCAGATGGCTGTCACTAAAGGGAAACTCACGGGCAAAAATAAATTATGAAAC
AAAACAAAAACAGACTTTAAAAAAAATACAAAGTACACAGGAATATCAAACTGAAAAATAGCCATAGATA
ATATTTCAGCCAGAAGGAAAATGTACCAGTAAGGCAGAAATAGCATGTGAGAAGCAGTGGAAAGCAAAGA
AATCCATAAAATACATTGGCAAACCTAAGCAAGTTTTGATGATGATTGTTCACAAGATATAAACAAATAA
CCAAAGATTTTTGCAAGGGTTCAGAAACATAGTAGAACTAAAATGTTAGTACTAACAACTAAGAAGGGGC
AGCTAGGCTGATTAAGAAAGTAAGTATTCAAGACCATATCTTATTTAGGAAGCTAAAGATACTCATCTTA
AAAATATAACTGTGTCAGTCACTAATTCATTACCTCTCAGCTTCAAATTGACGTCTGTCTCCTTTGCTGC
TTTCCCGGCAGGCTGATGTTTGGCTTTGCCAATAGGGGGCGATGGAAGGACACTCTATAGCAGGAAGAAG
GGGCTTTTCTTCTGATGTTTTCCATGGCTGCTAGCTAATTGGCATACAGAGCTGTTCTCAATCAATCACC
TAAGTACCCCCACAGTTTACCCTTGGCTCATGGCCTTCATGTTTTATCAGCAGGCAACACCTTTTGGCAA
GTTTCTTAGCCACAAGTGGGTTGCATGGTCCTATGGCTGCCAGCGGAAATCTAAATCTCAGCCTGGTAGG
GAGGCACTTAGTTTCTAACTTCCTTTCTTGTTCACTCTCCCTTACCCCTGGGGGTAAGGATCTGCATCCT
GAAGTTGATATTCTGTATCCCCTAGAGTCCACTTCTACCCCGTTTAGTAGTAAATACAAGAATTGCTAAC
TAGTAAAAGGTGGTTAAGTTTTTCAAAAAAAAAAAAAAAAA
**SEQ ID NO: 60 - Human extended synaptotagmin-like protein 3 transcript 2 (NM_001322831 as at the filing date)** 
ACTCACTGGAGCCGAGAACTGGGGCGGCGCGGCGCGGCGCGGTGCATTTCCAGGCGCTGCTCTCCGTCGC
AGAGAACCCTGAGCTCGGCGCGCCGAGAGTCCCAGCAGGGCAAGGGGGCGCGGCGTCCTGGTCCTCGAGC
TTGGGAGACAGATGCGCATGGGCGTGGGGGCATGCGGACCTAAGCTCGGGTGAAGCTCTCGGGAAGGGCA
AGACTGCGGCGACGAGATGCGAGCAGAGGAGCCCTGCGCCCCCGGGGCCCCCAGCGCCCTGGGAGCCCAG
CGCACGCCGGGCCCCGAGCTGCGCCTGTCCAGCCAGCTGCTGCCCGAGCTCTGTACCTTCGTGGTGCGCG
TGCTGTTCTACCTGGGGCCTGTCTACCTAGCTGGCTACCTGGGGCTCAGCATAACCTGGTTGCTGCTCGG
CGCCCTGCTGTGGATGTGGTGGCGCAGGAACCGCCGCGGGAAGCTTGGGCGCCTGGCCGCCGCCTTCGAA
TTCCTTGACAATGAACGCGAGTTCATCAGCCGCGAGCTGCGGGGCCAGCACCTGCCAGCCTGGATCCACT
TCCCGGACGTGGAGCGGGTCGAGTGGGCCAACAAGATCATCTCTCAGACCTGGCCCTACCTAAGCATGAT
CATGGAAAGCAAGTTCCGGGAGAAACTTGAGCCCAAGATCCGAGAGAAGAGCATCCACCTGAGGACCTTT
ACCTTTACCAAGCTCTACTTTGGACAGAAGTGTCCCAGGGTCAACGGTGTCAAGGCACACACTAATACGT
GCAACCGAAGACGTGTGACTGTGGACCTGCAGATCTGCTACATCGGGGACTGTGAGATCAGTGTGGAGCT
GCAGAAGATTCAGGCTGGTGTGAACGGGATCCAGTTGCAGGGCACCCTGCGGGTCATCCTGGAGCCCCTC
CTAGTGGACAAGCCCTTTGTGGGAGCCGTGACTGTGTTCTTCCTTCAGAAGCCGCACCTACAGATCAACT
GGACTGGCCTGACCAACCTGCTGGATGCGCCGGGAATCAATGATGTGTCAGACAGCTTACTGGAGGACCT
CATTGCCACCCACCTGGTGCTGCCCAACCGTGTGACTGTGCCTGTGAAGAAGGGGCTGGATCTGACCAAC
CTGCGCTTCCCTCTGCCCTGTGGGGTGATCAGAGTGCACTTGCTGGAGGCAGAGCAGCTGGCCCAGAAGG
ACAACTTTCTGGGGCTCCGAGGCAAGTCAGATCCCTACGCCAAGGTGAGCATCGGCCTACAGCATTTCCG
GAGTAGGACCATCTACAGGAACCTGAACCCCACCTGGAACGAAGTGTTTGAGTTCATGGTGTACGAAGTC
CCTGGACAGGACCTGGAGGTAGACCTGTATGATGAGGATACCGACAGGGATGACTTCCTGGGCAGCCTGC
AGATCTGCCTTGGAGATGTCATGACCAACAGAGTGGTGGATGAGTGGTTTGTCCTGAATGACACAACCAG
CGGGCGGCTGCACCTGCGGCTGGAGTGGCTTTCATTGCTTACTGACCAAGAAGTTCTGACTGAGGACCAT
GGTGGCCTTTCCACTGCCATTCTCGTGGTCTTCTTGGAGAGTGCCTGCAACTTGCCGAGAAACCCTTTTG
ACTACCTGAATGGTGAATATCGAGCCAAAAAACTCTCCAGGTTTGCCAGAAACAAGGTCAGCAAAGACCC
TTCTTCCTATGTCAAACTATCTGTAGGCAAGAAGACACATACAAGTAAGACCTGTCCCCACAACAAGGAC
CCTGTGTGGAGCCAGGTGTTCTCCTTCTTTGTGCACAATGTGGCCACTGAGCGGCTCCATCTGAAGGTGC
TTGATGATGACCAGGAGTGTGCTCTGGGAATGCTGGAGGTCCCCCTGTGCCAGATCCTCCCCTATGCTGA
CCTCACTCTTGAGCAGCGCTTTCAGCTGGACCACTCAGGCCTGGACAGCCTCATCTCCATGAGGCTGGTG
CTTCGGTTCCTGCAAGTGGAGGAACGAGAGCTGGGGAGCCCATACACAGGACCTGAAGCCCTAAAGAAAG
GCCCTCTGCTCATCAAGAAAGTGGCTACCAACCAGGGTCCCAAAGCCCAACCTCAGGAAGAAGGCCCTAC
AGATTTGCCATGTCCCCCAGACCCTGCTTCTGATACTAAGGACGTATCCAGGAGTACCACAACCACCACC
AGTGCTACCACCGTTGCCACTGAGCCCACATCCCAAGAGACAGGCCCAGAGCCTAAAGGCAAGGACAGTG
CCAAAAGGTTCTGTGAGCCCATCGGGGAGAAGAAGAGTCCAGCCACCATCTTCCTGACTGTCCCAGGTCC
CCACTCTCCAGGGCCCATCAAGTCACCCAGACCCATGAAATGCCCTGCCTCCCCATTCGCATGGCCGCCC
AAGAGGCTGGCTCCCAGCATGTCCTCGCTCAACTCCTTGGCCTCTTCTTGCTTTGACCTGGCAGATATCA
GCCTCAACATTGAAGGTGGGGACCTCAGGCGACGGCAGCTGGGTGAGATTCAGCTCACAGTGCGCTATGT
GTGTCTGCGGCGCTGCCTCAGCGTGCTAATCAATGGCTGCAGAAACCTAACACCATGTACCAGCAGTGGA
GCTGATCCCTACGTCCGTGTCTACTTGTTGCCAGAAAGGAAGTGGGCATGTCGTAAGAAGACTTCAGTGA
 
AGCGGAAGACCTTGGAACCCCTGTTTGATGAGACATTTGAATTTTTTGTTCCCATGGAAGAAGTAAAGAA
GAGGTCACTAGATGTTGCAGTGAAAAATAGTAGGCCACTTGGCTCACACAGAAGAAAGGAGTTAGGAAAA
GTACTGATTGACTTATCAAAAGAAGATCTGATTAAGGGCTTTTCACAATGGTATGAGCTGACTCCAAATG
GACAGCCCAGAAGCTGATGATGAGAATTCTTATCACTCACCTTTATATTAAAATACTTTACAAGACCAAA
TATACTTCTCATCCCCTCCAACATTCCTGGACCTCCTCTTCTCTCTTACCAACACCAACACTACCTGCCG
TCAGTTTTCTGTCTCTGCCTCTGCCTTCACTGCCACTTTTCTGAAGCAGGGCAACATTCGAGAACAAAGT
TTTGTCATGTTACTCTTATTCCAGTTTCCTGCCATCTGATTCACATGCAGATGTTCTATTCCAGAGTAGT
CCAAATACTATGCTCTGCTGACTCAAGTCATGTCTCAAACCTATTTCCTCTATTAAGTAAGTCAGTCAAT
CCATAGTGAAATGGACTCTTCCTACCGCCTGTAGCATTTACTGTTGATACTGAGTATTTTCCATTTTAAT
AGTTCCCAGCCTTTATAACACTAAGTTTTAAGAAATGTTGATTAGGCATCTGCTAAGGTTACATGCTAAG
CAGCTACTTATATTGTATTTGGTATTTAATTTTGTTTATTCTCCAGTTGAACAGTAGGGAACAATTCAAG
CTACTTGAATTGTTTTTCACCCTCACCATCCAAGACCGTGTTAAGTACATAGAAGATGCATGGTGAAATT
CGTTTCTGCTGAGGTTTCTATTTTAAATGGCAAATTCTCATAATAAAATGGAGCATTGATGCCTACCCTG
TCTACCTTATGATGGCATTCTAATTGCCCCCTAAATAGACAAGTGCCTTGAAAATGGAGAAGCACCAGAG
AAATAAGGGAATGTATCATATGTACATCGAAACATTAGCAACCCTAAAACAGCTAATCAAAATGGCAGAT
AAGTGAAAACCTACCCCTCTTCTTCCAAACTTAAACCACAAACATTTATTTTAAACGTGGTCAATTGAAC
CGTTTTTACCTGTGTCAGGCAACACAGGTAAATACCTGGTGATGGTCTGAAGAGGAGTTAAAATGTTGAA
GGGAACCAGAATTTTAGTACACAGCAGACAGGAGGCAAGGTTATATAGGCGCTGTGTGAGGGGCAGGGAG
CCTGAAAAGTTGTTAATACCTTGGCTGCCACTGAGGACAAGAATTGGTTTTCAGCAGTGGGTGGAAACAA
TCAGCTAATATGCAGGAGCATAGGGTCTGAAGTTAACATCATCTTGGTACAGAAACCCCCAAACCTAAGA
CGGAATGTGAATATTGGTTTAGAACCTGGAAGCCTGTGAAGCCTTGATGGAGGCAACCATAAAATTGGCC
CCACAGGGTTGCCACTCCCATCCAGGGCACATATGCGACAACTGCAGCAGATGGCTGTCACTAAAGGGAA
ACTCACGGGCAAAAATAAATTATGAAACAAAACAAAAACAGACTTTAAAAAAAATACAAAGTACACAGGA
ATATCAAACTGAAAAATAGCCATAGATAATATTTCAGCCAGAAGGAAAATGTACCAGTAAGGCAGAAATA
GCATGTGAGAAGCAGTGGAAAGCAAAGAAATCCATAAAATACATTGGCAAACCTAAGCAAGTTTTGATGA
TGATTGTTCACAAGATATAAACAAATAACCAAAGATTTTTGCAAGGGTTCAGAAACATAGTAGAACTAAA
ATGTTAGTACTAACAACTAAGAAGGGGCAGCTAGGCTGATTAAGAAAGTAAGTATTCAAGACCATATCTT
ATTTAGGAAGCTAAAGATACTCATCTTAAAAATATAACTGTGTCAGTCACTAATTCATTACCTCTCAGCT
TCAAATTGACGTCTGTCTCCTTTGCTGCTTTCCCGGCAGGCTGATGTTTGGCTTTGCCAATAGGGGGCGA
TGGAAGGACACTCTATAGCAGGAAGAAGGGGCTTTTCTTCTGATGTTTTCCATGGCTGCTAGCTAATTGG
CATACAGAGCTGTTCTCAATCAATCACCTAAGTACCCCCACAGTTTACCCTTGGCTCATGGCCTTCATGT
TTTATCAGCAGGCAACACCTTTTGGCAAGTTTCTTAGCCACAAGTGGGTTGCATGGTCCTATGGCTGCCA
GCGGAAATCTAAATCTCAGCCTGGTAGGGAGGCACTTAGTTTCTAACTTCCTTTCTTGTTCACTCTCCCT
TACCCCTGGGGGTAAGGATCTGCATCCTGAAGTTGATATTCTGTATCCCCTAGAGTCCACTTCTACCCCG
TTTAGTAGTAAATACAAGAATTGCTAACTAGTAAAAGGTGGTTAAGTTTTTCAAAAAAAAAAAAAAAAAA
**SEQ ID NO: 61 - Human extended synaptotagmin-like protein 3 transcript 3 (NM_001322834 as at the filing date)** 
ACTCACTGGAGCCGAGAACTGGGGCGGCGCGGCGCGGCGCGGTGCATTTCCAGGCGCTGCTCTCCGTCGC
AGAGAACCCTGAGCTCGGCGCGCCGAGAGTCCCAGCAGGGCAAGGGGGCGCGGCGTCCTGGTCCTCGAGC
TTGGGAGACAGATGCGCATGGGCGTGGGGGCATGCGGACCTAAGCTCGGGTGAAGCTCTCGGGAAGGGCA
AGACTGCGGCGACGAGATGCGAGCAGAGGAGCCCTGCGCCCCCGGGGCCCCCAGCGCCCTGGGAGCCCAG
CGCACGCCGGGCCCCGAGCTGCGCCTGTCCAGCCAGCTGCTGCCCGAGCTCTGTACCTTCGTGGTGCGCG
TGCTGTTCTACCTGGGGCCTGTCTACCTAGCTGGCTACCTGGGGCTCAGCATAACCTGGTTGCTGCTCGG
CGCCCTGCTGTGGATGTGGTGGCGCAGGAACCGCCGCGGGAAGCTTGGGCGCCTGGCCGCCGCCTTCGAA
TTCCTTGACAATGAACGCGAGTTCATCAGCCGCGAGCTGCGGGGCCAGCACCTGCCAGCCTGGATCCACT
TCCCGGACGTGGAGCGGGTCGAGTGGGCCAACAAGATCATCTCTCAGACCTGGCCCTACCTAAGCATGAT
CATGGAAAGCAAGTTCCGGGAGAAACTTGAGCCCAAGATCCGAGAGAAGAGCATCCACCTGAGGACCTTT
ACCTTTACCAAGCTCTACTTTGGACAGAAGTGTCCCAGGGTCAACGGTGTCAAGGCACACACTAATACGT
GCAACCGAAGACGTGTGACTGTGGACCTGCAGATCTGCTACATCGGGGACTGTGAGATCAGTGTGGAGCT
GCAGAAGATTCAGGCTGGTGTGAACGGGATCCAGTTGCAGGGCACCCTGCGGGTCATCCTGGAGCCCCTC
CTAGTGGACAAGCCCTTTGTGGGAGCCGTGACTGTGTTCTTCCTTCAGAAGCCGCACCTACAGATCAACT
GGACTGGCCTGACCAACCTGCTGGATGCGCCGGGAATCAATGATGTGTCAGACAGCTTACTGGAGGACCT
CATTGCCACCCACCTGGTGCTGCCCAACCGTGTGACTGTGCCTGTGAAGAAGGGGCTGGATCTGACCAAC
CTGCGCTTCCCTCTGCCCTGTGGGGTGATCAGAGTGCACTTGCTGGAGGCAGAGCAGCTGGCCCAGAAGG
ACAACTTTCTGGGGCTCCGAGGCAAGTCAGATCCCTACGCCAAGGTGAGCATCGGCCTACAGCATTTCCG
GAGTAGGACCATCTACAGGAACCTGAACCCCACCTGGAACGAAGTGTTTGAGTTCATGGTGTACGAAGTC
CCTGGACAGGACCTGGAGGTAGACCTGTATGATGAGGATACCGACAGGGATGACTTCCTGGGCAGCCTGC
 
AGATCTGCCTTGGAGATGTCATGACCAACAGAGTGGTGGATGAGTGGTTTGTCCTGAATGACACAACCAG
CGGGCGGCTGCACCTGCGGCTGGAGTGGCTTTCATTGCTTACTGACCAAGAAGTTCTGACTGAGGACCAT
GGTGGCCTTTCCACTGCCATTCTCGTGGTCTTCTTGGAGAGTGCCTGCAACTTGCCGAGAAACCCTTTTG
ACTACCTGAATGGTGAATATCGAGCCAAAAAACTCTCCAGGTTTGCCAGAAACAAGGTCAGCAAAGACCC
TTCTTCCTATGTCAAACTATCTGTAGGCAAGAAGACACATACAAGTAAGACCTGTCCCCACAACAAGGAC
CCTGTGTGGAGCCAGGTGTTCTCCTTCTTTGTGCACAATGTGGCCACTGAGCGGCTCCATCTGAAGGTGC
TTGATGATGACCAGGAGTGTGCTCTGGGAATGCTGGAGGTCCCCCTGTGCCAGATCCTCCCCTATGCTGA
CCTCACTCTTGAGCAGCGCTTTCAGCTGGACCACTCAGGCCTGGACAGCCTCATCTCCATGAGGCTGGTG
CTTCGGTTCCTGCAAGTGGAGGAACGAGAGCTGGGGAGCCCATACACAGGACCTGAAGCCCTAAAGAAAG
GCCCTCTGCTCATCAAGAAAGTGGCTACCAACCAGGGTCCCAAAGCCCAACCTCAGGAAGAAGGCCCTAC
AGATTTGCCATGTCCCCCAGACCCTGCTTCTGATACTAAGGACGTATCCAGGAGTACCACAACCACCACC
AGTGCTACCACCGTTGCCACTGAGCCCACATCCCAAGAGACAGGCCCAGAGCCTAAAGGCAAGGACAGTG
CCAAAAGGTTCTGTGAGCCCATCGGGGAGAAGAAGAGTCCAGCCACCATCTTCCTGACTGTCCCAGGTCC
CCACTCTCCAGGGCCCATCAAGTCACCCAGACCCATGAAATGCCCTGCCTCCCCATTCGCATGGCCGCCC
AAGAGGCTGGCTCCCAGCATGTCCTCGCTCAACTCCTTGGCCTCTTCTTGCTTTGACCTGGCAGATATCA
GCCTCAACATTGAAGGTGGGGACCTCAGGCGACGGCAGCTGGGTGAGATTCAGCTCACAGTGCGCTATGT
GTGTCTGCGGCGCTGCCTCAGCGTGCTAATCAATGGCTGCAGAAACCTAACACCATGTACCAGCAGTGGA
GCTGATCCCTACGTCCGTGTCTACTTGTTGCCAGAAAGGAAGTGGGCATGTCGTAAGAAGACTTCAGTGA
AGCGGAAGACCTTGGAACCCCTGTTTGATGAGACATTTGAATTTTTTGTTCCCATGGAAGAAGTAAAGAA
GAGGTCACTAGATGTTGCAGTGAAAAATAGTAGGCCACTTGGCTCACACAGAAGAAAGGAGTTAGGAAAA
GTACTGATTGACTTATCAAAAGAAGATCTGATTAAGGGCTTTTCACAATGACTTTACAAGACCAAATATA
CTTCTCATCCCCTCCAACATTCCTGGACCTCCTCTTCTCTCTTACCAACACCAACACTACCTGCCGTCAG
TTTTCTGTCTCTGCCTCTGCCTTCACTGCCACTTTTCTGAAGCAGGGCAACATTCGAGAACAAAGTTTTG
TCATGTTACTCTTATTCCAGTTTCCTGCCATCTGATTCACATGCAGATGTTCTATTCCAGAGTAGTCCAA
ATACTATGCTCTGCTGACTCAAGTCATGTCTCAAACCTATTTCCTCTATTAAGTAAGTCAGTCAATCCAT
AGTGAAATGGACTCTTCCTACCGCCTGTAGCATTTACTGTTGATACTGAGTATTTTCCATTTTAATAGTT
CCCAGCCTTTATAACACTAAGTTTTAAGAAATGTTGATTAGGCATCTGCTAAGGTTACATGCTAAGCAGC
TACTTATATTGTATTTGGTATTTAATTTTGTTTATTCTCCAGTTGAACAGTAGGGAACAATTCAAGCTAC
TTGAATTGTTTTTCACCCTCACCATCCAAGACCGTGTTAAGTACATAGAAGATGCATGGTGAAATTCGTT
TCTGCTGAGGTTTCTATTTTAAATGGCAAATTCTCATAATAAAATGGAGCATTGATGCCTACCCTGTCTA
CCTTATGATGGCATTCTAATTGCCCCCTAAATAGACAAGTGCCTTGAAAATGGAGAAGCACCAGAGAAAT
AAGGGAATGTATCATATGTACATCGAAACATTAGCAACCCTAAAACAGCTAATCAAAATGGCAGATAAGT
GAAAACCTACCCCTCTTCTTCCAAACTTAAACCACAAACATTTATTTTAAACGTGGTCAATTGAACCGTT
TTTACCTGTGTCAGGCAACACAGGTAAATACCTGGTGATGGTCTGAAGAGGAGTTAAAATGTTGAAGGGA
ACCAGAATTTTAGTACACAGCAGACAGGAGGCAAGGTTATATAGGCGCTGTGTGAGGGGCAGGGAGCCTG
AAAAGTTGTTAATACCTTGGCTGCCACTGAGGACAAGAATTGGTTTTCAGCAGTGGGTGGAAACAATCAG
CTAATATGCAGGAGCATAGGGTCTGAAGTTAACATCATCTTGGTACAGAAACCCCCAAACCTAAGACGGA
ATGTGAATATTGGTTTAGAACCTGGAAGCCTGTGAAGCCTTGATGGAGGCAACCATAAAATTGGCCCCAC
AGGGTTGCCACTCCCATCCAGGGCACATATGCGACAACTGCAGCAGATGGCTGTCACTAAAGGGAAACTC
ACGGGCAAAAATAAATTATGAAACAAAACAAAAACAGACTTTAAAAAAAATACAAAGTACACAGGAATAT
CAAACTGAAAAATAGCCATAGATAATATTTCAGCCAGAAGGAAAATGTACCAGTAAGGCAGAAATAGCAT
GTGAGAAGCAGTGGAAAGCAAAGAAATCCATAAAATACATTGGCAAACCTAAGCAAGTTTTGATGATGAT
TGTTCACAAGATATAAACAAATAACCAAAGATTTTTGCAAGGGTTCAGAAACATAGTAGAACTAAAATGT
TAGTACTAACAACTAAGAAGGGGCAGCTAGGCTGATTAAGAAAGTAAGTATTCAAGACCATATCTTATTT
AGGAAGCTAAAGATACTCATCTTAAAAATATAACTGTGTCAGTCACTAATTCATTACCTCTCAGCTTCAA
ATTGACGTCTGTCTCCTTTGCTGCTTTCCCGGCAGGCTGATGTTTGGCTTTGCCAATAGGGGGCGATGGA
AGGACACTCTATAGCAGGAAGAAGGGGCTTTTCTTCTGATGTTTTCCATGGCTGCTAGCTAATTGGCATA
CAGAGCTGTTCTCAATCAATCACCTAAGTACCCCCACAGTTTACCCTTGGCTCATGGCCTTCATGTTTTA
TCAGCAGGCAACACCTTTTGGCAAGTTTCTTAGCCACAAGTGGGTTGCATGGTCCTATGGCTGCCAGCGG
AAATCTAAATCTCAGCCTGGTAGGGAGGCACTTAGTTTCTAACTTCCTTTCTTGTTCACTCTCCCTTACC
CCTGGGGGTAAGGATCTGCATCCTGAAGTTGATATTCTGTATCCCCTAGAGTCCACTTCTACCCCGTTTA
GTAGTAAATACAAGAATTGCTAACTAGTAAAAGGTGGTTAAGTTTTTCAAAAAAAAAAAAAAAAA
**SEQ ID NO: 62 -Sense primer for MMP13 (NM_002427)**
   **ATGAAGACCCCAACCCTAAACA**
**SEQ ID NO: 63 - Anti-sense primer for MMP13 (NM_002427)**
   CGGAGACTGGTAATGGCATCA
**SEQ ID NO: 64 - Sense primer for STMN2 (HM_001199214)**
   AAGCCCCACGAACTTTAG
**SEQ ID NO: 65 - Anti-sense primer for STMN2 (NM_001199214)**
   CGTGTTCCCTCTTCTCTG
**SEQ ID NO: 66 - Sense primer for THBS4 (NM_003248)**
   AGAGAGGCAGGGGGATGTG
**SEQ ID NO: 67 - Anti-sense primer for THBS4 (NM_003248)**
   TTCTTCGTCGGGGTAACTGTC
**SEQ ID NO: 68 - Sense primer for QMD positive tissue control (NM_005014)**
   CAGTAACAGATTAGAATCAATGCC
**SEQ ID NO: 69 - Anti-sense primer for OMD positive tissue control (NM_005014)**
   GACATTCTTAGAGTATGAAGTTTTGGA

### ASPECTS OF THE INVENTION

1. A method of evaluating osteoarthritis pain in a patient, said method comprising measuring the amount of thromospondin-4 and/or one or more other biomarkers identified in Table 1 in a sample from the patient.
2. The method of aspect 1, wherein the method diagnoses that joint pain in a patient is a result of osteoarthritis.
3. The method of aspect 1, wherein the method identifies that a patient is at risk of developing osteoarthritis pain.
4. The method of aspect 1, wherein the method monitors disease progression.
5. The method of aspect 1, wherein the method monitors the efficacy of an anti-osteoarthritis therapy.
6. The method of any one of the preceding aspects, wherein the method comprises measuring the amount of thrombospondin 4 and/or one or more other biomarkers in Table 2.
7. The method of any one of the preceding aspects, wherein the method comprises measuring the amount of one or more biomarkers selected from the group consisting of thrombospondin 4, stathmin 2, ATP binding cassette subfamily B (MDR/TAP) member 5, matrix metalloproteinase 13, adenylate kinase 5, ATPase H+ transporting lysosomal 38 kDa, V0 subunit D2 (V-type proton ATPase subunit d2), DIRAS family, GTP-binding RAS-like 2 (gene name DIRAS2), syntaxin binding protein 5 like, PC4 and SFRS 1 interacting protein 1, neuronal tyrosine-phosphorylated phosphoinositide-3-kinase adaptor 2, catenin delta, PERM and PDZ containing 4 and extended synaptotagmin-like protein 3.
8. The method of any one of the preceding aspects, wherein the method comprises measuring the amount of thrombospondin 4 and/or stathmin 2.
9. The method of aspect 8, which is a method of diagnosing that joint pain in a patient is a result of osteoarthritis, wherein an increased amount of thrombospondin 4 and/or an increased amount of stathmin 2 is indicative that the pain is a result of osteoarthritis.
10. The method of aspect 9, wherein the method comprises:
   (a) measuring the amount of thrombospondin 4 and/or stathmin 2 in a sample from the patient;
   (b) comparing the amount of thrombospondin 4 and/or stathmin 2 in the sample with a control amount of thrombospondin 4 and/or stathmin 2; and
   (c) outputting the results,
   wherein an increased amount of thrombospondin 4 and/or stathmin 2 relative to the control is indicative that the pain is a result of osteoarthritis.
11. The method of aspect 10, wherein the control amount is derived from subjects known not to have osteoarthritis.
12. The method according to aspect 8, which is a method of identifying that a patient is at risk of developing osteoarthritis pain, wherein an increased amount of thrombospondin 4 and/or an increased amount of stathmin 2 is indicative that a patient is at risk of developing osteoarthritis pain.
13. The method according to aspect 12, wherein said method comprises:
   (a) measuring the amount of thrombospondin 4 and/or stathmin 2in a sample from the patient;
   (b) comparing the amount of thrombospondin 4 and/or stathmin 2 in the sample with a control amount of thrombospondin 4 and/or stathmin 2 ; and
   (c) outputting the results,
   wherein an increased amount of thrombospondin 4 and/or stathmin 2 relative to the control is indicative that a patient is at risk of developing osteoarthritis pain.
14. The method of aspect 13, wherein the control amount is derived from subjects known not to have osteoarthritis.
15. The method of any one of the preceding aspects, wherein mRNA amounts are measured.
16. The method of any one of the preceding aspects 1-14, wherein protein amounts are measured.
17. The method of aspect 16, wherein the amount protein is measured using an antibody/antibodies.
18. The method of aspect 17, wherein the amount of protein is measured using an ELISA assay.
19. The method of any one of the preceding aspects, wherein the sample from the patient is a blood, plasma, serum or urine sample.
20. Use of thrombopsondin 4 and/or one or more other biomarkers identified in Table 1 in evaluating osteoarthritis pain in a patient.
21. Use of thrombopsondin 4 and/or one or more other biomarkers identified in Table 1 in evaluating the size and/or severity of bone marrow lesions.
22. A kit for evaluating osteoarthritis pain, comprising a reagent for measuring the amount of thrombospondin 4 and/or one or more other biomarkers of Table 1 and instructions for use of the kit.
23. The kit according to aspect 22, which comprises a reagent for measuring the amount of thrombospondin 4 and/or stathmin 2.
24. A method of treating or preventing osteoarthritis pain in a patient, said method comprising administering an inhibitor of the NOTCH 1/thrombospondin 4 signalling pathway.
25. The method of aspect 24, wherein the inhibitor of the NOTCH 1/thrombospondin 4 signalling pathway is a γ-secretase inhibitor.
26. The method of aspect 25, wherein the γ-secretase inhibitor is N-[N-(3,5-Difluorophenacetyl)-L-alanyl]-S-phenylglycine t-butyl ester.
27. The method of aspect 26, wherein the γ-secretase inhibitor is semgacestat.
28. The method of aspect 24, therein the inhibitor of the NOTCH 1/thrombospondin 4 signalling pathway is a thrombospondin 4 inhibitor.
29. The method of aspect 28, wherein the inhibitor of thrombospondin 4 is an anti-thrombospondin 4 antibody, a small molecule or an oligonucleotide.
30. The method of aspect 28 or 29, wherein the method comprises measuring the amount of thrombospondin 4 in a sample from the patient and administering a thrombospondin 4 inhibitor if an increased amount of thrombospondin 4 is measured.
31. The method of aspect 24, wherein the inhibitor is a gamma-aminobutyric acid derivative.
32. The method of aspect 31, wherein the inhibitor is a gabapentinoid.
33. The method of aspect 32, wherein the gabapentinoid is pregabalin.
34. A method of treating or preventing osteoarthritis pain in a patient, said method comprising administering an inhibitor of microtubule activity to the patient.
35. The method of aspect 34, wherein the inhibitor of microtubule activity is a taxane.
36. The method of aspect 34, wherein the inhibitor of microtubule activity is a vinca alkaloid.
37. The method of aspect 34, wherein the inhibitor of microtubule activity is colchicine.
38. The method of aspect 34, wherein the inhibitor of microtubule activity is podophyllotoxin.
39. The method of aspect 35, wherein the inhibitor of microtubule activity is a stathmin 2 inhibitor.
40. The method of aspect 39, wherein the stathmin 2 inhibitor is an anti-stathmin 2 antibody, a small molecule or an oligonucleotide.
41. The method of aspect 39 or 40, wherein said method comprises measuring the amount of stathmin 2 in a sample from the patient and administering a stathmin 2 inhibitor if an increased amount of stathmin 2 is measured.
42. A method of screening for agents for treating or preventing osteoarthritis pain, said method comprising:
   (i) identifying agents which interact with thrombospondin 4 or stathmin 2; and
   (ii) assessing whether agents identified in part (i) inhibit thrombospondin 4 orstathmin 2.
43. A method of treating or preventing osteoarthritis pain in a patient, said method comprising:
   (i) performing the method of aspect 42 and identifying agents which inhibit thrombospondin 4 and/or stathmin 2; and
   (ii) administering the agents identified in part (i) to a patient in need thereof.

## Claims

1. A method of evaluating osteoarthritis pain in a patient, said method comprising measuring the amount of thromospondin-4 in a sample from the patient.

2. The method of claim 1, wherein the method:
(a) diagnoses that joint pain in a patient is a result of osteoarthritis;
(b) identifies that a patient is at risk of developing osteoarthritis pain;
(c) monitors disease progression; or
(d) monitors the efficacy of an anti-osteoarthritis therapy

3. The method of claim 1 or 2, wherein:
(a) the method comprises measuring the amount of thrombospondin 4 and one or more other biomarkers in Table 1;
(b) the method comprises measuring the amount of thrombospondin 4 and one or more other biomarkers in Table 2; or
(c) the method comprises measuring the amount of thrombospondin 4 and one or more biomarkers selected from the group consisting of ATP binding cassette subfamily B (MDR/TAP) member 5, matrix metalloproteinase 13, adenylate kinase 5, ATPase H+ transporting lysosomal 38 kDa, V0 subunit D2 (V-type proton ATPase subunit d2), DIRAS family, GTP-binding RAS-like 2 (gene name DIRAS2), syntaxin binding protein 5 like, PC4 and SFRS1 interacting protein 1, neuronal tyrosine-phosphorylated phosphoinositide-3-kinase adaptor 2, catenin delta, FERM and PDZ containing 4 and extended synaptotagmin-like protein 3.

4. The method of any one of the preceding claims, which is:
(a) a method of diagnosing that joint pain in a patient is a result of osteoarthritis, wherein an increased amount of thrombospondin 4 is indicative that the pain is a result of osteoarthritis, wherein the method optionally comprises:
(i) measuring the amount of thrombospondin 4 in a sample from the patient;
(ii) comparing the amount of thrombospondin 4 in the sample with a control amount of thrombospondin 4, such as a control amount derived from subjects known not to have osteoarthritis; and
(iii) outputting the results,
wherein an increased amount of thrombospondin 4 relative to the control is indicative that the pain is a result of osteoarthritis; or
(b) a method of identifying that a patient is at risk of developing osteoarthritis pain, wherein an increased amount of thrombospondin 4 is indicative that a patient is at risk of developing osteoarthritis pain, wherein the method optionally comprises:
(i) measuring the amount of thrombospondin 4 in a sample from the patient;
(ii) comparing the amount of thrombospondin 4 in the sample with a control amount of thrombospondin 4, such as a control amount derived from subjects known not to have osteoarthritis; and
(c) outputting the results,
wherein an increased amount of thrombospondin 4 relative to the control is indicative that a patient is at risk of developing osteoarthritis pain.

5. The method of any one of the preceding claims, wherein:
(a) mRNA amounts are measured; or
(b) protein amounts are measured, optionally wherein the amount of protein is measured using an antibody/antibodies, such as using an ELISA assay

6. The method of any one of the preceding claims, wherein the sample from the patient is a blood, plasma, serum or urine sample.

7. Use of thrombopsondin 4 and optionally one or more other biomarkers identified in Table 1 in:
(a) evaluating osteoarthritis pain in a patient.; or
(b) evaluating the size and/or severity of bone marrow lesions.

8. A kit for evaluating osteoarthritis pain, comprising a reagent for measuring the amount of thrombospondin 4 and optionally one or more other biomarkers of Table 1 and instructions for use of the kit.

9. An inhibitor of the NOTCH 1/thrombospondin 4 signalling pathway for use in a method of treating or preventing osteoarthritis pain in a patient.

10. The inhibitor of the NOTCH 1/thrombospondin 4 signalling pathway for use according to claim 9, wherein the inhibitor of the NOTCH 1/thrombospondin 4 signalling pathway:
(a) is a γ-secretase inhibitor, optionally N-[N-(3,5-Difluorophenacetyl)-L-alanyl]-S-phenylglycine t-butyl ester or semgacestat.
(b) is a thrombospondin 4 inhibitor, wherein optionally:
(i) the inhibitor of thrombospondin 4 is an anti-thrombospondin 4 antibody, a small molecule or an oligonucleotide; and/or
(ii) the method comprises measuring the amount of thrombospondin 4 in a sample from the patient and administering a thrombospondin 4 inhibitor if an increased amount of thrombospondin 4 is measured;
(c) is a *gamma*-aminobutyric acid derivative, optionally a gabapentinoid such as pregabalin.
